(19) Europäisches Patentamt
European Patent Office
Office européen des brevets

(11) EP 0 609 437 B1

(12) **EUROPEAN PATENT SPECIFICATION**

(45) Date of publication and mention of the grant of the patent: **07.07.1999 Bulletin 1999/27**

(21) Application number: **92909489.4**

(22) Date of filing: **28.04.1992**

(51) Int Cl.[6]: **C07H 15/10**, C07H 15/04, A61K 31/70

(86) International application number: **PCT/JP92/00561**

(87) International publication number: **WO 93/05055 (18.03.1993 Gazette 1993/08)**

(54) **NOVEL SPHINGOGLYCOLIPID AND USE THEREOF**

NEUE SPHINGOGLYCOLIPIDE UND VERWENDUNG DAVON

NOUVEAU SPHINGOGLYCOLIPIDE ET SON UTILISATION

(84) Designated Contracting States:
**AT BE CH DE DK ES FR GB GR IT LI LU MC NL SE**

(30) Priority: **29.08.1991 JP 24438491**

(43) Date of publication of application:
**10.08.1994 Bulletin 1994/32**

(73) Proprietor: **KIRIN BEER KABUSHIKI KAISHA Tokyo-To (JP)**

(72) Inventors:
- **HIGA, Tatsuo**
  **Naha-shi Okinawa 903 (JP)**
- **AKIMOTO, Koji c/o Kirin Beer K.K.**
  **Takasaki-shi Gunma 370-12 (JP)**
- **KOEZUKA, Yasuhiko**
  **Kirin Beer Kabushiki Kaisha**
  **Takasaki-shi Gunma 370-12 (JP)**
- **SAKAI, Teruyuki Kirin Beer Kabushiki Kaisha**
  **Takasaki-shi Gunma 370-12 (JP)**
- **MORITA, Masahiro Kirin Beer Kabushiki Kaisha**
  **Takasaki-shi Gunma 370-12 (JP)**
- **NATORI, Takenori Kirin Beer Kabushiki Kaisha**
  **Takasaki-shi Gunma 370-12 (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 371 414** **JP-A- 1 093 562**
**JP-A- 61 057 594**

- **LIEBIGS ANNALEN DER CHEMIE no. 1 , January 1988 , WEINHEIM, DE pages 19 - 24 Y. KAWANO ET AL 'Isolation and structure of six new cerebrosides'**
- **LIEBIGS ANNALEN DER CHEMIE no. 7 , July 1990 , WEINHEIM, DE pages 659 - 663 R. HIGUCHI ET AL 'Structures of three new cerebrosides, astrocerebroside A, B, and C and of related nearly homogeneous cerebrosides.'**
- **CHEMICAL AND PHARMACEUTICAL BULLETIN vol. 39, no. 6 , June 1991 , TOKYO, JP pages 1385 - 1391 M. HONDA ET AL 'Synthesis of a new cerebroside from a chondropsis sp. sponge'**
- **TETRAHEDRON vol. 45, no. 12 , December 1989 , OXFORD, GB pages 3897 - 3906 S. HIRSCH AND Y. KASHMAN 'New glycosphingolipids from marine organisms'**
- **Analytical Chemistry, Vol. 59, No. 13, pages 1652 to 1659 (1987), SATO K. et al., "High-performance tandem mass spectrometry", alpha-galactopyranosyl (CAS Reg. No. (107982-40-1)) is described.**
- **J. Chem. Soc. Perkin Transaction I, 1991, No. 1, pages 189 to 194, MUNESADA K. et al., "Cerebrosides of Frog brain".**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Background of the Invention

#### Field of the Art

The present invention relates to novel glycosphingolipids having effective antitumor activity and immuno-stimulating activity, to a process for producing them and to the use thereof.

#### Related Art

In respect to α-galactosylceramide, a study on a mass analysis has been reported in Analytical Chemistry, 59, 1652 (1987), in which the structure of α-galactosylceramide is described. The compound corresponding to the α-galactosylceramide has been isolated from a cestode by B.N. Singh and reported in Molecular and Biochemical Parasitology, 26, 99 (1987). However, the stereo chemical configuration of the sugar is not described in the study. In other reports, two α-galactosylceramides have been extracted and isolated from the marine sponge Agelas mauritianus by the present inventors (Japanese Patent Application Nos. 303314/1990 and 244385/1991).

On the other hand, it is only those described in Japanese Patent Laid-Open Publication No. 93562/1989 other than the invention according to the present inventors as far as we know that antitumor activity is found for galactosylceramides. Moreover, all of the galactosylceramides in which antitumor activity is shown in Examples of the above cited specification are β-galactosylceramides, and the dose is as high as 0.5-2 mg per mouse. Furthermore, there is no example in which a galactosylceramide has been used in practice as an antitumor agent or an immuno stimulator.

The galactosylceramides derived from marine sponges are described in Japanese Patent Laid-Open Publication No. 57594/1986 and Pure & Applied Chemistry, 58(3), 387-394 (1980). All of these galactosylceramides are, however, β-galactosylceramides, the antitumor activity of which have not been reported.

In general, the physiological activities of chemical substances depend largely on their chemical structures, and it is always desired to obtain novel compounds having antitumor activity and immuno-stimulating activity.

The object of the present invention is to realize the aforementioned desires.

### Outline of the Invention

The present inventors have extracted specific α-galactosylceramides from a marine sponge Agelas mauritianus and found that the compounds exhibit antitumor activity and immuno-stimulating activity. The present inventors have further created the method for synthesizing the related compounds and found that these related compounds also have the similar activities. The present invention have been achieved on the basis of these informations.

That is, the novel glycosphingolipids according to the present invention include an α-galactose residue and are represented by the following formula (A):

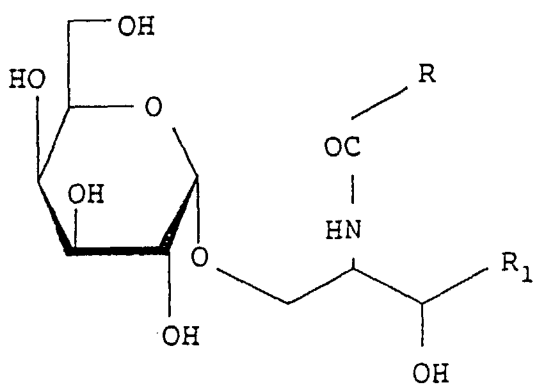

(A)

wherein

R represents

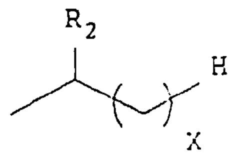

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17.

In the aforementioned formula (A),

(1) the compound in which R represents

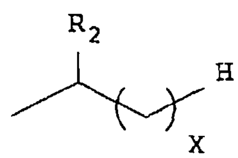

is represented by the formula (I):

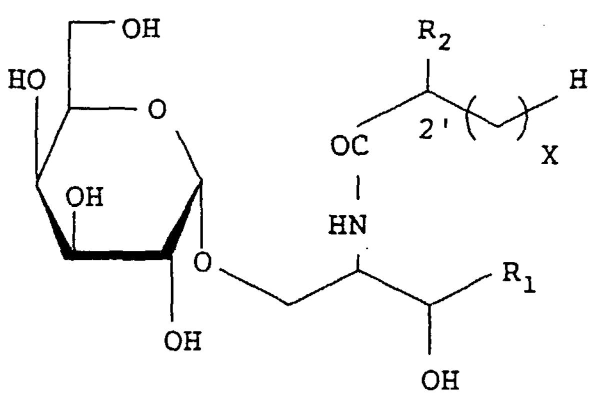

(I)

and
(2) the compound in which R represents
$-(CH_2)_7CH=CH(CH_2)_7CH_3$ is represented by the formula (XXI):

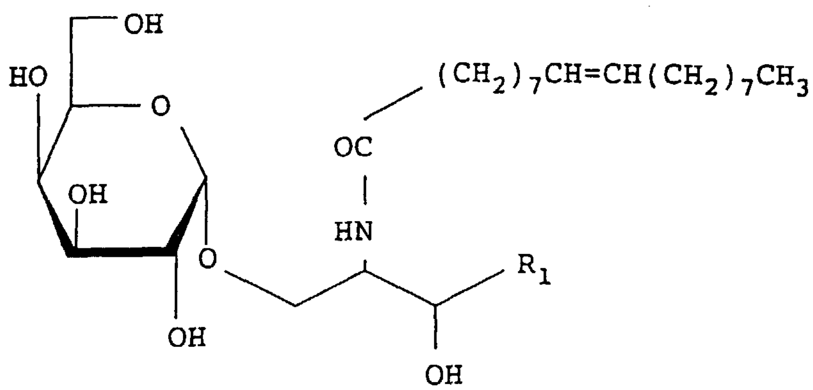

(XXI).

The present invention also relates to a process for preparing the compounds represented by the formula (I) and specified below. That is, the process for preparing the glycosphingolipid represented by the formula (I) according to the present invention comprises collecting the marine sponge Agelas mauritianus, subjecting it to an extraction operation with an organic solvent and isolating from the extract the glycosphingolipids represented by the formula (I) and specified below:

(1) (2S,3S,4R)-1-(α-D-galactopyranosyloxy)-2-[(R)-2-hydroxytetracosanoylamino]-3,4-heptadecanediol,
(2) (2S,3S,4R)-1-(α-D-galactopyranosyloxy)-2-[(R)-2-hydroxytetracosanoylamino]-3,4-hexadecanediol,
(3) (2S,3S,4R)-1-(α-D-galactopyranosyloxy)-2-[(R)-2-hydroxytricosanoylamino]-16-methyl-3,4-heptadecanediol, and
(4) (2S,3S,4R)-1-(α-D-galactopyranosyloxy)-2-[(R)-2-hydroxypentacosanoylamino]-16-methyl-3,4-octadecanediol.

The compound of the present invention represented by the formula (A) (i.e. formulae (I) and (XXI)) can be also synthesized chemically according to the process schemes or reaction route schemes described below.
The present invention further relates to the use of the compounds represented by the formula (A) (formulae (I) and (XXI)). That is, the antitumor agent and the immuno stimulator according to the present invention each contains one or more glycosphingolipids represented by the formula (A) (formulae (I) and (XXI)) as effective ingredients or contain the effective amount thereof and a pharmaceutically acceptable carrier or diluent.
Furthermore, the present invention relates to the therapeutic method comprising administering the effective amounts of one or more of the aforementioned compounds to patients who need inhibiting the proliferation of tumor or activating immunity.

Brief Description of the Drawings

Fig. 1 (a and b) shows the reaction route scheme (synthetic route A) for synthesizing the compounds represented by the formula (A) from an aldehyde compound as a starting material;
Fig. 2 also shows the reaction route scheme (synthetic route B) which is a route for synthesizing the compounds represented by the formula (A) from an aldehyde compound as a starting material as well as Fig. 1 and has less steps than the reaction route A;
Fig. 3 shows the reaction route scheme (synthetic route C) for deriving the compounds represented by the formula (A) by applying a variety of chemical modifications to the sphingosine;
Fig. 4(a-c) shows the reaction route scheme (synthetic route D) for synthesizing a derivative of the compound represented by the formula (A) from an aldehyde compound as a starting material, which has a hydroxyl group at the C-4 of the long chain base;
Fig. 5(a and b) shows the reaction route scheme which illustrates a preferred method for synthesizing the compound 9 ((2S,3R)-1-(α-D-galactopyranosyloxy)-2-tetracosanoylamino-3-tetradecanol);

Fig. 6 shows the reaction route scheme which illustrates a preferred method for synthesizing the compound 7 ((2S, 3R)-1-(α-D-galactopyranosyloxy)-2-octanoylamino-3-octadecanol);

Fig. 7 shows the reaction route scheme which illustrates a preferred method for synthesizing the compound 5 ((2S, 3R)-1-(α-D-galactopyranosyloxy)-2-tetradecanoylamino-3-octadecanol);

Fig. 8 shows the reaction route scheme which illustrates a preferred method for synthesizing the compound 1 ((2S, 3R)-1-(α-D-galactopyranosyloxy)-2-tetracosanoylamino-3-octadecanol);

Fig. 9 shows the reaction route scheme which illustrates another preferred method for synthesizing the compound 5; and

Fig. 10(a-c) shows the reaction route scheme which illustrates a preferred method for synthesizing the compound 22 ((2S,3S,4R)-1-(α-D-galactopyranosyloxy)-2-[(R)-2-hydroxyltetracosanoylamino]-3,4-heptadecanediol).

## Detailed Description of the Invention

### Glycosphingolipids

The glycosphingolipids -according to the present invention, as described above, are represented by the formula (A) (i.e. formulae (I) and (XXI)), and $R_1$ in the formula (I) is preferably represented by the following (a)-(e):

(a) $-CH_2(CH_2)_YCH_3$,

wherein, when $R_2$ represents H, it is preferable that X denote an integer of 0-24 and Y denote an integer of 7-15; when $R_2$ represents OH, it is preferable that X denote an integer of 20-24 and Y denote an integer of 11-15; when $R_2$ represents H, it is particularly preferable that X denote an integer of 8-22 and Y denote an integer of 9-13; and when $R_2$ represents OH, it is particularly preferable that X denote an integer of 21-23 and Y denote an integer of 12-14;

(b) $-CH(OH)(CH_2)_YCH_3$,

wherein, when $R_2$ represents H, it is preferable that X denote an integer of 18-26 and Y denote an integer of 5-15; when $R_2$ represents OH, it is preferable that X denote an integer of 18-26 and Y denote an integer of 5-17; further when $R_2$ represents H, it is particularly preferable that X denote an integer of 21-25 and Y denote an integer of 6-14; and when $R_2$ represents OH, it is particularly preferable that X denote an integer of 21-25 and Y denote an integer of 6-16;

(c) $-CH(OH)(CH_2)_YCH(CH_3)_2$,

wherein when $R_2$ represents H, it is preferable that X denote an integer of 20-24 and Y denote an integer of 9-13; when $R_2$ represents OH, it is preferable that X denote an integer of 18-24 and Y denote an integer of 9-13; further when $R_2$ represents H, it is particularly preferable that X denote an integer of 21-23 and Y denote an integer of 10-12; and when $R_2$ represents OH, it is particularly preferable that X denote an integer of 20-23 and Y denote an integer of 10-12;

(d) $-CH=CH(CH_2)_YCH_3$,

wherein $R_2$ represents H and it is preferable that X denote an integer of 10-18 and Y denote an integer of 10-14; and it is particularly preferable that X denote an integer of 11-17 and Y denote an integer of 11-13; and

(e) $-CH(OH)(CH_2)_YCH(CH_3)CH_2CH_3$,

wherein $R_2$ represents OH and it is preferable that X denote an integer of 21-25 and Y denote an integer of 9-13; and it is particularly preferable that X denote an integer of 22-24 and Y denote an integer of 10-12.

On the other hand, $R_1$ in the formula (XXI) preferably represents $-CH_2(CH_2)_YCH_3$, wherein Y denotes preferably an integer of 11-15, particularly 12-14.

A compound of the present invention which has the configurations at 2- and 3-positions as shown in the following formula (II) is particularly preferred.

Furthermore, when the synthetic route described below is used, the glycosphingolipid represented by the formula (IV) hereinafter wherein X denotes an integer of 8-22 and Y denotes an integer of 9-13 is the most preferred from the standpoint of easy availability of the raw material.

The more concrete form and the preferred form of the compound of the present invention represented by the formula (A) (formulae (I) and (XXI)) can be defined by the following definitions (1)-(4):

(1) the glycosphingolipids of the formula (I) represented by the formula (II):

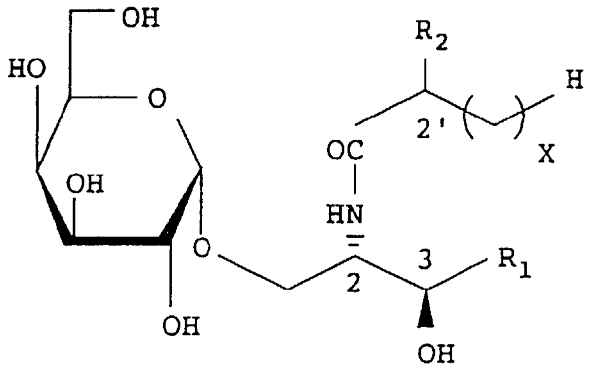

(II)

wherein $R_1$ represents any one of the substituents defined by the following (a)-(e), $R_2$ represents H or OH and X is defined in the following (a)-(e);

(a) $-CH_2(CH_2)_YCH_3$,

wherein, when $R_2$ represents H, X denotes an integer of 0-24 and Y denotes an integer of 7-15; and when $R_2$ represents OH, X denotes an integer of 20-24 and Y denotes an integer of 11-15;

(b) $-CH(OH)(CH_2)_YCH_3$,

wherein, when $R_2$ represents H, X denotes an integer of 18-26 and Y denotes an integer of 5-15; and when $R_2$ represents OH, X denotes an integer of 18-26 and Y denotes an integer of 5-17;

(c) $-CH(OH)(CH_2)_YCH(CH_3)_2$,

wherein, when $R_2$ represents H, X denotes an integer of 20-24 and Y denotes an integer of 9-13; and when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13;

(2) the glycosphingolipids of the formula (I) represented by the formula (III):

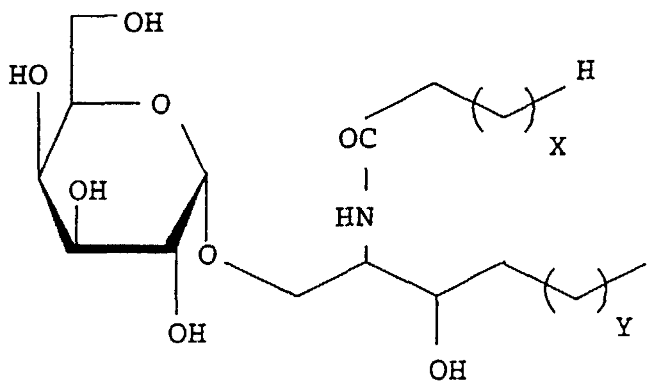

(III)

wherein X denotes an integer of 0-24 and Y denotes an integer of 7-15;
(3) the glycosphingolipids described in the above (2), wherein more preferably X denotes an integer of 8-22 and Y denotes an integer of 9-13;
(4) the glycosphingolipids described in the above (2) which is more preferably represented by the formula (IV):

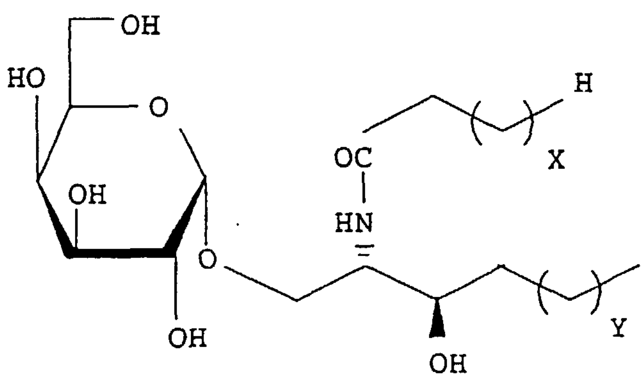

(IV)

wherein X denotes an integer of 0-24 and Y denotes an integer of 7-15;
(5) the glycosphingolipids described in the above (4), wherein most preferably X denotes an integer of 8-22 and Y denotes an integer of 9-13;
(6) the glycosphingolipids of the formula (I) represented by the formula (V):

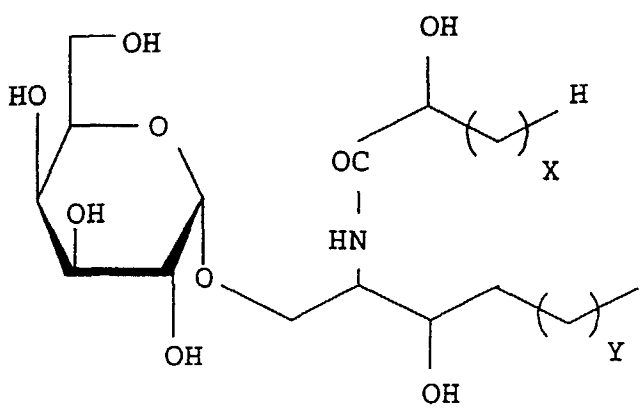

(V)

wherein X denotes an integer of 20-24 and Y denotes an integer of 11-15;
(7) the glycosphingolipids described in the above (6), wherein more preferably X denotes an integer of 21-23 and Y denotes an integer of 12-14;
(8) the glycosphingolipids described in the above (6), represented more preferably by the formula (VI):

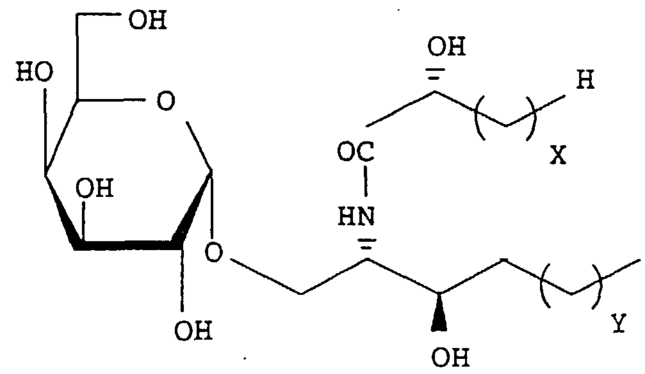

(VI)

wherein X denotes an integer of 20-24 and Y denotes an integer of 11-15;
(9) the glycosphingolipids described in the above (8), wherein most preferably X denotes an integer of 21-23 and Y denotes an integer of 12-14;
(10) the glycosphingolipids of the formula (I) represented by the formula (VII):

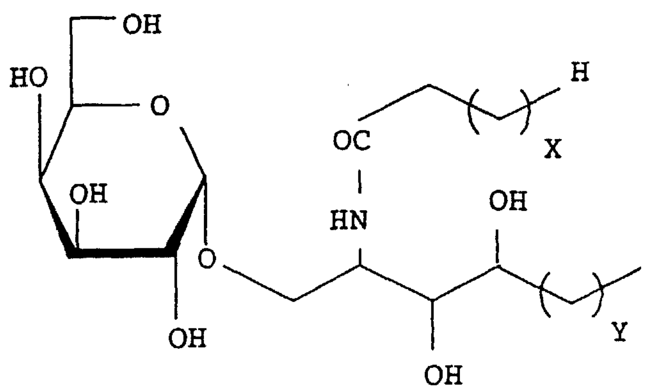

(VII)

wherein X denotes an integer of 18-26 and Y denotes an integer of 5-15;
(11) the glycosphingolipids described in the above (10), wherein more preferably X denotes an integer of 21-25 and Y denotes an integer of 6-14;
(12) the glycosphingolipids described in the above (10) which is represented more preferably by the formula (VIII):

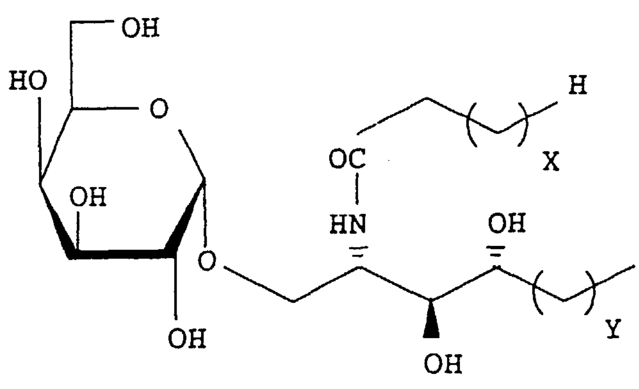

(VIII)

wherein X denotes an integer of 18-26 and Y denotes an integer of 5-15;
(13) the glycosphingolipids described in the above (12), wherein most preferably X denotes an integer of 21-25 and Y denotes an integer of 6-14;
(14) the glycosphingolipids of the formula (I) represented by the formula (IX):

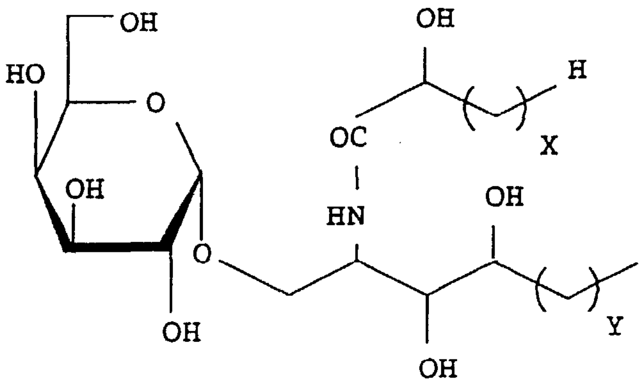

(IX)

wherein X denotes an integer of 18-26 and Y denotes an integer of 5-17;
(15) the glycosphingolipids described in the above (14), wherein more preferably X denotes an integer of 21-25 and Y denotes an integer of 6-16;
(16) the glycosphingolipids described in the above (14) represented more preferably by the formula (X):

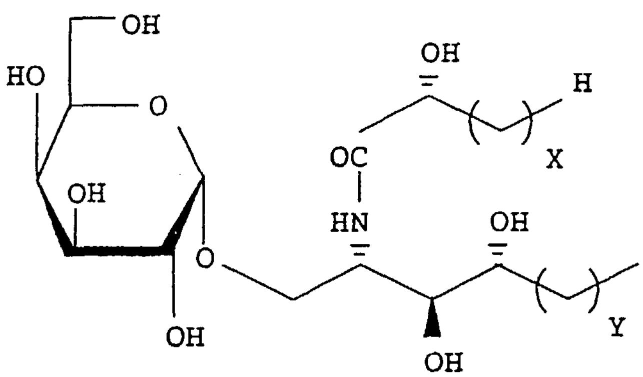

(X)

wherein X denotes an integer of 18-26 and Y denotes an integer of 5-17;
(17) the glycosphingolipids described in the above (14) represented more preferably by the formula (X'):

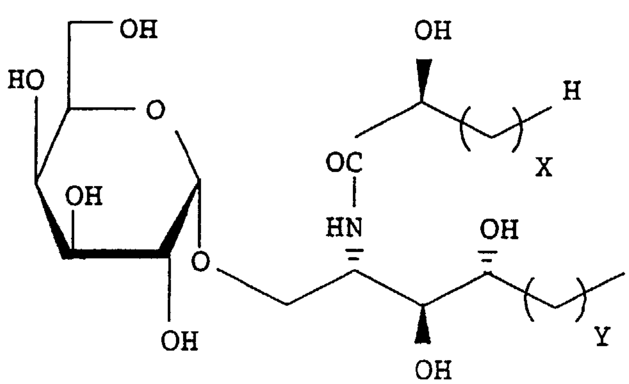

(X')

wherein X denotes an integer of 20-24 and Y denotes an integer of 10-14;
(18) the glycosphingolipids described in the above (16), wherein most preferably X denotes an integer of 21-25 and Y denotes an integer of 6-16;
(19) the glycosphingolipids described in the above (17), wherein most preferably X denotes an integer of 21-23 and Y denotes an integer of 11-13;
(20) the glycosphingolipids of the formula (I) represented by the formula (XI):

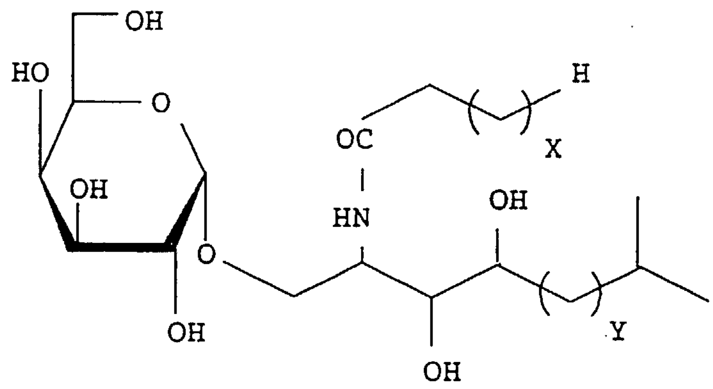

(XI)

wherein X denotes an integer of 20-24 and Y denotes an integer of 9-13;
(21) the glycosphingolipids described in the above (20), wherein more preferably X denotes an integer of 21-23 and Y denotes an integer of 10-12;
(22) the glycosphingolipids described in the above (20) more preferably represented by the formula (XII):

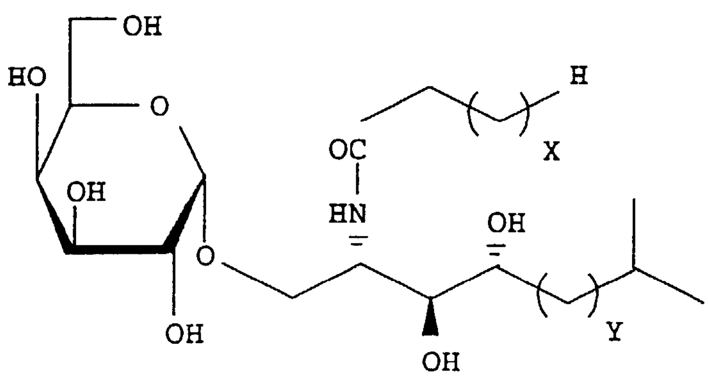

(XII)

wherein X denotes an integer of 20-24 and Y denotes an integer of 9-13;
(23) the glycosphingolipids described in the above (22), wherein more preferably X denotes an integer of 21-23 and Y denotes an integer of 10-12;
(24) the glycosphingolipids of the formula (I) represented by the formula (XIII):

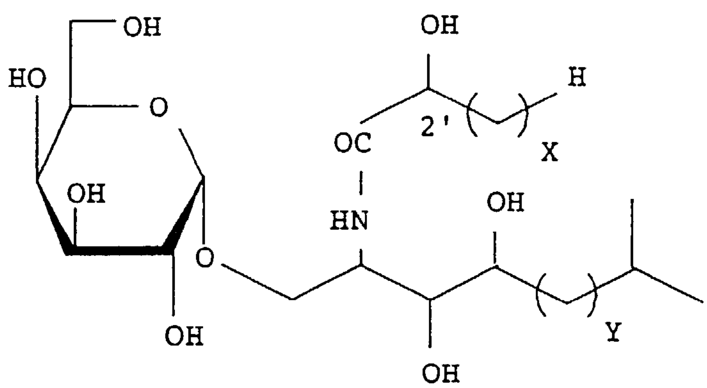

(XIII)

wherein X denotes an integer of 18-24 and Y denotes an integer of 9-13;
(25) the glycosphingolipids described in the above (24), wherein more preferably X denotes an integer of 20-23 and Y denotes an integer of 10-12;
(26) the glycosphingolipids described in the above (24), more preferably represented by the formula (XIV):

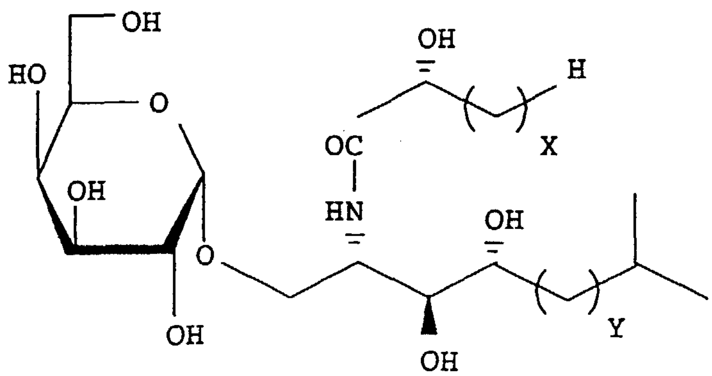

(XIV)

wherein X denotes an integer of 19-23 and Y denotes an integer of 9-13;
(27) the glycosphingolipids described in the above (24), more preferably represented by the formula (XIV'):

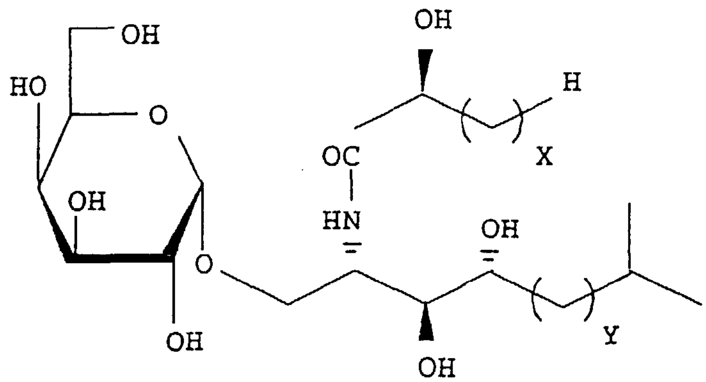

(XIV')

wherein X denotes an integer of 20-24 and Y denotes an integer of 9-13;
(28) the glycosphingolipids described in the above (26), wherein most preferably X denotes an integer of 20-22 and Y denotes an integer of 10-12;
(29) the glycosphingolipids described in the above (27), wherein most preferably X denotes an integer of 21-23 and Y denotes an integer of 10-12;
(30) the glycosphingolipids of the formula (I) represented by the formula (XV):

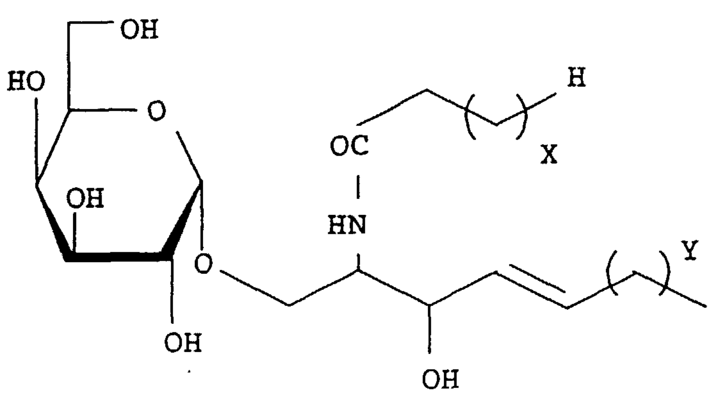

(XV)

wherein X denotes an integer of 10-18 and Y denotes an integer of 10-14;
(31) the glycosphingolipids described in the above (30), wherein more preferably X denotes an integer of 11-17 and Y denotes an integer of 11-13;
(32) the glycosphingolipids described in the above (30) more preferably represented by the formula (XVI):

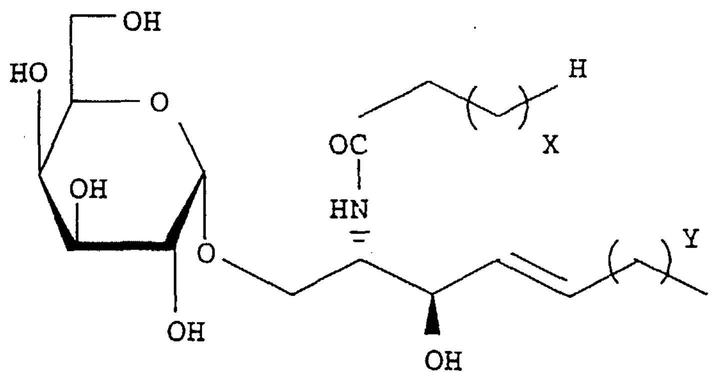

(XVI)

wherein X denotes an integer of 10-18 and Y denotes an integer of 10-14;
(33) the glycosphingolipids described in the above (32), wherein most preferably X denotes an integer of 11-17 and Y denotes an integer of 11-13;
(34) the glycosphingolipids of the formula (I) represented by the formula (XVII):

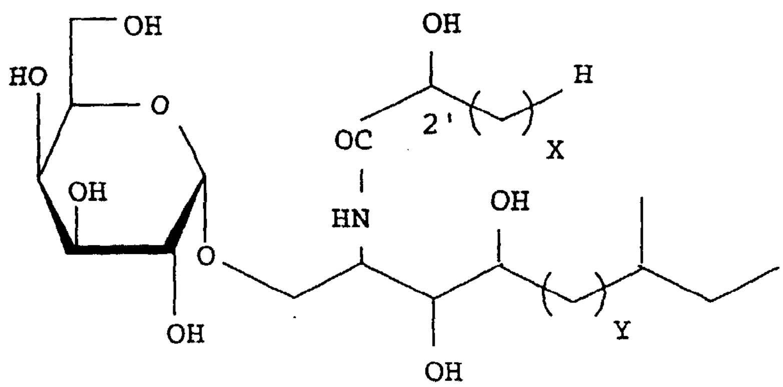

(XVII)

wherein X denotes an integer of 21-25 and Y denotes an integer of 9-13;
(35) the glycosphingolipids described in the above (34), wherein more preferably X denotes an integer of 22-24 and Y denotes an integer of 10-12;
(36) the glycosphingolipids described in the above (34) more preferably represented by the formula (XVIII):

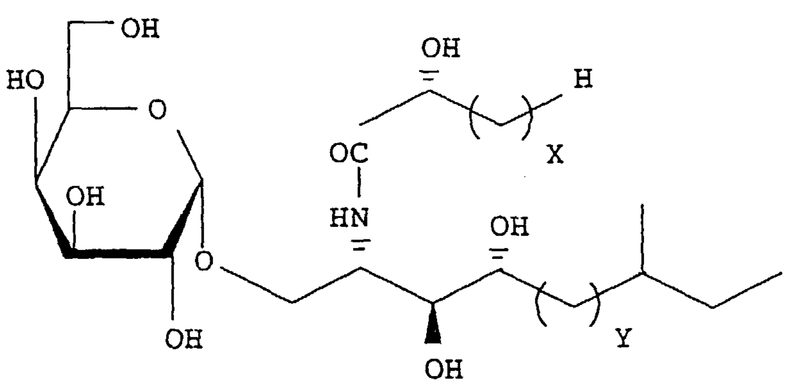

(XVIII)

wherein X denotes an integer of 21-25 and Y denotes an integer of 9-13;
(37) the glycosphingolipids described in the above (36), wherein most preferably X denotes an integer of 22-24 and Y denotes an integer of 10-12;
(38) the glycosphingolipids of the formula (XXI) represented by the formula (XIX):

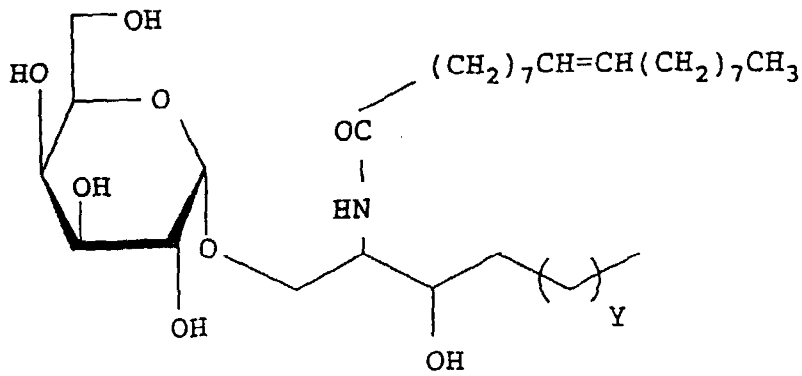

(XIX)

wherein Y denotes an integer of 11-15;

(39) the glycosphingolipids described in the above (38), wherein more preferably Y denotes an integer of 12-14;

(40) the glycosphingolipid described in the above (38) more preferably represented by the formula (XX):

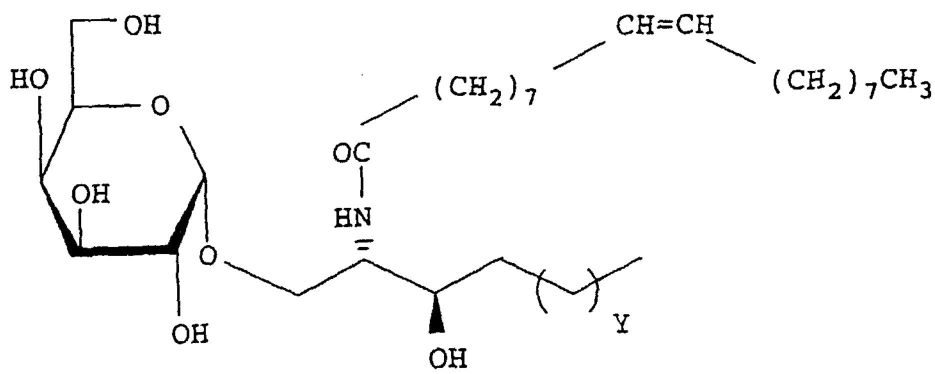

(XX)

wherein Y denotes an integer of 11-15; and

(41) the glycosphingolipids described in the above (40), wherein most preferably Y denotes an integer of 12-14.

Concrete preferred examples of compounds included in the present invention represented by the formula (A) (formulae (I) and (XXI)) are shown below. In respective formulae, X and Y are defined as above.

(1) The compounds represented by the following formulae (IV) and (VI)

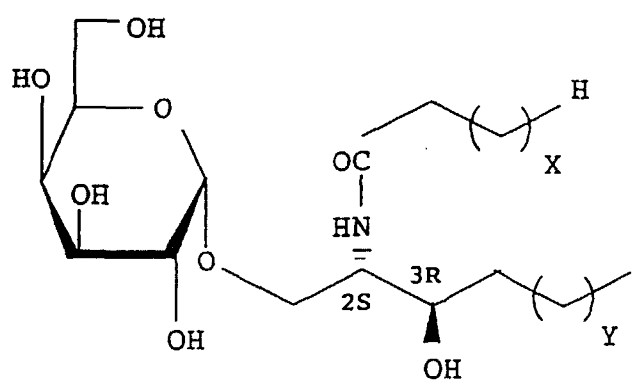

(IV)

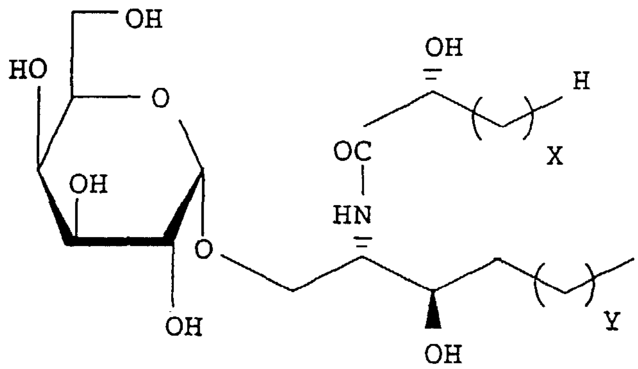

(VI)

Compound 1:
(2S,3R)-1-(α-D-galactopyranosyloxy)-2-tetracosanoylamino-3-octadecanol,
Compound 2:
(2S,3R)-2-docosanoylamino-1-(α-D-galactopyranosyloxy)-3-octadecanol,
Compound 3:
(2S,3R)-1-(α-D-galactopyranosyloxy)-2-icosanoylamino-3-octadecanol,
Compound 4:
(2S,3R)-1-(α-D-galactopyranosyloxy)-2-octadecanoylamino-3-octadecanol,
Compound 5:
(2S,3R)-1-(α-D-galactopyranosyloxy)-2-tetradecanoylamino-3-octadecanol,
Compound 6:
(2S,3R)-2-decanoylamino-1-(α-D-galactopyranosyloxy)-3-octadecanol,
Compound 7:
(2S,3R)-1-(α-D-galactopyranosyloxy)-2-octanoylamino-3-octadecanol,
Compound 8:
(2S,3R)-2-acetamino-1-(α-D-galactopyranosyloxy)-3-octadecanol,
Compound 9:
(2S,3R)-1-(α-D-galactopyranosyloxy)-2-tetracosanoylamino-3-tetradecanol,
Compound 10:
(2S,3R)-1-(α-D-galactopyranosyloxy)-2-tetradecanoylamino-3-hexadecanol,
Compound 11:
(2R,3S)-1-(α-D-galactopyranosyloxy)-2-tetradecanoylamino-3-hexadecanol,
Compound 12:
(2S,3S)-1-(α-D-galactopyranosyloxy)-2-tetradecanoylamino-3-hexadecanol,
Compound 13:
(2R,3R)-1-(α-D-galactopyranosyloxy)-2-tetradecanoylamino-3-hexadecanol,
Compound 14:
(2S,3R)-1-(α-D-galactopyranosyloxy)-2-[(R)-2-hydroxytetracosanoylamino]-3-octadecanol.

Among these compounds, the compounds 1-10 and 14 are preferred in consideration of the configuration at 2- and 3-positions.

(2) The compounds represented by the following formula (XVI)

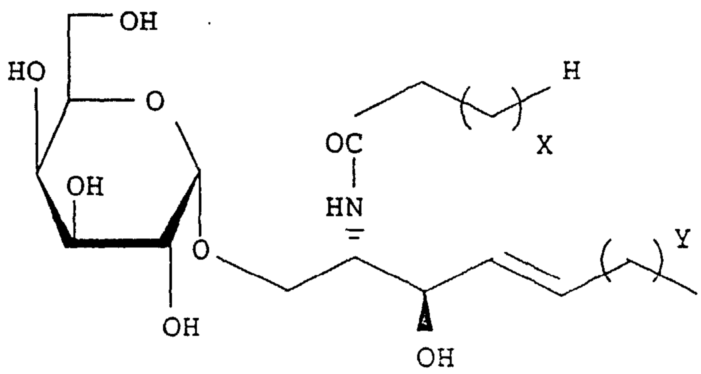

(XVI)

Compound 15:
(2S,3R,4E)-1-(α-D-galactopyranosyloxy)-2-octadecanoylamino-4-octadecen-3-ol,
Compound 35:
(2S,3R,4E)-1-(α-D-galactopyranosyloxy)-2-tetradecanoylamino-4-octadecen-3-ol.

(3) The compounds represented by the following formula (VIII)

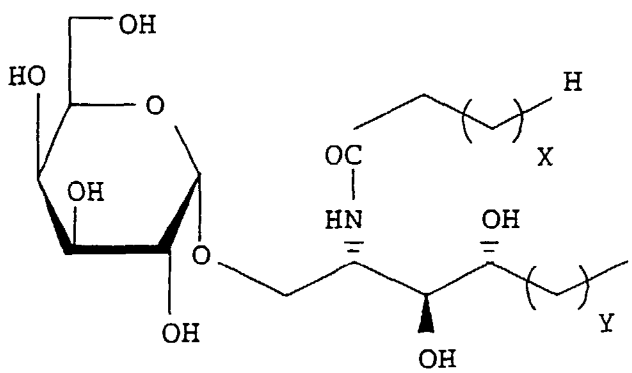

(VIII)

Compound 16:
(2S,3S,4R)-1-(α-D-galactopyranosyloxy)-2-tetracosanoylamino-3,4-octadecanediol,
Compound 17:
(2S,3S,4R)-1-(α-D-galactopyranosyloxy)-2-tetracosanoylamino-3, 4-heptadecanediol,
Compound 18:
(2S,3S,4R)-1-(α-D-galactopyranosyloxy)-2-tetracosanoylamino-3,4-pentadecanediol,
Compound 19:
(2S,3S,4R)-1-(α-D-galactopyranosyloxy)-2-tetracosanoylamino-3,4-undecanediol,
Compound 20:
(2S,3S,4R)-1-(α-D-galactopyranosyloxy)-2-hexacosanoylamino-3,4-heptadecanediol.

(4) The compounds represented by the following formulae (X) and (X')

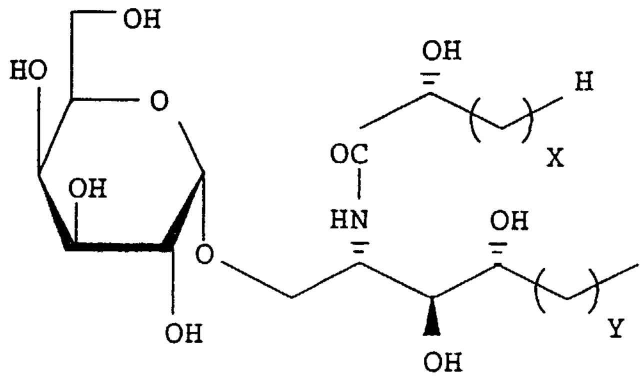

(X)

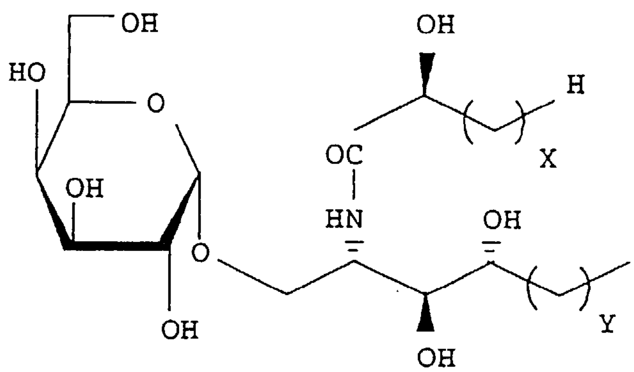

(X')

Compound 21:
(2S,3S,4R)-1-(α-D-galactopyranosyloxy)-2-[(R)-2-hydroxytetracosanoylamino]-3,4-octadecanediol,
Compound 22:
(2S,3S,4R)-1-(α-D-galactopyranosyloxy)-2-[(R)-2-hydroxytetracosanoylamino]-3,4-heptadecanediol,
Compound 23:
(2S,3S,4R)-1-(α-D-galactopyranosyloxy)-2-[(R)-2-hydroxytetracosanoylamino]-3,4-pentadecanediol,
Compound 24:
(2S,3S,4R)-1-(α-D-galactopyranosyloxy)-2-[(R)-2-hydroxytetracosanoylamino]-3,4-undecanediol,
Compound 25:
(2S,3S,4R)-1-(α-D-galactopyranosyloxy)-2-[(R)-2-hydroxyhexacosanoylamino]-3,4-octadecanediol,
Compound 26:
(2S,3S,4R)-1-(α-D-galactopyranosyloxy)-2-[(R)-2-hydroxyhexacosanoylamino]-3,4-nonadecanediol,
Compound 27:
(2S,3S,4R)-1-(α-D-galactopyranosyloxy)-2-[(R)-2-hydroxyhexacosanoylamino]-3,4-icosanediol,
Compound 28:
(2S,3S,4R)-1-(α-D-galactopyranosyloxy)-2-[(S)-2-hydroxytetracosanoylamino]-3,4-heptadecanediol,
Compound 32:
(2S,3S,4R)-1-(α-D-galactopyranosyloxy)-2-[(R)-2-hydroxytetracosanoylamino]-3,4-hexadecanediol.

(4) The compounds represented by the following formulae (XII), (XIV) and (XIV')

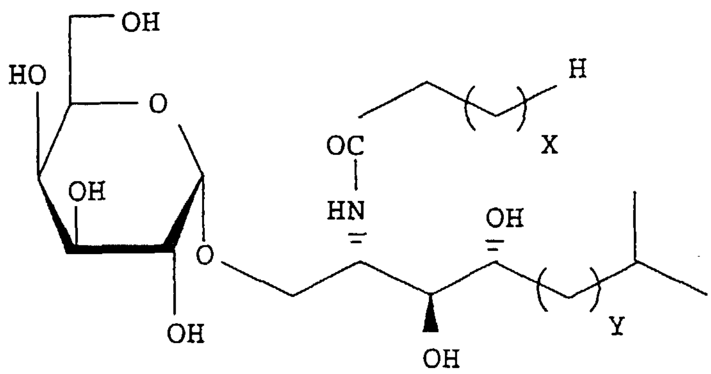

(XII)

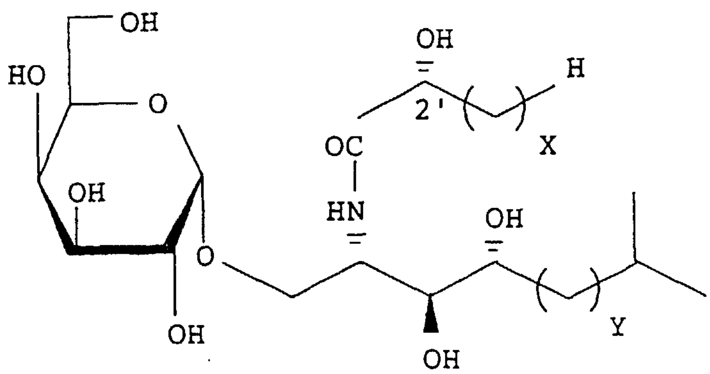

(XIV)

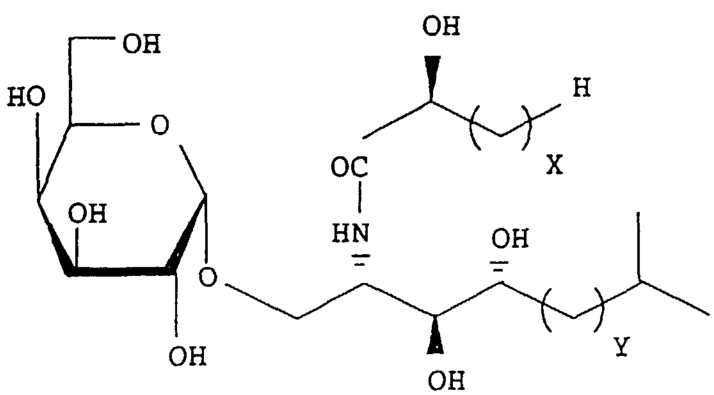

(XIV')

Compound 30:
(2S,3S,4R)-1-(α-D-galactopyranosyloxy)-2-[(S)-2-hydroxytetracosanoylamino]-16-methyl-3,4-heptadecanediol,
Compound 31:
(2S,3S,4R)-1-(α-D-galactopyranosyloxy)-16-methyl-2-tetracosanoylamino-3,4-heptadecanediol,
Compound 33:
(2S,3S,4R)-1-(α-D-galactopyranosyloxy)-2-[(R)-2-hydroxytricosanoylamino]-16-methyl-3,4-heptadecanediol.

(5) The compound represented by the following formula (XVIII)

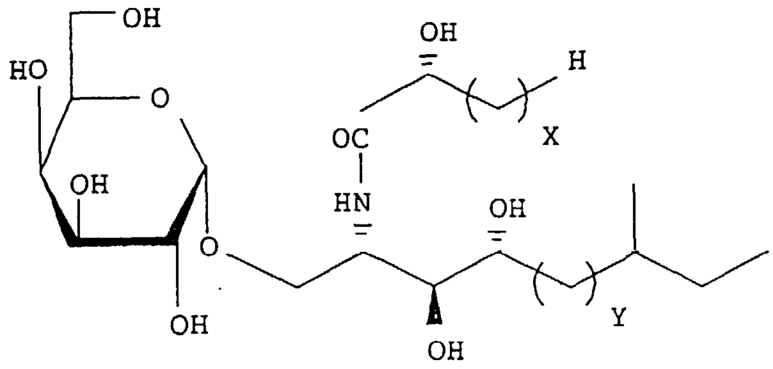

(XVIII)

Compound 34:
(2S,3S,4R)-1-(α-D-galactopyranosyloxy)-2-[(R)-2-hydroxypentacosanoylamino]-16-methyl-3,4-octadecanediol.

(6) The compound represented by the following formula (XIX)

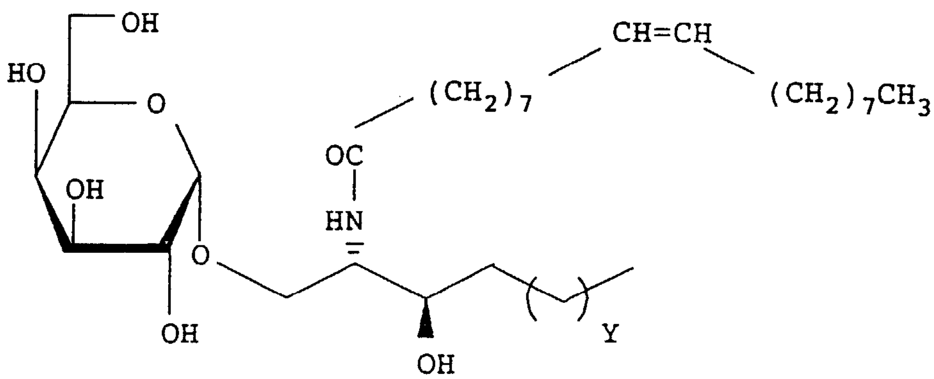

(XIX)

Compound 29:
(2S,3R)-1-(α-D-galactopyranosyloxy)-2-oleoylamino-3-octadecanol.

Process for preparing the compounds of the present invention

(i) Process for obtaining the compounds from a marine sponge

Among the compounds represented by the formula (I), the glycosphingolipids such as the compounds 22, 32, 33 and 34 can be obtained by extraction from some sponges with an organic solvent.

Fundamentally the process for obtaining the compounds from marine sponges comprises A: a step for collecting the sponges, B: an extraction step for contacting the sponges with at least one appropriate organic solvent in order to obtain a crude extract containing the compound of the present invention, and C: a step for isolating the compound of the present invention from the crude extract obtained in the step B.

(Step A)

This step is one for collecting marine sponges.

A preferred example of a species of marine sponges is Agelas mauritianus, which can be collected from the sea of Kumeshima in Okinawa Prefecture of Japan.

(Step B)

This step is one for extracting the compound of the present invention as a crude extract from the sponge with at least one appropriate organic solvent or water.

An organic solvent is preferable as the extracting solvent. It is sufficient that an appropriate organic solvent for extraction is a solvent which can extract the compound of the present invention from the sponge. Preferred examples of the solvent are esters such as ethyl acetate and butyl acetate and so on; alcohols such as methanol, ethanol and isopropyl alcohol and so on; aliphatic hydrocarbons such as heptane, hexane and isooctane and so on; ketones such as acetone and methyl ethyl ketone and so on; aromatic compounds such as benzene and toluene and so on; ethers such as diethyl ether and t-butyl methyl ether and so on; and substituted lower aliphatic hydrocarbon compounds such as methylene chloride, chloroform and 1,2-dichloroethane and so on. In the present invention, these solvents can be used respectively alone or in combination of the two or more thereof.

Among the organic solvents mentioned above, more preferable examples are ethanol, acetone, ethyl acetate and the like, and the preferred examples of combination of a plurality of the solvents are methanol and chloroform, methanol and methylene chloride, acetone and toluene and the like.

As the method for extraction, well-known methods in relation to the extraction of physiologically active substances, particularly a glycosphingolipid, from a living material such as an animal or a plant or a microorganism, for example, the methods described in Liebigs Annalen der Chemie, 51, (1990); Tetrahedron, 45 (12), 3897, (1989); or Zeitschrift fuer Naturforschung Teil B, 42 (11), 1476, (1987) can be applied. These extraction methods can be used respectively alone or in combination of the two or more thereof.

Specifically, for example, a marine sponge is applied as it is or after the preliminary treatments such as homogenization and lyophilization, and the extraction operation is carried out preferably with stirring at a temperature of 0-80°C, preferably around room temperature for an extraction period of 1-72 hours, preferably 12-36 hours. If necessary, the aforementioned extraction operation can be repeated at desired times.

(Step C)

This step is one for isolating the compound of the present invention from the crude extract which is obtained in the step B and contains the compound of the present invention.

As the isolating method, well-known methods in relation to the fractionation and isolation of a physiologically active substances, particularly a glycosphingolipids, from a variety of living materials as described above can be used. The general descriptions concerning such a method are given for example in Liebigs Annalen der Chemie, 51, (1990).

More specifically, as the fractionation method, the examples include fractionating method with use of the difference of solubilities (for example, by the combination of water and methanol), distributing method (involving the countercurrent distribution method; for example, by the combination of ethylacetate and water) with use of the difference of distribution rates, and the like. The aforementioned crude extract can be treated by these fractionation methods to recover the objective fraction, and thus the aforementioned four compounds of the present invention can be obtained as the crude products.

In order to further purify the resulting crude products of the compounds of the present invention, the combination of the aforementioned fractionation methods with the isolating methods as described below may be carried out at desired times. If necessary, the purified product of the compound of the present invention can also be obtained by subjecting the crude extract obtained in the step B to an appropriate operation of an isolating method at necessary times.

The examples of such isolating methods are the methods for eluting the objective product by chromatography such as adsorption chromatography, distribution chromatography, thin layer chromatography, high performance liquid chromatography or gel filtration and the like. A concrete example of chromatography is the column chromatography in which a stationary phase such as silica gel, ODS, TOYOPEARL HW-40® (TOSO, Japan) or Sephadex LH-20® (Pharmacia) is employed, and as a mobile phase an organic solvent as described above in the paragraph of the step B or water is used alone or in combination of the two or more thereof. As the preferable concrete examples of the eluent, mentioned are methanol, chloroform and the like as the single eluent, and methanol and chloroform, methanol and water and the like as the mixture.

(ii) Process by chemical synthesis

While the compounds according to the present invention, that is, the glycosphingolipids represented by the formula (A) (formulae (I) and (XXI)) can be derived from a variety of the chemical modifications of sphingosine, they can be also prepared by the overall synthesis with the chemical synthetic means which is a combination of a variety of general chemical reactions required for the synthesis of the glycosphingolipids. The route of the overall synthesis is not the only one, and the glycosphingolipid can be prepared via an alternative route from a different starting material. It can be also synthesized, for example, by applying the method described in Agricultural and Biological Chemistry, 54 (3), 663, 1990 which is an example of the chemical synthetic means. It can be also synthesized, for example, by applying the method described in Liebigs Annalen der Chemie, 663, 1988 which is an example of using a variety of sugars as the starting materials. Although a protective group is removed after a sugar is bonded to a ceramide in these

synthetic methods, it is also possible to use the method for synthesizing a cerebroside in which a sugar is first bonded to a long chain base and an amino group is then introduced to form an amide, as described in Liebigs Annalen der Chemie, 663, 1988.

(Synthetic route A)

As an example of the synthesis as described above, the compounds represented by the formulae (III), (V) and (XIX) can be synthesized also via the following steps (cf. Figs. 1a and 1b).
In Fig. 1, the following abbreviations are used:

Bn: benzyl,
$R_4$: a hydroxyl group or a formyloxy group,
Ms: methanesulfonyl,
$R_5$: a hydrogen atom or an acyloxy group,
Tr: triphenylmethyl, and
Bz: benzoyl.

An aldehyde as a raw material has one or two asymmetric centers. An amino acid or a sugar can also be employed as the asymmetric sources. While a benzyl group is employed as the protective group of a hydroxyl group in this example, any appropriate groups such as an isopropylidene group may be also employed.
In this route scheme, particularly many reaction methods are known for the amidation. An acid chloride or an acid anhydride can also be employed in place of a carboxylic acid.
The reaction with a carboxylic acid is a condensation reaction in the presence of an appropriate condensation agent. Suitable condensation agent used herein are dicyclohexylcarbodiimide (DCC), 2-ethoxy-1-ethoxycarbonyl-1,2-dihydroquinoline (EEDQ), 1-ethyl-3-(3-dimethylaminopropyl)-carbodiimide (WSC), chlorocarbonates, onium salts and the like. In order to progress the reaction rapidly, an organic base such as triethylamine, pyridine, N-methylmorpholine, dimethylaniline, 4-dimethylaminopyridine, N-methylpiperidine or N-methylpyrrolidine is added. Any inert solvents which do not participate in the reaction may be used as the solvent.
The reaction with an acid chloride satisfactorily proceeds generally in the presence of a solvent. Although the reaction is generally conducted with use of an appropriate solvent, the reaction which proceeds slowly can be progressed rapidly in the absence of the solvent. Any inert solvents which do not participate in the reaction may be used as the solvent. If the reaction proceeds slowly, it may be progressed rapidly by the addition of an organic solvent such as triethylamine, pyridine, N-methylmorpholine, dimethylaniline or 4-dimethylaminopyridine.
The reaction with an acid anhydride is preferably conducted in the presence of an appropriate base. As the base herein used, triethylamine, pyridine or the like is usually used as the solvent concurrently.
Many methods of reaction for glycosylation are also known as described in the following references: (1) YUKI GOSEI KAGAKU, 38 (5), 473, 1980; (2) YUKI GOSEI KAGAKU, 41 (8), 701, 1983; (3) Pure and Applied Chemistry, 61 (7), 1257, 1989; (4) Pharmacia, 27 (1), 50, 1991.
Any of the reactions described above may be used, but a method for obtaining preferentially an α-galactoside such as the one described in Chemistry Letters, 431-432, 1981 is preferred. If the α-isomer is not obtained alone, its separation from the β-isomer is carried out. When such a separation is difficult, the α-isomer and the β-isomer can be separated by introducing the hydroxyl group into an acyl derivative (e.g. an acetyl derivative).

(Synthetic route B)

It is also possible to show the following reaction route scheme as a shorter process starting from the same raw material as in the synthetic route A. The compounds represented by the formulae (III), (V) and (XIX) can be synthesized also by this method (see Fig. 2). In Fig. 2, the same abbreviations as described above are used. This route is characterized in that the steps are successfully reduced by performing simultaneously the reduction of the azide group, the removal of the benzyl group and the reduction of the double bond. The four isomers of the 2-amino-1,3-alkanediol which are intermediates obtained by the reduction can be obtained alone, respectively, by selecting the asymmetric sources of the aldehyde as the starting material depending on the purposes. The isomers are individually subjected to the subsequent amidation. A variety of the methods as described in the route A can be employed in this step. Subsequently, glycosylation and deprotection can be conducted in the similar way to the route A to obtain the objective product.

(Synthetic route C)

As an example of the synthesis introduced by a variety of chemical modifications of sphingosine, the compounds represented by the formulae (IV), (VI), (XVI) and (XX) in which the long chain base portion has 18 carbon atoms can be also synthesized via the following process (see Fig. 3). In Fig. 3, the same abbreviations as described above are used. While sphingosine can be obtained by the extraction from natural materials, it is commercially available from Sigma Chemical Company or Funakoshi Corporation, Japan. It can be also synthesized by a variety of synthetic methods as described in Pharmacia, 27, 1164, 1991 or Journal of the Chemical Society Perkin Transaction 1, 2279, 1991. The isomers having steric configurations different from those of the natural materials can be also synthesized by applying the method described in Helvetica Chimica Acta, 40, 1145, 1957 or Journal of the Chemical Society, Chemical Communications, 820, 1991. In the latter reference, many examples of the synthesis are reported. In this route, the double bond can be left also after the glycosylation. That is, if catalytic reduction is employed, a compound having no double bond is obtained, and if metallic sodium is reacted in liquid ammonia, a compound retaining a double bond is produced. Thus, it is possible to prepare the products suitable for the purpose.

(Synthetic route D)

Furthermore, the compounds represented by the formulae (VII), (IX), (XI), (XIII) and (XVII) among the compounds represented by the formula (A) in which the long chain base has a hydroxyl group at C-4 can also be synthesized via the following process (see Figs. 4a-4c). In Fig. 4, the same abbreviations as described above are used.

The starting aldehyde can be obtained alone as any isomers by selecting appropriately the asymmetric source of a raw material. The isomers are separately subjected to the subsequent Wittig reaction. The terminal of the Wittig's salts can be easily formed into the iso type, the anteiso type or the straight chain type. Generally, the Wittig reaction with such unstable ylid give a compound having a cis-double bond as main product, which is however contaminated by the trans-isomer. The double bonds in the mixture are however reduced to a single bond during the step of the catalytic reduction, and thus the mixture will cause no problem as it is. By subsequent mesylation and azide inversion, the product is reduced to an amino derivative, which is amidated in the subsequent step to give a ceramide. The intermediate ceramide having protective groups is also obtained by protecting a commercially available Cerebrine E (Alfred Bader Chemicals or K&K Laboratories Inc.) as the raw material with any appropriate protective group. Furthermore, in order to discriminate the hydroxyl group to which the sugar is bonded, protection and selective deprotection followed by glycosylation and deprotection can be conducted to obtain the objective product (see Figs. 4b-4c).

The use of the compounds of the present invention

The compounds of the present invention represented by the formula (A) (formulae (I) and (XXI)) have the following physiological activities, that is, an antitumor activity and an immuno-stimulating activity and can be used as an antitumor agent and an immunostimulator.

(1) Antitumor activity

The compounds of the present invention exhibited antitumor activities against the B16 mouse melanoma cells inoculated s.c. in mouse as shown in Experimental Example 2 below.

(2) Immuno-stimulating activity

The compounds of the present invention exhibited the stimulating effect on mixed lymphocyte culture reaction (MLR) in the test of mouse MLR as described in Experimental Example 3 below.

(3) Antitumor agent and immuno-stimulatory agent

As described above, the compound of the present invention has the antitumor activity and the immuno-stimulating activity and can be employed as an antitumor agent and an immunostimulator.

While the compounds of the present invention may be employed alone, these compounds may be used also in combination with the chemotherapy or the radiotherapy. Their uses have been reviewed in Pharmaceutical Society of Japan, Pharmacia Review, No. 23, Chemistry for Controlling Cancer, Second Series, 105-113, 1987; Medicalview Co., Ltd., Illustrative Clinic, "Cancer" series No. 19, GAN TO MENEKI, 159-169, 1987; IGAKU NO AYUMI, 150 (14), 1018-1021, 1989.

Since the compounds of the present invention exhibit such an immuno-stimulating activity as described above,

they are also employed as an immunostimulator against disorders other than cancer such as various infectious diseases, acquired immunodeficiency syndrome or the like. These uses have been described as the general in Medicalview Co., Ltd., Illustrative Clinic, "Cancer" series No. 19, GAN TO MENEKI, 45-50, 1987 and RINSHO KAGAKU, 23 (10), 1299-1305, 1987.

The compounds of the present invention as an antitumor agent and the immunostimulator can be administered via any appropriate dosage route in drug form determined by the dosage route adopted. As the drug, it takes generally a form which is diluted and molded with a pharmaceutically acceptable additive (carrier or diluent). When the compounds of the present invention are used as antitumor agent or immunostimulator, they can be administered orally or parenterally to human or mammal. For example, the compound of the present invention can be administered by dissolving, suspending or emulsifying it in an appropriate solvent for injection (e.g. distilled water for injection) and injecting it intravenously, intramuscularly or subcutaneously. The compound of the present invention can be administered orally by adding an appropriate additive (e.g. any compounds which are usually used for this purpose such as starch, lactose, crystalline cellulose, hydroxypropylcellulose (HPC), calcium carboxymethylcellulose (CMC-Ca), magnesium stearate and the like) and forming the mixture into powder, tablet, granule, capsule, troche, dry syrup or the like.

The dose of the compound of the present invention is determined to ensure that the dose administered continuously or intermittently will not exceed a certain amount in consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the patient or the disease, and the appropriate dose and dosage times under the certain conditions must be determined by the test for determining the appropriate dose by a medical specialist based on the above-described indices. In this connection, the minimal dose required for developing the activity of the compound of the present invention is generally in the range of ca. 0.0001 mg - 100 mg per 1 kg of the body weight of a host.

Experimental Examples

The present will now be described in detail with reference to the following Experimental Examples. In the Examples which follow, the names of the following products are Registered Trade Marks:

Molecular Seives-4A
Dowex 50W
Wako Gel C-200

Experimental Example 1-A: Preparation from a natural material

Preparation of the compounds 22, 32, 33 and 34:

The sponge Agelas mauritianus collected from the sea of Kumeshima in Okinawa Prefecture of Japan was subjected to homogenization and lyophilization to give a product (1,077.6 g). It was extracted with methanol-chloroform (1:1) as the first solvent to give an extract, which was then concentrated under reduced pressure to give a residue (178.53 g). The residue was distributed between ethyl acetate as the first distribution solvent and water. The upper ethyl acetate layer was dried over sodium sulfate anhydrous, and the lower aqueous layer was extracted with 1-butanol. The ethyl acetate soluble fractions and the 1-butanol soluble fractions containing the compounds 32, 33, 22 and 34 were combined together and concentrated under reduced pressure to give a residue (125.22 g), which was washed with 30% aqueous methanol and extracted with methanol. The extract was concentrated under reduced pressure to give a brown solid product in the yield of 37.50 g. The solid product was applied to silica gel column chromatography (Wako Gel C-200), and separated by eluting with chloroform initially and then with chloroform-methanol with gradually increasing the ratio of methanol. Eluent with chloroform containing 5%-8% methanol afforded an active fraction (20.05 g), which was further extracted with methanol and concentrated under reduced pressure to give a brown solid product. The solid product was applied to a ODS column (YMC-ODS-A) and washed with 30% aqueous methanol and then eluted with methanol to give an active fraction (1.2127 g), which was applied to reversed phase high performance liquid chromatography (Rp-HPLC) on a YMC-D-ODS-5 (manufactured from K.K. YMC) detected with an RI detector, eluting with 100% methanol at 11 ml/min flow rate to afford the compounds of the present invention 32 (24.0 mg), 33 (29.5 mg), 22 (20.9 mg) and 34 (9.8 mg) at the retention times of 39, 41, 46 and 74 minutes, respectively.

The compounds 32, 33, 22 and 34 have the following spectral data:

Compound 32

$[\alpha]^{28}_D$ = +61.6° (1-PrOH, c = 1.0)

MS: negative FABMS 816.
IR: ($cm^{-1}$, Kbr)
3400, 2950, 2870, 1645, 1535, 1475, 1080.
mp: 193.5 - 195.0°C
NMR: $^{1}H$ (500 MHz, $C_5D_5N$; 27°C)

δ (ppm)
8.49 (1H, d, J=9.2 Hz), 7.53 (1H, bs), 7.04 (1H, bs), 6.71 (1H, d, J = 6.7 Hz), 6.68 (1H, bs), 6.52 (1H, bs), 6.32 (1H, bs), 6.09 (1H, d, J=6.1 Hz), 5.58 (1H, d, J=3.7 Hz), 5.26 (1H, m), 4.62 (2H, m), 4.57 (1H, m), 4.52 (1H, bs), 4.48 (2H, m), 4.37 (1H, m), 4.34 (2H, m), 4.32 (1H, m), 4.26 (1H, m), 2.28 (1H, m), 2.18 (1H, m), 1.98 (1H, m), 1.87 (2H, m), 1.73 (1H, m), 1.66 (2H, m), 1.10-1.46 (56H, m), 0.85 (6H, t, J = 7.3 Hz).
$^{13}C$ (125 MHz, $C_5D_5N$; 27°C)
δ (ppm)
175.0 (s), 101.2 (d), 76.5 (d), 73.0 (d), 72.4 (d), 72.3 (d), 71.6 (d), 70.9 (d), 70.1 (d), 68.1 (t), 62.6 (t), 50.4 (d), 35.5 (t), 34.4 (t), 32.1 (t), 30.3 (t), 30.1 (t), 30.0 (t), 29.9 (t), 29.5 (t), 26.4 (t), 25.8 (t), 22.9 (t), 14.2 (q).

Compound 33

$[\alpha]^{28}_D$ = +65.4° (1-PrOH, c = 1.0)

MS: negative FABMS 830.
IR: ($cm^{-1}$, Kbr)
3400, 2950, 2870, 1645, 1535, 1475, 1080.
mp: 203.0 - 205.0°C
NMR: $^{1}H$ (500 MHz, $C_5D_5N$; 27°C)

δ (ppm)
8.49 (1H, d, J=9.2 Hz), 7.53 (1H, bs), 7.04 (1H, bs), 6.71 (1H, d, J=6.7 Hz), 6.68 (1H, bs), 6.52 (1H, bs), 6.32 (1H, bs), 6.09 (1H, d, J = 6.1 Hz), 5.58 (1H, d, J=3.7 Hz), 5.26 (1H, m), 4.62 (2H, m), 4.57 (1H, m), 4.51 (1H, bs), 4.48 (2H, m), 4.36 (1H, m), 4.33 (3H, m), 4.25 (1H, m), 2.29 (1H, m), 2.18 (1H, m), 1.99 (1H, m), 1.88 (2H, m), 1.73 (1H, m), 1.66 (2H, m), 1.46 (2H, m), 1.10-1.42 (53H, m), 0.84 (9H, m).
$^{13}C$ (125 MHz, $C_5D_5N$; 27°C)
δ (ppm)
175.0 (s), 101.2 (d), 76.5 (d), 73.1 (d), 72.4 (d), 72.4 (d), 71.6 (d), 70.9 (d), 70.2 (d), 68.2 (t), 62.6 (t), 50.6 (d), 39.2 (t), 35.5 (t), 34.4 (t), 32.1 (t), 30.3 (t), 30.1 (t), 30.0 (t), 29.8 (t), 29.7 (t), 29.5 (t), 28.2 (d), 27.8 (t), 27.4 (t), 26.4 (t), 25.8 (t), 23.0 (t), 22.8 (q), 14.2 (q).

Compound 22

$[\alpha]^{28}_D$ = +69.2° (1-PrOH, c = 1.0)

MS: negative FABMS 830.
IR: ($cm^{-1}$, Kbr)
3400, 2950, 2870, 1645, 1535, 1475, 1080.
mp: 201.0 - 203.5°C
NMR: $^{1}H$ (500 MHz, $C_5D_5N$; 27°C)

δ (ppm)
8.48 (1H, d, J=9.2 Hz), 7.53 (1H, bs), 7.03 (1H, bs), 6.71 (1H, d, J=6.7 Hz), 6.67 (1H, bs), 6.53 (1H, bs), 6.32 (1H, bs), 6.09 (1H, bs), 5.59 (1H, d, J=3.7 Hz), 5.27 (1H, m), 4.63 (2H, m), 4.58 (1H, m), 4.52 (1H, bs), 4.47 (2H, m), 4.38 (1H, m), 4.32 (3H, m), 4.26 (1H, m), 2.27 (1H, m), 2.18 (1H, m), 1.98 (1H, m), 1.88 (2H, m), 1.73 (1H, m), 1.65 (2H, m), 1.10-1.46 (58H, m), 0.85 (6H, t, J=7.3 Hz).
$^{13}C$ (125 MHz, $C_5D_5N$; 27°C)
δ (ppm)
175.0 (s), 101.2 (d), 76.5 (d), 73.0 (d), 72.4 (d), 72.3 (d), 71.6 (d), 70.9 (d), 70.2 (d), 68.3 (t), 62.6 (t), 50.4 (d), 35.5 (t), 34.4 (t), 32.1 (t), 30.3 (t), 30.1 (t), 29.9 (t), 29.6 (t), 29.5 (t), 26.4 (t), 25.9 (t), 22.9 (t), 14.2 (q).

Compound 34

$[\alpha]^{28}_D$ = +59.4° (1-PrOH, c = 1.0)

MS: negative FABMS 872.
IR: ($cm^{-1}$, Kbr)
3400, 2950, 2870, 1645, 1535, 1475, 1080.
mp: 215.5 - 218.0°C
NMR: $^1H$ (500 MHz, $C_5D_5N$; 27°C)

δ (ppm)

8.50 (1H, d, J=9.2 Hz), 7.53 (1H, bs), 7.02 (1H, bs), 6.71 (1H, d, J=6.7 Hz), 6.66 (1H, bs), 6.52 (1H, bs), 6.31 (1H, bs), 6.09 (1H, d, J=3.9 Hz), 5.59 (1H, d, J=3.7 Hz), 5.27 (1H, m), 4.62 (2H, m), 4.58 (1H, m), 4.52 (1H, bs), 4.47 (2H, m), 4.38 (1H, m), 4.33 (3H, m), 4.26 (1H, m), 2.28 (1H, m), 2.18 (1H, m), 1.99 (1H, m), 1.87 (2H, m), 1.73 (1H, m), 1.66 (2H, m), 1.10-1.42 (61H, m), 0.85 (9H, m).

$^{13}C$ (125 MHz, $C_5D_5N$; 27°C)

δ (ppm)

175.0 (s), 101.2 (d), 76.5 (d), 73.0 (d), 72.4 (d), 72.3 (d), 71.6 (d), 70.9 (d), 70.1 (d), 68.2 (t), 62.6 (t), 50.5 (d), 36.8 (t), 35.5 (t), 34.5 (d), 34.4 (t), 32.0 (t), 30.3 (t), 30.3 (t), 30.1 (t), 30.0 (t), 29.8 (t), 29.7 (t), 29.5 (t), 27.3 (t), 26.4 (t), 25.8 (t), 22.9 (t), 19.3 (q), 14.2 (q), 11.5 (q).

Experimental Example 1-B: Preparation by the synthetic methods

The methods for synthesizing the compounds of the present invention and the physico-chemical properties thereof are shown below (see reaction route schemes 1-10).

(1) Synthetic route A

While this scheme is shown specifically with reference to the aforementioned compound 9, the compounds 1-8 and 10-14 according to the present invention can also be synthesized by applying this method (see Figs. 5a and 5b).

In the above scheme, the following abbreviations are used.

DMAP: 4-dimethylaminopyridine,
TsOH: p-toluenesulfonic acid,
MS-4A: Molecular Sieves-4A (dehydrating agent).

The other abbreviations have the same meanings as in the previous route schemes.

Furthermore, the compound 29 leaving a double bond unreacted therein can be synthesized by the use of a fatty acid having a double bond as a starting material and by the deprotection at the final step with liquid ammonia and metallic sodium.

[Synthesis of the compound 9 (Figs. 5a and 5b)]

The compound A1 can be synthesized in accordance with the method described in Synthesis, 961-963, 1984.

(i) Synthesis of the compound A2

To a solution of the compound A1 (2.89 g) in 2-methyl-2-propanol (25 ml) was added a 5% aqueous sulfuric acid solution (25 ml), and the mixture was stirred at 45°C for 15 hours. After being neutralized with powdery sodium hydrogen carbonate under ice-cooling, the reaction mixture was concentrated. The residue, to which water (30 ml) was added, was extracted with ethyl acetate (three times), and the organic layer was concentrated. Purification on a silica gel column (Wako Gel C-200, 100 g) using hexane-acetone (2:1) as an eluent afforded a diol in an amount of 2.28 g (yield: 88.5%).

MS: FDMS 330.

The mixture of the diol (2.25 g) with ethanol (50 ml), water (12 ml) and sodium metaperiodate (2.33 g) was stirred at room temperature for 10 hours. Precipitates were removed by filtration, and the filtrate was concentrated.

The residue was diluted with chloroform and washed with brine. The organic layer was concentrated to give an aldehyde (compound A2) in an amount of 1.31 g. The aldehyde was directly used for the next reaction without purification.

(ii) Synthesis of the compound A3

To decanetriphenylphosphonium bromide (8.0 g) was added tetrahydrofuran (20 ml) under an argon atmosphere. After adding a 2.8 N solution of n-butyllithium in hexane (6.2 ml) to the mixture at -10°C, stirring was continued for 30 minutes. After the addition of the aldehyde (compound A2, 1.31 g) dissolved in tetrahydrofuran (5 ml), the mixture was allowed to warm to room temperature and stirred for 15 hours and concentrated. The reaction mixture was diluted with brine, and extracted twice with ethyl acetate. The organic layer was washed with brine and concentrated. Purification of the residue on a silica gel column (Wako Gel C-200, 100 g) by eluting with hexane-ethyl acetate (5:1) gave the alcohol (compound A3) in an amount of 1.47 g (yield, 51.0%).
Data of the compound A3

MS: FDMS 426.
NMR: $^1H$ (500 MHz, $CDCl_3$; 27°C)

δ (ppm)
7.25-7.35 (10H, m), 5.69-5.79 [1H, (5.75, dt, J=7.3, 11.0 Hz), (5.72, dt, J=6.7, 15.2 Hz)], 5.31-5.38 [1H, (5.36, bt, J=8.5 Hz), (5.33, bt, J=9.8 Hz)], 4.34-4.62 [2H, (4.61 & 4.35, ABq, J=11.6 Hz), (4.56 & 4.50, ABq, J=12.2 Hz), (4.55 & 4.52, ABq, J=11.6 Hz)], 4.28 (0.7H, dd, J=6.7, 9.7 Hz), 3.85 (0.3H, bt, J=7.9 Hz), 3.74-3.78 (1H, m), 3.56-3.60 [1H (3.59, dd, J=3.1, 9.8 Hz), (3.58, overlapped)], 3.47 (1H, dd, J=5.5, 9.8 Hz), 1.96-2.11 (1H, m), 1.25-1.57 (14H, m), 0.88 (3H, t, J=6.7 Hz).

(iii) Synthesis of the compound A4

The alcohol (compound A3, 0.83 g) was dissolved in tetrahydrofuran (10 ml). 10% Palladium on charcoal (1.0 g) was added, and the reaction vessel was purged with hydrogen. After the mixture was stirred at room temperature for 12 hours, it was filtered through celite and the filtrate was concentrated. Purification on a silica gel column (Wako Gel C-200, 30 g) eluting with hexane-ethyl acetate (5:1) afforded a reduction product (compound A4) in an amount of 0.81 g (yield, 97.1%).
Data of the compound A4

MS: FDMS 428.
NMR: $^1H$ (500 MHz, $CDCl_3$; 27°C)

δ (ppm)
7.25-7.46 (10H, m), 4.50 & 4.62 (2H, ABq, J=11.0 Hz), 4.54 (2H, s), 3.79-3.83 (1H, m), 3.48-3.56 (3H, m), 2.42 (1H, d, J=6.1 Hz), 1.26-2.04 (20H, m), 0.88 (3H, t, J=7.3 Hz).

(iv) Synthesis of the compound A5

After adding methanesulfonyl chloride (0.29 ml) to the reduction product (compound A4, 0.80 g) in pyridine (15 ml), the mixture was stirred at room temperature for 16 hours. The reaction mixture was concentrated and distilled azeotropically with toluene. The residue dissolved in diethyl ether was washed with brine and concentrated. Purification on a silica gel column (Wako Gel C-200, 30 g) eluting with hexane-acetone (6:1) afforded a mesylated product (compound A5) in an amount of 0.87 g (yield, 91.9%).
Data of the compound A5

MS: FDMS 504.
NMR: $^1H$ (500 MHz, $CDCl_3$; 27°C)

δ (ppm)
7.27-7.38 (10H, m), 4.81-4.84 (1H, m), 4.59 (2H, s), 4.55 & 4.50 (2H, ABq, J=11.6 Hz), 3.75 (1H, dd, J=3.1, 11.0 Hz), 3.71 (1H, dd, J=6.7, 11.0 Hz), 3.67 (1H, dt, J=4.3, 8.5 Hz), 2.99 (3H, s), 1.24-1.64 (20H, m), 0.88 (3H, t, J=7.3 Hz).

(v) Synthesis of the compound A6

To the mesylated product (compound A5, 0.86 g) were added dimethylformamide (10 ml) and sodium azide (885 mg), and the mixture was stirred at 120°C for 15 hours. The reaction mixture was diluted with brine, extracted with ethyl acetate (three times), and then concentrated. Purification on a silica gel column (Wako Gel C-200, 30 g) eluting with hexane-ethyl acetate (40:1) afforded an azide (compound A6) in an amount of 0.73 g (yield, 94.3%).
Data of the compound A6

MS: FDMS 453.
NMR: $^1H$ (500 MHz, $CDCl_3$; 27°C)

δ (ppm)
7.27-7.44 (10H, m), 4.54 & 4.58 (2H, ABq, J=12.2 Hz), 4.52 & 4.57 (2H, ABq, J=11.0 Hz), 3.68-3.70 (2H, m), 3.63 (1H, dd, J=8.5, 11.0 Hz), 3.53 (1H, dt, J=4.3, 8.6 Hz), 1.25-1.64 (20H, m), 0.88 (3H, t, J=6.7 Hz).

(vi) Synthesis of the compound A7

To the azide (compound A6, 0.72 g) were added tetrahydrofuran (7 ml) and 10% palladium on charcoal (70 mg), and the mixture was stirred at room temperature after the reaction vessel was purged with hydrogen. The reaction mixture was filtered through celite, and the filtrate was concentrated. Purification on a silica gel column (Wako Gel C-200, 15 g) eluting with hexane-acetone (6:1) afforded an amine (compound A7) in an amount of 0.62 g (yield, 91.5%).
Data of the compound A7

MS: FDMS 427.
NMR: $^1H$ (500 MHz, $CDCl_3$; 27°C)

δ (ppm)
7.27-7.36 (10H, m), 4.51 & 4.54 (2H, ABq, J=11.6 Hz), 4.52 (2H, s), 3.58 (1H, dd, J=3.7, 9.2 Hz), 3.41-3.45 (2H, m), 3.20 (1H, dt, J=4.3, 7.3 Hz), 1.26-1.63 (20H, m), 0.88 (3H, t, J=6.7 Hz).

(vii) Synthesis of the compound A8

To the amine (compound A7, 0.61 g) were added methylene chloride (20 ml), 2-chloro-1-methylpyridinium iodide (483 mg) and n-tributylamine (0.45 ml). Tetracosanic acid (597 mg) was further added, and the mixture was heated under reflux for 2 hours. The reaction mixture was cooled to room temperature, washed sequentially with 5% aqueous sodium thiosulfate solution, 5% aqueous sodium hydrogen carbonate solution and brine, and then concentrated. Purification on silica gel column (Wako Gel C-200, 20 g) eluting with hexane-acetone (20:1) afforded an amide (compound A8) in an amount of 0.56 g (yield, 51.2%).
Data of the compound A8

MS: FDMS 777.
NMR: $^1H$ (500 MHz, $CDCl_3$; 27°C)

δ (ppm)
7.28-7.35 (10H, m), 5.66 (1H, d, J=9.2 Hz), 4.45 & 4.58 (2H, ABq, J=11.6 Hz), 4.48 (2H, s), 4.25-4.30 (1H, m), 3.73 (1H, dd, J=4.9, 9.8 Hz), 3.57 (1H, dt, J=5.5, 6.7 Hz), 3.52 (1H, dd, J=4.3, 9.8 Hz), 2.08 (2H, dt, J=3.1, 10.4 Hz), 1.26-1.58 (64H, m), 0.88 (6H, t, J=6.7 Hz).

(viii) Synthesis of the compound A9

To the amide (compound A8, 0.55 g) were added tetrahydrofuran (15 ml) and palladium black (55 mg). The reaction vessel was purged with hydrogen, and the mixture was stirred at room temperature for 16 hours. The reaction mixture was filtered through celite, and the filtrate was concentrated. Purification on a silica gel column (Wako Gel C-200, 20 g) eluting with chloroform-methanol (20:1) afforded a diol (compound A9) in an amount of 302 mg (yield, 71.6%).
Data of the compound A9

MS: FDMS 597.
NMR: $^1H$ (500 MHz, $C_5D_5N$; 27°C)

δ (ppm)

8.34 (1H, d, J=7.9 Hz), 4.62-4.67 (1H, m), 4.46 (1H, dd, J=4.9, 11.0 Hz), 4.30 (1H, dd, J=5.8, 11.6 Hz), 4.25-4.32 (1H, m), 2.48 (2H, dt, J=2.4, 7.3 Hz), 1.23-1.97 (62H, m), 0.88 (6H, t, J=6.7 Hz).

(ix) Synthesis of the compound A10

To the diol (compound A9, 70 mg) were added pyridine (5 ml), triphenylmethyl chloride (261 mg) and 4-dimethylaminopyridine (5 mg), and the mixture was stirred at 60°C for 2 hours. The reaction mixture was diluted with chloroform, washed with brine and concentrated. Purification on a silica gel column (Wako Gel C-200, 10 g) eluting with chloroform-acetone (100:1) afforded a tritylated derivative (compound A10) in an amount of 90.2 mg (yield, 91.6%).
Data of the compound A10

MS: FDMS 837.
NMR: $^1$H (500 MHz, $CDCl_3$; 27°C)

δ (ppm)

7.25-7.47 (15H, m), 6.28 (1H, d, J=7.9 Hz), 3.93-3.96 (1H, m), 3.58-3.61 (1H, m), 3.52 (1H, dd, J=3.1, 9.8 Hz), 3.26 (1H, dd, J=3.7, 9.8 Hz), 2.95 (1H, d, J=9.2 Hz), 2.24 (2H, t, J=7.3 Hz), 1.25-1.70 (62H, m), 0.88 (6H, t, J=7.3 Hz).

(x) Synthesis of the compound A11

To the trityl derivative (compound A10, 87 mg) in pyridine (3.0 ml) were added benzoyl chloride (24 μℓ) and 4-dimethylaminopyridine (3 mg), and the mixture was stirred for 4 hours. After the mixture to which ice-water had been added was stirred for 30 minutes, it was diluted with chloroform, washed with water and concentrated. Purification on a silica gel column (Wako Gel C-200, 10 g) eluting with hexane-ethyl acetate (10:1) afforded a benzoyl derivative (compound A11) in an amount of 83.4 mg (yield, 85.3%).
Data of the compound A11

MS: FDMS 941.
NMR: $^1$H (500 MHz, $CDCl_3$; 27°C)

δ (ppm)

7.16-7.93 (20H, m), 5.74 (1H, d, J=9.2 Hz), 5.34-5.37 (1H, m), 4.39-4.48 (1H, m), 3.40 (1H, dd, J=3.7, 9.8 Hz), 3.19 (1H, dd, J=3.7, 9.8 Hz), 2.09 (2H, dt, J=2.5, 9.8 Hz), 1.25-1.74 (64H, m), 0.88 & 0.87 (each 3H, t, J=7.3 Hz).

(xi) Synthesis of the compound A12

To the benzoyl derivative (compound A11, 80 mg) were added methylene chloride (1.0 ml) and methanol (0.5 ml). p-Toluenesulfonic acid monohydrate (20 mg) was added, and the mixture was stirred at room temperature for 2 hours. The reaction mixture was diluted with ethyl acetate, washed with a 5% aqueous sodium hydrogen carbonate and brine, and then concentrated. Purification on a silica gel column (Wako Gel C-200, 5 g) eluting with hexane-ethyl acetate (2:1) afforded an alcohol (compound A12) in an amount of 58 mg (yield, 93.6%).
Data of the compound A12

MS: FDMS 701.
NMR: $^1$H (500 MHz, $CDCl_3$; 27°C)

δ (ppm)

7.46-8.06 (5H, m), 6.25 (1H, d, J=8.5 Hz), 5.06-5.09 (1H, m), 4.15-4.19 (1H, m), 3.58-3.68 (2H, m), 2.23 (2H, t, J=6.7 Hz), 1.22-1.77 (62H, m), 0.88 & 0.87 (each 3H, t, J=7.3 Hz).

(xii) Synthesis of the compound A14

A solution of the alcohol (compound A12, 58 mg) in tetrahydrofuran (3.0 ml) was stirred with stannous chloride (37 mg), silver perchlorate (41 mg) and Molecular Sieves 4A powder (300 mg). After stirring for 30 minutes, the mixture was cooled to -10°C, and a solution of benzyl galactosyl fluoride (compound A13, 68 mg) in tetrahydrofuran (1.5 ml) was added. The mixture was allowed to warm gradually to room temperature, stirred for 2 hours and filtered through celite. The filtrate was concentrated. Purification on a silica gel column (Wako Gel C-200, 5 g) eluting with hexane-ethyl acetate (5:1) afforded an α-galactoside (compound A14) in an amount of 62.6 mg (yield, 61.8%).
Data of the compound A14

MS: FDMS 1224.
NMR: $^1$H (500 MHz, $CDCl_3$; 27°C)

δ (ppm)
8.02 (2H, d, J=7.3 Hz), 7.56 (1H, t, J=7.9 Hz), 7.43 (2H, t, J=7.9 Hz), 7.23-7.39 (20H, m), 6.58 (1H, d, J=9.2 Hz), 5.30 (1H, dt, J=3.7, 7.9 Hz), 4.90 & 4.55 (2H, ABq, J=11.6 Hz), 4.77 & 4.69 (2H, ABq, J=11.6 Hz), 4.75 (1H, d, J=3.7 Hz), 4.73 & 4.65 (2H, ABq, J=12.2 Hz), 4.47 & 4.38 (2H, ABq, J=12.2 Hz), 4.30-4.34 (1H, m), 4.10-4.12 (1H, m), 4.01 (1H, dd, J=3.7, 9.8 Hz), 3.97 (1H, dd, J=3.7, 12.2 Hz), 3.84-3.93 (2H, m), 3.57 (1H, dd, J=3.1, 12.2 Hz), 3.52 (1H, dd, J=7.3, 9.2 Hz), 3.29 (1H, dd, J=4.3, 9.8 Hz), 1.98-2.09 (2H, m), 1.18-1.68 (62H, m), 0.88 (3H, t, J=6.7 Hz), 0.86 (3H, t, J=7.3 Hz).

(xiii) Synthesis of the compound A15

To the α-galactoside (compound A14, 56 mg) were added tetrahydrofuran (4.0 ml) and palladium black (15 mg), and the mixture was stirred at room temperature for 16 hours after the reaction vessel was purged with hydrogen. The reaction mixture was filtered through celite, concentrated and purified on a silica gel column (Wako Gel C-200, 2 g) eluting with chloroform-methanol (20:1) to give a tetraol (compound A15) in an amount of 37.4 mg (yield, 94.7%).
Data of the compound A15

MS: FDMS 863.
NMR: $^1$H (500 MHz, $CDCl_3$; 27°C)

δ (ppm)
8.04 (2H, d, J=7.9 Hz), 7.62 (1H, t, J=7.9 Hz), 7.48 (2H, t, J=7.3 Hz), 6.16 (1H, d, J=9.2 Hz), 5.21-5.24 (1H, m), 4.81 (1H, d, J=2.4 Hz), 4.45-4.46 (1H, m), 4.08 (1H, bs), 3.91-3.94 (1H, m), 3.87 (1H, dd, J=2.4, 10.4 Hz), 3.75-3.85 (4H, m), 3.57 (1H, dd, J=5.5, 11.6 Hz), 2.22 (2H, dt, J=1.8, 7.3 Hz), 1.22-1.79 (62H, m), 0.88 (3H, t, J=7.3 Hz), 0.87 (3H, t, J=6.7 Hz).

(xiv) Synthesis of the compound 9

To the tetraol (compound A15, 36.0 mg) were added methanol (3 ml) and a 1N methanolic sodium methoxide solution (0.3 ml), and the mixture was stirred for 2 hours. The mixture was neutralized with resins (Dowex 50W, X8; manufactured by The Dow Chemical Company), and then filtered. The solids removed was washed sufficiently with chloroform-methanol (1:1), and the extract was combined with the filtrate, and then concentrated. Purification on a silica gel column (Wako Gel C-200, 2 g) eluting with chloroform-methanol (10:1) afforded the compound 9 in an amount of 29.7 mg (yield, 94.0%).
Data of the compound 9
$[\alpha]^{23}_D$ = +49.0° (pyridine, c = 1.31)

MS: FDMS 759.
IR: ($cm^{-1}$, Kbr)
3200, 2870, 2800, 1630, 1530, 1450, 1080.
mp: 151-155°C
NMR: $^1$H (500 MHz, $C_5D_5N$; 27°C)

δ (ppm)
8.49 (1H, d, J=8.6 Hz), 6.11-6.52 (5H, m), 5.45 (1H, d, J=3.7 Hz), 4.73 (1H, m), 4.65 (1H, dd, J=3.8, 10.4 Hz), 4.53-4.57 (2H, m), 4.43-4.49 (4H, m), 4.36 (1H, dd, J=5.5, 10.4 Hz), 4.27 (1H, m), 2.47 (2H, t, J=6.7 Hz), 1.83-1.91 (4H, m), 1.23-1.56 (58H, m), 0.88 (6H, t, J=7.3 Hz).
$^{13}$C (125 MHz, $C_5D_5N$; 27°C)
δ (ppm)
173.4 (s), 102.1 (d), 73.1 (d), 71.9 (d), 71.7 (d), 71.0 (d), 70.5 (d), 69.7 (t), 62.7 (t), 54.9 (d), 36.8 (t), 35.1 (t), 32.1 (t), 30.2 (t), 30.1 (t), 30.0 (t), 29.9 (t), 29.8 (t), 29.7 (t), 29.6 (t), 26.6 (t), 26.4 (t), 22.9 (t), 14.3 (q).

(2) Synthetic route B

While this reaction route scheme specifically illustrates the synthetic routes of the aforementioned compounds 7 and 5, the compounds according to the present invention (1-4, 6, 8-14) can also be synthesized by applying this method.

[Synthesis of the compound 7 (Fig. 6)]

Abbreviations in the aforementioned scheme are the same as those in the previously described scheme.

(i) Synthesis of the compound B1

To tetradecanetriphenylphosphonium bromide (213.7 g) was added tetrahydrofuran (630 ml), and the reaction vessel was purged with argon. A 2.3N solution of n-butyl lithium in hexane (173 ml) was added at -30°C, and the mixture was stirred for 3.5 hours. A (2R,3R)-aldehyde (compound A2, 31.73 g) dissolved in tetrahydrofuran (630 ml) was added dropwise, and the mixture was stirred for 2 hours, and then concentrated. The residue was diluted with ethyl acetate, washed with water and brine, and then concentrated. Purification on a silica gel column (Wako Gel C-200, 850 g) eluting with hexane-ethyl acetate (9:1) afforded an alcohol (compound B1) in an amount of 36.31 g (yield, 79.0%).
Data of the compound B1

MS: FDMS 481.
NMR: $^1H$ (500 MHz, $CDCl_3$; 27°C)

δ (ppm)
7.26-7.46 (10H, m), 5.69-5.78 (1H, m), 5.31-5.38 (1H, m), 4.34-4.63 (5H, m), 4.28 (0.7H, dd, J=6.7, 9.2 Hz), 3.85 (0.3H, t, J=7.3 Hz), 3.75-3.78 (1H, m), 3.56-3.60 (1H, m), 3.47 (1H, dd, J=5.5, 10.4 Hz), 1.98-2.11 (2H, m), 1.26-1.34 (22H, m), 0.88 (3H, t, J=6.7 Hz).

(ii) Synthesis of the compound B2

To a solution of the alcohol (compound B1, 5.03 g) in pyridine (50 ml) was added methanesulfonyl chloride (1.62 ml), and the mixture was stirred at room temperature for 16 hours. The mixture was concentrated and a residual acid chloride was distilled azeotropically together with toluene. The residue was diluted with diethyl ether, washed with brine, and then concentrated. Purification on a silica gel column (Wako Gel C-200, 200 g) eluting with hexane-acetone (10:1) afforded a mesyl derivative (compound B2) in an amount of 5.20 g (yield, 88.9%).
Data of the compound B2

MS: FDMS 558.
NMR: $^1H$ (500 MHz, $CDCl_3$; 27°C)

δ (ppm)
7.23-7.35 (10H, m), 5.77-5.83 (1H, m), 5.26-5.35 (1H, m), 4.71-4.77 (1H, m), 4.33-4.62 (5H, m), 4.06 (0.3H, t, J=8.1 Hz), 3.74 (0.7H, dd, J=3.1, 11.0 Hz), 3.65-3.70 (1H, m), 2.964 (0.9H, s), 2.956 (2.1H, s), 1.99-2.17 (2H, m), 1.26-1.37 (22H, m), 0.88 (3H, t, J=6.8 Hz).

(iii) Synthesis of the compound B3

To the mesyl derivative (compound B2, 1.52 g) were added dimethylformamide (20 ml) and sodium azide (1.42 g). After stirring at 120°C for 12 hours, the mixture was diluted with brine, extracted with ethyl acetate (three times), and then concentrated. Purification on a silica gel column (Wako Gel C-200, 50 g) eluting with hexane-ethyl acetate (40:1) afforded an azide derivative (compound B3) in an amount of 1.07 g (yield, 77.7%).
Data of the compound B3

IR: ($cm^{-1}$, Kbr)
2870, 2810, 2050, 1490, 1440.
NMR: $^1H$ (500 MHz, $CDCl_3$; 27°C)

δ (ppm)

7.25-7.35 (10H, m), 5.69-5.82 (1H, m), 5.35-5.43 (1H, m), 4.30-4.74 (4H, m), 3.89 (0.3H, dd, J=5.5, 8.5 Hz), 3.55-3.70 (3.7H, m), 1.97-2.10 (2H, m), 1.25-1.36 (22H, m), 0.88 (3H, t, J=6.8 Hz).

(iv) Synthesis of the compound B5

To a solution of the azide (compound B3, 0.45 g) in tetrahydrofuran (10 ml) were added a 10% methanolic hydrochloric acid solution (2 ml) and palladium black (0.25 g). After the reaction vessel was purged with hydrogen, the mixture was stirred at room temperature for 12 hours, and then filtered through celite. The filtrate was concentrated to give a white powdery amine (compound B4, 301 mg). Tetrahydrofuran (10 ml), p-nitrophenyl octanoate (260 mg) and triethylamine (0.15 ml) were added to the amine, the mixture was stirred at 60°C for 12 hours. The reaction mixture was concentrated to give a syrup. Purification of the syrup on a silica gel column (Wako Gel C-200, 50 g) eluting with chloroform-methanol (20:1) afforded an amide derivative (compound B5) in an amount of 166 mg (yield based on the compound B3, 43.6%).
Data of the compound B5

MS: FDMS 429.
NMR: $^1H$ (500 MHz, $C_5D_5N$; 27°C)

δ (ppm)

8.37 (1H, d, J=7.9 Hz), 4.63-4.69 (1H, m), 4.44-4.49 (1H, m), 4.25-4.35 (2H, m), 2.46 (2H, dt, J=3.1, 7.9 Hz), 1.78-1.95 (4H, m), 1.16-1.59 (34H, m), 0.87 & 0.82 (each 3H, t, J=6.7 Hz).

(v) Synthesis of the compound B6

To a solution of the amide (compound B5, 48 mg) in tetrahydrofuran (1.0 ml) were added stannous chloride (75 mg), silver perchlorate (82 mg) and powdery Molecular Sieves 4A (200 mg), and the mixture was stirred for 30 minutes. The mixture was cooled to -10°C, and a solution of benzylgalactosyl fluoride (compound A13, 67 mg) in tetrahydrofuran (2.0 ml) was added thereto. The mixture was allowed to warm gradually to room temperature, stirred for 2 hours, and then filtered through celite. The solids removed were washed with a small amount of acetone and combined with the filtrate, and then concentrated. Purification on a silica gel column (Wako Gel C-200, 5 g) eluting with hexane-ethyl acetate (3:1) afforded a crude α-galactoside (compound B6), which was subjected to the subsequent reaction.

(vi) Synthesis of the compound 7

To a solution of the α-galactoside (compound B6, 47 mg) in ethyl acetate (1.5 ml) was added palladium black (15 mg). After the reaction vessel was purged with hydrogen, the mixture was stirred at room temperature for 16 hours. The mixture was filtered through celite, and the filtrate was concentrated. Purification on a silica gel column (Wako Gel C-200, 2 g) eluting with chloroform-methanol (10:1) afforded the compound 7 in an amount of 25.1 mg (yield based on the compound B5, 37.9%).
Data of the compound 7
$[\alpha]^{23}_D = +58.2°$ (pyridine, c = 0.56)

MS: FDMS 591.
IR: ($cm^{-1}$, Kbr)
3300, 2870, 2810, 1640, 1535, 1460, 1060.
mp: 155-157°C
NMR: $^1H$ (500 MHz, $C_5D_5N$; 27°C)

δ (ppm)

8.49 (1H, d, J=8.6 Hz), 6.52 (2H, m), 6.42 (1H, m), 6.33 (1H, bs), 6.12 (1H, bd, J=6.7 Hz), 5.46 (1H, d, J=3.7 Hz), 4.73 (1H, m), 4.65 (1H, m), 4.53-4.57 (2H, m), 4.40-4.49 (5H, m), 4.36 (1H, dd, J=5.5, 10.4 Hz), 4.27 (1H, m), 2.45 (2H, dt, J=5.5, 7.9 Hz), 1.80-1.92 (4H, m), 1.18-1.58 (34H, m), 0.87 & 0.81 (each 3H, t, J=6.7 Hz).
$^{13}C$ (125 MHz, $C_5D_5N$; 27°C)

δ (ppm)

173.4 (s), 102.2 (d), 73.1 (d), 72.0 (d), 71.7 (d), 71.0 (d), 70.8 (d), 70.5 (d), 69.7 (t), 62.7 (t), 54.9 (d), 36.8 (t), 35.1 (t), 32.1 (t), 31.9 (t), 30.2 (t), 30.1 (t), 30.0 (t), 29.9 (t), 29.64 (t), 29.61 (t), 29.4 (t), 26.6 (t), 26.4 (t), 22.93 (t), 22.86

(t), 14.3 (q), 14.2 (q).

[Synthesis of the compound 5 (Fig. 7)]

Abbreviations in the aforementioned scheme are the same as those in the previously described scheme.

(i) Synthesis of the compound B7

To a solution of the azide (compound B3, 3.9 g) in ethyl acetate (50 ml) was added 10% palladium on charcoal (1.2 g). After the reaction vessel was purged with hydrogen, the mixture was stirred at room temperature for 16 hours. The catalyst was filtered off, and the filtrate was concentrated and purified on a silica gel column (Wako Gel C-200, 300 g, hexane-acetone (6:1)) to give an amine (compound B7) in an amount of 3.22 g (yield, 86.7%).

MS: FDMS 480.
NMR: $^1H$ (500 MHz, $CDCl_3$; 27°C)

δ (ppm)

7.24-7.35 (10H, m), 5.79 (0.7H, dt, J=7.3, 11.6 Hz), 5.71 (0.3 H, dt, J=6.7, 15.3 Hz), 5.34-5.41 (1H, m), 4.30-4.58 (4H, m), 4.17 (0.7H, dd, J=6.7, 9.8 Hz), 3.72 (0.3H, dd, J=6.7, 8.5 Hz), 3.42-3.66 (2H, m), 3.06-3.10 (1H, m), 2.01-2.14 (2H, m), 1.26-1.50 (22H, m), 0.88 (3H, t, J=6.7 Hz).

(ii) Synthesis of the compound B8

To a solution of the amine (compound B7, 2.22 g) in methylene chloride (50 ml), 2-chloro-1-methylpyridinium iodide (1.88 g) were added n-tributylamine (1.75 ml) and myristic acid (1.47 g), and the mixture was heated under reflux and stirred for 2 hours. The reaction mixture was washed sequentially with a 5% aqueous sodium thiosulfate solution and brine, and then concentrated. Purification on a silica gel column (Wako Gel C-200, 100 g) eluting with chloroform-acetone (200:1) afforded an amide (compound B8) in an amount of 2.41 g (yield, 75.6%).

MS: FDMS 691.
NMR: $^1H$ (500 MHz, $CDCl_3$; 27°C)

δ (ppm)

7.26-7.32 (10H, m), 5.64-5.73 (2H, m), 5.33-5.41 (1H, m), 4.19-4.59 (6H, m), 3.79-3.89 (1H, m), 3.51-3.58 (1H, m), 1.98-2.13 (2H, m), 1.26-1.58 (46H, m), 0.88 (6H, t, J=6.7 Hz).

(iii) Synthesis of the compound B9

To the amide (compound B8, 3.50 g) were added 1-propanol (15 ml), tetrahydrofuran (15 ml), 10% palladium on charcoal (1.2 g) and formic acid (3.0 ml). The mixture was stirred at 45°C for 16 hours under the nitrogen atmosphere. The catalyst was removed by filtration, and the filtrate was concentrated. Crystallization of the residue from chloroform-acetone afforded a ceramide (compound B9) in an amount of 2.08 g (yield, 80.4%).
$[\alpha]^{24}_D$ = +3.5° (pyridine, c = 1.87)

MS: FDMS 513.
mp: 104-105°C
NMR: $^1H$ (500 MHz, $C_5D_5N$; 27°C)

δ (ppm)

8.35 (1H, d, J=9.2 Hz), 6.36 (1H, t, J=4.9 Hz), 6.24 (1H, d, J=6.1 Hz), 4.62-4.67 (1H, m), 4.46 (1H, dt, J=4.9, 11.0 Hz), 4.25-4.33 (2H, m), 2.47 (2H, dt, J=1.8, 7.3 Hz), 1.25-1.95 (50H, m), 0.88 (6H, t, J=6.7 Hz).

(iv) Synthesis of the compound B10

To a solution of the ceramide (compound B9, 1.0 g) in tetrahydrofuran (30 ml) were added stannous chloride (1.29 g), silver perchlorate (1.41 g) and powdery Molecular Sieves 4A (1.5 g), and the mixture was stirred for 30 minutes. The mixture was cooled to -10°C, and a solution of benzylgalactosyl fluoride (compound A13, 1.11 g) in tetrahydrofuran (10 ml) was added. The resulting mixture was allowed to warm gradually to room temperature, stirred for 2 hours, and

then filtered through celite. The solids removed were washed with a small amount of acetone, and the extract was combined with the filtrate, and then concentrated and purified on a silica gel column (Wako Gel C-200, 150 g, hexane-ethyl acetate (3:1)) to give an α-galactoside (compound B10) in an amount of 646 mg (yield, 32.0%).

MS: FDMS 1035.
NMR: $^1H$ (500 MHz, $CDCl_3$; 27°C)

δ (ppm)
7.23-7.37 (20H, m), 6.49 (1H, d, J=7.9 Hz), 4.92 (1H, d, J=11.3 Hz), 4.84 (1H, d, J=12.2 Hz), 4.73-4.78 (3H, m), 4.67 (1H, d, J=11.6 Hz), 4.46 (1H, d, J=11.6 Hz), 4.37 (1H, d, J=11.6 Hz), 4.03 (1H, dd, J=3.7, 9.8 Hz), 3.96 (1H, bs), 3.83-3.92 (4H, m), 3.70 (1H, dd, J=3.1, 10.4 Hz), 3.47-3.58 (3H, m), 3.40 (1H, d, J=9.8 Hz), 2.12 (2H, dt, J=1.8, 7.9 Hz), 1.25-1.61 (51H, m), 0.88 (6H, t, J=6.7 Hz).

(v) Synthesis of the compound 5

To a solution of the galactoside (compound B10, 1.59 g) in tetrahydrofuran (30 ml) was added palladium black (290 mg). After the reaction vessel was purged with hydrogen, the mixture was stirred at room temperature for 16 hours. The catalyst was removed by filtration, and the filtrate was concentrated. Purification on a silica gel column (Wako Gel C-200, 100 g) eluting with chloroform-methanol (5:1) afforded the compound 5 in an amount of 984 mg (yield, 95.0 %).
Data of the compound 5
$[\alpha]^{24}_D$ = +57.8° (pyridine, c = 1.69)

MS: FDMS 674.
IR: ($cm^{-1}$, Kbr)
3400, 3270, 2920, 2850, 1640, 1550, 1465, 1135, 1075, 1045.
mp: 159.0-161.0°C
NMR: $^1H$ (500 MHz, $C_5D_5N$; 27°C)

δ (ppm)
8.52 (1H, d, J=8.6 Hz), 6.51 (1H, m), 6.44 (1H, m), 6.33 (1H, m), 6.15 (1H, m), 5.45 (1H, d, J=3.7 Hz), 4.73 (1H, m), 4.65 (1H, m), 4.40-4.58 (6H, m), 4.36 (1H, dd, J=5.5, 10.0 Hz), 4.28 (1H, m), 2.48 (2H, t, J=7.0 Hz), 1.80-1.95 (4H, m), 1.57 (1H, m), 1.18-1.43 (49H, m), 0.88 (6H, t, J=6.7 Hz).
$^{13}C$ (125 MHz, $C_5D_5N$; 27°C)
δ (ppm)
173.4 (s), 102.2 (d), 73.1 (d), 71.9 (d), 71.7 (d), 71.0 (d), 70.5 (d), 69.7 (t), 62.7 (t), 54.9 (d), 36.8 (t), 35.1 (t), 32.1 (t), 30.2 (t), 30.1 (t), 30.02 (t), 29.97 (t), 29.91 (t), 29.87 (t), 29.8 (t), 29.7 (t), 29.6 (t), 26.6 (t), 26.4 (t), 22.9 (t), 14.3 (q).

(3) Synthetic route C

A specific synthetic route with use of a sphingosine can be illustrated by the following scheme. While the reaction route scheme is illustrated specifically with reference to the aforementioned compounds 1 and 5, the compounds (2-4, 6-8, 14) according to the present invention can also be synthesized by applying this method. Furthermore, the compounds 15 and 35 having a double bond can be synthesized by conducting the deprotection with use of liquid ammonia and metallic sodium.

[Synthesis of the compound 1 (Fig. 8)]

Abbreviations in the aforementioned scheme are the same as those in the previously described schemes.

(i) Synthesis of the compound C2

To a solution of sphingosine (25 mg) in tetrahydrofuran (1 ml) were added p-nitrophenyl tetracosanate (81.8 mg) and 4-dimethylaminopyridine (2.5 mg), and the mixture was stirred at 40°C for 12 hours and directly concentrated. Purification on a silica gel column (Wako Gel C-200, 10 g) eluting with chloroform-methanol (4:1) afforded an amide (compound C2) in an amount of 23.2 mg (yield, 42.7%).
Data of the compound C2
$[\alpha]^{23}_D$ = -11.3° (pyridine, c = 1.03)

MS: FDMS 651.
IR: ($cm^{-1}$, Kbr)
3280, 2910, 2840, 1635, 1540, 1465.
mp: 87.5-89.5°C
NMR: $^1H$ (500 MHz, $CDCl_3+CD_3OD$ (ldrop); 27°C)

δ (ppm)

5.76 (1H, dt, J=6.7, 15.3 Hz), 5.49 (1H, dd, J=6.7, 15.3 Hz), 4.24 (1H, bs), 3.82-3.91 (2H, m), 3.67 (1H, m), 2.21 (2H, t, J=7.6 Hz), 1.9-2.1 (2H, m), 1.62 (2H, m), 1.2-1.4 (62H, m), 0.88 (6H, t, J=6.7 Hz).

(ii) Synthesis of the compound C3

To a solution of the amide (compound C2, 33.8 mg) in tetrahydrofuran (1.5 ml) were added stannous chloride (33 mg), silver perchlorate (36 mg) and powdered Molecular Sieves 4A (140 mg), and the mixture was stirred for 30 minutes. The mixture was next cooled to -10°C, a solution of benzylgalactosyl fluoride (compound A13, 28 mg) in tetrahydrofuran (0.5 ml) was added to it. The resulting mixture was allowed to gradually warm to room temperature. After being stirred for 3 hours, the mixture was diluted with acetone, filtered through celite, and the filtrate was concentrated. Purification on a silica gel column (Wako Gel C-200, 10 g) eluting with hexane-ethyl acetate (3:1) afforded an α-galactoside (compound C3) in an amount of 19.7 mg (yield, 32.4%).
Data of the compound C3
$[\alpha]^{23}_D = +25.1°$ ($CHCl_3$, $\underline{c}$ = 0.47)

MS: FDMS 1173.
IR: ($cm^{-1}$, Kbr)
3210, 2920, 2850, 1640, 1590, 1545, 1495, 1465, 1450, 1335, 1290, 1110.
mp: 63.0-64.5°C
NMR: $^1H$ (500 MHz, $CDCl_3$; 27°C)

δ (ppm)

7.23-7.37 (20H, m), 6.40 (1H, d, J=7.9 Hz), 5.65 (1H, m), 5.42 (1H, dd, J=6.1, 15.3 Hz), 4.91, 4.85, 4.70, 4.55, 4.47 & 4.38 (each 1H, d, J=11.6 Hz), 4.75 (2H, s), 4.12 (1H, m), 3.95-4.06 (3H, m), 3.79-3.92 (3H, m), 3.4-3.71 (3H, m), 2.12 (2H, dt, J=3.4, 7.6 Hz), 1.90-2.01 (3H, m), 1.1-1.6 (63H, m), 0.88 (6H, t, J=6.7 Hz).

(iii) Synthesis of the compound 1

To a solution of the α-galactoside (compound C3, 9.7 mg) in tetrahydrofuran (1.0 ml) was added a 5% palladium on barium sulfate (5 mg). After the reaction vessel was purged with hydrogen, the mixture was stirred at room temperature for 16 hours, and then filtered through celite. The filtrate was concentrated and purified on a silica gel column (Wako Gel C-200, 10 g, chloroform-methanol (10:1)) to give the compound 1 in an amount of 3.0 mg (yield, 44.5 mg).
Data of the compound 1
$[\alpha]^{23}_D = +50.0°$ (pyridine, $\underline{c}$ = 0.26)

MS: FDMS 814.
IR: ($cm^{-1}$, Kbr)
3260, 2910, 2850, 1645, 1545, 1470, 1350, 1125, 1065.
mp: 184.5-186.5°C
NMR: $^1H$ (500 MHz, $C_5D_5N$; 27°C)

δ (ppm)

8.52 (1H, d, J=8.6 Hz), 5.46 (1H, d, J=3.7 Hz), 4.74 (1H, m), 4.66 (1H, dd, J=3.6, 9.8 Hz), 4.54-4.60 (2H, m), 4.40-4.52 (4H, m), 4.37 (1H, dd, J=5.5, 10.4 Hz), 4.29 (1H, m), 2.48 (2H, t, J=7.3 Hz), 1.8-2.0 (4H, m), 1.58 (1H, m), 1.20-1.45 (65H, m), 0.881 & 0.877 (each 3H, t, J=7.3 Hz).
$^{13}C$ (125 MHz, $C_5D_5N$; 27°C)

δ (ppm)

173.4 (s), 102.2 (d), 73.1 (d), 71.9 (d), 71.7 (d), 71.0 (d), 70.5 (d), 69.7 (t), 62.7 (t), 54.9 (d), 36.8 (t), 35.1 (t), 32.1 (t), 30.2 (t), 30.1 (t), 30.0 (t), 29.9 (t), 29.83 (t), 29.76 (t), 29.6 (t), 26.6 (t), 26.4 (t), 22.9 (t), 14.3 (q).

[Synthesis of the compound 5 (Fig. 9)]

Abbreviations in the aforementioned scheme are the same as those in the previously described schemes.

(i) Synthesis of the compound C4

To a solution of sphingosine (75 mg) in tetrahydrofuran (1.5 ml) were added p-nitrophenyl myristate (175 mg) and 4-dimethylaminopyridine (7.6 mg), and the mixture was stirred at 46°C for 12 hours. The reaction mixture was concentrated directly and purified on a silica gel column (Wako Gel C-200, 10 g, hexane-acetone (3:1)) to give an amide (compound C4) in an amount of 112.6 mg (yield, 88.3%).
Data of the compound C4
$[\alpha]^{23}_D$ = -11.4° (pyridine, c = 0.58)

MS: FDMS 510.
IR: ($cm^{-1}$, Kbr)
3300, 2910, 2850, 1640, 1620, 1550, 1470, 1380, 1265, 1240, 1040.
mp: 96.5-98.0°C
NMR: $^1H$ (500 MHz, $C_5D_5N$; 27°C)

δ (ppm)
8.33 (1H, d, J=8.5 Hz), 6.7 (1H, m), 6.05 (1H, dd, J=6.4, 15.9 Hz), 5.96 (1H, dt, J=6.4, 15.9 Hz), 4.85 (1H, t, J=6.7 Hz), 4.75 (1H, m), 4.47 (1H, dd, J=4.9, 11.0 Hz), 4.30 (1H, dd, J=4.0, 10.7 Hz), 2.47 (2H, t, J=7.6 Hz), 2.10 (2H, m), 1.85 (2H, m), 1.39 (4H, m), 1.20-1.33 (38H, m), 0.88 (6H, t, J=6.7 Hz).
$^{13}C$ (125 MHz, $C_5D_5N$; 27°C)
δ (ppm)
173.5 (s), 132.4 (d), 132.3 (d), 73.3 (d), 62.2 (t), 56.9 (d), 36.9 (t), 32.7 (t), 32.1 (t), 29.99 (t), 29.96 (t), 29.93 (t), 29.87 (t), 29.8 (t), 29.7 (t), 29.61 (t), 29.55 (d), 26.4 (t), 22.9 (t), 14.3 (q).

(ii) Synthesis of the compound C5

To a solution of the amide (compound C4, 106.8 mg) in tetrahydrofuran (4.5 ml) was added a powdered Molecular Sieves 4A (400 mg), and the mixture was stirred for 10 minutes. Stannous chloride (133 mg) and silver perchlorate (146 mg) were added, and the mixture was further stirred for 30 minutes. The reaction mixture was cooled to -10°C, and a solution of benzylgalactosyl fluoride (compound A13, 113 mg) in tetrahydrofuran (1.5 ml) was added thereto. After 30 minutes, it was allowed to warm to room temperature, stirred for 30 minutes, and then diluted with chloroform-methanol (1:1), filtered through celite, and the filtrate was concentrated. Purification of the residue on a silica gel column (Wako Gel C-200, 15 g) eluting with hexane-ethyl acetate (5:2) afforded an α-galactoside (compound C5) in an amount of 76.0 mg (yield, 35.2%).
Data of the compound C5
$[\alpha]^{24}_D$ = +32.7° ($CHCl_3$, c = 2.26)

MS: FDMS 1033.
IR: ($cm^{-1}$, Kbr)
3320, 2920, 2850, 1640, 1615, 1545, 1465, 1450, 1350, 1105, 1045.
mp: 66.0-68.0°C
NMR: $^1H$ (500 MHz, $CDCl_3$; 27°C)

δ (ppm)
7.25-7.37 (20H, m), 6.40 (1H, d, J=7.9 Hz), 5.66 (1H, dt, J=7.9, 15.3 Hz), 5.42 (1H, dd, J=5.5, 15.3 Hz), 4.91, 4.85, 4.70, 4.55, 4.47 & 4.38 (each 1H, d, J=11.6 Hz), 4.752 (2H, s), 4.747 (1H, d, J=4.9 Hz), 4.13 (1H, m), 4.03 (1H, dd, J=3.7, 10.4 Hz), 3.95-4.01 (2H, m), 3.79-3.89 (4H, m), 3.69 (1H, dd, J=3.7, 10.3 Hz), 3.45-3.55 (2H, m), 2.12 (2H, dt, J=3.7, 7.9 Hz), 1.99 (2H, m), 1.58 (2H, m), 1.2-1.4 (42H, m), 0.88 (6H, t, J=7.0 Hz).
$^{13}C$ (125 MHz, $CDCl_3$; 27°C)
δ (ppm)
173.3 (s), 138.5 (s), 138.4 (s), 138.0 (s), 137.6 (s), 133.0 (d), 129.2 (d), 128.44 (d), 128.41 (d), 128.3 (d), 128.13 (d), 128.10 (d), 127.90 (d), 127.86 (d), 127.6 (d), 127.4 (d), 126.1 (d), 99.1 (d), 79.2 (d), 75.9 (d), 74.8 (t), 74.4 (d), 74.2 (t), 74.0 (d), 73.6 (t), 72.7 (t), 69.8 (d), 69.0 (t), 68.7 (t), 52.8 (d), 36.7 (t), 32.3 (t), 31.9 (t), 29.68 (t), 29.65 (t), 29.5 (t), 29.41 (t), 29.36 (t), 29.32 (t), 29.26 (t), 25.8 (t), 22.7 (t), 14.1 (q).

(iii) Synthesis of the compound 5

To a solution of the galactoside (compound C5, 7.3 mg) in tetrahydrofuran (2.0 ml) was added palladium black (1.5 mg). After the reaction vessel was purged with hydrogen, the mixture was stirred at room temperature for 16 hours, and then filtered through celite. The filtrate was concentrated. Purification on a silica gel column (Wako Gel C-200, 2 g) eluting with chloroform-methanol (8:1) afforded the compound 5 in an amount of 4.4 mg (yield, 90.9%).
Data of the compound 5 was the same as those described above.
The compounds other than those described above (1-14) were synthesized by using appropriate carboxylic acids or combining Wittig's salts having alkyl groups of a variety of lengths in accordance with the synthetic methods of the compounds (9, 7, 5, 1) (synthetic routes A-C). The compounds 15, 35 and 29 had double bonds unreduced by conducting the reduction at the final stage with liquid ammonia and metallic sodium. Examples of the synthesis of these compounds are illustrated below.

Compound 2

The compound 2 was obtained by reacting the sphingosine C1 with p-nitrophenyl docosanoate in place of p-nitrophenyl tetracosanoate in the synthesis of the compound 1 and conducting synthesis by applying the route C.
As an alternative method, the compound 2 was obtained by reacting the amine B4 with p-nitrophenyl docosanoate in place of p-nitrophenyl octanoate in the synthesis of the compound 7 and conducting synthesis by applying the route B.
[Data]
$[\alpha]^{25}_{D}$ = +50.7° (pyridine, c = 0.82)

MS: FDMS 787.
IR: ($cm^{-1}$, Kbr)
3390, 3220, 2870, 2810, 1635, 1535, 1455, 1080, 1055.
mp: 147.0-149.5°C
NMR: $^{1}H$ (500 MHz, $C_5D_5N$; 27°C)

δ (ppm)
8.53 (1H, d, J=8.6 Hz), 5.46 (1H, d, J=3.1 Hz), 4.74 (1H, m), 4.66 (1H, m), 4.4-4.6 (6H, m), 4.37 (1H, dd, J=5.8, 10.1 Hz), 4.29 (1H, m), 2.48 (2H, t, J=7.3 Hz), 1.80-1.97 (4H, m), 1.58 (1H, m), 1.20-1.45 (61H, m), 0.880 & 0.876 (each 3H, t, J=7.3 Hz).
$^{13}C$ (125 MHz, $C_5D_5N$; 27°C)
δ (ppm)
173.4 (s), 102.2 (d), 73.1 (d), 72.0 (d), 71.7 (d), 71.0 (d), 70.6 (d), 69.7 (t), 62.7 (t), 54.9 (d), 36.8 (t), 35.1 (t), 32.1 (t), 30.2 (t), 30.1 (t), 30.0 (t), 29.95 (t), 29.92 (t), 29.83 (t), 29.76 (t), 29.62 (t), 29.61 (t), 26.6 (t), 26.4 (t), 22.9 (t), 14.3 (q).

Compound 3

The compound 3 was obtained by reacting the sphingosine C1 with p-nitrophenyl icosanoate in place of p-nitrophenyl tetracosanoate in the synthesis of the compound 1 and conducting synthesis by applying the route C.
As an alternative method, the compound 3 was obtained by reacting the amine B4 with p-nitrophenyl icosanoate in place of p-nitrophenyl octanoate in the synthesis of the compound 7 and conducting further synthesis by applying the route B.
[Data]
$[\alpha]^{25}_{D}$ = +47.3° (pyridine, c = 1.76)

MS: FDMS 759.
IR: ($cm^{-1}$, Kbr)
3390, 3220, 2880, 2810, 1635, 1530, 1455, 1080, 1055.
mp: 151.5-153.0°C
NMR: $^{1}H$ (500 MHz, $C_5D_5N$; 27°C)

δ (ppm)
8.52 (1H, d, J=8.6 Hz), 5.46 (1H, d, J=4.3 Hz), 4.73 (1H, m), 4.66 (1H, dd, J=4.5, 10.1 Hz), 4.4-4.6 (6H, m), 4.37 (1H, dd, J=5.5, 10.4 Hz), 4.29 (1H, m), 2.48 (2H, t, J=7.3 Hz), 1.80-1.97 (4H, m), 1.58 (1H, m), 1.20-1.42 (57H, m), 0.879 & 0.876 (each 3H, t, J=7.3 Hz).

$^{13}C$ (125 MHz, $C_5D_5N$; 27°C)
δ (ppm)
173.4 (s), 102.1 (d), 73.1 (d), 71.9 (d), 71.6 (d), 71.0 (d), 70.5 (d), 69.7 (t), 62.7 (t), 54.9 (d), 36.8 (t), 35.1 (t), 32.1 (t), 30.2 (t), 30.1 (t), 30.0 (t), 29.9 (t), 29.8 (t), 29.7 (t), 29.6 (t), 26.6 (t), 26.4 (t), 22.9 (t), 14.3 (q).

Compound 4

The compound 4 was obtained by reacting the sphingosine C1 with p-nitrophenyl stearate in place of p-nitrophenyl tetracosanoate in the synthesis of the compound 1 and conducting further synthesis by applying the route C.
As an alternative method, the compound 4 was obtained by reacting the amine B4 with p-nitrophenyl stearate in place of p-nitrophenyl octanoate in the synthesis of the compound 7 and conducting further synthesis by applying the route B.
[Data]
$[\alpha]^{25}_D$ = +55.5° (pyridine, c = 0.84)

MS: FDMS 731.
IR: ($cm^{-1}$, Kbr)
3230, 2940, 2830, 1640, 1540, 1465, 1345, 1120, 1090, 1060.
mp: 157.5-159.5°C
NMR: $^1H$ (500 MHz, $C_5D_5N$; 27°C)

δ (ppm)
8.52 (1H, d, J=8.6 Hz), 5.46 (1H, d, J=3,7 Hz), 4.73 (1H, m), 4.66 (1H, dd, J=3.7, 9.8 Hz), 4.57 (1H, d, J=2.5 Hz), 4.55 (1H, t, J=6.1 Hz), 4.40-4.51 (4H, m), 4.37 (1H, dd, J=5.8, 10.7 Hz), 4.29 (1H, m), 2.48 (2H, t, J=7.3 Hz), 1.80-1.96 (4H, m), 1.59 (1H, m), 1.2-1.44 (53H, m), 0.88 (6H, t, J=6.7 Hz).
$^{13}C$ (125 MHz, $C_5D_5N$; 27°C)
δ (ppm)
173.4 (s), 102.1 (d), 73.1 (d), 71.9 (d), 71.7 (d), 71.0 (d), 70.5 (d), 69.7 (t), 62.7 (t), 54.9 (d), 36.8 (t), 35.1 (t), 32.1 (t), 30.2 (t), 30.1 (t), 30.0 (t), 29.9 (t), 29.8 (t), 29.7 (t), 29.6 (t), 26.6 (t), 26.4 (t), 22.9 (t), 22.8 (t), 14.3 (q).

Compound 6

The compound 6 was obtained by reacting the sphingosine C1 with p-nitrophenyl decanoate in place of p-nitrophenyl tetracosanoate in the synthesis of the compound 1 and conducting further synthesis by applying the route C.
As an alternative method, the compound 6 was obtained by reacting the amine B4 with p-nitrophenyl decanoate in place of p-nitrophenyl octanoate in the synthesis of the compound 7 and conducting further synthesis by applying the route B.
[Data]
$[\alpha]^{25}_D$ = +54.8° (pyridine, c = 0.93)

MS: FDMS 619.
IR: ($cm^{-1}$, Kbr)
3245, 2900, 2840, 1635, 1540, 1460, 1345, 1120, 1090, 1060.
mp: 151.0-154.0°C
NMR: $^1H$ (500 MHz, $C_5D_5N$; 27°C)

δ (ppm)
8.52 (1H, d, J=9.2 Hz), 6.14 (1H, m), 5.45 (1H, d, J=3.7 Hz), 4.74 (1H, m), 4.65 (1H, dd, J=4.0, 10.1 Hz), 4.57 (1H, d, J=3.4 Hz), 4.54 (1H, t, J=5.8 Hz), 4.40-4.50 (4H, m), 4.36 (1H, dd, J=5.5, 11.0 Hz), 4.28 (1H, m), 2.47 (2H, dt, J=1.5, 7.6 Hz), 1.80-1.95 (4H, m), 1.57 (1H, m), 1.15-1.40 (37H, m), 0.87 & 0.85 (each 3H, t, J=6.7 Hz).
$^{13}C$ (125 MHz, $C_5D_5N$; 27°C)
δ (ppm)
173.4 (s), 102.1 (d), 73.1 (d), 71.9 (d), 71.6 (d), 71.0 (d), 70.5 (d), 69.7 (t), 62.7 (t), 54.9 (d), 36.8 (t), 35.1 (t), 32.12 (t), 32.05 (t), 30.2 (t), 30.1 (t), 30.0 (t), 29.9 (t), 29.8 (t), 29.7 (t), 29.61 (t), 29.55 (t), 26.6 (t), 26.4 (t), 22.93 (t), 22.90 (t), 14.3 (q).

Compound 8

The compound 8 was obtained by reacting the sphingosine C1 with acetic anhydride in place of p-nitrophenyl tetracosanoate in the synthesis of the compound 1 and conducting further synthesis by applying the route C.

As an alternative method, the compound 8 was obtained by reacting the amine B4 with acetic anhydride in place of p-nitrophenyl octanoate in the synthesis of the compound 7 and conducting further synthesis by applying the route B.

[Data]

$[\alpha]^{25}_D$ = +74.3° (pyridine, c = 1.36)

MS: FDMS 507.

IR: ($cm^{-1}$, Kbr)

3230, 2890, 2830, 1630, 1540, 1465, 1370, 1140.

mp: 171.0-172.0°C

NMR: $^1H$ (500 MHz, $C_5D_5N$; 27°C)

δ (ppm)

8.63 (1H, d, J=8.6 Hz), 6.1 (2H, m), 5.43 (1H, d, J=3.7 Hz), 4.70 (1H, m), 4.64 (1H, dd, J=4.0, 10.1 Hz), 4.55 (1H, d, J=2.4 Hz), 4.52 (1H, t, J=6.1 Hz), 4.46 (1H, dd, J=3.7, 10.4 Hz), 4.38-4.44 (3H, m), 4.31 (1H, dd, J=6.1, 10.4 Hz), 4.26 (1H, m), 2.13 (3H, s), 1.77-1.90 (3H, m), 1.55 (1H, m), 1.20-1.40 (24H, m), 0.87 (3H, t, J=7.0 Hz).

$^{13}C$ (125 MHz, $C_5D_5N$; 27°C)

δ (ppm)

170.3 (s), 102.0 (d), 73.0 (d), 71.9 (d), 71.6 (d), 70.9 (d), 70.5 (d), 69.4 (t), 62.6 (t), 55.0 (d), 35.0 (t), 32.1 (t), 30.1 (t), 30.04 (t), 29.97 (t), 29.9 (t), 29.6 (t), 26.6 (t), 23.3 (q), 22.9 (t), 14.3 (q).

Compound 10

In the synthesis of the compound 7, the aldehyde A2 was reacted with dodecanetriphenylphosphonium bromide in place of tetradecanetriphenylphosphonium bromide. Next, the amine obtained in the reduction was reacted with p-nitrophenyl myristate in place of p-nitrophenyl octanoate, and synthesis was further conducted by applying the route B to give the compound 10.

[Data]

$[\alpha]^{24}_D$ = +74.3° (pyridine, c = 0.35)

MS: FDMS 646.

IR: ($cm^{-1}$, Kbr)

3250, 2900, 2830, 1640, 1540, 1460, 1120, 1085, 1060.

mp: 153.5-156.0°C

NMR: $^1H$ (500 MHz, $C_5D_5N$; 27°C)

δ (ppm)

8.52 (1H, d, J=8.6 Hz), 6.1 (1H, m), 5.47 (1H, d, J=3.7 Hz), 4.75 (1H, m), 4.67 (1H, dd, J=3.7, 9.8 Hz), 4.34-4.60 (7H, m), 4.29 (1H, m), 2.48 (2H, dt, J=1.2, 7.3 Hz), 1.80-1.95 (4H, m), 1.58 (1H, m), 1.20-1.42 (41H, m), 0.87 (6H, t, J=6.8 Hz).

$^{13}C$ (125 MHz, $C_5D_5N$; 27°C)

δ (ppm)

173,4 (s), 102.1 (d), 73.1 (d), 72.0 (d), 71.7 (d), 71.0 (d), 70.6 (d), 69.7 (t), 62.7 (t), 54.9 (d), 36.8 (t), 35.1 (t), 32.1 (t), 30.2 (t), 30.1 (t), 30.00 (t), 29.97 (t), 29.9 (t), 29.8 (t), 29.7 (t), 29.6 (t), 26.6 (t), 26.4 (t), 22.9 (t), 14.3 (q).

Compound 11

In the synthesis of the compound 10, the (2S,3S)-aldehyde was used in place of the aldehyde A2, and the synthesis was conducted by applying the route B to give the compound 11.

[Data]

$[\alpha]^{24}_D$ = +62.0° (pyridine, c = 0.50)

MS: FDMS 646.

IR: ($cm^{-1}$, Kbr)

3290, 2910, 2840, 1640, 1615, 1540, 1465, 1140, 1050.
mp: 145.0-147.0°C
NMR: $^1H$ (500 MHz, $C_5D_5N$; 27°C)

δ (ppm)
8.40 (1H, d, J=8.5 Hz), 6.28 (1H, m), 5.47 (1H, d, J=3.7 Hz), 4.66-4.76 (3H, m), 4.10-4.62 (7H, m), 2.48 (2H, dt, J=1.8, 7.3 Hz), 1.80-2.00 (3H, m), 1.70 (1H, m), 1.57 (1H, m), 1.20-1.42 (41H, m), 0.88 (6H, t, J=6.7 Hz).

Compound 12

In the synthesis of the compound 10, the (2S,3R)-aldehyde was used in place of the aldehyde A2, and the synthesis was conducted by applying the route B to give the compound 12.
[Data]
$[\alpha]^{23}_D$ = +52.5° (pyridine, c = 0.75)

MS: FDMS 646.
IR: ($cm^{-1}$, Kbr)
3480, 3240, 2910, 2840, 1630, 1560, 1460, 1070, 1005.
mp: 148.5-152.5°C
NMR: $^1H$ (500 MHz, $C_5D_5N$; 27°C)

δ (ppm)
8.10 (1H, d, J=8.6 Hz), 5.46 (1H, d, J=3.7 Hz), 4.79 (1H, m), 4.66 (1H, dd, J=3.7, 9.8 Hz), 4.34-4.56 (7H, m), 4.12 (1H, t, J=6.1 Hz), 4.07 (1H, dd, J=6.1, 9.8 Hz), 2.49 (2H, t, J=6.5 Hz), 1.75-1.92 (3H, m), 1.69 (1H, m), 1.55 (1H, m), 1.20-1.42 (41H, m), 0.88 (6H, t, J=6.7 Hz).
$^{13}C$ (125 MHz, $C_5D_5N$; 27°C)
δ (ppm)
173.6 (s), 101.4 (d), 73.0 (d), 71.8 (d), 71.1 (d), 70.6 (d), 70.4 (d), 69.8 (t), 62.8 (t), 53.1 (d), 36.8 (t), 35.3 (t), 32.1 (t), 30.2 (t), 30.0 (t), 29.93 (t), 29.89 (t), 29.8 (t), 29.7 (t), 29.6 (t), 26.6 (t), 26.5 (t), 22.9 (t), 14.3 (q).

Compound 13

In the synthesis of the compound 10, the (2R,3S)-aldehyde was used in place of the aldehyde A2, and the synthesis was conducted by applying the route B to give the compound 13.
[Data]
$[\alpha]^{24}_D$ = +80.7° (pyridine, c = 0.27)

MS: FDMS 646.
IR: ($cm^{-1}$, Kbr)
3300, 2900, 2820, 1635, 1520, 1460, 1065, 1005.
mp: 149.0-150.5°C
NMR: $^1H$ (500 MHz, $C_5D_5N$; 27°C)

δ (ppm)
8.04 (1H, d, J=8.6 Hz), 6.4 (1H, m), 5.49 (1H, d, J=3.7 Hz), 4.80 (1H, m), 4.68 (1H, dd, J=3.7, 9.8 Hz), 4.65 (1H, bd, J=2.4 Hz), 4.36-4.58 (6H, m), 4.16 (1H, dd, J=6.7, 10.4 Hz), 2.50 (2H, t, J=7.3 Hz), 1.75-1.92 (3H, m), 1.69 (1H, m), 1.53 (1H, m), 1.20-1.42 (41H, m), 0.88 (6H, t, J=7.0 Hz).

Compound 14

The compound 14 was obtained by reacting the sphingosine C1 with p-nitrophenyl (R)-2-acetoxytetracosanoate in place of p-nitrophenyl tetracosanoate in the synthesis of the compound 1 and further conducting the synthesis by applying the route C.
As an alternative method, the compound 14 was obtained by reacting the amine B4 with p-nitrophenyl (R)-2-acetoxytetracosanoate in place of p-nitrophenyl octanoate in the synthesis of the compound 7 and conducting further synthesis by applying the route B.
[Data]

MS: FDMS 831.
NMR: $^1H$ (500 MHz, $C_5D_5N$; 27°C)

δ (ppm)
8.45 (1H, d, J=9.2 Hz), 5.44 (1H, d, J=3.7 Hz), 4.71 (1H, m), 4.64 (2H, m), 4.53 (3H, m), 4.40 (3H, m), 4.25 (1H, m), 2.22 (1H, m), 2.09 (1H, m), 1.70-1.95 (4H, m), 1.54 (1H, m), 1.2-1.45 (63H, m), 0.884 & 0.876 (each 3H, t, J=6.7 Hz).
$^{13}C$ (125 MHz, $C_5D_5N$; 27°C)
δ (ppm)
175.1 (s), 101.9 (d), 73.2 (d), 72.4 (d), 71.7 (d), 71.0 (d), 70.5 (d), 69.4 (t), 62.7 (t), 54.1 (d), 35.6 (t), 35.2 (t), 32.1 (t), 30.3 (t), 30.04 (t), 29.97 (t), 29.9 (t), 29.64 (t), 29.61 (t), 26.5 (t), 25.8 (t), 22.9 (t), 14.3 (q).

Compound 15

The compound 15 was obtained by reacting the sphingosine C1 with p-nitrophenyl stearate in place of p-nitrophenyl tetracosanoate in the synthesis of the compound 1 and further conducting the synthesis by applying the route C. The compound 15 as the deprotected derivative was obtained by conducting the deprotection in the final step by wetting the raw material with a small amount of tetrahydrofuran and adding thereto liquid ammonia and next metallic sodium.
[Data]
$[\alpha]^{25}_D$ = +41.4° (pyridine, c = 0.14)

MS: FDMS 729.
IR: ($cm^{-1}$, Kbr)
3230, 2880, 2810, 1630, 1535, 1460, 1375, 1065, 1040.
mp: 169.0-172.0°C
NMR: $^1H$ (500 MHz, $C_5D_5N$; 27°C)

δ (ppm)
8.50 (1H, d, J=8.6 Hz), 6.01 (2H, bs), 5.47 (1H, d, J=3.7 Hz), 4.86 (2H, m), 4.67 (1H, dd, J=4.0, 10.1 Hz), 4.59 (1H, d, J=2.4 Hz), 4.54 (1H, t, J=5.8 Hz), 4.40-4.50 (5H, m), 4.37 (1H, m), 2.46 (2H, dt, J=3.1, 7.6 Hz), 2.09 (2H, bs), 1.84 (2H, m), 1.15-1.45 (50H, m), 0.88 (6H, t, J=6.4 Hz).

Compound 29

The synthesis was conducted by reacting the amine A7 with oleic acid in place of tetracosanoic acid in the synthesis of the compound 9 and further continuing the synthesis by applying the route C. The compound 29 as the deprotected derivative was obtained by conducting the deprotection in the final step by wetting the raw material with a small amount of tetrahydrofuran and then adding thereto liquid ammonia and metallic sodium.
[Data]
$[\alpha]^{24}_D$ = +46.6° (pyridine, c = 0.17)

MS: FDMS 728.
IR: ($cm^{-1}$, Kbr)
3400, 2900, 2820, 1640, 1540, 1460, 1060.
mp: 134-136°C
NMR: $^1H$ (500 MHz, $C_5D_5N$; 27°C)

δ (ppm)
8.52 (1H, d, J=8.6 Hz), 6.54 (1H, bs), 6.45 (1H, bs), 6.35 (1H, bs), 6.15 (1H, bs), 5.44 (3H, m), 4.73 (1H, m), 4.66 (1H, dd, J=3.7, 9.8 Hz), 4.33-4.58 (7H, m), 4.27 (1H, m), 2.45 (2H, m), 2.06 (3H, m), 1.75-1.92 (2H, m), 1.55 (1H, m), 1.14-1.42 (48H, m), 0.84 (6H, m).
$^{13}C$ (125 MHz, $C_5D_5N$; 27°C)
δ (ppm)
173.3 (s), 130.1 (d), 130.1 (d), 102.0 (d), 73.0 (d), 71.8 (d), 71.6 (d), 70.9 (d), 70.4 (d), 69.6 (t), 62.6 (t), 54.9 (d), 36.7 (t), 35.0 (t), 32.0 (t), 32.0 (t), 30.1 (t), 30.0 (t), 29.9 (t), 29.8 (t), 29.7 (t), 29.6 (t), 29.6 (t), 29.5 (t), 29.5 (t), 29.4 (t), 27.4 (t), 26.5 (t), 26.3 (t), 22.9 (t), 14.2 (q).

Compound 35

The synthesis was conducted by reacting the sphingosine C1 with p-nitrophenyl myristate in place of p-nitrophenyl tetracosanoate in the synthesis of the compound 1 and further by applying the route C. The compound 29 as the deprotected derivative was obtained by conducting the deprotection in the final step by wetting the raw material with a small amount of tetrahydrofuran and then adding thereto liquid ammonia and metallic sodium.
[Data]
$[\alpha]^{24}_D$ = +48.9° (pyridine, c = 0.45)

MS: FDMS 673.
IR: ($cm^{-1}$, Kbr)
3320, 2920, 2855, 1640, 1545, 1470, 1345, 1150.
mp: 158.0-160.0°C
NMR: $^1H$ (500 MHz, $C_5D_5N$; 27°C)

δ (ppm)
8.46 (1H, d, J=7.3 Hz), 6.59 (1H, m), 6.41 (1H, m), 6.33 (1H, m), 6.00 (2H, bs), 5.46 (1H, d, J=3.7 Hz), 4.85 (2H, m), 4.65 (1H, dd, J=3.7, 9.8 Hz), 4.58 (1H, m), 4.53 (1H, t, J=6.1 Hz), 4.40-4.50 (4H, m), 4.35 (1H, dd, J=5.2, 10.1 Hz), 2.45 (2H, dt, J=3.1, 7.3 Hz), 2.08 (2H, m), 1.84 (2H, m), 1.37 (4H, m), 1.20-1.32 (38H, m), 0.88 (6H, t, J=6.7 Hz).
$^{13}C$ (125 MHz, $C_5D_5N$; 27°C)
δ (ppm)
173.5 (s), 132.4 (d), 132.0 (d), 102.1 (d), 73.0 (d), 71.7 (d), 70.9 (d), 70.6 (d), 69.4 (t), 62.7 (t), 55.1 (d), 36.8 (t), 32.7 (t), 32.1 (t), 30.01 (t), 29.99 (t), 29.96 (t), 29.63 (t), 29.87 (t), 29.83 (t), 29.76 (t), 29.73 (t), 29.6 (t), 26.4 (t), 22.9 (t), 14.3 (q).

(4) Synthetic route D

The specific method for synthesizing a compound having a hydroxyl group at C-4 of the long chain base in formula (A) can be illustrated by the following reaction route scheme. Although the reaction route scheme specifically illustrates the method with reference to the compound 22, the compounds according to the present invention including 16-34 except for 22 and 29 can also be synthesized by applying the method (synthesis of the compound 22 (Figs. 10a-10c)).
In the aforementioned scheme, the following abbreviations are used:
EEDQ: 2-ethoxy-1-ethoxycarbonyl-1,2-dihydroquinoline.
The other abbreviations are the same as those in the previous reaction schemes.

(i) Synthesis of the compound D1

The compound D1 can be synthesized by applying the method described in Agricultural and Biological Chemistry, 54 (3), 663-667, 1990.

(ii) Synthesis of the compound D3

To the Wittig's salt (compound D2, 32.07 g) was added tetrahydrofuran (40 ml), and the reaction vessel was purged with argon. A 2N solution of n-butyl lithium in hexane (30 ml) was added, and the mixture was stirred for 15 minutes. A solution of the aldehyde (compound D1, 13.18 g) in tetrahydrofuran (20 ml) was dropwise added to the mixture, which was then allowed to warm to room temperature and stirred for 15 hours. To the reaction mixture were added methanol (3 ml) followed by 20% aqueous methanol (300 ml), and the mixture was extracted thrice with n-hexane. The extracts were washed with brine and concentrated. Purification on a silica gel column (Wako Gel C-200, 400 g) eluting with hexane-ethyl acetate (9:1) afforded an alcohol (compound D3) in an amount of 9.31 g (yield, 51.9%).
Data of the compound D3
$[\alpha]^{24}_D$ = -38.2° ($CHCl_3$, c = 1.0)

MS: FDMS 573, 301.
NMR: $^1H$ (500 MHz, $CDCl_3$; 27°C)

δ (ppm)
7.20-7.35 (15H, m), 5.72 (1H, m), 5.46 (1H, bt, J=9.2 Hz), 4.68 (1H, d, J=11.2 Hz), 4.60 (1H, d, J=11.7 Hz),

4.47-4.52 (3H, m), 4.44 (1H, dd, J=5.5, 9.8 Hz), 4.33 (1H, d, J=11.7 Hz), 4.08 (1H, m), 3.56 (1H, dd, J=2.4, 5.5 Hz), 3.51 (2H, d, J=6.1 Hz), 3.01 (1H, d, J=5.5 Hz), 1.85-2.01 (2H, m), 1.17-1.36 (18H, m), 0.88 (3H, t, J=6.7 Hz).

(iii) Synthesis of the compound D4

To a solution of the alcohol (compound D3, 9.31 g) in tetrahydrofuran (30 ml) was added 10% palladium on charcoal (0.53 g). After the reaction vessel was purged with hydrogen, and the mixture was stirred at room temperature for 15 hours, and then filtered through celite. The filtrate was concentrated to give a reduced product (compound D4) in an amount of 9.34 g (yield, quantitatively).
Data of the compound D4
$[\alpha]^{24}_{D}$ = -35.1° ($CHCl_3$, c = 0.5)

MS: FDMS 575.
NMR: $^{1}H$ (500 MHz, $CDCl_3$; 27°C)

δ (ppm)
7.22-7.34 (15H, m), 4.69 (1H, d, J=11.6 Hz), 4.65 (1H, d, J=11.6 Hz), 4.55 (1H, d, J=11.0 Hz), 4.52 (1H, d, J=11.6 Hz), 4.50 (1H, d, J=11.0 Hz), 4.48 (1H, d, J=12.2 Hz), 4.04 (1H, m), 3.68 (1H, m), 3.61 (1H, m), 3.54 (2H, m), 3.17 (1H, d, J=4.9 Hz), 1.85 (3H, m), 1.65 (2H, m), 1.56 (1H, m), 1.41 (1H, m), 1.16-1.35 (17H, m), 0.88 (3H, t, J=7.3 Hz).

(iv) Synthesis of the compound D5

To a solution of the reduced product (compound D4, 9.34 g) in pyridine (70 ml) was added methanesulfonyl chloride (2.5 ml), and the mixture was stirred at room temperature for 2 hours, and then concentrated. After the residual acid chloride was distilled azeotropically with toluene, the residue was taken into diethyl ether and washed with brine. The organic layer was concentrated and purified on a silica gel column (Wako Gel C-200, 500 g, hexane-ethyl acetate (9:1)) to give a mesyl derivative (compound D5) in an amount of 9.74 g (yield, 91.8%).
Data of the compound D5
$[\alpha]^{24}_{D}$ = +6.5° ($CHCl_3$, c = 1.0)

MS: FDMS 653.
NMR: $^{1}H$ (500 MHz, $CDCl_3$; 27°C)

δ (ppm)
7.25-7.38 (15H, m), 4.91 (1H, dt, J=3.9, 5.6 Hz), 4.76 (1H, d, J=11.2 Hz), 4.62 (1H, d, J=11.2 Hz), 4.58 (1H, d, J=11.5 Hz), 4.55 (1H, d, J=11.7 Hz), 4.48 (1H, d, J=11.2 Hz), 4.48 (1H, d, J=11.7 Hz), 3.89 (1H, t, J=4.9 Hz), 3.67-3.76 (2H, m), 3.61 (1H, m), 2.91 (3H, s), 1.72 (1H, m), 1.54 (1H, m), 1.41 (1H, m), 1.16-1.35 (21H, m), 0.88 (3H, t, J=7.3 Hz).

(v) Synthesis of the compound D6

To the solution of the mesyl derivative (compound D5, 9.74 g) in dimethylformamide (100 ml) was added sodium azide (9.70 g), and the mixture was stirred at 120°C for 16 hours, then concentrated, taken into ethyl acetate and washed with water and brine. The organic layer was concentrated and purified on a silica gel column (Wako Gel C-200, 200 g, hexane-ethyl acetate (98:2)) to give an azide derivative (compound D6) in an amount of 6.75 g (yield, 75.4%).
Data of the compound D6
$[\alpha]^{24}_{D}$ = +8.2° ($CHCl_3$, c = 1.0)

MS: FDMS 600, 573, 450.
NMR: $^{1}H$ (500 MHz, $CDCl_3$; 27°C)

δ (ppm)
7.25-7.40 (15H, m), 4.69 (1H, d, J=11.2 Hz), 4.60 (1H, d, J=11.2 Hz), 4.55 (1H, d, J=11.2 Hz), 4.48-4.53 (3H, m), 3.75-3.81 (2H, m), 3.65-3.72 (2H, m), 3.60 (1H, dt, J=3.7, 7.3 Hz), 1.66 (1H, m), 1.56 (1H, m), 1.41 (1H, m), 1.19-1.36 (21H, m), 0.88 (3H, t, J=6.7 Hz).

(vi) Synthesis of the compound D7

To the solution of the azide derivative (compound D6, 605.5 mg) in tetrahydrofuran (6 ml) was added 10% palladium on charcoal (60 mg). After the reaction vessel was purged with hydrogen, the mixture was stirred at room temperature for 15 hours, filtered through celite, and the filtrate was concentrated and purified on a silica gel column (Wako Gel C-200, 30 g, hexane-ethyl acetate (7:3)) to give an amine (compound D7) in an amount of 459.9 mg (yield, 79.4%).
Data of the compound D7
$[\alpha]^{24}_D$ = -7.0° ($CHCl_3$, c = 0.5)

MS: FDMS 574.
NMR: $^1H$ (500 MHz, $CDCl_3$; 27°C)

δ (ppm)
7.23-7.36 (15H, m), 4.74 (1H, d, J=11.2 Hz), 4.63 (1H, d, J=11.5 Hz), 4.53 (1H, d, J=11.5 Hz), 4.52 (1H, d, J=11.5 Hz), 4.49 (2H, d, J=1.8 Hz), 3.71 (2H, m), 3.57 (1H, dd, J=3.7, 6.7 Hz), 3.49 (1H, m), 3.16 (1H, m), 1.82 (1H, m), 1.69 (1H, m), 1.58 (1H, m), 1.49 (1H, m), 1.20-1.35 (20H, bs), 0.88 (3H, t, J=7.3 Hz).

(vii) Synthesis of the compound D8

(R)-2-Acetoxytetracosanoic acid (compound D8) is obtained, for example, by reacting (R)-2-α-hydroxytetracosanoic acid which is synthesized by applying the method described in Agricultural and Biological Chemistry, 54 (12), 3337-3338, 1990 with acetic anhydride in pyridine.
Data of the compound D8
$[\alpha]^{20}_D$ = +8.5° ($CHCl_3$, c = 1.0)

(viii) Synthesis of the compound D9

The amine (compound D7, 153.3 mg) and (R)-2-acetoxytetracosanoic acid (compound D8, 113.8 mg) were dissolved in tetrahydrofuran (4 ml), and 2-ethoxy-1-ethoxycarbonyl-1,2-dihydroquinoline (EEDQ, 99.0 mg) was added to the solution. The mixture was stirred at room temperature for 60 hours, and then concentrated and purified on a silica gel column (Wako Gel C-200, 10 g, hexane-ethyl acetate (9:1)) to give a benzylceramide (compound D9) in an amount of 205.6 mg (yield, 78.3%).
Data of the compound D9
$[\alpha]^{23}_D$ = +2.1° ($CHCl_3$, c = 0.6)

MS: FDMS 983.
NMR: $^1H$ (500 MHz, $CDCl_3$; 27°C)

δ (ppm)
7.22-7.36 (15H, m), 6.50 (1H, d, J=9.2 Hz), 5.05 (1H, dd, J=4.9, 7.3 Hz), 4.82 (1H, d, J=11.6 Hz), 4.62 (1H, d, J=11.6 Hz), 4.55 (1H, d, J=11.6 Hz), 4.52 (1H, d, J=11.6 Hz), 4.42 (2H, s), 4.23 (1H, m), 3.84 (2H, m), 3.51 (1H, m), 3.48 (1H, dd, J=3.7, 9.8 Hz), 1.98 (3H, s), 1.60-1.82 (2H, m), 1.50 (1H, m), 1.20-1.35 (63H, m), 0.88 (6H, t, J=7.3 Hz).

(ix) Synthesis of the compound D10

To the solution of the benzylceramide (compound D9, 317.7 mg) in tetrahydrofuran-n-propanol (1:1) (6 ml) were added 10% palladium on charcoal (167.4 mg) and formic acid (0.6 ml). After the reaction vessel was purged with hydrogen, the mixture was stirred at 40°C for 5 hours. The reaction mixture was diluted with chloroform (10 ml), filtered through celite, and the filtrate was concentrated. Purification on a silica gel column (Wako Gel C-200, 15 g) eluting with chloroform-methanol (98:2) afforded a ceramide (compound D10) in an amount of 191.6 mg (yield, 83.2%).
Data of the compound D10
$[\alpha]^{23}_D$ = +6.0° ($CHCl_3$, c = 0.1)

MS: FDMS 713.
NMR: $^1H$ (500 MHz, $C_5D_5N$; 27°C)

δ (ppm)

8.63 (1H, d, J=8.5 Hz), 6.56 (2H, m), 6.13 (1H, bd, J=5.7 Hz), 5.54 (1H, dd, J=5.5, 7.3 Hz), 5.07 (1H, m), 4.47 (1H, m), 4.43 (1H, m), 4.38 (1H, m), 4.28 (1H, m), 2.20 (1H, m), 2.07 (2H, m), 2.04 (3H, s), 1.90 (2H, m), 1.68 (1H, m), 1.15-1.60 (60H, m), 0.85 (6H, t, J=6.7 Hz).

(x) Synthesis of the compound D11

To the solution of the ceramide (compound D10, 99.7 mg) in pyridine (3 ml) were added triphenylmethyl chloride (390.3 mg) and 4-dimethylaminopyridine (5.0 mg), and the mixture was stirred at 60°C for 3 hours. After dilution with chloroform (30 ml), the mixture was washed with brine and concentrated. Purification on a silica gel column (Wako Gel C-200, 5 g) eluting with chloroform afforded a trityl derivative (compound D11) in an amount of 111.7 mg (yield, 83.6%).
Data of the compound D11
$[\alpha]^{23}_D$ = -13.3° ($CHCl_3$, $\underline{c}$ = 0.1)

NMR: $^1H$ (500 MHz, $CDCl_3$; 27°C)

δ (ppm)
7.21-7.40 (15H, m), 6.89 (1H, d, J=8.6 Hz), 5.21 (1H, dd, J=5.1, 6.6 Hz), 4.27 (1H, m), 3.60 (1H, m), 3.43 (1H, dd, J=3.2, 7.1 Hz), 3.36 (1H, dd, J=4.2, 7.1 Hz), 3.34 (1H, m), 3.01 (1H, m), 2.08 (1H, m), 2.05 (3H, s), 1.85 (1H, m), 1.75 (1H, m), 1.68 (1H, m), 1.10-1.50 (62H, m), 0.88 (6H, t, J=7.3 Hz).

(xi) Synthesis of the compound D12

To the solution of the trityl derivative (compound D11, 166.5 mg) in pyridine (3 ml) were added benzoyl chloride (0.18 ml) and 4-dimethylaminopyridine (5.0 mg). After stirring at room temperature for 36 hours, the mixture was diluted with brine, extracted with chloroform and concentrated. Purification on a silica gel column (Wako Gel C-200, 15 g) eluting with hexane-ethyl acetate (95:5) afforded a benzoyl derivative (compound D12) in an amount of 193.9 mg (yield, 95.6%).
Data of the compound D12
$[\alpha]^{23}_D$ = +7.3° ($CHCl_3$, $\underline{c}$ = 0.5)

MS: FDMS 1162, 920.
NMR: $^1H$ (500 MHz, $CDCl_3$; 27°C)

δ (ppm)
7.04-8.16 (25H, m), 5.91 (1H, dd, J=2.4, 9.0 Hz), 5.45 (1H, dt, J=2.9, 9.8 Hz), 5.37 (1H, t, J=7.3 Hz), 4.68 (1H, m), 3.34 (1H, dd, J=3.7, 9.8 Hz), 3.26 (1H, dd, J=2.9, 9.8 Hz), 2.02 (3H, s), 1.12-2.02 (66H, m), 0.87 (6H, m).

(xii) Synthesis of the compound D13

To the solution of benzoyl derivative (compound D12, 193.9 mg) in a solution of methylene chloride-methanol (2:1) (3 ml) was added p-toluenesulfonic acid monohydrate (63.4 mg). After being stirred at room temperature for 1.5 hours, the mixture was concentrated. The residue was dissolved in ethyl acetate and washed with aqueous sodium hydrogen carbonate and brine, and then concentrated. Purification on a silica gel column (Wako Gel C-200, 15 g) eluting with hexane-ethyl acetate (8:2) afforded an alcohol (compound D13) in an amount of 113.1 mg (yield, 73.7%).
Data of the compound D13
$[\alpha]^{23}_D$ = +27.3° ($CHCl_3$, $\underline{c}$ = 0.1)

MS: FDMS 921.
NMR: $^1H$ (500 MHz, $CDCl_3$; 27°C)

δ (ppm)
8.06 (2H, d, J=7.3 Hz), 7.96 (2H, d, J=7.3 Hz), 7.64 (1H, t, J=7.3 Hz), 7.54 (1H, t, J=7.6 Hz), 7.50 (2H, t, J=7.9 Hz), 7.39 (2H, t, J=7.9 Hz), 7.06 (1H, d, J=9.2 Hz), 5.48 (1H, dd, J=2.4, 9.1 Hz), 5.38 (1H, dt, J=3.1, 9.8 Hz), 5.19 (1H, t, J=6.1 Hz), 4.37 (1H, m), 3.57-3.68 (2H, m), 2.20 (3H, s), 2.02 (2H, m), 1.92 (2H, m), 1.16-1.50 (62H, m), 0.88 (6H, m).

(xiii) Synthesis of the compound D14

To the solution of the alcohol (compound D13, 113.1 mg) in tetrahydrofuran (2 ml) were added stannous chloride (54.8 mg), silver perchlorate (59.9 mg) and powdered Molecular Sieves 4A (500 mg), and the mixture was stirred at room temperature for 30 minutes. After the mixture was cooled to -10°C, a solution of benzylgalactosyl fluoride (compound A13, 313.4 mg) in tetrahydrofuran (2 ml) was added. The resulting mixture was allowed to warm to room temperature, stirred for 2 hours, and then diluted with acetone, filtered through celite. The filtrate was evaporated under reduced pressure, and the residue was suspended in ethyl acetate, washed with brine and concentrated. Purification on a silica gel column (Wako Gel C-200, 10 g) eluting with hexane-ethyl acetate (19:1) afforded an α-galactoside (compound D14) in an amount of 148.0 mg (yield, 83.5%).
Data of the compound D14
$[\alpha]^{23}_D$ = +21.0° ($CHCl_3$, c = 0.1)

MS: FDMS 1443.
NMR: $^1H$ (500 MHz, $CDCl_3$; 27°C)

δ (ppm)
8.03 (2H, d, J=7.9 Hz), 7.90 (2H, d, J=7.9 Hz), 7.73 (1H, d, J=8.3 Hz), 7.59 (1H, t, J=6.4 Hz), 7.50 (1H, t, J=6.4 Hz), 7.45 (2H, t, J=7.6 Hz), 7.15-7.40 (22H, m), 5.78 (1H, dd, J=2.6, 9.8 Hz), 5.40 (1H, m), 5.10 (1H, dd, J=5.2, 7.6 Hz), 4.88 (1H, d, J=11.3 Hz), 4.53-4.76 (7H, m), 4.48 (1H, d, J=11.8 Hz), 4.40 (1H, d, J=11.8 Hz), 4.09 (1H, t, J=7.2 Hz), 3.99 (1H, dd, J=3.3, 10.4 Hz), 3.93 (1H, m), 3.90 (1H, m), 3.82 (1H, dd, J=2.4, 9.8 Hz), 3.59 (1H, dd, J=2.3, 12.1 Hz), 3.53 (1H, dd, J=6.4, 8.9 Hz), 3.45 (1H, dd, J=6.7, 9.2 Hz), 2.44 (1H, bs), 2.02 (3H, s), 1.89 (3H, m), 1.40 (2H, m), 1.10-1.35 (61H, m), 0.88 (6H, m).

(xiv) Synthesis of the compound D15

To the solution of the α-galactoside (compound D14, 147.1 mg) in ethyl acetate (3 ml) was added palladium black (15 mg). After the reaction vessel was purged with hydrogen, the mixture was stirred at room temperature for 4 hours, filtered through celite, and the filtrate was concentrated to give a tetraol (compound D15) in an amount of 106.6 mg (yield, 96.6%).
Data of the compound D15
$[\alpha]^{23}_D$ = +26.0° ($CHCl_3$, c = 0.1)

MS: FDMS 1083, 921.
NMR: $^1H$ (500 MHz, $CDCl_3$; 27°C)

δ (ppm)
7.99 (2H, d, J=7.9 Hz), 7.90 (2H, d, J=7.9 Hz), 7.75 (1H, d, J=8.3 Hz), 7.60 (1H, t, J=6.4 Hz), 7.53 (1H, t, J=6.4 Hz), 7.48 (2H, t, J=7.6 Hz), 7.38 (2H, t, J=7.6 Hz), 5.78 (1H, dd, J=2.4, 9.8 Hz), 5.26 (1H, m), 5.07 (1H, t, J=6.7 Hz), 4.70 (1H, d, J=3.7 Hz), 4.57 (1H, m), 3.98 (1H, bs), 3.90 (1H, m), 3.80-3.90 (3H, m), 3.78 (1H, m), 3.70 (1H, m), 3.65 (1H, bd, J=10.4 Hz), 3.46 (2H, m), 3.13 (1H, bs), 2.78 (1H, m), 2.18 (3H, s), 1.81-1.95 (4H, m), 1.41 (2H, m), 1.16-1.35 (60H, m), 0.88 (6H, m).

(xv) Synthesis of the compound 22

To the solution of the tetraol (compound D15, 105.5 mg) in methanol (5 ml) was added slowly a 1N methanolic sodium methoxide solution (2 ml), and the mixture was stirred at room temperature for 30 minutes. A cation exchange resin (Dowex 50W, X8, manufactured by The Dow Chemical Company) was added to neutralize the mixture, and the resulting mixture was filtered. The solids removed were washed sufficiently with a chloroform-methanol (1:1) solution. The extract was combined with the filtrate, and concentrated. Purification on a silica gel column (Wako Gel C-200, 5 g) eluting with chloroform-methanol-water (90:10:1) afforded a cerebroside (compound 22) in an amount of 66.7 mg (yield, 82.2%).
Data of the compound 22
The various data of the compound 22 accorded with those of the product obtained from the natural material (Example 1-A).
The compounds (16-21, 23-28, 30-33) were synthesized by using various carboxylic acids or combining a variety of Wittig's salts by applying the method for synthesizing the compound 22 (reaction route D). Synthetic examples of these compounds are herein illustrated.

Compound 16

The aldehyde D1 was reacted with tridecanetriphenylphosphonium bromide in place of the Wittig's salt in the synthesis of the compound 22. Synthesis was further conducted by applying the route D. The amine obtained by reducing an azide group was reacted with tetracosanoic acid in place of (R)-2-acetoxytetracosanoic acid D8, and the synthetic process was followed by applying the route D to obtain the compound 16.
[Data]
$[\alpha]^{24}_D$ = +28.2° (pyridine, c = 0.27)

MS: FDMS 831.
IR: ($cm^{-1}$, Kbr)
3350, 2920, 2850, 1640, 1540, 1465.
mp: 146-147°C
NMR: $^1H$ (500 MHz, $C_5D_5N$; 27°C)

δ (ppm)
8.45 (1H, d, J=8.5 Hz), 5.55 (1H, d, J=3.7 Hz), 5.24 (1H, m), 4.64 (2H, m), 4.52 (1H, m), 4.48 (1H, m), 4.38 (4H, m), 4.28 (2H, bs), 2.41 (2H, t, J=6.3 Hz), 2.24 (1H, m), 1.88 (2H, m), 1.78 (2H, m), 1.64 (1H, m), 1.10-1.45 (62H, m), 0.85 (6H, t, J=6.7 Hz).
$^{13}C$ (125 MHz, $C_5D_5N$; 27°C)
δ (ppm)
173.2 (s), 101.5 (d), 76.7 (d), 73.0 (d), 72.5 (d), 71.6 (d), 71.0 (d), 70.3 (d), 68.7 (t), 62.7 (t), 51.5 (d), 36.8 (t), 34.3 (t), 32.1 (t), 30.4 (t), 30.1 (t), 30.0 (t), 29.9 (t), 29.9 (t), 29.8 (t), 29.7 (t), 29.6 (t), 26.5 (t), 26.4 (t), 22.9 (t), 14.3 (q).

Compound 17

The amine obtained by reducing an azide group by applying the route D in the synthesis of the compound 22 was reacted with tetracosanoic acid in place of (R)-2-acetoxytetracosanoic acid D8, and the synthetic process was followed by applying the route D to obtain the compound 17.
[Data]
$[\alpha]^{23}_D$ = +42.4° (pyridine, c = 0.8)

MS: FDMS 817.
IR: ($cm^{-1}$, Kbr)
3400, 2950, 2870, 1645, 1535, 1475, 1080.
mp: 166-168°C
NMR: $^1H$ (500 MHz, $C_5D_5N$; 27°C)

δ (ppm)
8.43 (1H, d, J=8.6 Hz), 5.55 (1H, d, J=3.7 Hz), 5.23 (1H, m), 4.64 (1H, dd, J=5.5, 10.4 Hz), 4.62 (1H, dd, J=4.3, 10.4 Hz), 4.52 (1H, m), 4.49 (1H, bt, J=6.1 Hz), 4.33-4.42 (4H, m), 4.30 (2H, m), 2.42 (2H, dd, J=6.7, 7.3 Hz), 2.26 (1H, m), 1.86 (2H, m), 1.78 (2H, m), 1.65 (1H, m), 1.16-1.46 (60H, m), 0.85 (6H, t, J=6.7 Hz).
$^{13}C$ (125 MHz, $C_5D_5N$; 27°C)
δ (ppm)
173.2 (s), 101.5 (d), 76.7 (d), 73.0 (d), 72.4 (d), 71.5 (d), 70.9 (d), 70.2 (d), 68.6 (t), 62.6 (t), 51.4 (d), 36.7 (t), 34.3 (t), 32.1 (t), 30.3 (t), 30.1 (t), 30.0 (t), 29.9 (t), 29.8 (t), 29.8 (t), 29.7 (t), 29.7 (t), 29.5 (t), 26.4 (t), 26.3 (t), 22.9 (t), 14.2 (q).

Compound 18

The aldehyde D1 was reacted with decanetriphenylphosphonium bromide in place of the Wittig's salt D2 in the synthesis of the compound 22. The subsequent synthetic process was followed by applying the route D. The amine obtained by reducing the azide group was reacted with tetracosanoic acid in place of (R)-2-acetoxytetracosanoic acid D8, and the subsequent steps were followed by applying the route D to obtain the compound 18.
[Data]
$[\alpha]^{24}_D$ = +30.0° (pyridine, c = 0.2)

MS: FDMS 789.

IR: ($cm^{-1}$, Kbr)
3350, 2920, 2840, 1640, 1540, 1465.
mp: 154-155°C
NMR: $^1H$ (500 MHz, $C_5D_5N$; 27°C)

δ (ppm)
8.45 (1H, d, J=8.5 Hz), 5.55 (1H, d, J=3.7 Hz), 5.24 (1H, m), 4.64 (2H, m), 4.53 (1H, m), 4.49 (1H, m), 4.39 (4H, m), 4.30 (2H, bs), 2.42 (2H, t, J=6.7 Hz), 2.25 (1H, m), 1.88 (2H, m), 1.78 (2H, m), 1.64 (1H, m), 1.15-1.45 (56H, m), 0.85 & 0.84 (each 3H, t, J=7.3 Hz).
$^{13}C$ (125 MHz, $C_5D_5N$; 27°C)
δ (ppm)
173.3 (s), 101.5 (d), 76.7 (d), 73.0 (d), 72.5 (d), 71.6 (d), 71.0 (d), 70.3 (d), 68.7 (t), 62.7 (t), 51.5 (d), 36.8 (t), 34.3 (t), 32.1 (t), 30.3 (t), 29.6-30.1, 26.5 (t), 26.4 (t), 22.9 (t), 14.3 (q).

Compound 19

The aldehyde D1 was reacted with hexanetriphenylphosphonium bromide in place of the Wittig's salt D2 in the synthesis of the compound 22. The subsequent synthetic process was followed by applying the route D. The amine obtained by reducing the azide group was reacted with tetracosanoic acid in place of (R)-2-acetoxytetracosanoic acid D8, and the subsequent steps were followed by applying the route D to obtain the compound 19.
[Data]

MS: FDMS 732.
NMR: $^1H$ (500 MHz, $C_5D_5N$; 27°C)

δ (ppm)
8.45 (1H, d, J=8.6 Hz), 6.97 (1H, bs), 6.62 (1H, bs), 6.52 (1H, m), 6.43 (1H, bs), 6.29 (1H, d, J=3.7 Hz), 6.06 (1H, bs), 5.58 (1H, d, J=3.7 Hz), 5.26 (1H, m), 4.66-4.68 (2H, m), 4.55 (1H, bs), 4.51 (1H, m), 4.38-4.42 (4H, m), 4.30 (1H, bs), 2.44 (2H, t, J=7.3 Hz), 1.80-1.88 (4H, m), 1.19-1.59 (50H, m), 0.88 & 0.81 (each 3H, t, J=6.7 Hz).

Compound 20

Synthesis was conducted by applying the route D in the synthesis of the compound 22. The amine obtained by reducing the azide group was reacted with hexacosanoic acid in place of (R)-2-acetoxytetracosanoic acid D8, and the subsequent steps were followed by applying the route D to obtain the compound 20.
[Data]
$[\alpha]^{25}_D$ = +37.7° (pyridine, c = 0.97)

MS: FDMS 845.
IR: ($cm^{-1}$, Kbr)
3380, 2920, 2840, 1635, 1545, 1465, 1065.
mp: 156-158°C
NMR: $^1H$ (500 MHz, $C_5D_5N$; 27°C)

δ (ppm)
8.46 (1H, d, J=8.6 Hz), 6.42 (1H, m), 6.09 (1H, m), 5.57 (1H, d, J=3.7 Hz), 5.26 (1H, m), 4.66 (2H, m), 4.55 (1H, m), 4.51 (1H, t, J=5.8 Hz), 4.41 (4H, m), 4.32 (2H, m), 2.44 (2H, t, J=7.0 Hz), 2.28 (1H, m), 1.90 (2H, m), 1.81 (2H, m), 1.68 (1H, m), 1.15-1.45 (64H, m), 0.88 (6H, t, J=6.7 Hz).
$^{13}C$ (125 MHz, $C_5D_5N$; 27°C)
δ (ppm)
173.2 (s), 101.5 (d), 76.7 (d), 73.0 (d), 72.5 (d), 71.6 (d), 71.0 (d), 70.3 (d), 68.7 (t), 62.7 (t), 51.5 (d), 36.8 (t), 34.4 (t), 32.1 (t), 30.4 (t), 30.1 (t), 30.03 (t), 29.99 (t), 29.93 (t), 29.87 (t), 29.81 (t), 29.76 (t), 29.6 (t), 26.5 (t), 26.4 (t), 22.9 (t), 14.3 (q).

Compound 21

The aldehyde D1 was reacted with decanetriphenylphosphonium bromide in place of the Wittig's salt D1 in the synthesis of the compound 22. The subsequent synthetic process was followed by applying the route D to obtain

the compound 21.
[Data]

MS: FDMS 847.
IR: ($cm^{-1}$, Kbr)
3400, 2950, 2870, 1645, 1535, 1475, 1080.
NMR: $^{1}H$ (500 MHz, $C_5D_5N$; 27°C)

δ (ppm)
8.50 (1H, d, J=9.2 Hz), 5.59 (1H, d, J=3.7 Hz), 5.27 (1H, m), 4.64 (2H, m), 4.58 (1H, m), 4.53 (1H, m), 4.48 (2H, m), 4.30-4.42 (4H, m), 4.27 (1H, m), 2.29 (1H, m), 2.18 (1H, m), 1.98 (1H, m), 1.87 (2H, m), 1.74 (1H, m), 1.67 (2H, m), 1.15-1.46 (60H, m), 0.84 (6H, t, J=6.7 Hz).
$^{13}C$ (125 MHz, $C_5D_5N$; 27°C)
δ (ppm)
174.9 (s), 101.2 (d), 76.5 (d), 73.0 (d), 72.4 (d), 72.3 (d), 71.6 (d), 70.9 (d), 70.1 (d), 68.1 (t), 62.6 (t), 50.4 (d), 35.5 (t), 34.4 (t), 32.1 (t), 30.3 (t), 30.1 (t), 30.0 (t), 29.9 (t), 29.5 (t), 26.4 (t), 25.8 (t), 22.9 (t), 14.2 (q).

Compound 23

The aldehyde D1 was reacted with decanetriphenylphosphonium bromide in place of the Wittig's salt D2 in the synthesis of the compound 22. The subsequent synthetic process was followed by applying the route D to obtain the compound 23.
[Data]
$[\alpha]^{24}_D$ = +59.2° (pyridine, c = 0.1)

MS: FDMS 805.
IR: ($cm^{-1}$, Kbr)
3400, 2950, 2870, 1645, 1535, 1475, 1080.
mp: 193-194°C
NMR: $^{1}H$ (500 MHz, $C_5D_5N$; 27°C)

δ (ppm)
8.50 (1H, d, J=9.2 Hz), 5.59 (1H, d, J=3.7 Hz), 5.28 (1H, m), 4.64 (2H, m), 4.58 (1H, m), 4.53 (1H, m), 4.48 (2H, m), 4.30-4.42 (4H, m), 4.27 (1H, m), 2.29 (1H, m), 2.18 (1H, m), 1.98 (1H, m), 1.87 (2H, m), 1.74 (1H, m), 1.66 (2H, m), 1.15-1.46 (54H, m), 0.84 (6H, t, J=6.7 Hz).
$^{13}C$ (125 MHz, $C_5D_5N$; 27°C)
δ (ppm)
174.9 (s), 101.2 (d), 76.5 (d), 73.0 (d), 72.4 (d), 72.3 (d), 71.6 (d), 70.9 (d), 70.1 (d), 68.1 (t), 62.6 (t), 50.4 (d), 35.5 (t), 34.4 (t), 32.1 (t), 30.3 (t), 30.1 (t), 30.0 (t), 29.9 (t), 29.5 (t), 26.4 (t), 25.8 (t), 22.9 (t), 14.2 (q).

Compound 24

The aldehyde D1 was reacted with hexanetriphenylphosphonium bromide in place of the Wittig's salt D2 in the synthesis of the compound 22. The subsequent synthetic process was followed by applying the route D to obtain the compound 24.
[Data]
$[\alpha]^{23}_D$ = +67.1° (pyridine, c = 1.32)

MS: FDMS 749.
IR: ($cm^{-1}$, Kbr)
3300, 2870, 2800, 1630, 1605, 1515, 1455, 1060.
mp: 145-147°C
NMR: $^{1}H$ (500 MHz, $C_5D_5N$; 27°C)

δ (ppm)
8.50 (1H, d, J=9.2 Hz), 6.70 (2H, bd, J=6.1 Hz), 6.53 (1H, bs), 6.31 (1H, bs), 6.08 (1H, bs), 5.61 (1H, d, J=3.7 Hz), 5.29 (1H, m), 4.64-4.67 (2H, m), 4.59 (1H, m), 4.54 (1H, m), 4.47-4.51 (2H, m), 4.32-4.43 (4H, m), 4.26 (1H, m), 1.64-2.27 (4H, m), 1.20-1.40 (50H, m), 0.87 & 0.82 (each 3H, t, J=6.7 Hz).

$^{13}C$ (125 MHz, $C_5D_5N$; 27°C)

δ (ppm)

175.0 (s), 101.2 (d), 76.5 (d), 73.0 (d), 72.4 (d), 72.3 (d), 71.6 (d), 70.9 (d), 70.1 (d), 68.1 (t), 62.6 (t), 50.4 (d), 35.5 (t), 34.4 (t), 32.0 (t), 30.2 (t), 29.9 (t), 29.8 (t), 29.7 (t), 29.5 (t), 26.3 (t), 25.8 (t), 22.9 (t), 22.8 (t), 14.21 (q), 14.18 (q).

Compound 25

The aldehyde D1 was reacted with tridecanetriphenylphosphonium bromide in place of the Wittig's salt D2 in the synthesis of the compound 22. The subsequent synthetic process was followed by applying the route D, and the amine obtained by reducing the azide group was reacted with (R)-2-acetoxyhexacosanoic acid in place of (R)-2-acetoxytetracosanoic acid D8 with the subsequent synthetic process by applying the route D to give the compound 25.

[Data]

$[\alpha]^{23}_D$ = +45.2° (pyridine, c = 1.0)

MS: FDMS 875.

IR: ($cm^{-1}$, Kbr)

3400, 2950, 2870, 1645, 1535, 1475, 1080.

mp: 198-199°C

NMR: $^1H$ (500 MHz, $C_5D_5N$; 27°C)

δ (ppm)

8.49 (1H, d, J=9.2 Hz), 7.53 (1H, bs), 7.02 (1H, bs), 6.70 (1H, d, J=6.1 Hz), 6.65 (1H, bs), 6.53 (1H, bs), 6.30 (1H, bs), 6.08 (1H, d, J=5.5 Hz), 5.57 (1H, d, J=3.7 Hz), 5.26 (1H, m), 4.62 (2H, dd, J=4.9, 10.4 Hz), 4.58 (1H, m), 4.51 (1H, bs), 4.46 (2H, m), 4.28-4.41 (4H, m), 4.26 (1H, m), 2.27 (1H, m), 2.17 (1H, m), 1.98 (1H, m), 1.87 (2H, m), 1.74 (1H, m), 1.66 (2H, m), 1.16-1.46 (64H, m), 0.85 (6H, t, J=6.1 Hz).

$^{13}C$ (125 MHz, $C_5D_5N$; 27°C)

δ (ppm)

175.0 (s), 101.2 (d), 76.5 (d), 73.0 (d), 72.4 (d), 72.3 (d), 71.6 (d), 70.9 (d), 70.1 (d), 68.2 (t), 62.6 (t), 50.5 (d), 35.5 (t), 34.4 (t), 32.1 (t), 30.3 (t), 30.1 (t), 29.9 (t), 29.9 (t), 29.6 (t), 26.4 (t), 25.8 (t), 22.9 (t), 14.2 (q).

Compound 26

The aldehyde D1 was reacted with tetradecanetriphenylphosphonium bromide in place of the Wittig's salt D2 in the synthesis of the compound 22. The subsequent synthetic process was followed by applying the route D, and the amine obtained by reducing the azide group was reacted with (R)-2-acetoxyhexacosanoic acid in place of (R)-2-acetoxytetracosanoic acid D8 with the subsequent synthetic process by applying the route D to give the compound 26.

[Data]

$[\alpha]^{23}_D$ = +46.5° (pyridine, c = 0.7)

MS: FDMS 889.

IR: ($cm^{-1}$, Kbr)

3400, 2950, 2870, 1645, 1535, 1475, 1080.

mp: 205-206°C

NMR: $^1H$ (500 MHz, $C_5D_5N$; 27°C)

δ (ppm)

8.50 (1H, d, J=9.2 Hz), 7.56 (1H, bs), 7.04 (1H, bs), 6.71 (1H, d, J=6.7 Hz), 6.66 (1H, bs), 6.54 (1H, bs), 6.32 (1H, bs), 6.10 (1H, d, J=5.5 Hz), 5.58 (1H, d, J=3.7 Hz), 5.27 (1H, m), 4.63 (2H, m), 4.58 (1H, m), 4.52 (1H, bs), 4.47 (2H, m), 4.28-4.41 (4H, m), 4.27 (1H, m), 2.27 (1H, m), 2.18 (1H, m), 1.99 (1H, m), 1.88 (2H, m), 1.74 (1H, m), 1.66 (2H, m), 1.16-1.46 (66H, m), 0.85 (6H, t, J=6.7 Hz).

$^{13}C$ (125 MHz, $C_5D_5N$; 27°C)

δ (ppm)

175.0 (s), 101.2 (d), 76.5 (d), 73.0 (d), 72.4 (d), 72.3 (d), 71.6 (d), 70.9 (d), 70.1 (d), 68.1 (t), 5 62.6 (t), 50.4 (d), 35.5 (t), 34.4 (t), 32.1 (t), 30.3 (t), 30.1 (t), 29.9 (t), 29.9 (t), 29.5 (t), 26.4 (t), 25.8 (t), 22.9 (t), 14.2 (q).

Compound 27

The aldehyde D1 was reacted with heptadecanetriphenylphosphonium bromide in place of the Wittig's salt

D2 in the synthesis of the compound 22. The subsequent synthetic process was followed by applying the route D, and the amine obtained by reducing the azide group was reacted with (R)-2-acetoxyhexacosanoic acid in place of (R)-2-acetoxytetracosanoic acid D8 with the subsequent synthetic process by applying the route D to give the compound 27.
[Data]
$[\alpha]^{23}_D$ = +46.0° (pyridine, c = 0.8)

MS: FDMS 903.
IR: ($cm^{-1}$, Kbr)
3400, 2950, 2870, 1645, 1535, 1475, 1080.
mp: 200-201°C
NMR: $^1H$ (500 MHz, $C_5D_5N$; 27°C)

δ (ppm)
8.49 (1H, d, J=9.2 Hz), 7.54 (1H, bs), 7.02 (1H, bs), 6.69 (1H, d, J=6.7 Hz), 6.66 (1H, bs), 6.53 (1H, bs), 6.30 (1H, bs), 6.08 (1H, d, J=4.9 Hz), 5.57 (1H, d, J=3.7 Hz), 5.25 (1H, m), 4.62 (2H, dd, J=4.9, 10.4 Hz), 4.57 (1H, m), 4.51 (1H, bs), 4.46 (2H, m), 4.28-4.40 (4H, m), 4.26 (1H, m), 2.26 (1H, m), 2.17 (1H, m), 1.98 (1H, m), 1.87 (2H, m), 1.73 (1H, m), 1.65 (2H, m), 1.16-1.46 (68H, m), 0.86 (6H, t, J=6.7 Hz).
$^{13}C$ (125 MHz, $C_5D_5N$; 27°C)
δ (ppm)
175.0 (s), 101.2 (d), 76.4 (d), 73.0 (d), 72.4 (d), 72.3 (d), 71.5 (d), 70.9 (d), 70.1 (d), 68.1 (t), 62.6 (t), 50.5 (d), 35.5 (t), 34.3 (t), 32.1 (t), 30.3 (t), 30.1 (t), 29.9 (t), 29.6 (t), 26.4 (t), 25.8 (t), 22.9 (t), 14.2 (q).

As the alternative methods for synthesizing the compounds 25, 26 and 27, Cerebrin E was employed. Cerebrin E which is a tetraol and commercially available from Alfred Baker Chemicals or K&K Laboratories, Inc. was used in place of the triol D10 in the synthesis of the compound 22. Synthesis was further conducted by applying the route D to obtain the compounds 25, 26 and 27. These compounds were separated by high performance liquid chromatography (D-ODS-5, manufactured by K.K. YMC, eluent: 100% methanol, 45°C).

Compound 28

In the synthesis of the compound 22, the route D was followed. The amine obtained by reducing the azide group was reacted with (S)-2-acetoxytetracosanoic acid in place of (R)-2-acetoxytetracosanoic acid D8 with the subsequent synthetic process by applying the route D to give the compound 28.
[Data]
$[\alpha]^{23}_D$ = +36.8° (pyridine, c = 2.0)

MS: FDMS 833.
IR: ($cm^{-1}$, Kbr)
3400, 2950, 2870, 1645, 1535, 1475, 1080.
mp: 174-176°C
NMR: $^1H$ (500 MHz, $C_5D_5N$; 27°C)

δ (ppm)
8.55 (1H, d, J=8.5 Hz), 5.61 (1H, d, J=4.3 Hz), 5.26 (1H, m), 4.68 (1H, dd, J=5.5, 10.4 Hz), 4.63 (1H, dd, J=3.7, 9.8 Hz), 4.56 (2H, bs), 4.49 (1H, t, J=5.5 Hz), 4.46 (1H, dd, J=3.7, 9.8 Hz), 4.38 (2H, m), 4.34 (1H, dd, J=4.3, 11.0 Hz), 4.31 (1H, bd, J=8.6 Hz), 4.20 (1H, dd, J=3.7, 7.9 Hz), 2.26 (1H, m), 2.19 (1H, m), 1.99 (1H, m), 1.84 (2H, m), 1.74 (1H, m), 1.58-1.70 (2H, m), 1.16-1.46 (58H, m), 0.85 (6H, t, J=6.7 Hz).
$^{13}C$ (125 MHz, $C_5D_5N$; 27°C)
δ (ppm)
175.0 (s), 101.2 (d), 76.7 (d), 73.0 (d), 72.5 (d), 72.4 (d), 71.6 (d), 70.9 (d), 70.1 (d), 68.0 (t), 62.6 (t), 50.5 (d), 35.6 (t), 34.6 (t), 32.1 (t), 30.3 (t), 30.1 (t), 29.9 (t), 29.9 (t), 29.6 (t), 26.3 (t), 25.8 (t), 22.9 (t), 14.2 (q).

Compound 30

The aldehyde D1 was reacted with 11-methyl-9-dodecenetriphenylphosphonium bromide in place of the Wittig's salt D2 in the synthesis of the compound 22. The subsequent synthetic process was followed by applying the route D, and the amine obtained by reducing the azide group was reacted with (S)-2-acetoxyhexacosanoic acid in place of (R)-2-acetoxytetracosanoic acid D8 with the subsequent synthetic process by applying the route D to give the compound 30.

[Data]
$[\alpha]^{25}_D$ = +46.2° (pyridine, c = 1.0)

MS: FDMS 847.
IR: ($cm^{-1}$, Kbr)
3400, 3250, 2870, 2810, 1640, 1525, 1455, 1355, 1320, 1275, 1145, 1060.
mp: 169.0-171.0°C
NMR: $^1H$ (500 MHz, $C_5D_5N$; 27°C)

δ (ppm)
8.57 (1H, d, J=9.2 Hz), 6.64 (2H, m), 6.45 (1H, m), 6.30 (1H, m), 6.11 (2H, m), 5.65 (1H, d, J=3.7 Hz), 5.29 (2H, m), 4.65-4.75 (2H, m), 4.59 (2H, m), 4.51 (2H, m), 4.30-4.45 (4H, m), 4.22 (1H, m), 2.30 (1H, m), 2.21 (1H, m), 2.02 (1H, m), 1.6-2.0 (5H, m), 1.49 (1H, m), 1.15-1.35 (56H, m), 0.89 (3H, t, J=6.1 Hz), 0.87 (6H, d, J=6.1 Hz).
$^{13}C$ (125 MHz, $C_5D_5N$; 27°C)
δ (ppm)
175.0 (s), 101.3 (d), 76.7 (d), 73.0 (d), 72.4 (d), 72.3 (d), 71.6 (d), 70.9 (d), 70.1 (d), 68.0 (t), 62.6 (t), 50.6 (d), 39.2 (t), 35.6 (t), 34.6 (t), 32.1 (t), 30.3 (t), 30.2 (t), 30.1 (t), 30.0 (t), 29.9 (t), 29.6 (t), 28.1 (d), 27.7 (t), 26.3 (t), 25.8 (t), 22.9 (t), 22.7 (q), 14.2 (q).

Compound 31

The aldehyde D1 was reacted with 11-methyl-9-dodecenetriphenylphosphonium bromide in place of the Wittig's salt D2 in the synthesis of the compound 22. The subsequent synthetic process was followed by applying the route D, and the amine obtained by reducing the azide group was reacted with tetracosanoic acid in place of (R)-2-acetoxytetracosanoic acid D8 with the subsequent synthetic process by applying the route D to give the compound 31.
[Data]
$[\alpha]^{25}_D$ = +43.6° (pyridine, c = 0.44)

MS: FDMS 831.
IR: ($cm^{-1}$, Kbr)
3300, 2880, 2810, 1630, 1535, 1455, 1055.
mp: 197.0-198.5°C
NMR: $^1H$ (500 MHz, $C_5D_5N$; 27°C)

δ (ppm)
8.44 (1H, d, J=8.6 Hz), 5.57 (1H, d, J=3.7 Hz), 5.25 (1H, m), 4.63-4.70 (2H, m), 4.54 (1H, d, J=3.1 Hz), 4.50 (1H, t, J=6.1 Hz), 4.35-4.45 (4H, m), 4.31 (2H, m), 2.44 (2H, t, J=7.3 Hz), 2.28 (1H, m), 1.90 (2H, m), 1.81 (2H, m), 1.68 (1H, m), 1.49 (1H, m), 1.2-1.45 (56H, m), 1.15 (2H, m), 0.88 (3H, t, J=6.7 Hz), 0.87 (6H, d, J=6.7 Hz).
$^{13}C$ (125 MHz, $C_5D_5N$; 27°C)
δ (ppm)
173.2 (s), 101.5 (d), 76.7 (d), 73.0 (d), 72.5 (d), 71.6 (d), 71.0 (d), 70.3 (d), 68.7 (t), 62.7 (t), 51.4 (d), 39.3 (t), 36.8 (t), 34.4 (t), 32.1 (t), 30.4 (t), 30.23 (t), 30.15 (t), 30.03 (t), 30.00 (t), 29.91 (t), 29.87 (t), 29.81 (t), 29.75 (t), 29.6 (d), 28.2 (d), 27.7 (t), 26.5 (t), 26.4 (t), 22.9 (t), 22.8 (q), 14.3 (q).

Experimental Example 2: Anti-tumor activity of the compounds of the present invention

Anti-tumor activity against B16 mouse melanoma inoculated subcutaneously.

Experiment was carried out with the groups of 6 female $BDF_1$ mice (6 weeks old) purchased from Japan SLC Inc., B16 mouse melanoma cells (1 × $10^6$ cells/mouse) were inoculated subcutaneously in the rear region of mice, and the sample prepared in a concentration of 0.1 mg/kg was administered intravenously at a dose of 0.2 ml/20g/mouse after 1, 5 and 9 days from inoculation (the day of inoculation being set as 0 day). The volume of the tumor at the hypodermis of the rear region [(longer diameter × shorter diameter × height)/2] was measured on 8, 12, 16 and 20 days after inoculation, and the tumor growth inhibition rate (TGIR) of each sample was determined. TGIR was calculated from the following equation:

$$TGIR (\%) = (1 - T/C) \times 100$$

wherein C represents a tumor volume of the control group and T represents a tumor volume of the group to which the sample was administered.

Table 1 shows the maximum TGIR during the test period of 20 days. In this connection, respective test runs were divided by broken lines.

Table 1

| Tumor growth inhibiting effects against B16 mouse melanoma cells | |
|---|---|
| Compound No | TGIR (%) |
| 31 | 83.4 |
| 14 | 84.0 |
| 23 | 94.1 |
| 24 | 52.5 |
| 30 | 57.7 |
| 21 | 57.9 |
| 17 | 58.0 |
| 22 | 82.4 |
| 28 | 76.2 |
| 16 | 65.0 |
| 19 | 80.2 |
| 1 | 91.4 |
| 9 | 71.5 |
| 4 | 78.1 |
| 6 | 73.7 |
| 15 | 61.9 |
| 20 | 73.7 |
| 2 | 53.1 |
| 3 | 56.9 |
| 7 | 18.5 |
| 8 | 22.1 |
| 18 | 66.3 |
| 35 | 63.0 |
| 29 | 79.7 |
| 25 | 92.8 |
| 26 | 72.3 |
| 27 | 92.8 |
| 5 | 92.1 |
| 12 | 41.8 |
| 13 | 28.2 |
| 10 | 76.5 |
| 11 | 55.9 |
| 32 | 73.2 |
| 33 | 76.5 |
| 34 | 88.9 |

As shown in Table 1, all of the compounds inhibited the growth of tumor.

Experimental Example 3: Immuno-stimulating activity of the compounds of the present invention

Lymphocyte mixed culture reaction

Experiment was carried out with the spleen cells of C57BL/6 mouse which had been treated with mitomycin C (50 µg/ml, 30 min) as the stimulator and with the pancreatic cells of BALB/c mouse as the responder. These pancreatic cells were suspended to a concentration of 2 × $10^6$ cells/ml with a culture medium of 10% FCS RPMI 1640, respectively. These cells (50 µl/well) and a sample (10 µl/well) were plated in a 96 well round-bottomed plate and cultured for 42 hours* under the condition of 37°C and 5% $CO_2$. $^3$H-thymidine ($^3$H-TdR) was added in a dose of 0.5 µCi/well. After 8 hours, the cells were harvested and subjected to the measurement of the uptake of $^3$H-TdR by a liquid scintillation counter.

Table 2

| Uptake rate of $^3$H-TdR in respective sample concentrations | | | |
|---|---|---|---|
| Sample/Concentration (µg/ml) (Compound) | Uptake of $^3$H-TdR (% of control) | | |
| | $10^0$ | $10^{-1}$ | $10^{-2}$ |
| 1 | 359 | 151 | 136 |
| 2 | 329 | 115 | 103 |
| 3 | 254 | 117 | 110 |
| 4 | 269 | 158 | 134 |
| 5 | 473 | 170 | 153 |
| 6 | 498 | 190 | 187 |
| 7 | 853 | 576 | 207 |
| 8 | 297 | 189 | 96 |
| 9 | 460 | 193 | 176 |
| 10 | 610 | 381 | 157 |
| 11 | 128 | 105 | 95 |
| 12 | 123 | 99 | 104 |
| 13 | 139 | 106 | 107 |
| 14 | 289 | 197 | 139 |
| 15 | 360 | 165 | 144 |
| 16 | 321 | 176 | 160 |
| 17 | 410 | 190 | 143 |
| 18 | 482 | 176 | 138 |
| 19 | 345 | 188 | 144 |
| 20 | 443 | 188 | 192 |
| 21 | 304 | 149 | 142 |
| 22 | 414 | 166 | 149 |
| 23 | 423 | 167 | 143 |
| 24 | 416 | 167 | 144 |
| 25 | 230 | 179 | 161 |
| 26 | 253 | 199 | 193 |
| 27 | 257 | 181 | 162 |
| 28 | 357 | 172 | 141 |
| 29 | 319 | 382 | 215 |
| 30 | 385 | 156 | 134 |
| 31 | 398 | 235 | 163 |
| 32* | - | 406 | 426 |
| 33* | - | 365 | 422 |
| 34* | - | 360 | 406 |
| 35 | 562 | 261 | 247 |

*The samples of the compounds 32, 33 and 34 were cultured for 4 days.

As shown in Table 2, all of these samples exhibited lymphocyte mixed culture reaction stimulating activities.

<u>Experimental Example 4</u>: Cytotoxicity

B16 melanoma cells which had been prepared in a concentration of $1 \times 10^5$ cells/ml and the compounds 1-35 which had been prepared in various concentrations were added to a 96 well flat-bottomed microplate in an amount of 100 µl/well and 10 µl/well, respectively. After culturing under the condition of 37°C and 5% $CO_2$ for 42 hours, $^3$H-TdR was added in a dose of 0.5 µCi/well. After further 8 hours, the cells were harvested, and the uptake of $^3$H-TdR was measured. None of the compounds even in the final concentration of 10 µg/ml influenced the proliferation of the cells.

<u>Experimental Example 5</u>: Acute toxicity

The compound 5 was once administered intravenously in doses of 0.1, 1.0 and 10 mg/kg to the groups of 6 male Crj:CD rats (5 weeks old), and the toxicity tests were conducted for 7 days after the administration of the compound 5.

As a result, even the dose of 10 mg/kg was not lethal to the animals, and no abnormality was observed on the autopsy, so that the $LD_{50}$ value of the compound is believed to be at least 10 mg/kg.

## Claims

**Claims for the following Contracting States : AT, BE, CH, LI, DE, DK, FR, GB, IT, LU, MC, NL, SE**

1. A glycosphingolipid including an alpha-galactose residue represented by the formula (A):

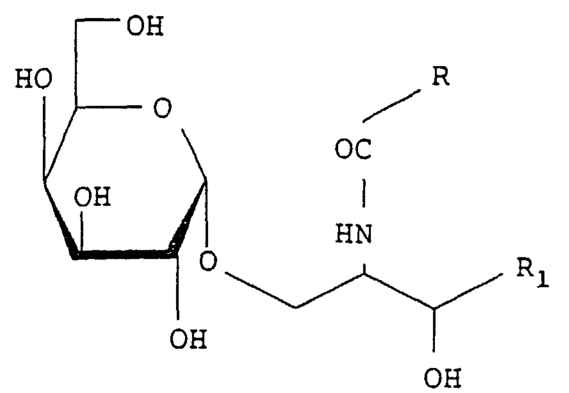

(A)

wherein

R represents

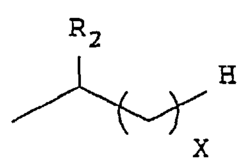

,

where $R_2$ represents H or OH and X denotes an integer of 0-26, or $-(CH_2)_7CH\text{-}CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17.

**2.** A glycosphingolipid according to claim 1, which is represented by the formula (I):

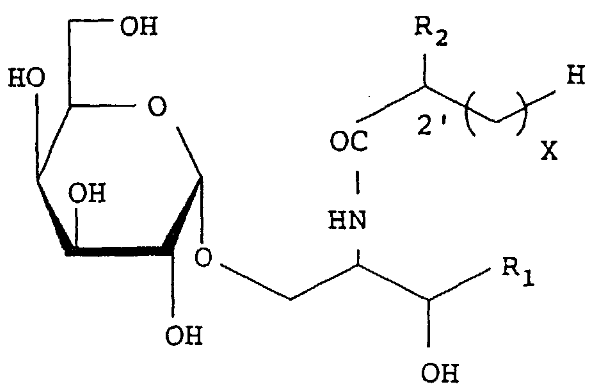

(I)

wherein

$R_1$ represents any one of the substituents defined by (a)-(e) below and $R_2$ represents H or OH (X is defined in the following (a)-(e)):

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH=CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**3.** A glycosphingolipid according to claim 2, which is represented by the formula (II):

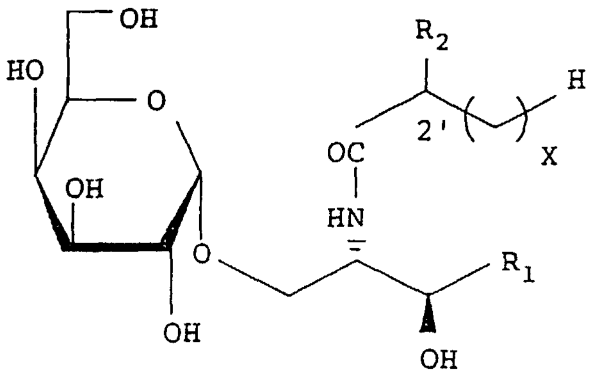

(II)

wherein

$R_1$ represents any one of the substituents defined by (a)-(e) below and $R_2$ represents H or OH (X is defined in the following (a)-(e));

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH=CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**4.** A glycosphingolipid according to claim 2, which is represented by the formula (III):

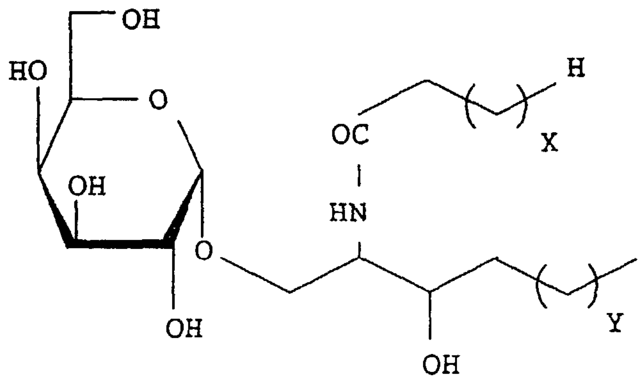

(III)

wherein X denotes an integer of 0-24 and Y denotes an integer of 7-15.

**5.** A glycosphingolipid according to claim 4, wherein X denotes an integer of 8-22 and Y denotes an integer of 9-13.

**6.** A glycosphingolipid according to claim 4, which is represented by the formula (IV):

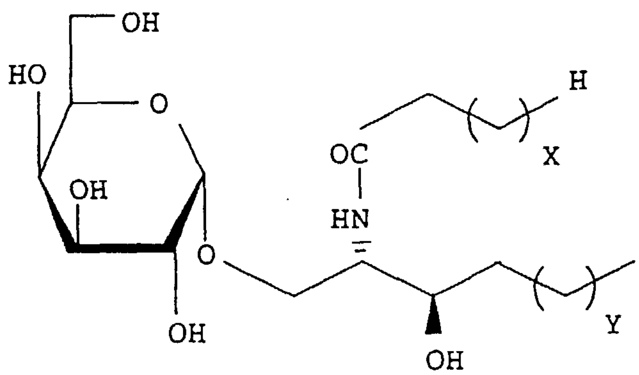

(IV)

wherein X denotes an integer of 0-24 and Y denotes an integer of 7-15.

**7.** A glycosphingolipid according to claim 6, wherein X denotes an integer of 8-22 and Y denotes an integer of 9-13.

**8.** A glycosphingolipid according to claim 2, which is represented by the formula (V):

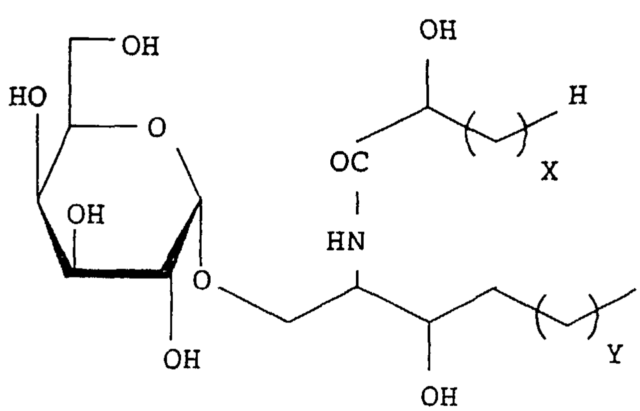

(V)

wherein X denotes an integer of 20-24 and Y denotes an integer of 11-15.

**9.** A glycosphingolipid according to claim 8, wherein X denotes an integer of 21-23 and Y denotes an integer of 12-14.

**10.** A glycosphingolipid according to claim 8, which is represented by the formula (VI):

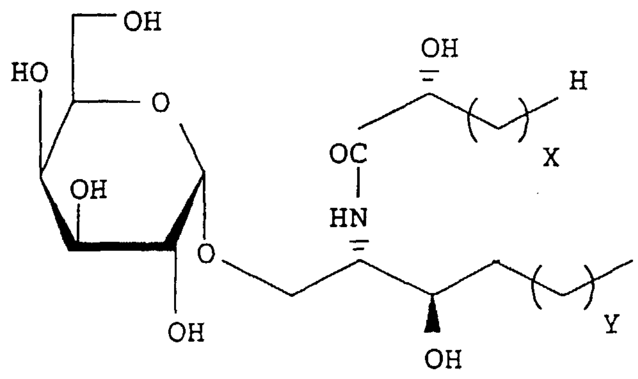

(VI)

wherein X denotes an integer of 20-24 and Y denotes an integer of 11-15.

**11.** A glycosphingolipid according to claim 10, wherein X denotes an integer of 21-23 and Y denotes an integer of 12-14.

**12.** A glycosphingolipid according to claim 2, which is represented by the formula (VII):

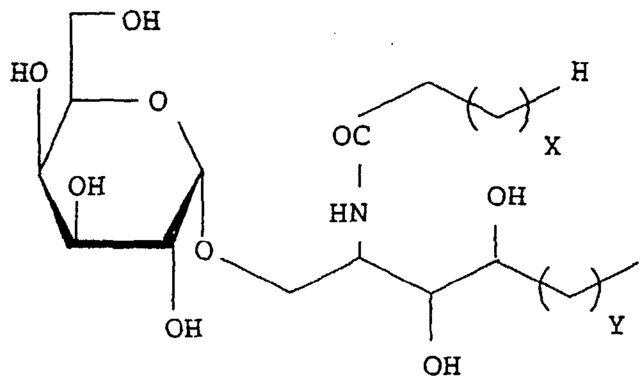

(VII)

wherein X denotes an integer of 18-26 and Y denotes an integer of 5-15.

**13.** A glycosphingolipid according to claim 12, wherein X denotes an integer of 21-25 and Y denotes an integer of 6-14.

**14.** A glycosphingolipid according to claim 12, which is represented by the formula (VIII):

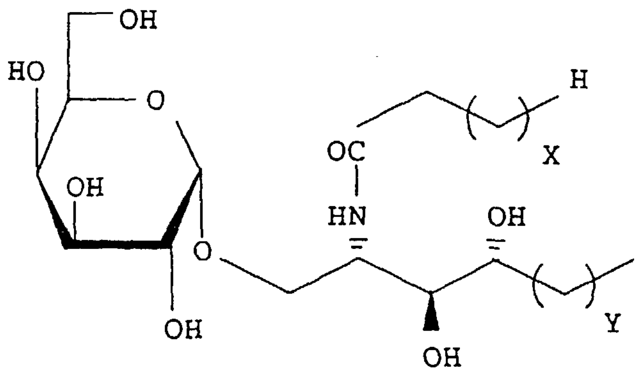

(VIII)

wherein X denotes an integer of 18-26 and Y denotes an integer of 5-15.

**15.** A glycosphingolipid according to claim 14, wherein X denotes an integer of 21-25 and Y denotes an integer of 6-14.

**16.** A glycosphingolipid according to claim 2, which is represented by the formula (IX):

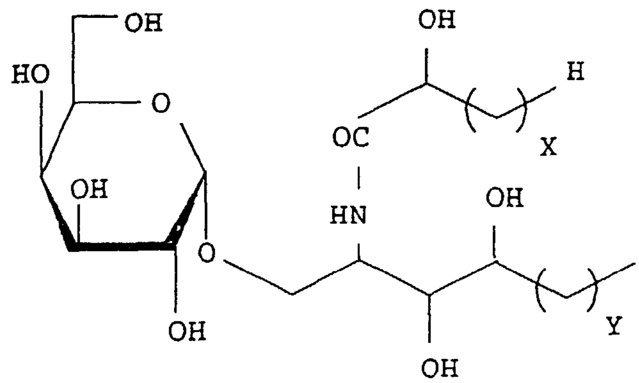

(IX)

wherein X denotes an integer of 18-26 and Y denotes an integer of 5-17.

**17.** A glycosphingolipid according to claim 16, wherein X denotes an integer of 21-25 and Y denotes an integer of 6-16.

**18.** A glycosphingolipid according to claim 16, which is represented by the formula (X):

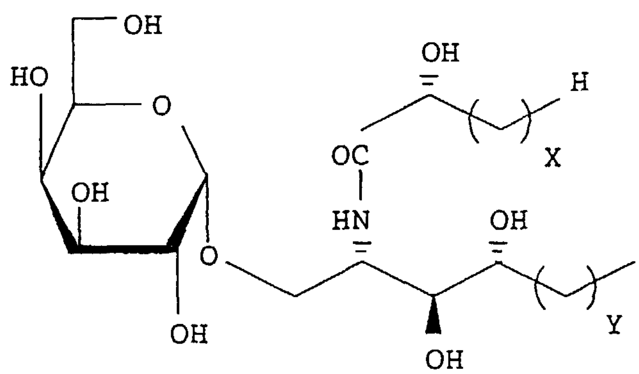

(X)

wherein X denotes an integer of 18-26 and Y denotes an integer of 5-17.

**19.** A glycosphingolipid according to claim 16, which is represented by the formula (X'):

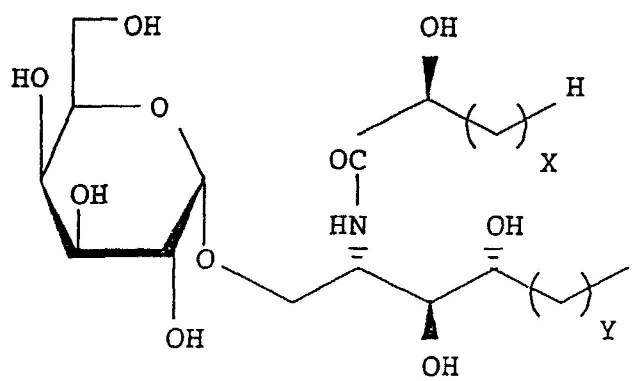

(X')

wherein X denotes an integer of 20-24 and Y denotes an integer of 10-14.

**20.** A glycosphingolipid according to claim 18, wherein X denotes an integer of 21-25 and Y denotes an integer of 6-16.

**21.** A glycosphingolipid according to claim 19, wherein X denotes an integer of 21-23 and Y denotes an integer of 11-13.

**22.** A glycosphingolipid according to claim 2, which is represented by the formula (XI):

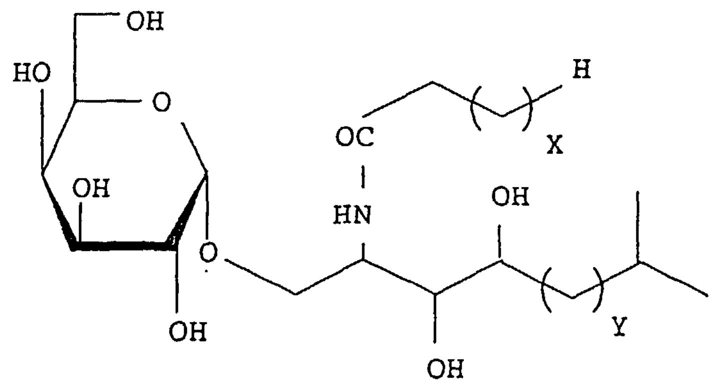

(XI)

wherein X denotes an integer of 20-24 and Y denotes an integer of 9-13.

**23.** A glycosphingolipid according to claim 22, wherein X denotes an integer of 21-23 and Y denotes an integer of 10-12.

**24.** A glycosphingolipid according to claim 22, which is represented by the formula (XII):

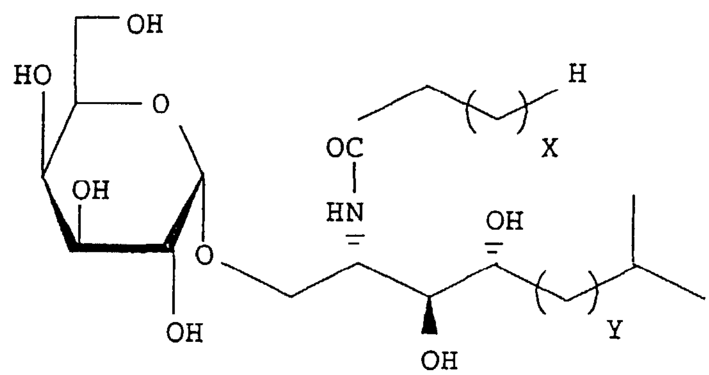

(XII)

wherein X denotes an integer of 20-24 and Y denotes an integer of 9-13.

**25.** A glycosphingolipid according to claim 24, wherein X denotes an integer of 21-23 and Y denotes an integer of 10-12.

**26.** A glycosphingolipid according to claim 2, which is represented by the formula (XIII):

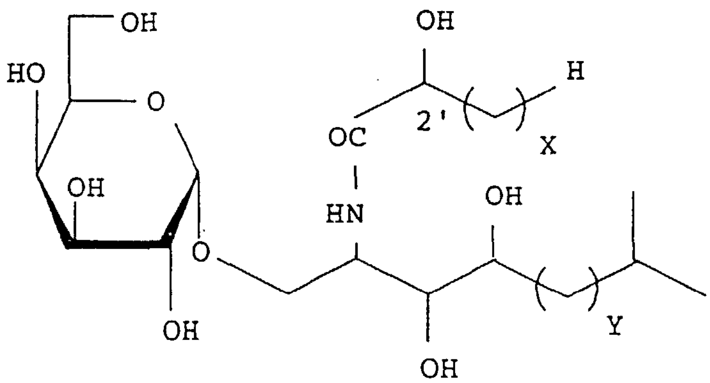

(XIII)

wherein X denotes an integer of 18-24 and Y denotes an integer of 9-13.

**27.** A glycosphingolipid according to claim 26, wherein X denotes an integer of 20-23 and Y denotes an integer of 10-12.

**28.** A glycosphingolipid according to claim 26, which is represented by the formula (XIV):

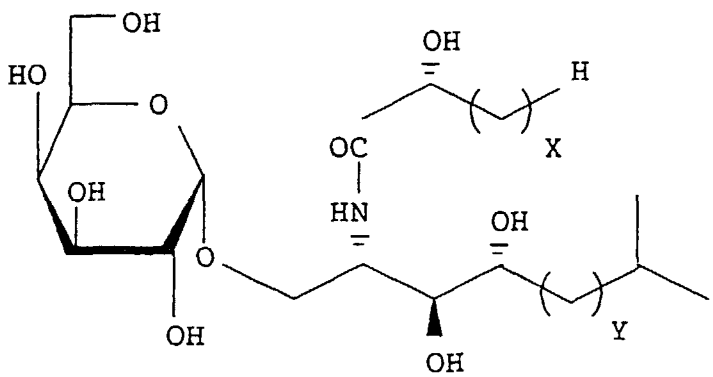

(XIV)

wherein X denotes an integer of 19-23 and Y denotes an integer of 9-13.

**29.** A glycosphingolipid according to claim 26, which is represented by the formula (XIV'):

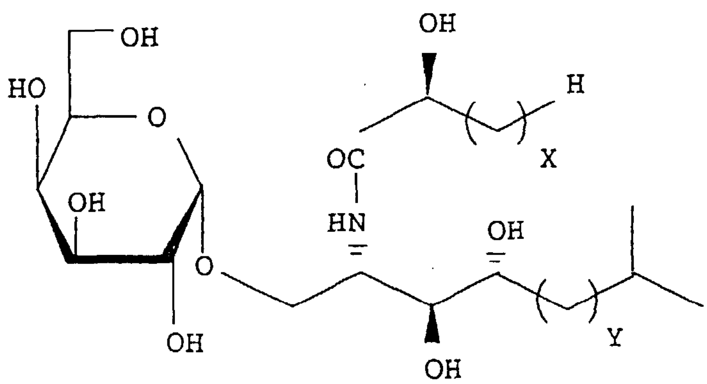

(XIV')

wherein X denotes an integer of 20-24 and Y denotes an integer of 9-13.

**30.** A glycosphingolipid according to claim 28, wherein X denotes an integer of 20-22 and Y denotes an integer of 10-12.

**31.** A glycosphingolipid according to claim 29, wherein X denotes an integer of 21-23 and Y denotes an integer of 10-12.

**32.** A glycosphingolipid according to claim 2, which is represented by the formula (XV):

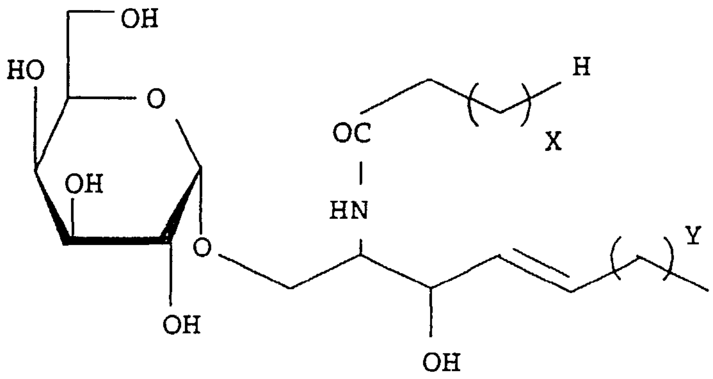

(XV)

wherein X denotes an integer of 10-18 and Y denotes an integer of 10-14.

**33.** A glycosphingolipid according to claim 32, wherein X denotes an integer of 11-17 and Y denotes an integer of 11-13.

**34.** A glycosphingolipid according to claim 32, which is represented by the formula (XVI):

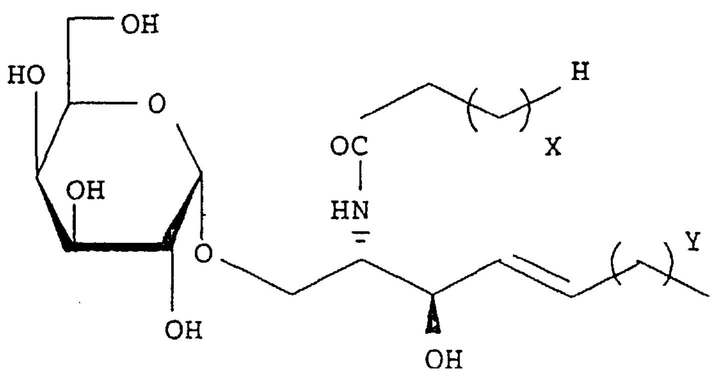

(XVI)

wherein X denotes an integer of 10-18 and Y denotes an integer of 10-14.

**35.** A glycosphingolipid according to claim 34, wherein X denotes an integer of 11-17 and Y denotes an integer of 11-13.

**36.** A glycosphingolipid according to claim 2, which is represented by the formula (XVII):

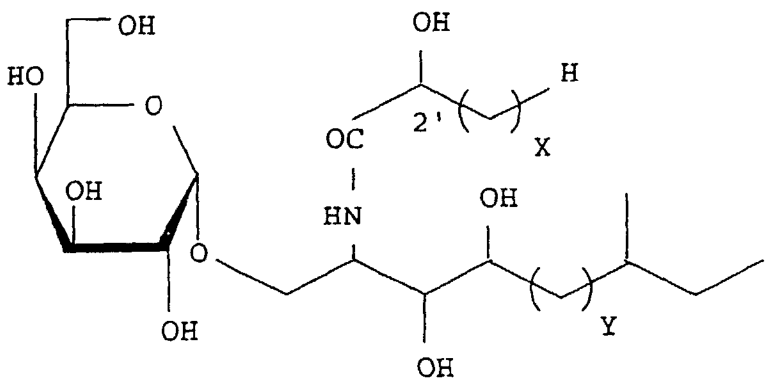

(XVII)

wherein X denotes an integer of 21-25 and Y denotes an integer of 9-13.

37. A glycosphingolipid according to claim 36, wherein X denotes an integer of 22-24 and Y denotes an integer of 10-12.

38. A glycosphingolipid according to claim 36, which is represented by the formula (XVIII):

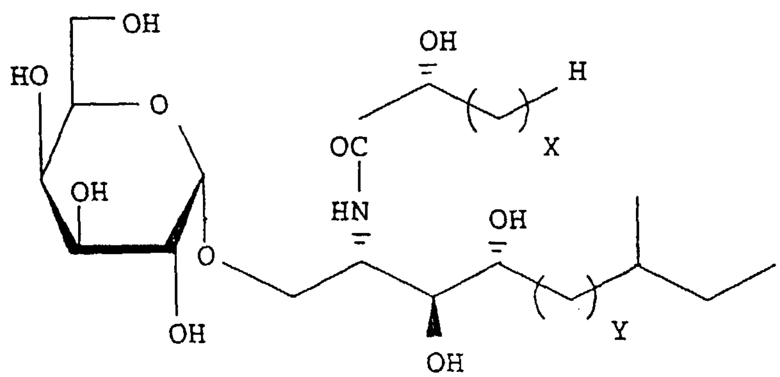

(XVIII)

wherein X denotes an integer of 21-25 and Y denotes an integer of 9-13.

39. A glycosphingolipid according to claim 38, wherein X denotes an integer of 22-24 and Y denotes an integer of 10-12.

40. A glycosphingolipid according to claim 1, which is represented by the formula (XIX):

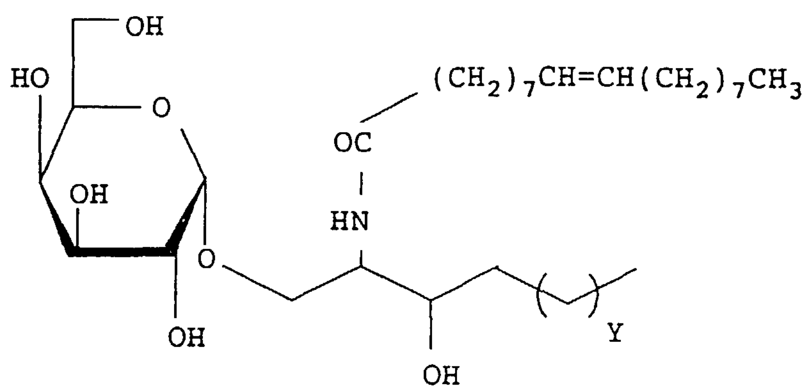

(XIX)

wherein Y denotes an integer of 11-15.

41. A glycosphingolipid according to claim 40, wherein Y denotes an integer of 12-14.

42. A glycosphingolipid according to claim 40, which is represented by the formula (XX):

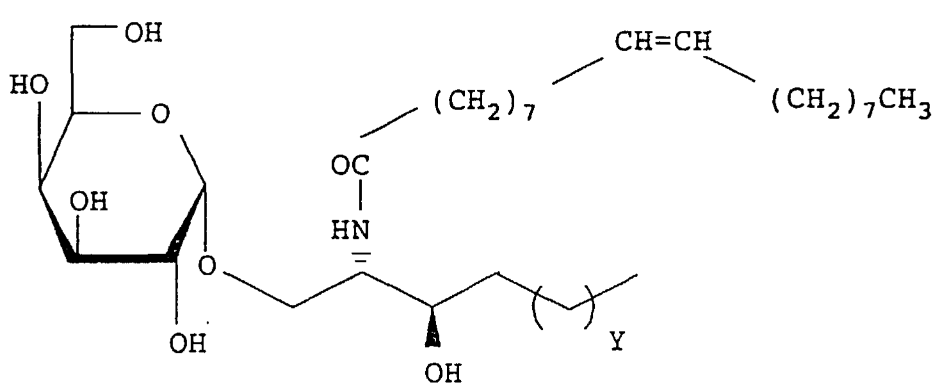

(XX)

wherein Y denotes an integer of 11-15.

**43.** A glycosphingolipid according to claim 42, wherein Y denotes an integer of 12-14.

**44.** A glycosphingolipid according to claim 1, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-octanoylamino-3-octadecanol,
(8) (2S,3R)-2-acetamino-1-(α-D-galactopyranosyloxy)-3-octadecanol,
(9) (2S,3R)-1-(α-D-galactopyranosyloxy)-2-tetracosanoylamino-3-tetradecanol,
(10) (2S,3R)-1-(α-D-galactopyranosyloxy)-2-tetradecanoylamino-3-hexadecanol,
(11) (2R,3S)-1-(α-D-galactopyranosyloxy)-2-tetradecanoylamino-3-hexadecanol,
(12) (2S, 3S)-1-(α-D-galactopyranosyloxy)-2-tetradecanoylamino-3-hexadecanol,
(13) (2R,3R)-1-(α-D-galactopyranosyloxy)-2-tetradecanoylamino-3-hexadecanol,
(14) (2S,3R)-1-(α-D-galactopyranosyloxy)-2-[(R)-2-hydroxytetracosanoylamino]-3-octadecanol,
(15) (2S,3R,4E)-1-(α-D-galactopyranosyloxy)-2-octadecanoylamino-4-octadecen-3-ol,
(16) (2S,3R,4E)-1-(α-D-galactopyranosyloxy)-2-tetradecanoylamino-4-octadecen-3-ol,
(17) (2S,3S,4R)-1-(α-D-galactopyranosyloxy)-2-tetracosanoylamino-3,4-octadecanediol,
(18) (2S,3S,4R)-1-(α-D-galactopyranosyloxy)-2-tetracosanoylamino-3,4-heptadecanediol,
(19) (2S,3S,4R)-1-(α-D-galactopyranosyloxy)-2-tetracosanoylamino-3,4-pentadecanediol,
(20) (2S,3S,4R)-1-(α-D-galactopyranosyloxy)-2-tetracosanoylamino-3,4-undecanediol,
(21) (2S,3S,4R)-1-(α-D-galactopyranosyloxy)-2-hexacosanoylamino-3,4-heptadecanediol,
(22) (2S,3S,4R)-1-(α-D-galactopyranosyloxy)-2-[(R)-2-hydroxytetracosanoylamino]-3,4-octadecanediol,
(23) (2S,3S,4R)-1-(α-D-galactopyranosyloxy)-2-[(R)-2-hydroxytetracosanoylamino]-3,4-heptadecanediol,
(24) (2S,3S,4R)-1-(α-D-galactopyranosyloxy)-2-[(R)-2-hydroxytetracosanoylamino]-3,4-pentadecanediol,
(25) (2S,3S,4R)-1-(α-D-galactopyranosyloxy)-2-[(R)-2-hydroxytetracosanoylamino]-3,4-undecanediol,
(26) (2S,3S,4R)-1-(α-D-galactopyranosyloxy)-2-[(R)-2-hydroxyhexacosanoylamino]-3,4-octadecanediol,
(27) (2S,3S,4R)-1-(α-D-galactopyranosyloxy)-2-[(R)-2-hydroxyhexacosanoylamino]-3,4-nonadecanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytetracosanoylamino]-3,4-heptadecanediol,
(30) (2S,3S,4R)-1-(α-D-galactopyranosyloxy)-2-[(R)-2-hydroxytetracosanoylamino]-3,4-hexadecanediol,
(31) (2S,3S,4R)-1-(α-D-galactopyranosyloxy)-2-[(S)-2-hydroxytetracosanoylamino]-16-methyl-3,4-heptadecanediol,
(32) (2S,3S,4R)-1-(α-D-galactopyranosyloxy)-16-methyl-2-tetracosanoylamino-3,4-heptadecanediol,
(33) (2S,3S,4R)-1-(α-D-galactopyranosyloxy)-2-[(R)-2-hydroxytricosanoylamino]-16-methyl-3,4-heptadecanediol,
(34) (2S,3S,4R)-1-(α-D-galactopyranosyloxy)-2-[(R)-2-hydroxypentacosanoylamino]-16-methyl-3,4-octadecanediol, and
(35) (2S,3R)-1-(α-D-galactopyranosyloxy)-2-oleoylamino-3-octadecanol.

**45.** A glycosphingolipid according to claim 44, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-octanoylamino-3-octadecanol,
(8) (2S,3R)-2-acetamino-1-(α-D-galactopyranosyloxy)-3-octadecanol,
(9) (2S,3R)-1-(α-D-galactopyranosyloxy)-2-tetracosanoylamino-3-tetradecanol, and
(10) (2S,3R)-1-(α-D-galactopyranosyloxy)-2-tetradecanoylamino-3-hexadecanol.

46. A glycosphingolipid according to claim 45, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-tetracosanoylamino-3-tetradecanol, and
(8) (2S,3R)-1-(α-D-galactopyranosyloxy)-2-tetradecanoylamino-3-hexadecanol.

47. A glycosphingolipid according to claim 46, which is selected from the group consisting of the following compounds:

(1) (2S,3R)-1-(α-D-galactopyranosyloxy)-2-octadecanoylamino-3-octadecanol,
(2) (2S,3R)-1-(α-D-galactopyranosyloxy)-2-tetradecanoylamino-3-octadecanol, and
(3) (2S,3R)-1-(α-D-galactopyranosyloxy)-2-tetradecanoylamino-3-hexadecanol.

48. A glycosphingolipid according to claim 44, which is (2S,3R)-1-(α-D-galactopyranosyloxy)-2-[(R)-2-hydroxytetracosanoylamino]-3-octadecanol.

49. A glycosphingolipid according to claim 44, which is selected from the group consisting of the following compounds:

(1) (2S,3R,4E)-1-(α-D-galactopyranosyloxy)-2-octadecanoylamino-4-octadecen-3-ol, and
(2) (2S,3R,4E)-1-(α-D-galactopyranosyloxy)-2-tetradecanoylamino-4-octadecen-3-ol.

50. A glycosphingolipid according to claim 44, which is selected from the group consisting of the following compounds:

(1) (2S,3S,4R)-1-(α-D-galactopyranosyloxy)-2-tetracosanoylamino-3,4-octadecanediol,
(2) (2S,3S,4R)-1-(α-D-galactopyranosyloxy)-2-tetracosanoylamino-3,4-heptadecanediol,
(3) (2S,3S,4R)-1-(α-D-galactopyranosyloxy)-2-tetracosanoylamino-3,4-pentadecanediol,
(4) (2S,3S,4R)-1-(α-D-galactopyranosyloxy)-2-tetracosanoylamino-3,4-undecanediol, and
(5) (2S,3S,4R)-1-(α-D-galactopyranosyloxy)-2-hexacosanoylamino-3,4-heptadecanediol.

51. A glycosphingolipid according to claim 44, which is selected from the group consisting of the following compounds:

(1) (2S,3S,4R)-1-(α-D-galactopyranosyloxy)-2-[(R)-2-hydroxytetracosanoylamino]-3,4-octadecanediol,
(2) (2S,3S,4R)-1-(α-D-galactopyranosyloxy)-2-[(R)-2-hydroxytetracosanoylamino]-3,4-heptadecanediol,
(3) (2S,3S,4R)-1-(α-D-galactopyranosyloxy)-2-[(R)-2-hydroxytetracosanoylamino]-3,4-pentadecanediol,
(4) (2S,3S,4R)-1-(α-D-galactopyranosyloxy)-2-[(R)-2-hydroxytetracosanoylamino]-3,4-undecanediol,
(5) (2S,3S,4R)-1-(α-D-galactopyranosyloxy)-2-[(R)-2-hydroxyhexacosanoylamino]-3,4-octadecanediol,
(6) (2S,3S,4R)-1-(α-D-galactopyranosyloxy)-2-[(R)-2-hydroxyhexacosanoylamino]-3,4-nonadecanediol,
(7) (2S,3S,4R)-1-(α-D-galactopyranosyloxy)-2-[(R)-2-hydroxyhexacosanoylamino]-3,4-icosanediol,
(8) (2S,3S,4R)-1-(α-D-galactopyranosyloxy)-2-[(S)-2-hydroxytetracosanoylamino]-3,4-heptadecanediol, and
(9) (2S,3S,4R)-1-(α-D-galactopyranosyloxy)-2-[(R)-2-hydroxytetracosanoylamino]-3,4-hexadecanediol.

52. A glycosphingolipid according to claim 44, which is selected from the group consisting of the following compounds:

(1) (2S,3S,4R)-1-(α-D-galactopyranosyloxy)-2-[(S)-2-hydroxytetracosanoylamino]-16-methyl-3,4-heptadecanediol,
(2) (2S,3S,4R)-1-(α-D-galactopyranosyloxy)-16-methyl-2-tetracosanoylamino-3,4-heptadecanediol, and
(3) (2S,3S,4R)-1-(α-D-galactopyranosyloxy)-2-[(R)-2-hydroxytricosanoylamino]-16-methyl-3,4-heptadecanediol.

53. A glycosphingolipid according to claim 44, which is (2S,3S,4R)-1-(α-D-galactopyranosyloxy)-2-[(R)-2-hydroxypentacosanoylamino]-16-methyl-3,4-octadecanediol.

54. A glycosphingolipid according to claim 44, which is (2S,3R)-1-(α-D-galactopyranosyloxy)-2-oleoylamino-3-octandecanol.

**55.** A process for a glycosphingolipid comprising collecting the marine sponge Agelas mauritianus, subjecting it to an extraction operation with an organic solvent and isolating from the extract at least one of the following glycosphingolipids represented by the formula (I) according to claim 2:

(1) (2S,3S,4R)-1-(α-D-galactopyranosyloxy)-2-[(R)-2-hydroxytetracosanoylamino]-3,4-heptadecanediol,
(2) (2S,3S,4R)-1-(α-D-galactopyranosyloxy)-2-[(R)-2-hydroxytetracosanoylamino]-3,4-hexadecanediol,
(3) (2S,3S,4R)-1-(α-D-galactopyranosyloxy)-2-[(R)-2-hydroxytricosanoylamino]-16-methyl-3,4-heptadecanediol, and
(4) (2S,3S,4R)-1-(α-D-galactopyranosyloxy)-2-[(R)-2-hydroxypentacosanoylamino]-16-methyl-3,4-octadecanediol.

**56.** An antitumor agent comprising one or more of the compounds according to claim 1 as effective ingredients.

**57.** An immunostimulator comprising one or more of the compounds according to claim 1 as effective ingredients.

**58.** An antitumor agent comprising an effective amount of one or more of the compounds according to claim 1 and a carrier or diluent.

**59.** An immunostimulator comprising an effective amount of one or more of the compounds according to claim 1 and a carrier or diluent.

**60.** The use of one or more of the compounds according to claim 1 in the preparation of an antitumor agent.

**61.** The use of one or more of the compounds according to claim 1 in the preparation of an immunostimulating medicament.

**Claims for the following Contracting State : ES**

**1.** A process for preparing a glycosphingolipid including an alpha-galactose residue represented by the formula (A):

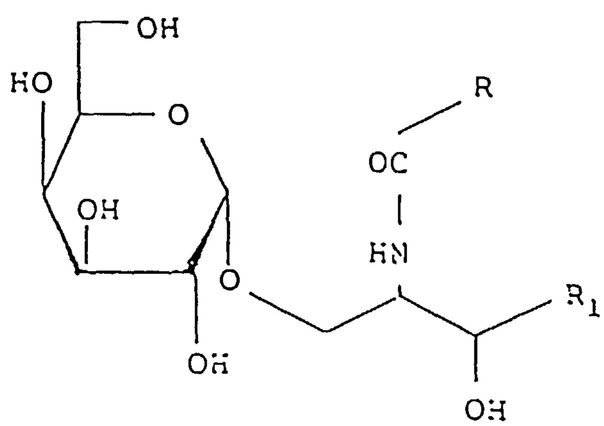

(A)

wherein

R represents

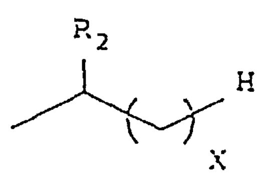

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17,

which comprises one of the following methods:

a) extracting (A) from a species of the marine sponge Algelas mauretianus with at least one appropriate organic solvent and water;
b) modifying sphingosine; and
c) general chemical reactions for the synthesis of glycosphingolipids.

**2.** A process according to claim 1, wherein a glycosphingolipid represented by the formula (I) is prepared:

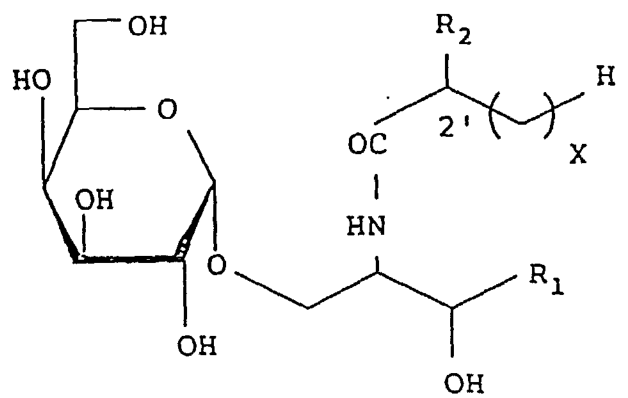

(I)

wherein

$R_1$ represents any one of the substituents defined by (a)-(e) below and $R_2$ represents H or OH (X is defined in the following (a)-(e)):

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

3. A process according to claim 2, wherein a glycosphingolipid represented by the formula (II) is prepared:

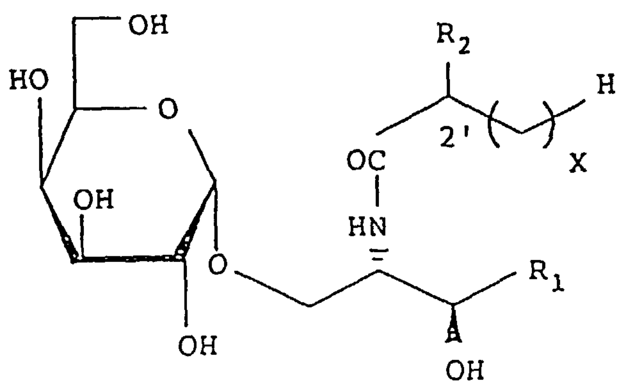

(II)

wherein

$R_1$ represents any one of the substituents defined by (a)-(e) below and $R_2$ represents H or OH (X is defined in the following (a)-(e));

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH=CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**4.** A process according to claim 2, wherein a glycosphingolipid represented by the formula (III) is prepared:

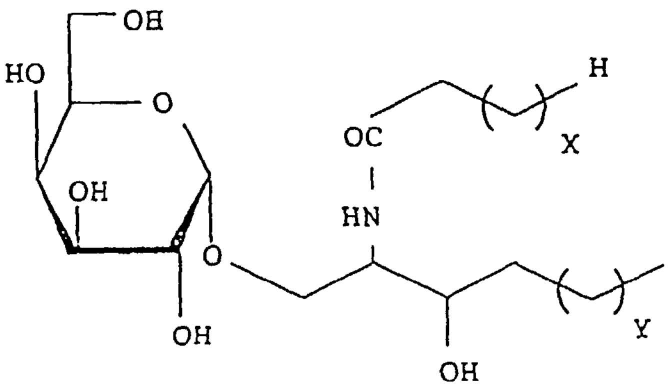

(III)

wherein X denotes an integer of 0-24 and Y denotes an integer of 7-15.

**5.** A process according to claim 4, wherein X denotes an integer of 8-22 and Y denotes an integer of 9-13.

**6.** A process according to claim 4, wherein a glycosphingolipid represented by the formula (IV) is prepared:

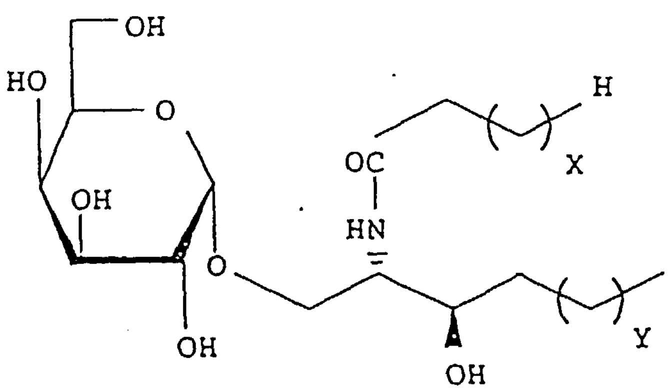

(IV)

wherein X denotes an integer of 0-24 and Y denotes an integer of 7-15.

**7.** A process according to claim 6, wherein X denotes an integer of 8-22 and Y denotes an integer of 9-13.

**8.** A process according to claim 2, wherein a glycosphingolipid represented by the formula (V) is prepared:

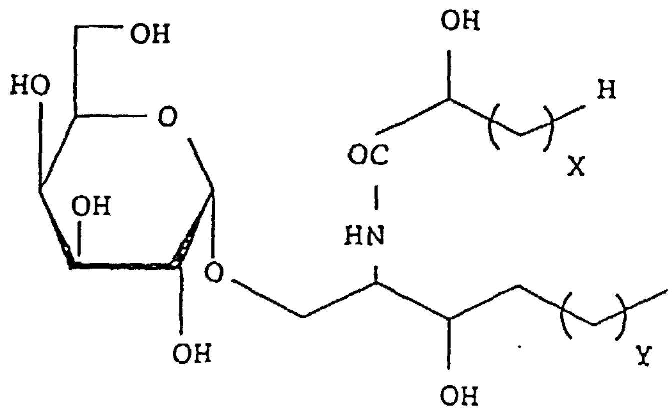

(V)

wherein X denotes an integer of 20-24 and Y denotes an integer of 11-15.

**9.** A process ccording to claim 8, wherein X denotes an integer of 21-23 and Y denotes an integer of 12-14.

**10.** A process according to claim 8, wherein a glycosphingolipid represented by the formula (VI) is prepared:

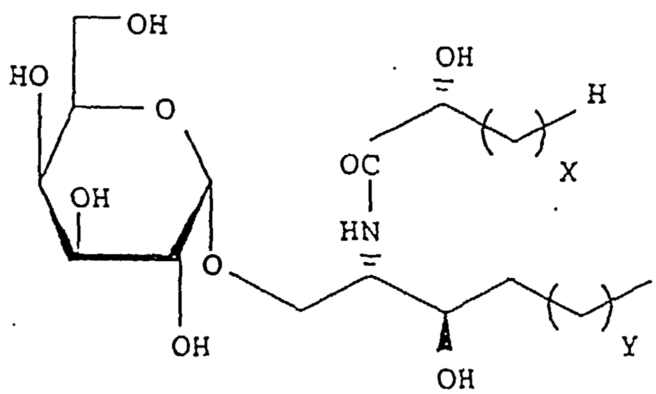

(VI)

wherein X denotes an integer of 20-24 and Y denotes an integer of 11-15.

**11.** A process according to claim 10, wherein X denotes an integer of 21-23 and Y denotes an integer of 12-14.

**12.** A process according to claim 2, wherein a glycosphingolipid represented by the formula (VII) is prepared:

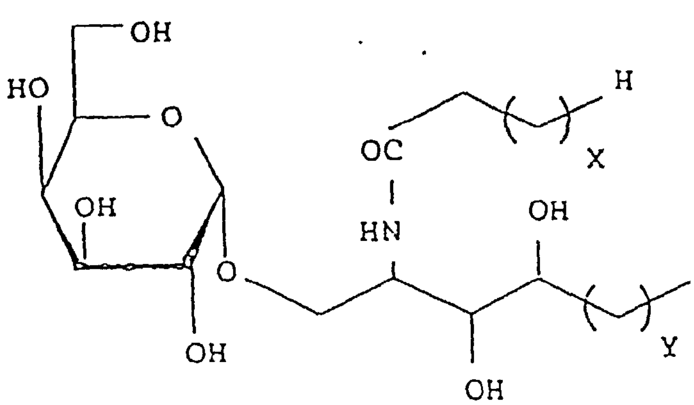

(VII)

wherein X denotes an integer of 18-26 and Y denotes an integer of 5-15.

**13.** A process according to claim 12, wherein X denotes an integer of 21-25 and Y denotes an integer of 6-14.

**14.** A process according to claim 12, wherein a glycosphingolipid represented by the formula (VIII) is prepared:

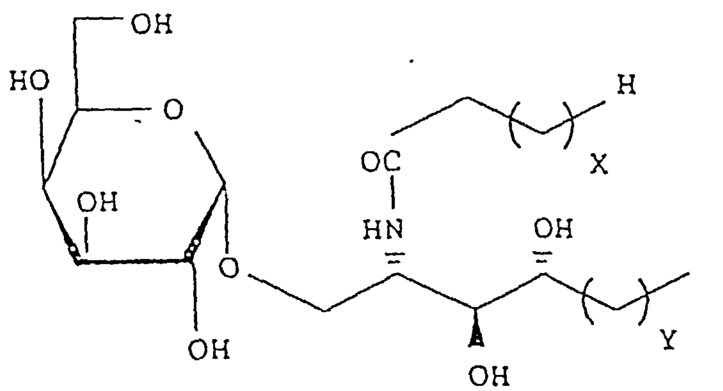

(VIII)

wherein X denotes an integer of 18-26 and Y denotes an integer of 5-15.

**15.** A process according to claim 14, wherein X denotes an integer of 21-25 and Y denotes an integer of 6-14.

**16.** A process according to claim 2, wherein a glycosphingolipid represented by the formula (IX) is prepared:

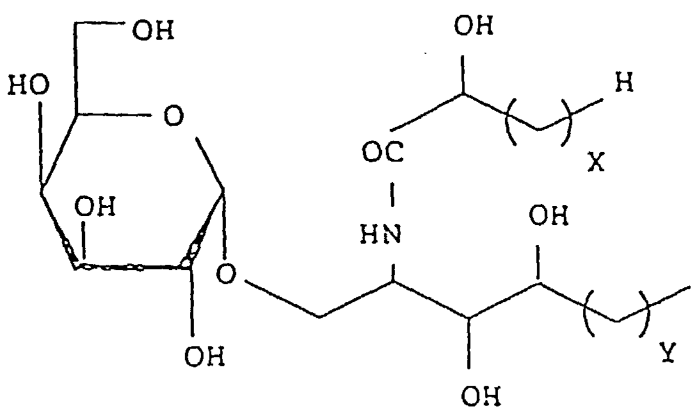

(IX)

wherein X denotes an integer of 18-26 and Y denotes an integer of 5-17.

**17.** A process according to claim 16, wherein X denotes an integer of 21-25 and Y denotes an integer of 6-16.

**18.** A process according to claim 16, wherein a glycosphingolipid represented by the formula (X) is prepared:

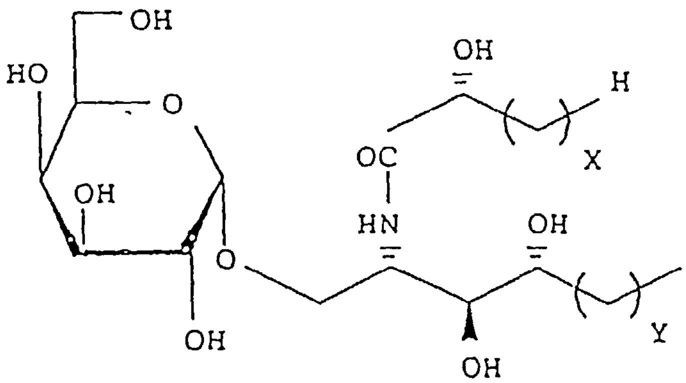

(X)

wherein X denotes an integer of 18-26 and Y denotes an integer of 5-17.

**19.** A process according to claim 16, wherein a glycosphingolipid represented by the formula (X') is prepared:

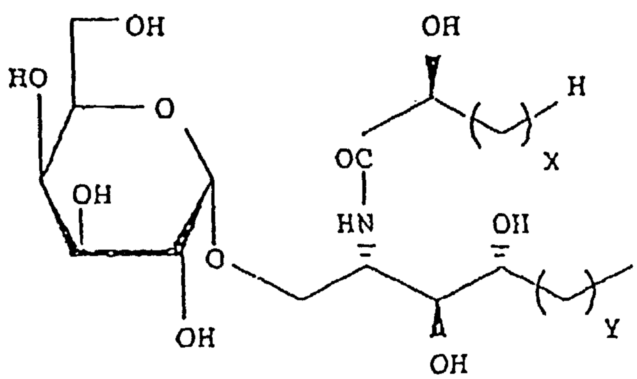

(X')

wherein X denotes an integer of 20-24 and Y denotes an integer of 10-14.

**20.** A process according to claim 18,
wherein X denotes an integer of 21-25 and Y denotes an integer of 6-16.

**21.** A process according to claim 19,
wherein X denotes an integer of 21-23 and Y denotes an integer of 11-13.

**22.** A process according to claim 2, wherein a glycosphingolipid represented by the formula (XI) is prepared:

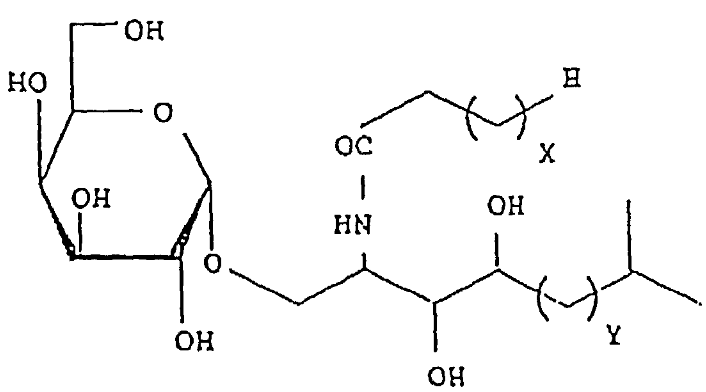

(XI)

wherein X denotes an integer of 20-24 and Y denotes an interger of 9-13.

**23.** A process according to claim 22,
wherein X denotes an integer of 21-23 and Y denotes an integer of 10-12.

**24.** A process according to claim 22, wherein a glycosphingolipid represented by the formula (XII) is prepared:

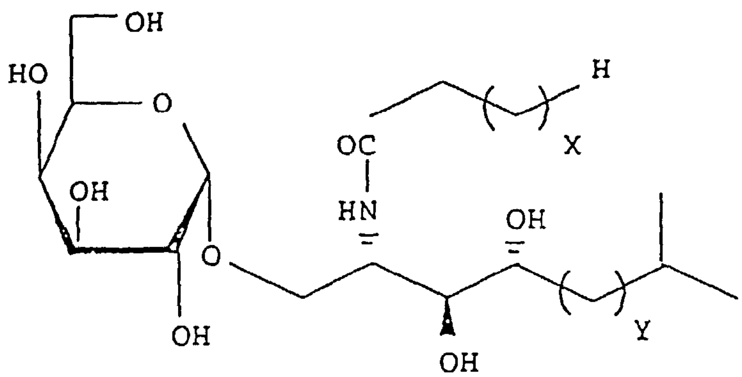

(XII)

wherein X denotes an integer of 20-24 and Y denotes an integer of 9-13.

**25.** A process according to claim 24,
wherein X denotes an integer of 21-23 and Y denotes an integer of 10-12.

**26.** A process according to claim 2, wherein a glycosphingolipid represented by the formula (XIII) is prepared:

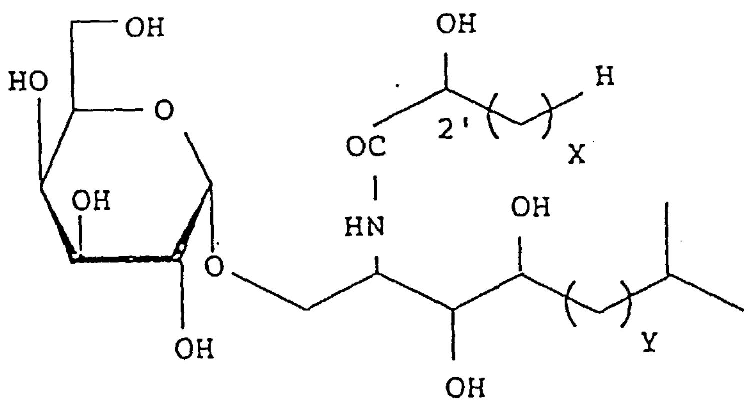

(XIII)

wherein X denotes an integer of 18-24 and Y denotes an integer of 9-13.

**27.** A process according to claim 26,
wherein X denotes an integer of 20-23 and Y denotes an integer of 10-12.

**28.** A process according to claim 26, wherein a glycosphingolipid represented by the formula (XIV) is prepared:

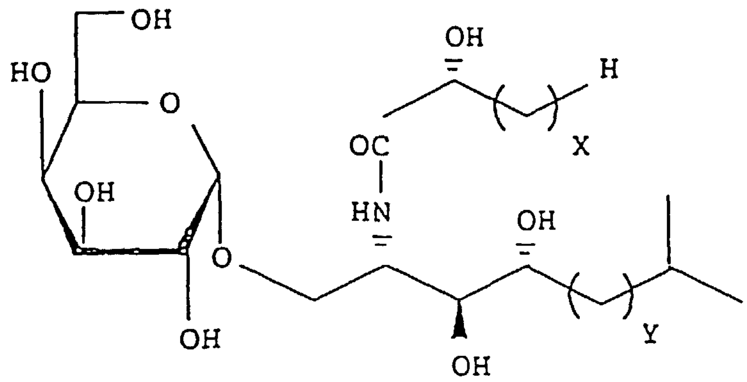

(XIV)

wherein X denotes an integer of 19-23 and Y denotes an integer of 9-13.

**29.** A process according to claim 26, wherein a glycosphingolipid represented by the formula (XIV') is prepared:

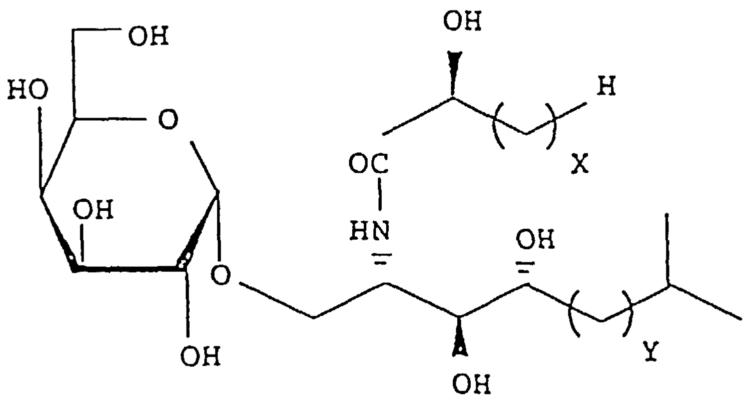

(XIV')

wherein X denotes an integer of 20-24 and Y denotes an integer of 9-13.

**30.** A process according to claim 28, wherein X denotes an integer of 20-22 and Y denotes an integer of 10-12.

**31.** A process according to claim 29, wherein X denotes an integer of 21-23 and Y denotes an integer of 10-12.

**32.** A process according to claim 2, wherein a glycosphingolipid represented by the formula (XV) is prepared:

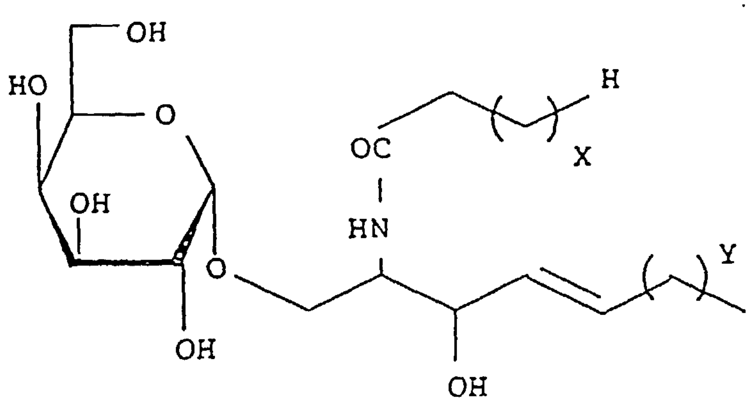

(XV)

wherein X denotes an integer of 10-18 and Y denotes an integer of 10-14.

**33.** A process according to claim 32, wherein X denotes an integer of 11-17 and Y denotes an integer of 11-13.

**34.** A process according to claim 32, wherein a glycosphingolipid represented by the formula (XVI) is prepared:

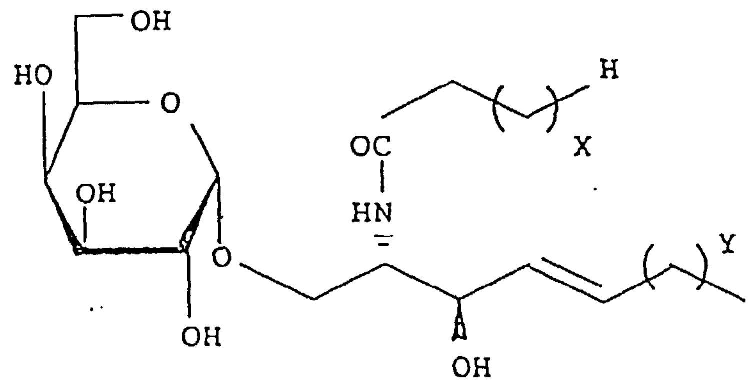

(XVI)

wherein X denotes an integer of 10-18 and Y denotes an integer of 10-14.

**35.** A process according to claim 34, wherein X denotes an integer of 11-17 and Y denotes an integer of 11-13.

**36.** A process according to claim 2, wherein a glycosphingolipid represented by the formula (XVII) is prepared:

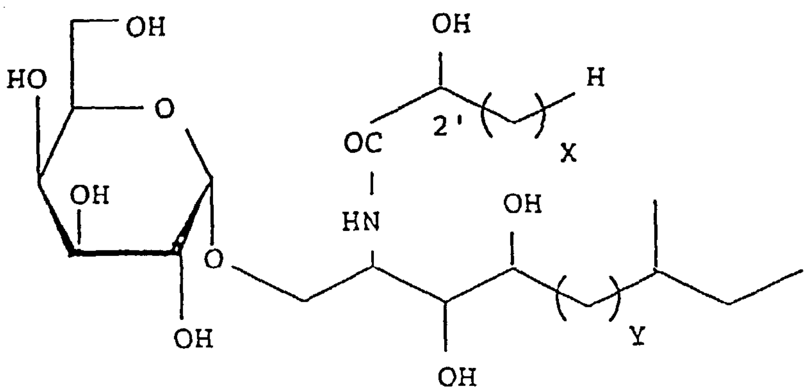

(XVII)

wherein X denotes an integer of 21-25 and Y denotes an integer of 9-13.

**37.** A process according to claim 36, wherein X denotes an integer of 22-24 and Y denotes an integer of 10-12.

**38.** A process according to claim 36, wherein a glycosphingolipid represented by the formula (XVIII) is prepared:

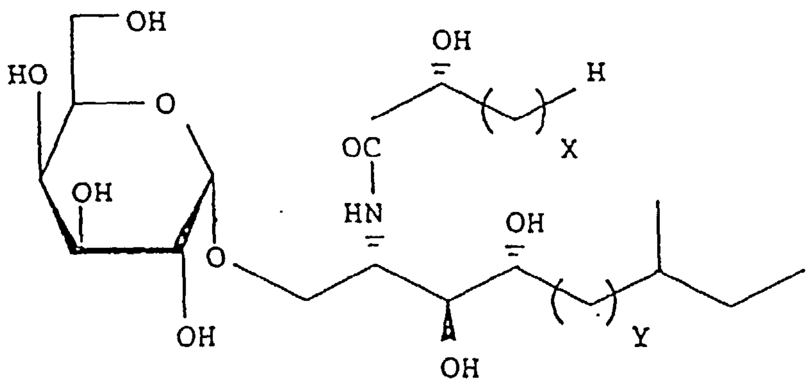

(XVIII)

wherein X denotes an integer of 21-25 and Y denotes an integer of 9-13.

**39.** A process according to claim 38, wherein X denotes an integer of 22-24 and Y denotes an integer of 10-12.

**40.** A process according to claim 1, wherein a glycosphingolipid represented by the formula (XIX) is prepared:

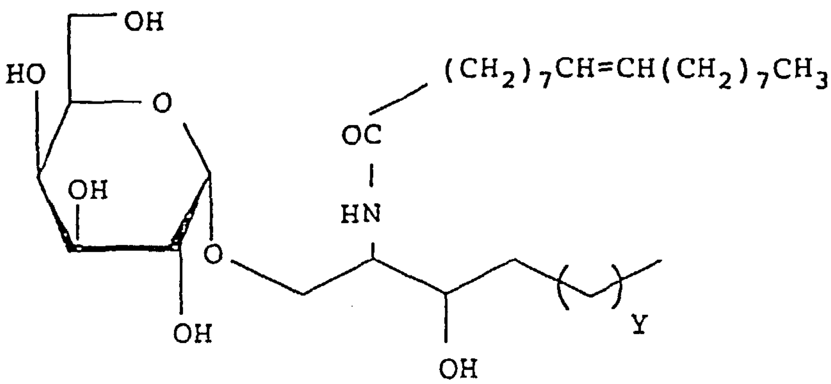

(XIX)

wherein Y denotes an integer of 11-15.

**41.** A process according to claim 40, wherein Y denotes an integer of 12-14.

**42.** A process according to claim 40, wherein a glycosphingolipid represented by the formula (XX) is prepared:

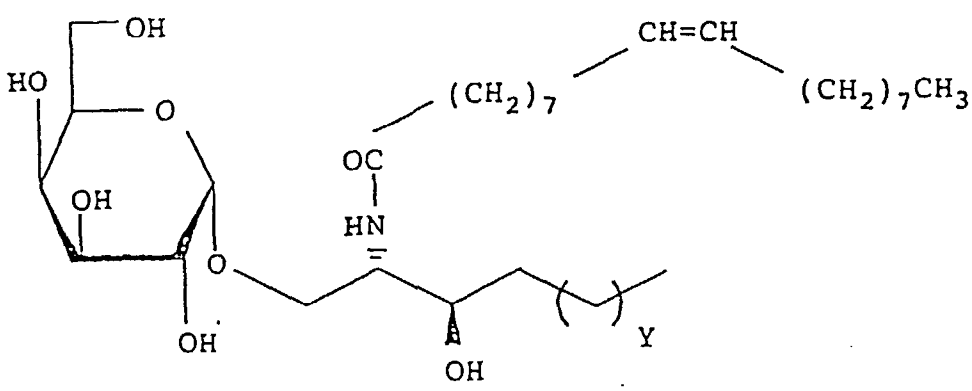

(XX)

wherein Y denotes an integer of 11-15.

**43.** A process according to claim 42, wherein Y denotes an integer of 12-14.

**44.** A process according to claim 1, wherein a glycosphingolipid selected from the group consisting of the following compounds is prepared:

(1) (2S, 3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S, 3R)-1-(α-D-galactopyranosyloxy)-2-octanoylamino-3-octadecanol,
(8) (2S,3R)-2-acetamino-1-(α-D-galactopyranosyloxy)-3-octadecanol,
(9) (2S,3R)-1-(α-D-galactopyranosyloy)-2-tetracosanoylamino-3-tetradecanol,
(10) (2S,3R)-1-(α-D-galactopyranosyloxy)-2-tetradecanoylamino-3-hexadecanol,
(11) (2R,3S)-1-(α-D-galactopyranosyloxy)-2-tetradecanoylamino-3-hexadecanol,
(12) (2S,3S)-1-(α-D-galactopyranosyloxy)-2-tetradecanoylamino-3-hexadecanol,
(13) (2R,3R)-1-(α-D-galactopyranosyloxy)-2-tetradecanoylamino-3-hexadecanol,
(14) (2S,3R)-1-(α-D-galactopyranosyloxy)-2-[(R)-2-hydroxytetracosanoylamino]-3-octadecanol,
(15) (2S,3R,4E)-1-(α-D-galactopyranosyloxy)-2-octadecanoylamino-4-octadecen-3-ol,
(16) (2S,3R,4E)-1-(α-D-galactopyranosyloxy)-2-tetradecanoylamino-4-octadecen-3-ol,
(17) (2S,3S,4R)-1-(α-D-galactopyranosyloxy)-2-tetracosanoylamino-3,4-octadecanediol,
(18) (2S,3S,4R)-1-(α-D-galactopyranosyloxy)-2-tetracosanoylamino-3,4-heptadecanediol,
(19) (2S,3S,4R)-1-(α-D-galactopyranosyloxy)-2-tetracosanoylamino-3,4-pentadecanediol,
(20) (2S,3S,4R)-1-(α-D-galactopyranosyloxy)-2-tetracosanoylamino-3,4-undecanediol,
(21) (2S,3S,4R)-1-(α-D-galactopyranosyloxy)-2-hexacosanoylamino-3,4-heptadecanediol,
(22) (2S,3S,4R)-1-(α-D-galactopyranosyloxy)-2-[(R)-2-hydroxytetracosanoylamino]-3,4-octadecanediol,
(23) (2S,3S,4R)-1-(α-D-galactopyranosyloxy)-2-[(R)-2-hydroxytetracosanoylamino]-3,4-heptadecanediol,
(24) (2S,3S,4R)-1-(α-D-galactopyranosyloxy)-2-[(R)-2-hydroxytetracosanoylamino]-3,4-pentadecanediol,
(25) (2S,3S,4R)-1-(α-D-galactopyranosyloxy)-2-[(R)-2-hydroxytetracosanoylamino]-3,4-undecanediol,
(26) (2S,3S,4R)-1-(-α-D-galactopyranosyloxy)-2-[(R)-2-hydroxyhexacosanoylamino]-3,4-octadecanediol,
(27) (2S,3S,4R)-1-(α-D-galactopyranosyloxy)-2-[(R)-2-hydroxyhexacosanoylamino]-3,4-nonadecanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytetracosanoylamino]-3,4-heptadecanediol,
(30) (2S,3S,4R)-1-(α-D-galactopyranosyloxy)-2-[(R)-2-hydroxytetracosanoylamino]-3,4-hexadecanediol,
(31) (2S,3S,4R)-1-(α-D-galactopyranosyloxy)-2-[(S)-2-hydroxytetracosanoylamino]-16-methyl-3,4-heptadecanediol,
(32) (2S,3S,4R)-1-(α-D-galactopyranosyloxy)-16-methyl-2-tetracosanoylamino-3,4-heptadecanediol,
(33) (2S,3S,4R)-1-(α-D-galactopyranosyloxy)-2-[(R)-2-hydroxytricosanoylamino]-16-methyl-3,4-heptadecanediol,
(34) (2S,3S,4R)-1-(α-D-galactopyranosyloxy)-2-[(R)-2-hydroxypentacosanoylamino]-16-methyl-3,4-octadecanediol, and
(35) (2S,3R)-1-(α-D-galactopyranosyloxy)-2-oleoylamino-3-octadecanol.

**45.** A process for a glycosphingolipid comprising collecting the marine sponge Agelas mauritianus, subjecting it to an extraction operation with an organic solvent and isolating from the extract at least one of the following glycosphingolipids represented by the formula (I) according to claim 2:

(1) (2S,3S,4R)-1-(α-D-galactopyranosyloxy)-2-[(R)-2-hydroxytetracosanoylamino]-3,4-heptadecanediol,
(2) (2S,3S,4R)-1-(α-D-galactopyranosyloxy)-2-[(R)-2-hydroxytetracosanoylamino]-3,4-hexadecanediol,
(3) (2S,3S,4R)-1-(α-D-galactopyranosyloxy)-2-[(R)-2-hydroxytricosanoylamino]-16-methyl-3,4-heptadecanediol, and
(4) (2S,3S,4R)-1-(α-D-galactopyranosyloxy)-2-[(R)-2-hydroxypentacosanoylamino]-16-methyl-3,4-octadecanediol.

**46.** Process for preparing an antitumor agent comprising the mixing of one or more compounds represented by the formula (A)

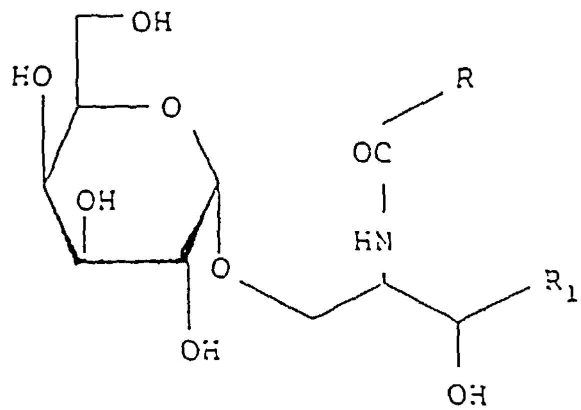

(A)

wherein

R represents

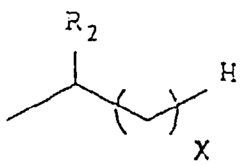

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17

as effective ingredients with pharmaceutically acceptable additives.

**47.** Process for preparing an immunostimulator comprising the mixing of one or more compounds represented by the formula (A)

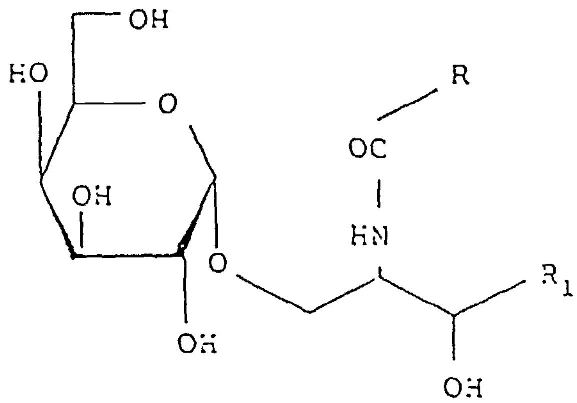

(A)

wherein

R represents

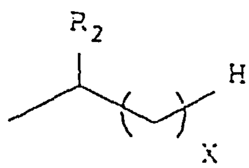

,

where $R_2$ represents H or OH and X denotes an integer of 0-26, or $-(CH_2)_7CH{=}CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$,

and

(e) $-CH(OH)(CH_2)_YCH(CH_3)CH_2CH_3$,

wherein Y denotes an integer of 5-17

as effective ingredients with pharmaceutically acceptable additives.

**48.** The use of one or more compounds represented by the formula (A)

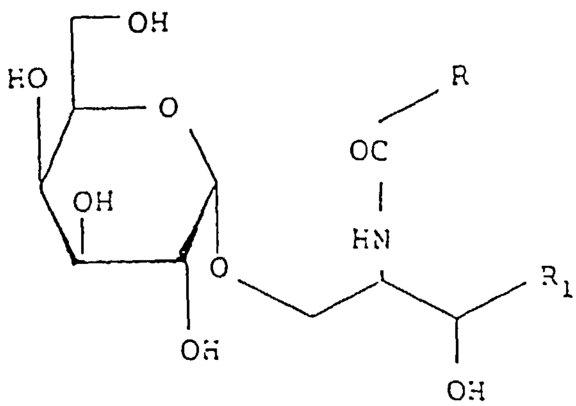

(A)

wherein

R represents

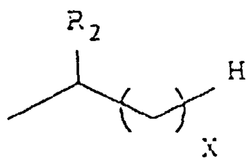

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17

in the preparation of an antitumor agent.

**49.** The use of one or more compounds represented by the formula (A)

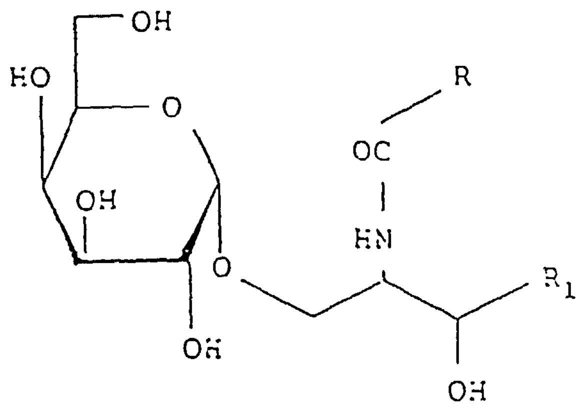

(A)

wherein

R represents

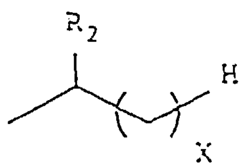

,

where $R_2$ represents H or OH and X denotes an integer of 0-26, or $-(CH_2)_7CH{=}CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$,

and

(e) $-CH(OH)(CH_2)_YCH(CH_3)CH_2CH_3$,

wherein Y denotes an integer of 5-17

in the preparation of an immunostimulating medicament.

**Claims for the following Contracting State : GR**

1. A glycosphingolipid including an alpha-galactose residue represented by the formula (A):

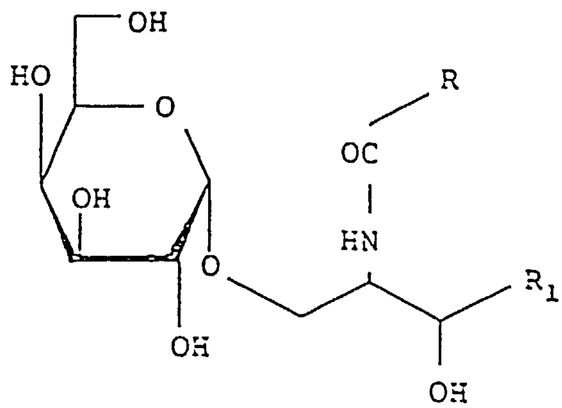

(A)

wherein

R represents

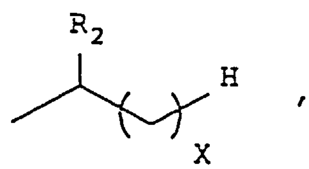

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17.

**2.** A glycosphingolipid according to claim 1, which is represented by the formula (I):

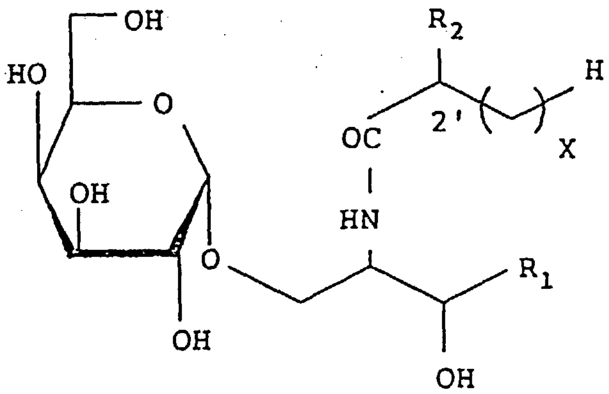

(I)

wherein

$R_1$ represents any one of the substituents defined by (a)-(e) below and $R_2$ represents H or OH (X is defined in the following (a)-(e)):

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**3.** A glycosphingolipid according to claim 2, which is represented by the formula (II):

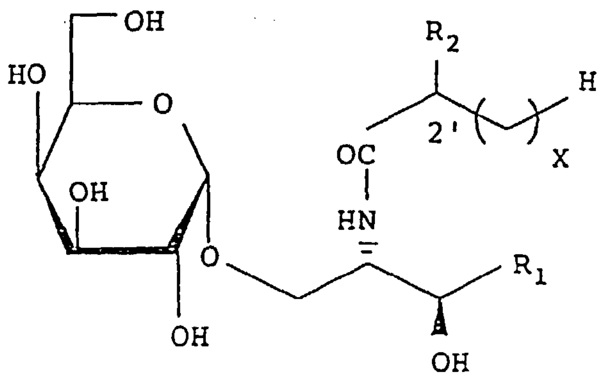

(II)

wherein

$R_1$ represents any one of the substituents defined by (a)-(e) below and $R_2$ represents H or OH (X is defined in the following (a)-(e));

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**4.** A glycosphingolipid according to claim 2, which is represented by the formula (III):

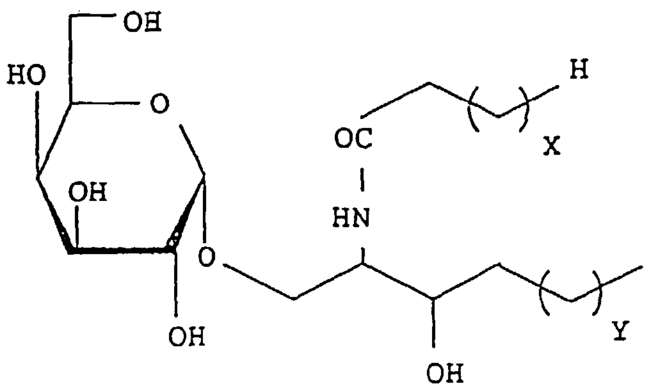

(III)

wherein X denotes an integer of 0-24 and Y denotes an integer of 7-15.

5. A glycosphingolipid according to claim 4, wherein X denotes an integer of 8-22 and Y denotes an integer of 9-13.

6. A glycosphingolipid according to claim 4, which is represented by the formula (IV):

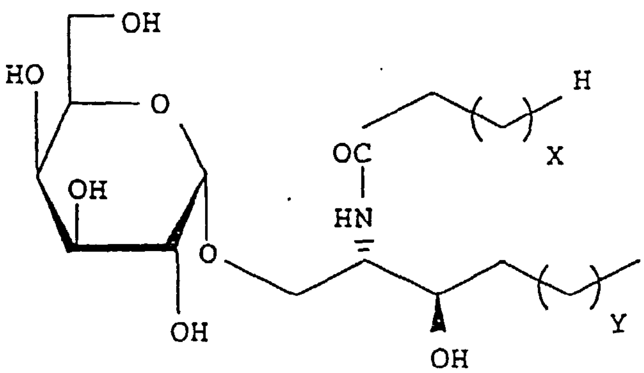

(IV)

wherein X denotes an integer of 0-24 and Y denotes an integer of 7-15.

7. A glycosphingolipid according to claim 6, wherein X denotes an integer of 8-22 and Y denotes an integer of 9-13.

8. A glycosphingolipid according to claim 2, which is represented by the formula (V):

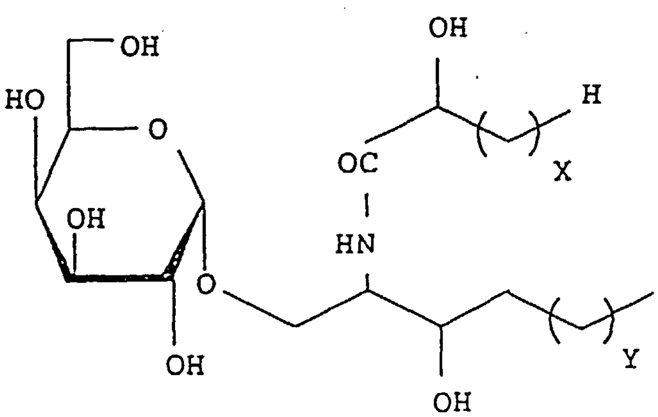

(V)

wherein X denotes an integer of 20-24 and Y denotes an integer of 11-15.

9. A glycosphingolipid according to claim 8, wherein X denotes an integer of 21-23 and Y denotes an integer of 12-14.

**10.** A glycosphingolipid according to claim 8, which is represented by the formula (VI):

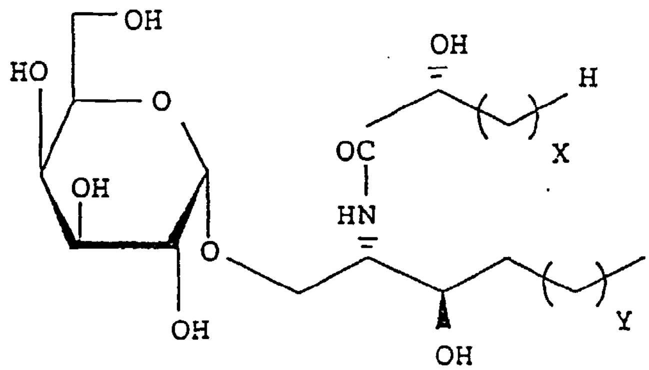

(VI)

wherein X denotes an integer of 20-24 and Y denotes an integer of 11-15.

**11.** A glycosphingolipid according to claim 10, wherein X denotes an integer of 21-23 and Y denotes an integer of 12-14.

**12.** A glycosphingolipid according to claim 2, which is represented by the formula (VII):

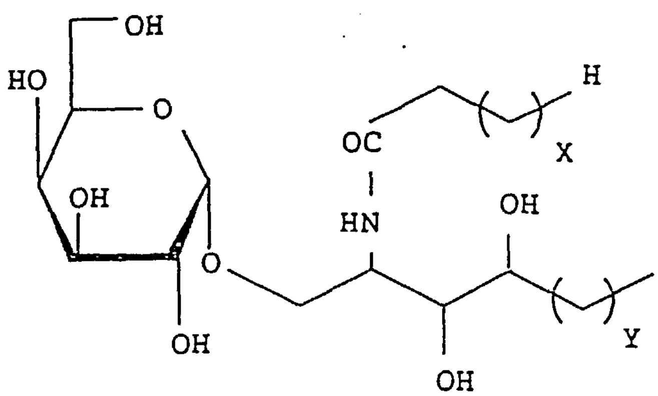

(VII)

wherein X denotes an integer of 18-26 and Y denotes an integer of 5-15.

**13.** A glycosphingolipid according to claim 12, wherein X denotes an integer of 21-25 and Y denotes an integer of 6-14.

**14.** A glycosphingolipid according to claim 12, which is represented by the formula (VIII):

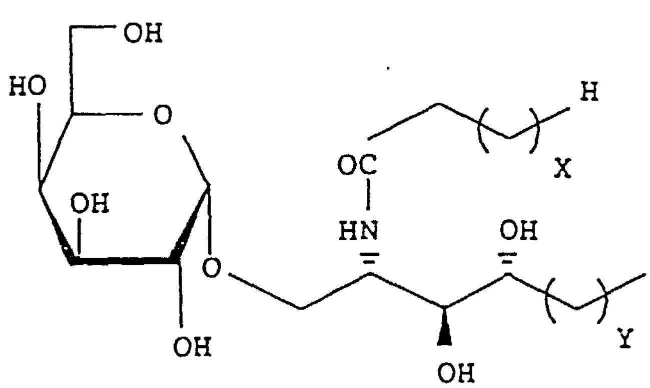

(VIII)

wherein X denotes an integer of 18-26 and Y denotes an integer of 5-15.

**15.** A glycosphingolipid according to claim 14, wherein X denotes an integer of 21-25 and Y denotes an integer of 6-14.

**16.** A glycosphingolipid according to claim 2, which is represented by the formula (IX):

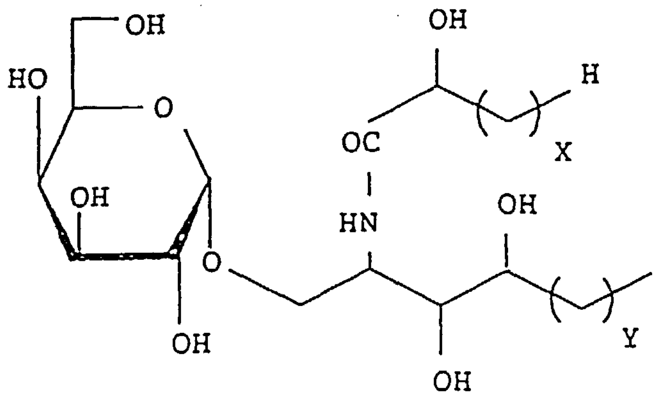

(IX)

wherein X denotes an integer of 18-26 and Y denotes an integer of 5-17.

**17.** A glycosphingolipid according to claim 16, wherein X denotes an integer of 21-25 and Y denotes an integer of 6-16.

**18.** A glycosphingolipid according to claim 16, which is represented by the formula (X):

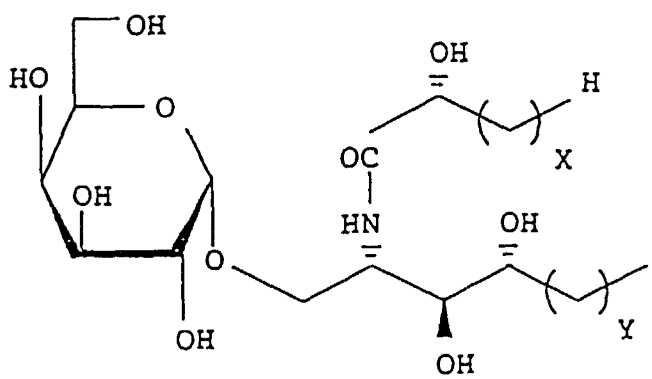

(X)

wherein X denotes an integer of 18-26 and Y denotes an integer of 5-17.

**19.** A glycosphingolipid according to claim 16, which is represented by the formula (X'):

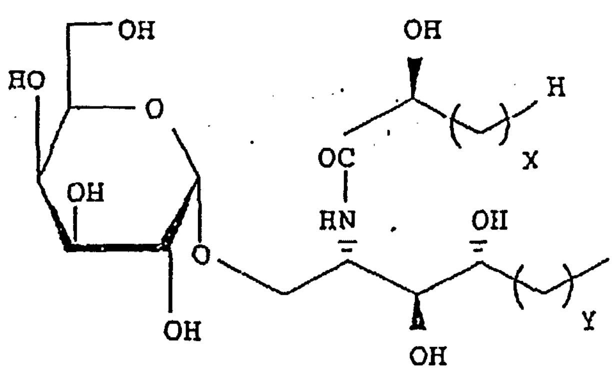

(X')

wherein X denotes an integer of 20-24 and Y denotes an integer of 10-14.

**20.** A glycosphingolipid according to claim 18, wherein X denotes an integer of 21-25 and Y denotes an integer of 6-16.

**21.** A glycosphingolipid according to claim 19, wherein X denotes an integer of 21-23 and Y denotes an integer of 11-13.

**22.** A glycosphingolipid according to claim 2, which is represented by the formula (XI):

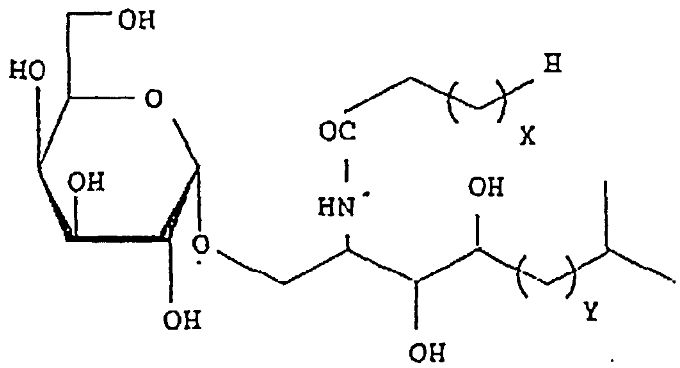

(XI)

wherein X denotes an integer of 20-24 and Y denotes an integer of 9-13.

**23.** A glycosphingolipid according to claim 22, wherein X denotes an integer of 21-23 and Y denotes an integer of 10-12.

**24.** A glycosphingolipid according to claim 22, which is represented by the formula (XII):

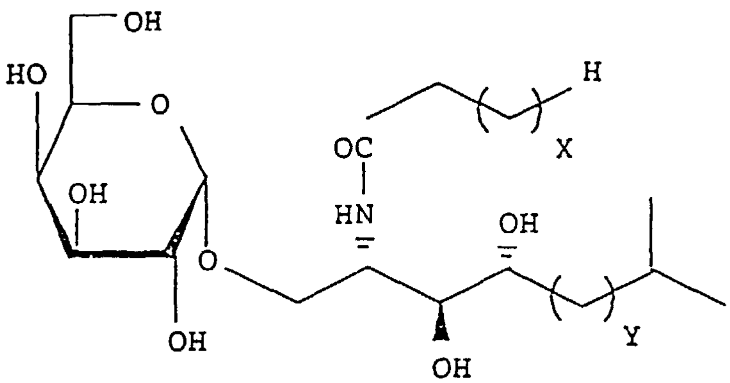

(XII)

wherein X denotes an integer of 20-24 and Y denotes an integer of 9-13.

**25.** A glycosphingolipid according to claim 24, wherein X denotes an integer of 21-23 and Y denotes an integer of 10-12.

**26.** A glycosphingolipid according to claim 2, which is represented by the formula (XIII):

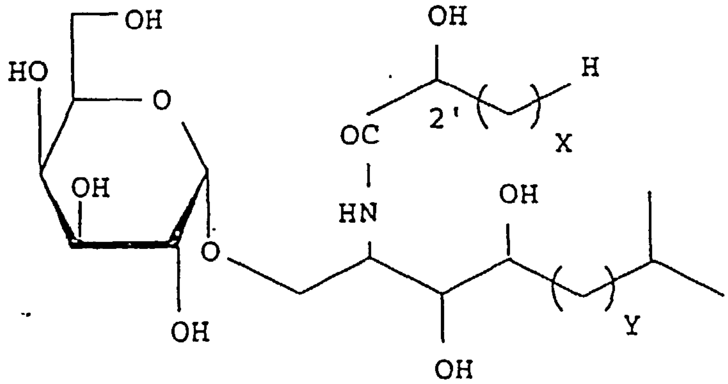

(XIII)

wherein X denotes an integer of 18-24 and Y denotes an integer of 9-13.

**27.** A glycosphingolipid according to claim 26, wherein X denotes an integer of 20-23 and Y denotes an integer of 10-12.

**28.** A glycosphingolipid according to claim 26, which is represented by the formula (XIV):

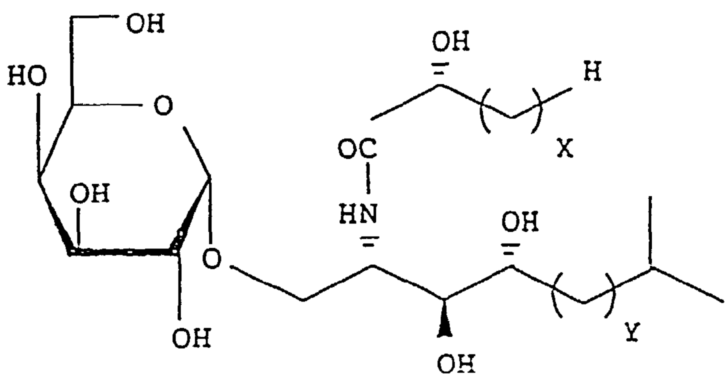

(XIV)

wherein X denotes an integer of 19-23 and Y denotes an integer of 9-13.

**29.** A glycosphingolipid according to claim 26, which is represented by the formula (XIV'):

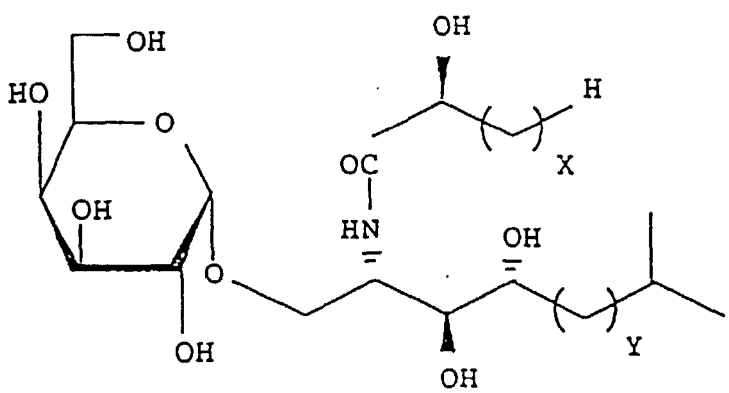

(XIV')

wherein X denotes an integer of 20-24 and Y denotes an integer of 9-13.

**30.** A glycosphingolipid according to claim 28, wherein X denotes an integer of 20-22 and Y denotes an integer of 10-12.

**31.** A glycosphingolipid according to claim 29, wherein X denotes an integer of 21-23 and Y denotes an integer of 10-12.

32. A glycosphingolipid according to claim 2, which is represented by the formula (XV):

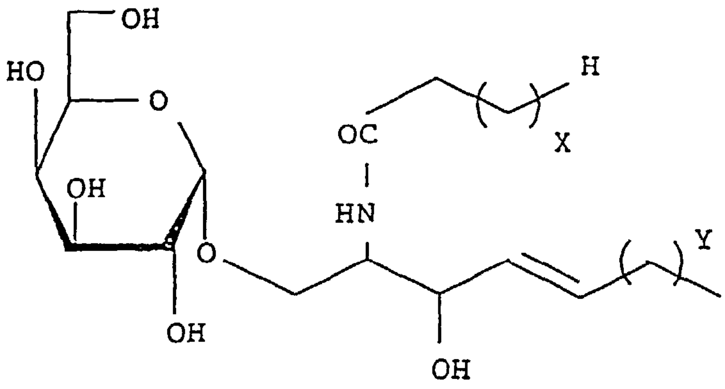

(XV)

wherein X denotes an integer of 10-18 and Y denotes an integer of 10-14.

33. A glycosphingolipid according to claim 32, wherein X denotes an integer of 11-17 and Y denotes an integer of 11-13.

34. A glycosphingolipid according to claim 32, which is represented by the formula (XVI):

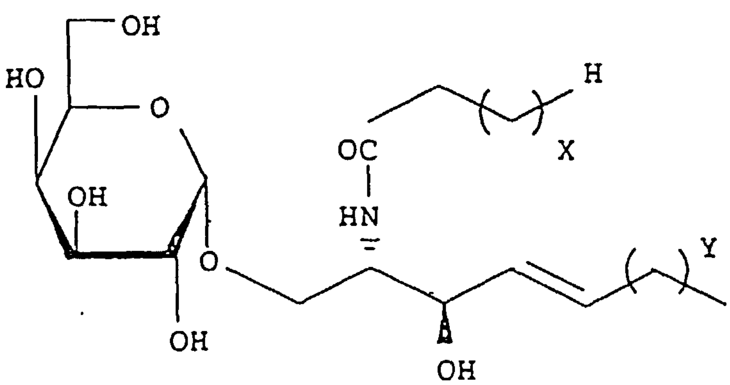

(XVI)

wherein X denotes an integer of 10-18 and Y denotes an integer of 10-14.

35. A glycosphingolipid according to claim 34, wherein X denotes an integer of 11-17 and Y denotes an integer of 11-13.

36. A glycosphingolipid according to claim 2, which is represented by the formula (XVII):

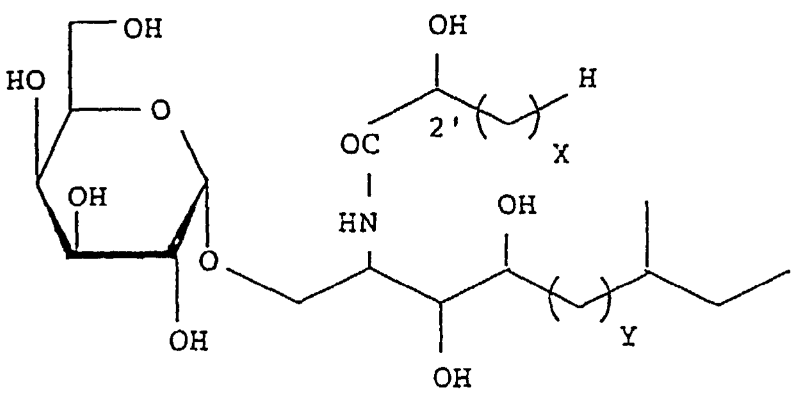

(XVII)

wherein X denotes an integer of 21-25 and Y denotes an integer of 9-13.

37. A glycosphingolipid according to claim 36, wherein X denotes an integer of 22-24 and Y denotes an integer of 10-12.

38. A glycosphingolipid according to claim 36, which is represented by the formula (XVIII):

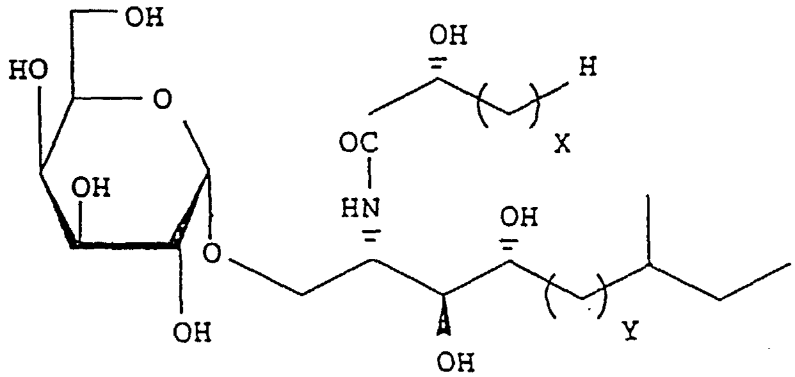

(XVIII)

wherein X denotes an integer of 21-25 and Y denotes an integer of 9-13.

39. A glycosphingolipid according to claim 38, wherein X denotes an integer of 22-24 and Y denotes an integer of 10-12.

40. A glycosphingolipid according to claim 1, which is represented by the formula (XIX):

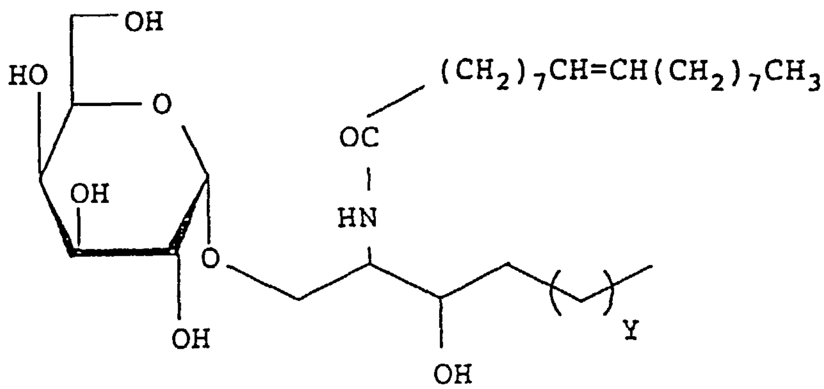

(XIX)

wherein Y denotes an integer of 11-15.

41. A glycosphingolipid according to claim 40, wherein Y denotes an integer of 12-14.

42. A glycosphingolipid according to claim 40, which is represented by the formula (XX):

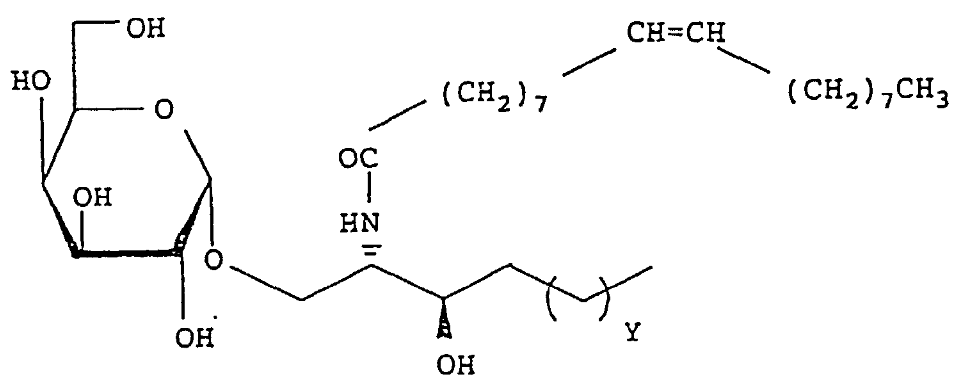

(XX)

wherein Y denotes an integer of 11-15.

**43.** A glycosphingolipid according to claim 42, wherein Y denotes an integer of 12-14.

**44.** A glycosphingolipid according to claim 1, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-octanoylamino-3-octadecanol,
(8) (2S,3R)-2-acetamino-1-(α-D-galactopyranosyloxy)-3-octadecanol,
(9) (2S,3R)-1-(α-D-galactopyranosyloxy)-2-tetracosanoylamino-3-tetradecanol,
(10) (2S,3R)-1-(α-D-galactopyranosyloxy)-2-tetradecanoylamino-3-hexadecanol,
(11) (2R,3S)-1-(α-D-galactopyranosyloxy)-2-tetradecanoylamino-3-hexadecanol,
(12) (2S,3S)-1-(α-D-galactopyranosyloxy)-2-tetradecanoylamino-3-hexadecanol,
(13) (2R,3R)-1-(α-D-galactopyranosyloxy)-2-tetradecanoylamino-3-hexadecanol,
(14) (2S,3R)-1-(α-D-galactopyranosyloxy)-2-[(R)-2-hydroxytetracosanoylamino]-3-octadecanol,
(15) (2S,3R,4E)-1-(α-D-galactopyranosyloxy)-2-octadecanoylamino-4-octadecen-3-ol,
(16) (2S,3R,4E)-1-(α-D-galactopyranosyloxy)-2-tetradecanoylamino-4-octadecen-3-ol,
(17) (2S,3S,4R)-1-(α-D-galactopyranosyloxy)-2-tetracosanoylamino-3,4-octadecanediol,
(18) (2S,3S,4R)-1-(α-D-galactopyranosyloxy)-2-tetracosanoylamino-3,4-heptadecanediol,
(19) (2S,3S,4R)-1-(α-D-galactopyranosyloxy)-2-tetracosanoylamino-3,4-pentadecanediol,
(20) (2S,3S,4R)-1-(α-D-galactopyranosyloxy)-2-tetracosanoylamino-3,4-undecanediol,
(21) (2S,3S,4R)-1-(α-D-galactopyranosyloxy)-2-hexacosanoylamino-3,4-heptadecanediol,
(22) (2S,3S,4R)-1-(α-D-galactopyranosyloxy)-2-[(R)-2-hydroxytetracosanoylamino]-3,4-octadecanediol,
(23) (2S,3S,4R)-1-(α-D-galactopyranosyloxy)-2-[(R)-2-hydroxytetracosanoylamino]-3,4-heptadecanediol,
(24) (2S,3S,4R)-1-(α-D-galactopyranosyloxy)-2-[(R)-2-hydroxytetracosanoylamino]-3,4-pentadecanediol,
(25) (2S,3S,4R)-1-(α-D-galactopyranosyloxy)-2-[(R)-2-hydroxytetracosanoylamino]-3,4-undecanediol,
(26) (2S,3S,4R)-1-(α-D-galactopyranosyloxy)-2-[(R)-2-hydroxyhexacosanoylamino]-3,4-octadecanediol,
(27) (2S,3S,4R)-1-(α-D-galactopyranosyloxy)-2-[(R)-2-hydroxyhexacosanoylamino]-3,4-nonadecanediol,
(28) (2S,3S,4R)-1-(α-D-galactopyranosyloxy)-2-[(R)-2-hydroxyhexacosanoylamino]-3,4-icosanediol,
(29) (25,3S,4R)-1-(α-D-galactopyranosyloxy)-2-[(S)-2-hydroxytetracosanoylamino]-3,4-heptadecanediol,
(30) (2S,3S,4R)-1-(α-D-galactopyranosyloxy)-2-[(R)-2-hydroxytetracosanoylamino]-3,4-hexadecanediol,
(31) (2S,3S,4R)-1-(α-D-galactopyranosyloxy)-2-[(S)-2-hydroxytetracosanoylamino]-16-methyl-3,4-heptadecanediol,
(32) (2S,3S,4R)-1-(α-D-galactopyranosyloxy)-16-methyl-2-tetracosanoylamino-3,4-heptadecanediol,
(33) (2S,3S,4R)-1-(α-D-galactopyranosyloxy)-2-[(R)-2-hydroxytricosanoylamino]-16-methyl-3,4-heptadecanediol,
(34) (2S,3S,4R)-1-(α-D-galactopyranosyloxy)-2-[(R)-2-hydroxypentacosanoylamino]-16-methyl-3,4-octadecanediol, and
(35) (2S,3R)-1-(α-D-galactopyranosyloxy)-2-oleoylamino-3-octadecanol.

**45.** A glycosphingolipid according to claim 44, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-octanoylamino-3-octadecanol,
(8) (2S,3R)-2-acetamino-1-(α-D-galactopyranosyloxy)-3-octadecanol,
(9) (2S,3R)-1-(α-D-galactopyranosyloxy)-2-tetracosanoylamino-3-tetradecanol, and
(10) (2S,3R)-1-(α-D-galactopyranosyloxy)-2-tetradecanoylamino-3-hexadecanol.

**46.** A glycosphingolipid according to claim 45, which is selected from the group consisting of the following compounds:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-tetracosanoylamino-3-tetradecanol, and
(8) (2S,3R)-1-(α-D-galactopyranosyloxy)-2-tetradecanoylamino-3-hexadecanol.

**47.** A glycosphingolipid according to claim 46, which is selected from the group consisting of the following compounds:

(1) (2S,3R)-1-(α-D-galactopyranosyloxy)-2-octadecanoylamino-3-octadecanol,
(2) (2S,3R)-1-(α-D-galactopyranosyloxy)-2-tetradecanoylamino-3-octadecanol, and
(3) (2S,3R)-1-(α-D-galactopyranosyloxy)-2-tetradecanoylamino-3-hexadecanol.

**48.** A glycosphingolipid according to claim 44, which is (2S,3R)-1-(α-D-galactopyranosyloxy)-2-[(R)-2-hydroxytetracosanoylamino]-3-octadecanol.

**49.** A glycosphingolipid according to claim 44, which is selected from the group consisting of the following compounds:

(1) (2S,3R,4E)-1-(α-D-galactopyranosyloxy)-2-octadecanoylamino-4-octadecen-3-ol, and
(2) (2S,3R,4E)-1-(α-D-galactopyranosyloxy)-2-tetradecanoylamino-4-octadecen-3-ol.

**50.** A glycosphingolipid according to claim 44, which is selected from the group consisting of the following compounds:

(1) (2S,3S,4R)-1-(α-D-galactopyranosyloxy)-2-tetracosanoylamino-3,4-octadecanediol,
(2) (2S,3S,4R)-1-(α-D-galactopyranosyloxy)-2-tetracosanoylamino-3,4-heptadecanediol,
(3) (2S,3S,4R)-1-(α-D-galactopyranosyloxy)-2-tetracosanoylamino-3,4-pentadecanediol,
(4) (2S,3S,4R)-1-(α-D-galactopyranosyloxy)-2-tetracosanoylamino-3,4-undecanediol, and
(5) (2S,3S,4R)-1-(α-D-galactopyranosyloxy)-2-hexacosanoylamino-3,4-heptadecanediol.

**51.** A glycosphingolipid according to claim 44, which is selected from the group consisting of the following compounds:

(1) (2S,3S,4R)-1-(α-D-galactopyranosyloxy)-2-[(R)-2-hydroxytetracosanoylamino]-3,4-octadecanediol,
(2) (2S,3S,4R)-1-(α-D-galactopyranosyloxy)-2-[(R)-2-hydroxytetracosanoylamino]-3,4-heptadecanediol,
(3) (2S,3S,4R)-1-(α-D-galactopyranosyloxy)-2-[(R)-2-hydroxytetracosanoylamino]-3,4-pentadecanediol,
(4) (2S,3S,4R)-1-(α-D-galactopyranosyloxy)-2-[(R)-2-hydroxytetracosanoylamino]-3,4-undecanediol,
(5) (2S,3S,4R)-1-(α-D-galactopyranosyloxy)-2-[(R)-2-hydroxyhexacosanoylamino]-3,4-octadecanediol,
(6) (2S,3S,4R)-1-(α-D-galactopyranosyloxy)-2-[(R)-2-hydroxyhexacosanoylamino]-3,4-nonadecanediol,
(7) (2S,3S,4R)-1-(α-D-galactopyranosyloxy)-2-[(R)-2-hydroxyhexacosanoylamino]-3,4-icosanediol,
(8) (2S,3S,4R)-1-(α-D-galactopyranosyloxy)-2-[(S)-2-hydroxytetracosanoylamino]-3,4-heptadecanediol, and
(9) (2S,3S,4R)-1-(α-D-galactopyranosyloxy)-2-[(R)-2-hydroxytetracosanoylamino]-3,4-hexadecanediol.

**52.** A glycosphingolipid according to claim 44, which is selected from the group consisting of the following compounds:

(1) (2S,3S,4R)-1-(α-D-galactopyranosyloxy)-2-[(S)-2-hydroxytetracosanoylamino]-16-methyl-3,4-heptadecanediol,
(2) (2S,3S,4R)-1-(α-D-galactopyranosyloxy)-16-methyl-2-tetracosanoylamino-3,4-heptadecanediol, and
(3) (2S,3S,4R)-1-(α-D-galactopyranosyloxy)-2-[(R)-2-hydroxytricosanoylamino]-16-methyl-3,4-heptadecanediol.

**53.** A glycosphingolipid according to claim 44, which is (2S,3S,4R)-1-(α-D-galactopyranosyloxy)-2-[(R)-2-hydroxypentacosanoylamino]-16-methyl-3,4-octadecanediol.

**54.** A glycosphingolipid according to claim 44, which is (2S,3R)-1-(α-D-galactopyranosyloxy)-2-oleoylamino-3-octandecanol.

**55.** A process for preparing a glycosphingolipid including an alpha-galactose residue represented by the formula (A):

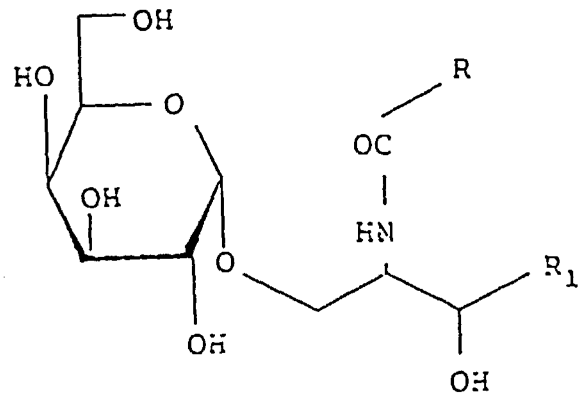

(A)

wherein

R represents

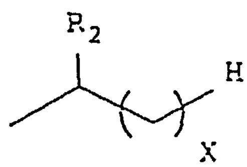

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17, which comprises

one of the following methods:

a) extracting (A) from a species of the marine sponge Algelas mauretianus with at least one appropriate organic solvent and water;
b) modifying sphingosine; and
c) general chemical reactions for the synthesis of glycosphingolipids.

**56.** A process according to claim 55, wherein a glycosphingolipid represented by the formula (I) is prepared:

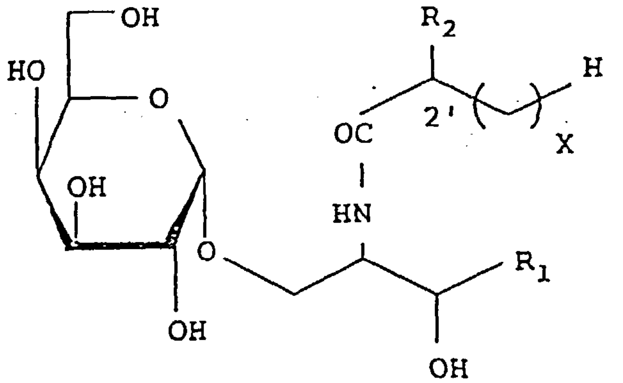

(I)

wherein

$R_1$ represents any one of the substituents defined by (a)-(e) below and $R_2$ represents H or OH (X is defined in the following (a)-(e)):

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**57.** A process according to claim 56, wherein a glycosphingolipid represented by the formula (II) is , prepared:

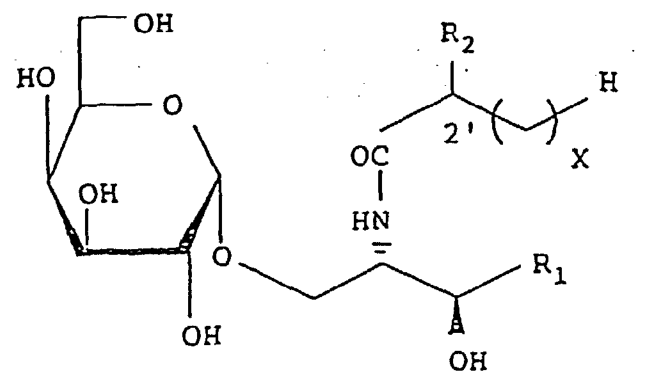

(II)

wherein

$R_1$ represents any one of the substituents defined by (a)-(e) below and $R_2$ represents H or OH (X is defined in the following (a)-(e));

(a) $-CH_2(CH_2)_YCH_3$,

wherein when $R_2$ represents H, X denotes an integer of 0-24 and Y denotes an integer of 7-15; when $R_2$ represents OH, X denotes an integer of 20-24 and Y denotes an integer of 11-15;

(b) $-CH(OH)(CH_2)_YCH_3$,

wherein when $R_2$ represents H, X denotes an integer of 18-26 and Y denotes an integer of 5-15; when $R_2$ represents OH, X denotes an integer of 18-26 and Y denotes an integer of 5-17;

(c) $-CH(OH)(CH_2)_YCH(CH_3)_2$,

wherein when $R_2$ represents H, X denotes an integer of 20-24 and Y denotes an integer of 9-13; when $R_2$ represents OH, X denotes an integer of 18-24 and Y denotes an integer of 9-13;

(d) $-CH{=}CH(CH_2)_YCH_3$,

wherein $R_2$ represents H, X denotes an integer of 10-18 and Y denotes an integer of 10-14; and

(e) $-CH(OH)(CH_2)_YCH(CH_3)CH_2CH_3$,

wherein $R_2$ represents OH, X denotes an integer of 21-25 and Y denotes an integer of 9-13.

**58.** A process according to claim 56, wherein a glycosphingolipid represented by the formula (III) is prepared:

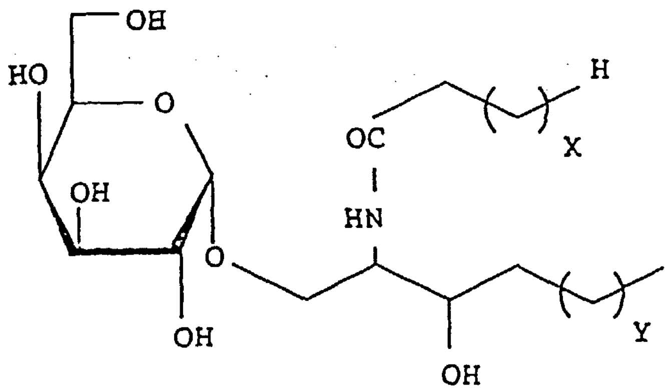

(III)

wherein X denotes an integer of 0-24 and Y denotes an integer of 7-15.

**59.** A process according to claim 58, wherein X denotes an integer of 8-22 and Y denotes an integer of 9-13.

**60.** A process according to claim 58, wherein a glycosphingolipid represented by the formula (IV) is prepared:

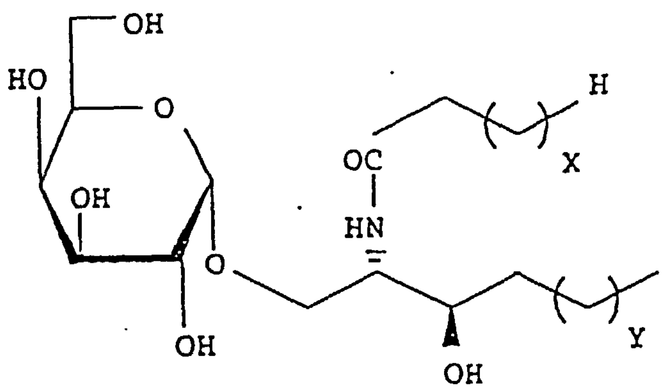

(IV)

wherein X denotes an integer of 0-24 and Y denotes an integer of 7-15.

**61.** A process according to claim 60, wherein X denotes an integer of 8-22 and Y denotes an integer of 9-13.

**62.** A process according to claim 56, wherein a glycosphingolipid represented by the formula (V) is prepared:

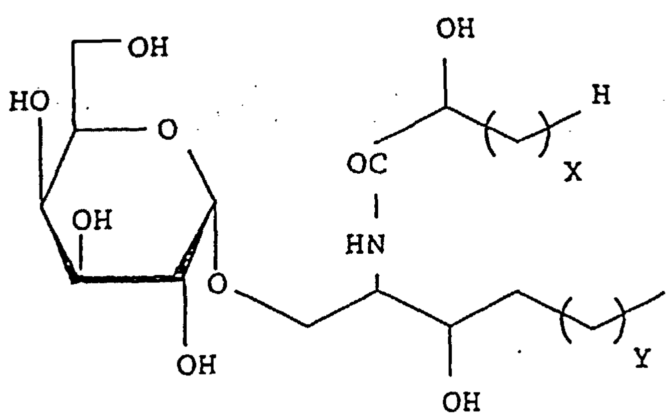

(V)

wherein X denotes an integer of 20-24 and Y denotes an integer of 11-15.

63. A process according to claim 62, wherein X denotes an integer of 21-23 and Y denotes an integer of 12-14.

64. A process according to claim 62, wherein a glycosphingolipid represented by the formula (VI) is prepared:

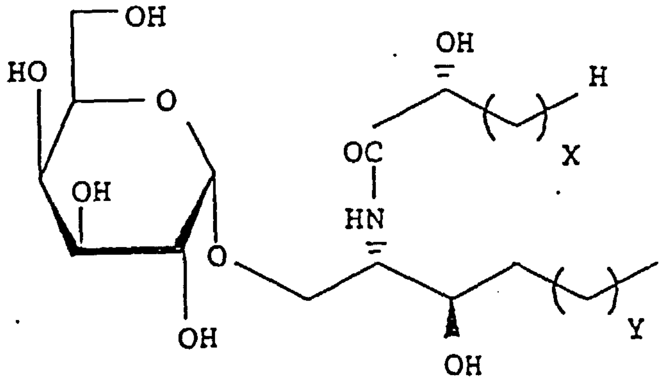

(VI)

wherein X denotes an integer of 20-24 and Y denotes an integer of 11-15.

65. A process according to claim 64, wherein X denotes an integer of 21-23 and Y denotes an integer of 12-14.

66. A process according to claim 56, wherein a glycosphingolipid represented by the formula (VII) is prepared:

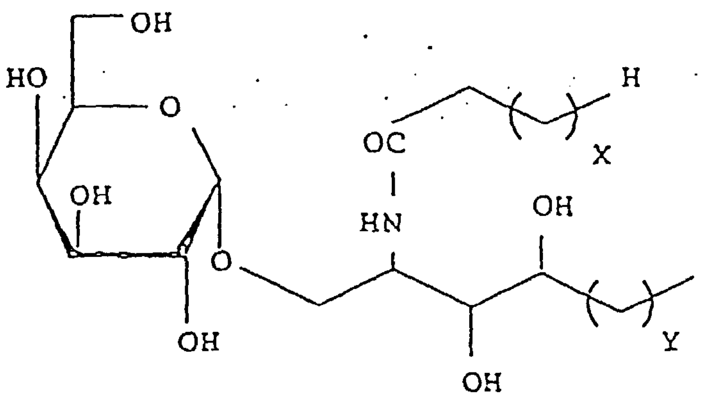

(VII)

wherein X denotes an integer of 18-26 and Y denotes an integer of 5-15.

67. A process according to claim 66, wherein X denotes an integer of 21-25 and y denotes an integer of 6-14.

68. A process according to claim 66, wherein a glycosphingolipid represented by the formula (VIII) is prepared:

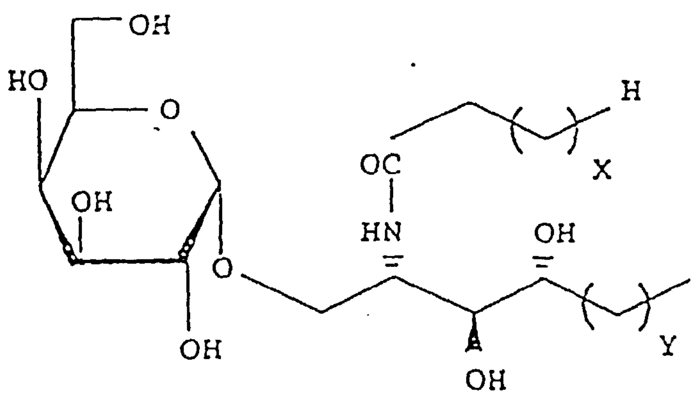

(VIII)

wherein X denotes an integer of 18-26 and Y denotes an integer of 5-15.

**69.** A process according to claim 68,
wherein X denotes an integer of 21-25 and Y denotes an integer of 6-14.

**70.** A process according to claim 56, wherein a glycosphingolipid represented by the formula (IX) is prepared:

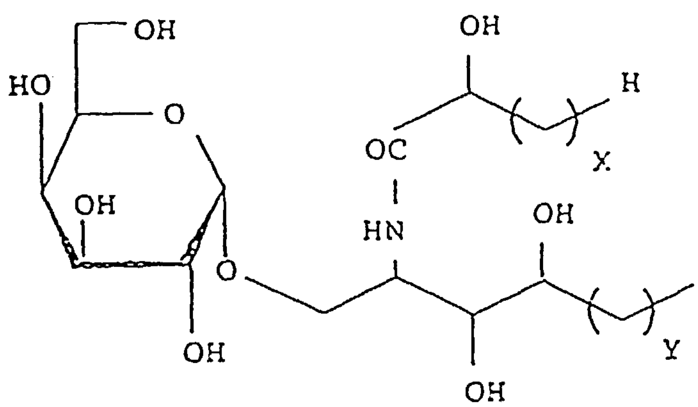

(IX)

wherein X denotes an integer of 18-26 and Y denotes an integer of 5-17.

**71.** A process according to claim 70,
wherein X denotes an integer of 21-25 and Y denotes an integer of 6-16.

**72.** A process according to claim 70, wherein a glycosphingolipid represented by the formula (X) is prepared:

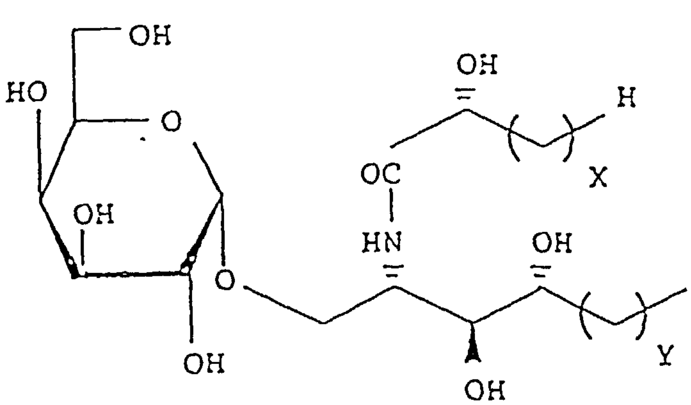

(X)

wherein X denotes an integer of 18-26 and Y denotes an integer of 5-17.

**73.** A process according to claim 70, wherein a glycosphingolipid represented by the formula (X') is prepared:

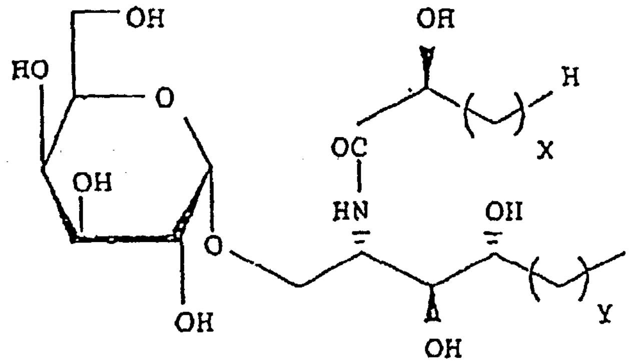

(X')

wherein X denotes an integer of 20-24 and Y denotes an integer of 10-14.

**74.** A process according to claim 72, wherein X denotes an integer of 21-25 and Y denotes an integer of 6-16.

**75.** A process according to claim 74, wherein X denotes an integer of 21-23 and Y denotes an integer of 11-13.

**76.** A process according to claim 56, wherein a glycosphingolipid represented by the formula (XI) is prepared:

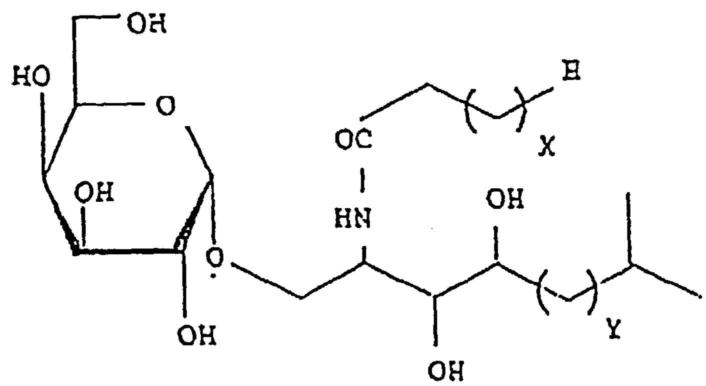

(XI)

wherein X denotes an integer of 20-24 and Y denotes an integer of 9-13.

**77.** A process according to claim 76, wherein X denotes an integer of 21-23 and Y denotes an integer of 10-12.

**78.** A process according to claim 76, wherein a glycosphingolipid represented by the formula (XII) is prepared:

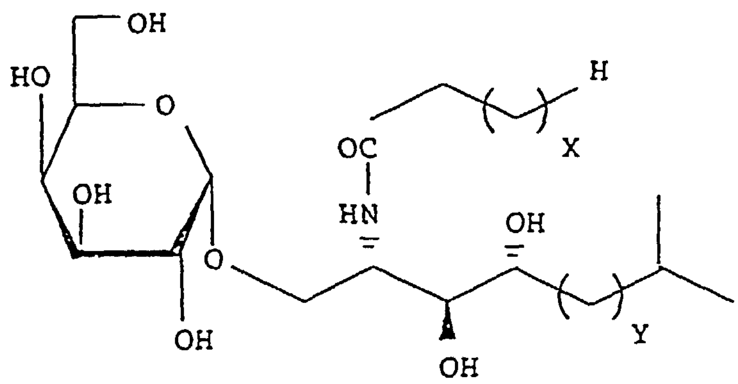

(XII)

wherein X denotes an integer of 20-24 and Y denotes an integer of 9-13.

**79.** A process according to claim 78,
wherein X denotes an integer of 21-23 and Y denotes an integer of 10-12.

**80.** A process according to claim 56, wherein a glycosphingolipid represented by the formula (XIII) is prepared:

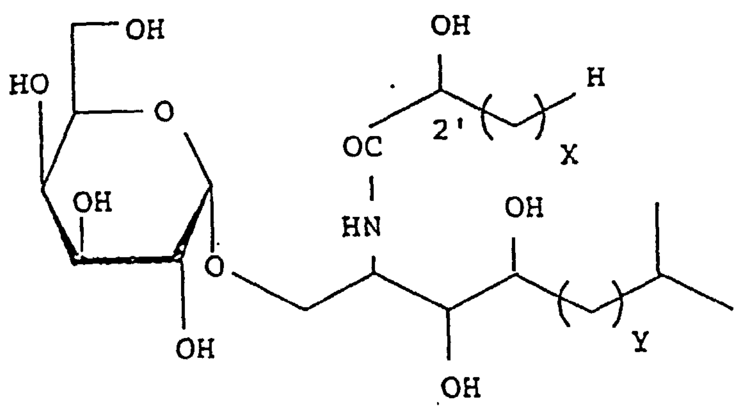

(XIII)

wherein X denotes an integer of 18-24 and Y denotes an integer of 9-13.

**81.** A process according to claim 80,
wherein X denotes an integer of 20-23 and Y denotes an integer of 10-12.

**82.** A process according to claim 80, wherein a glycosphingolipid represented by the formula (XIV) is prepared:

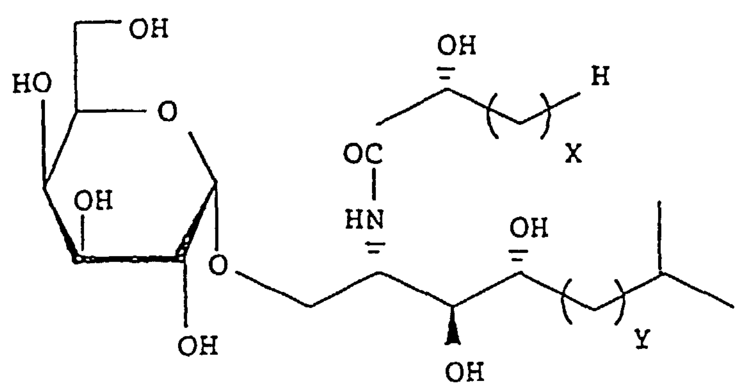

(XIV)

wherein X denotes an integer of 19-23 and Y denotes an integer of 9-13.

**83.** A process according to claim 80, wherein a glycosphingolipid represented by the formula (XIV') is prepared:

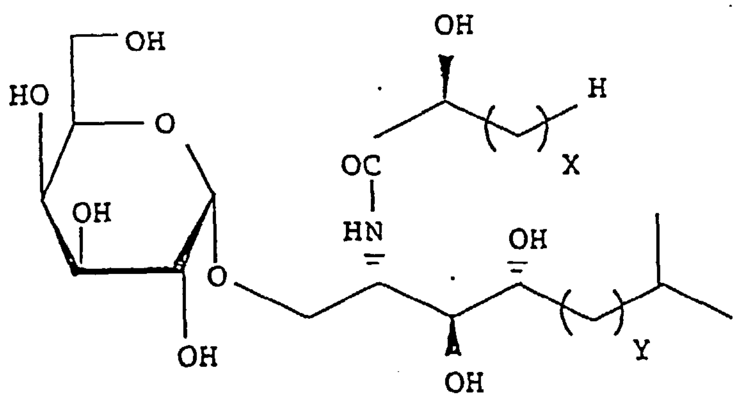

(XIV')

wherein X denotes an integer of 20-24 and Y denotes an integer of 9-13.

**84.** A process according to claim 82, wherein X denotes an integer of 20-22 and Y denotes an integer of 10-12.

**85.** A process according to claim 83, wherein X denotes an integer of 21-23 and Y denotes an integer of 10-12.

**86.** A process according to claim 56, wherein a glycosphingolipid represented by the formula (XV) is prepared:

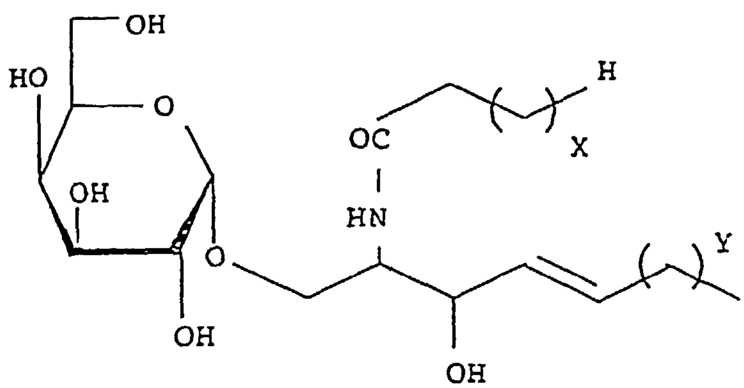

(XV)

wherein X denotes an integer of 10-18 and Y denotes an integer of 10-14.

**87.** A process according to claim 86, wherein X denotes an integer of 11-17 and Y denotes an integer of 11-13.

**88.** A process according to claim 86, wherein a glycosphingolipid represented by the formula (XVI) is prepared:

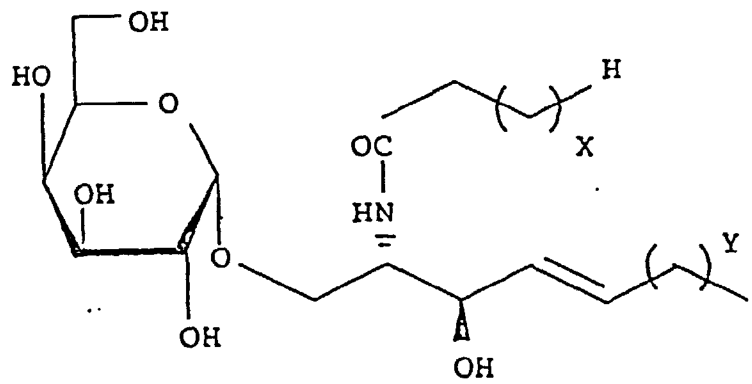

(XVI)

wherein X denotes an integer of 10-18 and Y denotes an integer of 10-14.

**89.** A process according to claim 88,
wherein X denotes an integer of 11-17 and Y denotes an integer of 11-13.

**90.** A process according to claim 56, wherein a glycosphingolipid represented by the formula (XVII) is prepared:

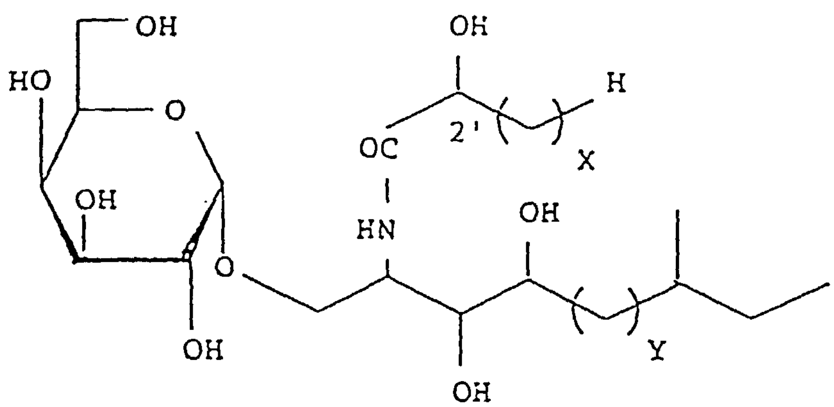

(XVII)

wherein X denotes an integer of 21-25 and Y denotes an integer of 9-13.

**91.** A process according to claim 90,
wherein X denotes an integer of 22-24 and Y denotes an integer of 10-12.

**92.** A process according to claim 90, wherein a glycosphingolipid represented by the formula (XVIII) is prepared:

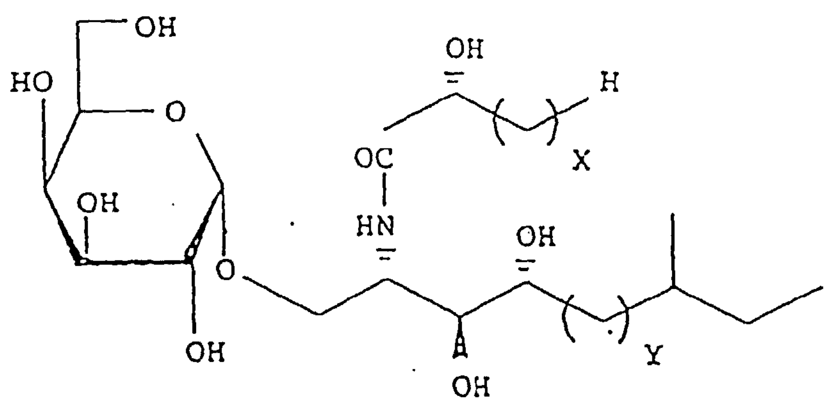

(XVIII)

wherein X denotes an integer of 21-25 and Y denotes an integer of 9-13.

**93.** A process according to claim 92,
wherein X denotes an -integer of 22-24 and Y denotes an integer of 10-12.

**94.** A process according to claim 55, wherein a glycosphingolipid represented by the formula (XIX) is prepared:

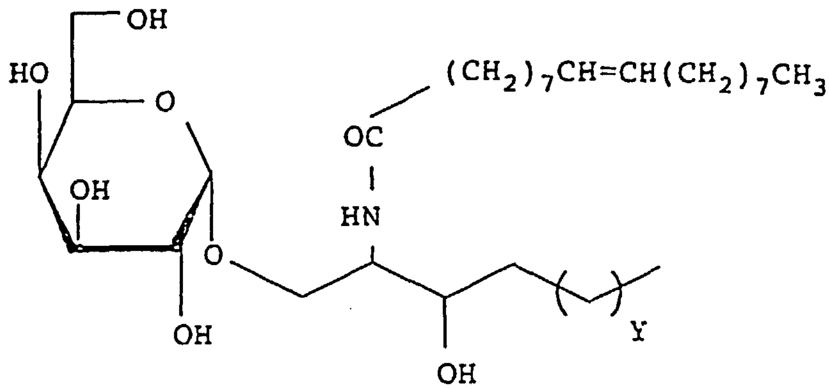

(XIX)

wherein Y denotes an integer of 11-15.

**95.** A process according to claim 94,
wherein Y denotes an integer of 12-14.

**96.** A process according to claim 94, wherein a glycosphingolipid represented by the formula (XX) is prepared:

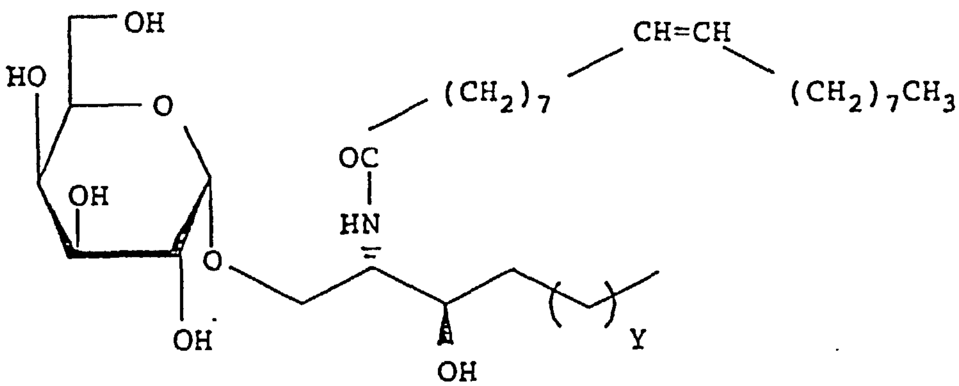

(XX)

wherein Y denotes an integer of 11-15.

**97.** A process according to claim 96,
wherein Y denotes an integer of 12-14.

**98.** A process according to claim 55, wherein a glycosphingolipid selected from the group consisting of the following compounds is prepared:

(1) (2S,3R)-1-(α-D-galactopyranosyloxy)-2-tetracosanoylamino-3-octadecanol,
(2) (2S,3R)-2-docosanoylamino-1-(α-D-galactopyranosyloxy)-3-octadecanol,
(3) (2S,3R)-1-(α-D-galactopyranosyloxy)-2-icosanoylamino-3-octadecanol,
(4) (2S,3R)-1-(α-D-galactopyranosyloxy)-2-octadecanoylamino-3-octadecanol,
(5) (2S,3R)-1-(α-D-galactopyranosyloxy)-2-tetradecanoylamino-3-octadecanol,
(6) (2S,3R)-2-decanoylamino-1-(α-D-galactopyranosyloxy)-3-octadecanol,
(7) (2S,3R)-1-(α-D-galactopyranosyloxy)-2-octanoylamino-3-octadecanol,
(8) (2S,3R)-2-acetamino-1-(α-D-galactopyranosyloxy)-3-octadecanol,

(9) (2S,3R)-1-(-α-D-galactopyranosyloxy)-2-tetracosanoylamino-3-tetradecanol,
(10) (2S,3R)-1-(α-D-galactopyranosyloxy)-2-tetradecanoylamino-3-hexadecanol,
(11) (2R,3S)-1-(α-D-galactopyranosyloxy)-2-tetradecanoylamino-3-hexadecanol,
(12) (2S,3S)-1-(α-D-galactopyranosyloxy)-2-tetradecanoylamino-3-hexadecanol,
(13) (2R,3R)-1-(α-D-galactopyranosyloxy)-2-tetradecanoylamino-3-hexadecanol,
(14) (2S,3R)-1-(α-D-galactopyranosyloxy)-2-[(R)-2-hydroxytetracosanoylamino]-3-octadecanol,
(15) (2S,3R,4E)-1-(α-D-galactopyranosyloxy)-2-octadecanoylamino-4-octadecen-3-ol,
(16) (2S,3R,4E)-1-(α-D-galactopyranosyloxy)-2-tetradecanoylamino-4-octadecen-3-ol,
(17) (2S,3S,4R)-1-(α-D-galactopyranosyloxy)-2-tetracosanoylamino-3,4-octadecanediol,
(18) (2S,3S,4R)-1-(α-D-galactopyranosyloxy)-2-tetracosanoylamino-3,4-heptadecanediol,
(19) (2S,3S,4R)-1-(α-D-galactopyranosyloxy)-2-tetracosanoylamino-3,4-pentadecanediol,
(20) (2S,3S,4R)-1-(α-D-galactopyranosyloxy)-2-tetracosanoylamino-3,4-undecanediol,
(21) (2S,3S,4R)-1-(α-D-galactopyranosyloxy)-2-hexacosanoylamino-3,4-heptadecanediol,
(22) (2S,3S,4R)-1-(α-D-galactopyranosyloxy)-2-[(R)-2-hydroxytetracosanoylamino]-3,4-octadecanediol,
(23) (2S,3S,4R)-1-(α-D-galactopyranosyloxy)-2-[(R)-2-hydroxytetracosanoylamino]-3,4-heptadecanediol,
(24) (2S,3S,4R)-1-(α-D-galactopyranosyloxy)-2-[(R)-2-hydroxytetracosanoylamino]-3,4-pentadecanediol,
(25) (2S,3S,4R)-1-(α-D-galactopyranosyloxy)-2-[(R)-2-hydroxytetracosanoylamino]-3,4-undecanediol,
(26) (2S,3S,4R)-1-(α-D-galactopyranosyloxy)-2-[(R)-2-hydroxyhexacosanoylamino]-3,4-octadecanediol,
(27) (2S,3S,4R)-1-(α-D-galactopyranosyloxy)-2-[(R)-2-hydroxyhexacosanoylamino]-3,4-nonadecanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytetracosanoylamino]-3,4-heptadecanediol,
(30) (2S,3S,4R)-1-(α-D-galactopyranosyloxy)-2-[(R)-2-hydroxytetracosanoylamino]-3,4-hexadecanediol,
(31) (2S,3S,4R)-1-(α-D-galactopyranosyloxy)-2-[(S)-2-hydroxytetracosanoylamino]-16-methyl-3,4-heptadecanediol,
(32) (2S,3S,4R)-1-(α-D-galactopyranosyloxy)-16-methyl-2-tetracosanoylamino-3,4-heptadecanediol,
(33) (2S,3S,4R)-1-(α-D-galactopyranosyloxy)-2-[(R)-2-hydroxytricosanoylamino]-16-methyl-3,4-heptadecanediol,
(34) (2S,3S,4R)-1-(α-D-galactopyranosyloxy)-2-[(R)-2-hydroxypentacosanoylamino]-16-methyl-3,4-octadecanediol, and
(35) (2S,3R)-1-(α-D-galactopyranosyloxy)-2-oleoylamino-3-octadecanol.

**99.** A process for a glycosphingolipid comprising collecting the marine sponge Agelas mauritianus, subjecting it to an extraction operation with an organic solvent and isolating from the extract at least one of the following glycolphingolipids represented by the formula (I) according to claim 56:

(1) (2S,3S,4R)-1-(α-D-galactopyranosyloxy)-2-[(R)-2-hydroxytetracosanoylamino]-3,4-heptadecanediol,
(2) (2S,3S,4R)-1-(α-D-galactopyranosyloxy)-2-[(R)-2-hydroxytetracosanoylamino]-3,4-hexadecanediol,
(3) (2S,3S,4R)-1-(α-D-galactopyranosyloxy)-2-[(R)-2-hydroxytricosanoylamino]-16-methyl-3,4-heptadecanediol, and
(4) (2S,3S,4R)-1-(α-D-galactopyranosyloxy)-2-[(R)-2-hydroxypentacosanoylamino]-16-methyl-3,4-octadecanediol.

**100.** Process for preparing an antitumor agent comprising the mixing of one or more compounds represented by the formula (A)

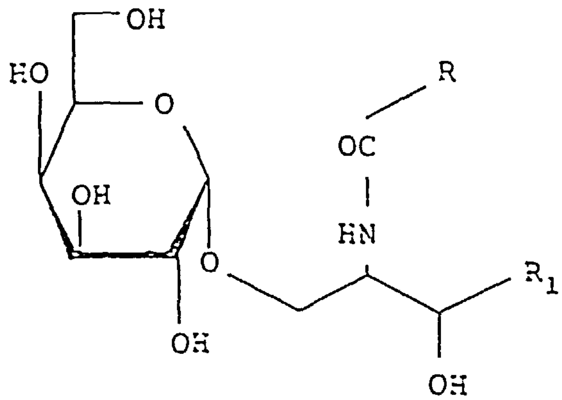

(A)

wherein

R represents

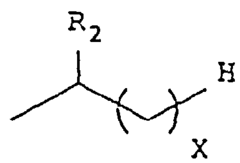

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17

as effective ingredients with pharmaceutically acceptable additives.

**101.** Process for preparing an immunostimulator comprising the mixing of one or more compounds represented by the formula (A)

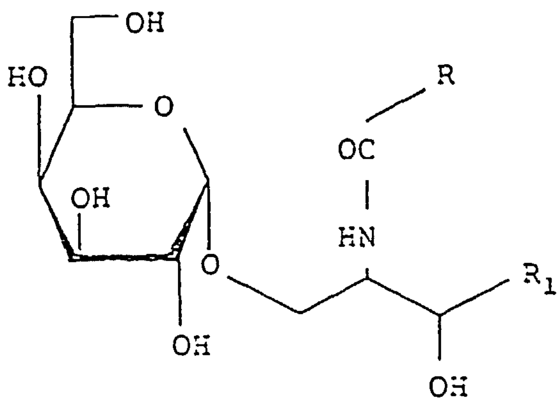

(A)

wherein

R represents

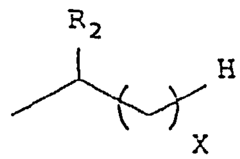

,

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17

as effective ingredients with pharmaceutically acceptable additives.

**102.** The use of one or more compounds represented by the formula (A)

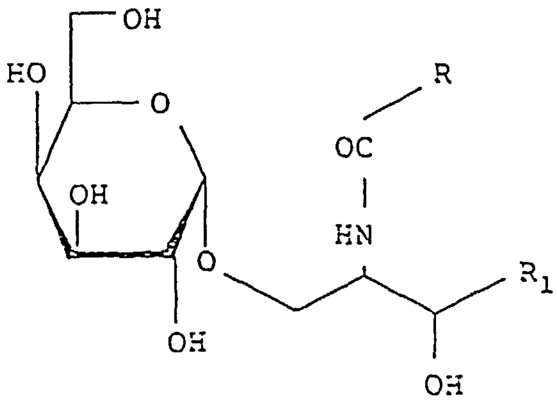

(A)

wherein

R represents

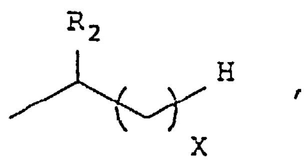

where $R_2$ represents H or OH and X denotes an integer of 0-26, or $-(CH_2)_7CH=CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$,

and

(e) $-CH(OH)(CH_2)_YCH(CH_3)CH_2CH_3$,

wherein Y denotes an integer of 5-17

in the preparation of an antitumor agent.

**103.** The use of one or more compounds represented by the formula (A)

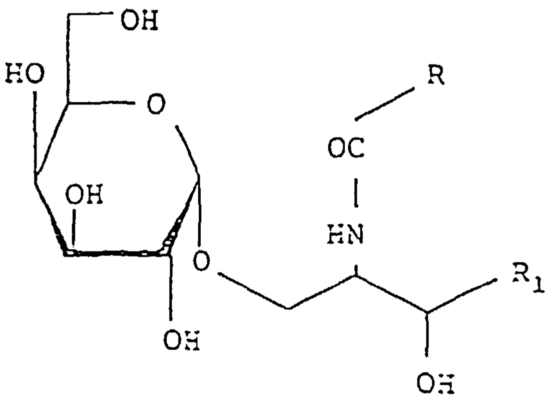

(A)

wherein

R represents

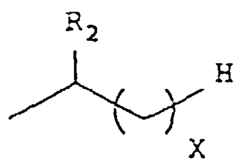

,

where $R_2$ represents H or OH and X denotes an integer of 0-26, or $-(CH_2)_7CH{=}CH(CH_2)_7CH_3$ and $R_1$ represents any one of the substituents defined by the following (a)-(e):

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$,

and

(e) $-CH(OH)(CH_2)_YCH(CH_3)CH_2CH_3$,

wherein Y denotes an integer of 5-17

in the preparation of an immunostimulating medicament.

**Patentansprüche**

**Patentansprüche für folgende Vertragsstaaten : AT, BE, CH, LI, DE, DK, FR, GB, IT, LU, MC, NL, SE**

**1.** Glycosphingolipid mit einem α-Galactoserest, dargestellt durch die Formel (A):

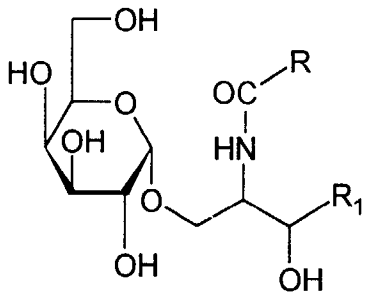

(A)

wobei R

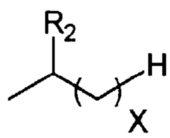

,

wobei $R_2$ H oder OH darstellt und X eine ganze Zahl von 0 bis 26 bezeichnet, oder $-(CH_2)_7CH=CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet.

2. Glycosphingolipid gemäss Anspruch 1, dargestellt durch die Formel (I):

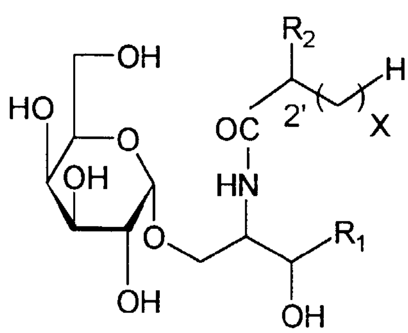

(I)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH=CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**3.** Glycosphingolipid gemäss Anspruch 2, dargestellt durch die Formel (II):

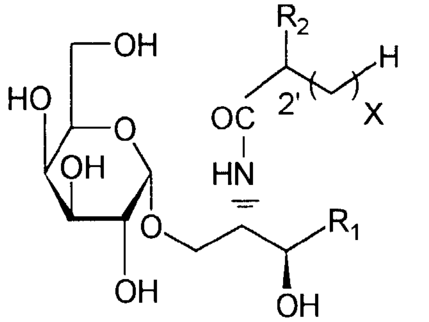

(II)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH{=}CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**4.** Glycosphingolipid gemäss Anspruch 2, dargestellt durch die Formel (III):

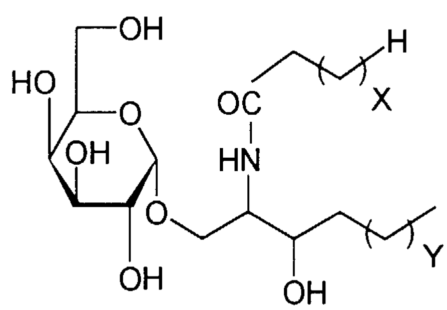

(III)

wobei X eine ganze Zahl von 0 bis 24 bezeichnet und Y eine ganze Zahl von 7 bis 15 bezeichnet.

**5.** Glycosphingolipid gemäss Anspruch 4, wobei X eine ganze Zahl von 8 bis 22 bezeichnet und Y eine ganze Zahl von 9 bis 13 bezeichnet.

**6.** Glycosphingolipid gemäss Anspruch 4, dargestellt durch die Formel (IV):

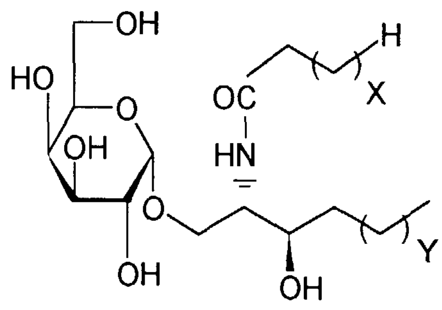

( IV )

wobei X eine ganze Zahl von 0 bis 24 bezeichnet und Y eine ganze Zahl von 7 bis 15 bezeichnet.

**7.** Glycosphingolipid gemäss Anspruch 6, wobei X eine ganze Zahl von 8 bis 22 bezeichnet und Y eine ganze Zahl von 9 bis 13 bezeichnet.

**8.** Glycosphingolipid gemäss Anspruch 2, dargestellt durch die Formel (V):

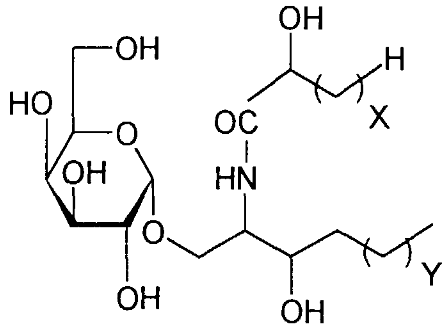

( V )

wobei X eine ganze Zahl von 20 bis 24 bezeichnet und Y eine ganze Zahl von 11 bis 15 bezeichnet.

**9.** Glycosphingolipid gemäss Anspruch 8, wobei X eine ganze Zahl von 21 bis 23 bezeichnet und Y eine ganze Zahl von 12 bis 14 bezeichnet.

**10.** Glycosphingolipid gemäss Anspruch 8, dargestellt durch die Formel (VI):

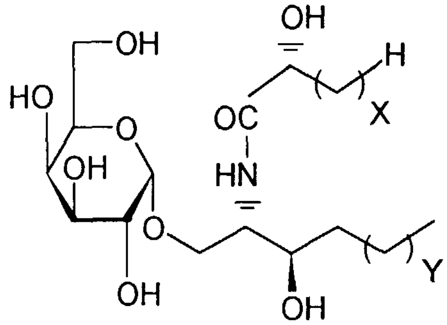

( VI )

wobei X eine ganze Zahl von 20 bis 24 bezeichnet und Y eine ganze Zahl von 11 bis 15 bezeichnet.

**11.** Glycosphingolipid gemäss Anspruch 10, wobei X eine ganze Zahl von 21 bis 23 bezeichnet und Y eine ganze Zahl von 12 bis 14 bezeichnet.

**12.** Glycosphingolipid gemäss Anspruch 2, dargestellt durch die Formel (VII):

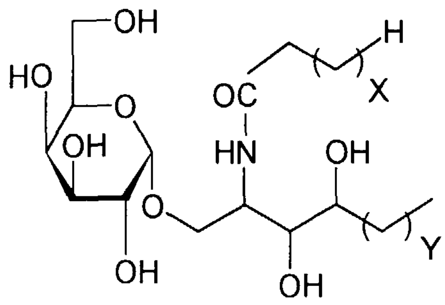

( VII )

wobei X eine ganze Zahl von 18 bis 26 bezeichnet und Y eine ganze Zahl von 5 bis 15 bezeichnet.

**13.** Glycosphingolipid gemäss Anspruch 12, wobei X eine ganze Zahl von 21 bis 25 bezeichnet und Y eine ganze Zahl von 6 bis 14 bezeichnet.

**14.** Glycosphingolipid gemäss Anspruch 12, dargestellt durch die Formel (VIII):

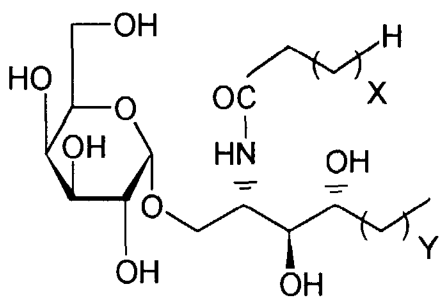

( VIII )

wobei X eine ganze Zahl von 18 bis 26 bezeichnet und Y eine ganze Zahl von 5 bis 15 bezeichnet.

**15.** Glycosphingolipid gemäss Anspruch 14, wobei X eine ganze Zahl von 21 bis 25 bezeichnet und Y eine ganze Zahl von 6 bis 14 bezeichnet.

**16.** Glycosphingolipid gemäss Anspruch 2, dargestellt durch die Formel (IX):

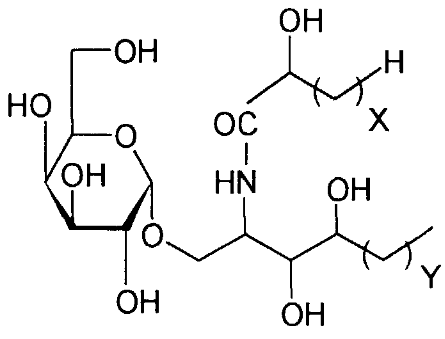

( IX )

wobei X eine ganze Zahl von 18 bis 26 bezeichnet und Y eine ganze Zahl von 5 bis 17 bezeichnet.

**17.** Glycosphingolipid gemäss Anspruch 16, wobei X eine ganze Zahl von 21 bis 25 bezeichnet und Y eine ganze Zahl von 6 bis 16 bezeichnet.

**18.** Glycosphingolipid gemäss Anspruch 16, dargestellt durch die Formel (X):

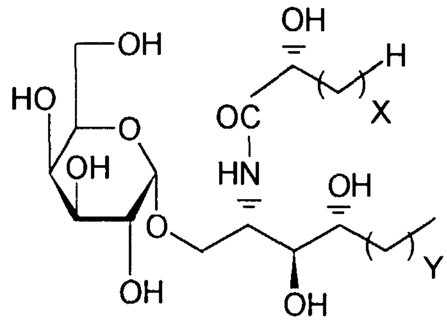

(X)

wobei X eine ganze Zahl von 18 bis 26 bezeichnet und Y eine ganze Zahl von 5 bis 17 bezeichnet.

**19.** Glycosphingolipid gemäss Anspruch 16, dargestellt durch die Formel (X'):

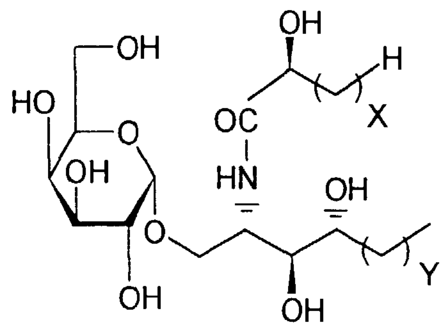

(X')

wobei X eine ganze Zahl von 20 bis 24 bezeichnet und Y eine ganze Zahl von 10 bis 14 bezeichnet.

**20.** Glycosphingolipid gemäss Anspruch 18, wobei X eine ganze Zahl von 21 bis 25 bezeichnet und Y eine ganze Zahl von 6 bis 16 bezeichnet.

**21.** Glycosphingolipid gemäss Anspruch 19, wobei X eine ganze Zahl von 21 bis 23 bezeichnet und Y eine ganze Zahl von 11 bis 13 bezeichnet.

**22.** Glycosphingolipid gemäss Anspruch 2, dargestellt durch die Formel (XI):

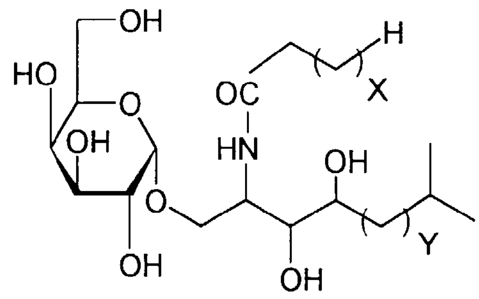

(XI)

wobei X eine ganze Zahl von 20 bis 24 bezeichnet und Y eine ganze Zahl von 9 bis 13 bezeichnet.

23. Glycosphingolipid gemäss Anspruch 22, wobei X eine ganze Zahl von 21 bis 23 bezeichnet und Y eine ganze Zahl von 10 bis 12 bezeichnet.

24. Glycosphingolipid gemäss Anspruch 22, dargestellt durch die Formel (XII):

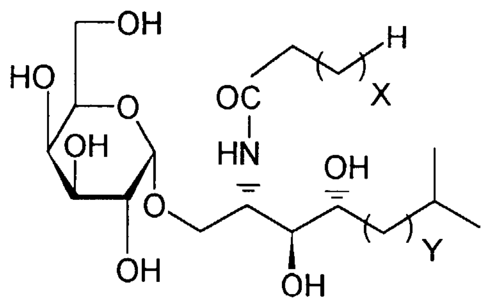

(XII)

wobei X eine ganze Zahl von 20 bis 24 bezeichnet und Y eine ganze Zahl von 9 bis 13 bezeichnet.

25. Glycosphingolipid gemäss Anspruch 24, wobei X eine ganze Zahl von 21 bis 23 bezeichnet und Y eine ganze Zahl von 10 bis 12 bezeichnet.

26. Glycosphingolipid gemäss Anspruch 2, dargestellt durch die Formel (XIII):

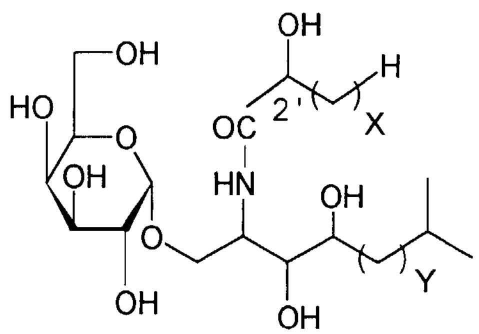

(XIII)

wobei X eine ganze Zahl von 18 bis 24 bezeichnet und Y eine ganze Zahl von 9 bis 13 bezeichnet.

27. Glycosphingolipid gemäss Anspruch 26, wobei X eine ganze Zahl von 20 bis 23 bezeichnet und Y eine ganze Zahl von 10 bis 12 bezeichnet.

28. Glycosphingolipid gemäss Anspruch 26, dargestellt durch die Formel (XIV):

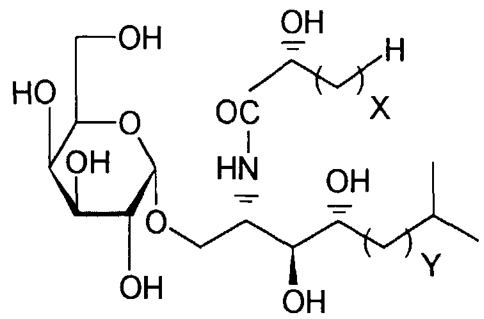

(XIV)

wobei X eine ganze Zahl von 19 bis 23 bezeichnet und Y eine ganze Zahl von 9 bis 13 bezeichnet.

**29.** Glycosphingolipid gemäss Anspruch 26, dargestellt durch die Formel (XIV'):

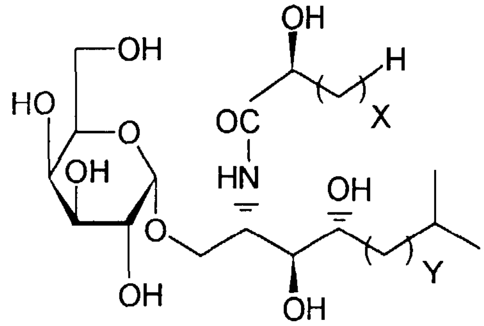

(XIV')

wobei X eine ganze Zahl von 20 bis 24 bezeichnet und Y eine ganze Zahl von 9 bis 13 bezeichnet.

**30.** Glycosphingolipid gemäss Anspruch 28, wobei X eine ganze Zahl von 20 bis 22 bezeichnet und Y eine ganze Zahl von 10 bis 12 bezeichnet.

**31.** Glycosphingolipid gemäss Anspruch 29, wobei X eine ganze Zahl von 21 bis 23 bezeichnet und Y eine ganze Zahl von 10 bis 12 bezeichnet.

**32.** Glycosphingolipid gemäss Anspruch 2, dargestellt durch die Formel (XV):

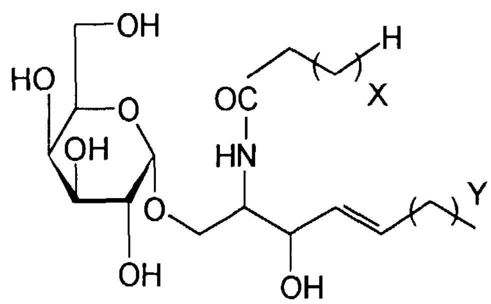

(XV)

wobei X eine ganze Zahl von 10 bis 18 bezeichnet und Y eine ganze Zahl von 10 bis 14 bezeichnet.

**33.** Glycosphingolipid gemäss Anspruch 32, wobei X eine ganze Zahl von 11 bis 17 bezeichnet und Y eine ganze Zahl von 11 bis 13 bezeichnet.

**34.** Glycosphingolipid gemäss Anspruch 32, dargestellt durch die Formel (XVI):

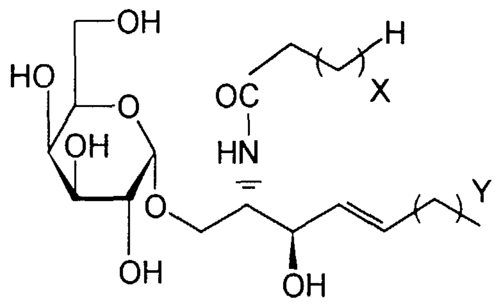

(XVI)

wobei X eine ganze Zahl von 10 bis 18 bezeichnet und Y eine ganze Zahl von 10 bis 14 bezeichnet.

**35.** Glycosphingolipid gemäss Anspruch 34, wobei X eine ganze Zahl von 11 bis 17 bezeichnet und Y eine ganze Zahl von 11 bis 13 bezeichnet.

**36.** Glycosphingolipid gemäss Anspruch 2, dargestellt durch die Formel (XVII):

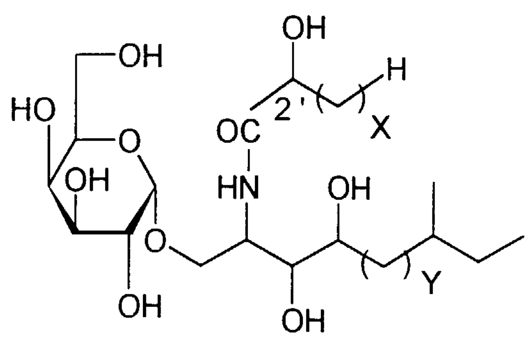

(XVII)

wobei X eine ganze Zahl von 21 bis 25 bezeichnet und Y eine ganze Zahl von 9 bis 13 bezeichnet.

**37.** Glycosphingolipid gemäss Anspruch 36, wobei X eine ganze Zahl von 22 bis 24 bezeichnet und Y eine ganze Zahl von 10 bis 12 bezeichnet.

**38.** Glycosphingolipid gemäss Anspruch 36, dargestellt durch die Formel (XVIII):

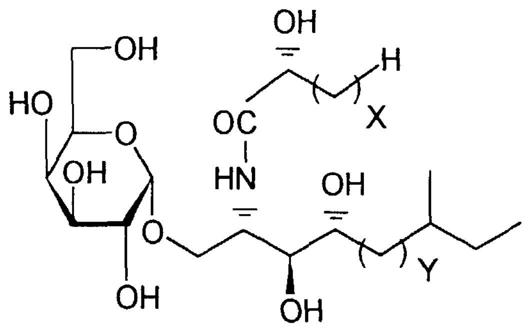

(XVIII)

wobei X eine ganze Zahl von 21 bis 25 bezeichnet und Y eine ganze Zahl von 9 bis 13 bezeichnet.

**39.** Glycosphingolipid gemäss Anspruch 38, wobei X eine ganze Zahl von 22 bis 24 bezeichnet und Y eine ganze Zahl von 10 bis 12 bezeichnet.

**40.** Glycosphingolipid gemäss Anspruch 1, dargestellt durch die Formel (XIX):

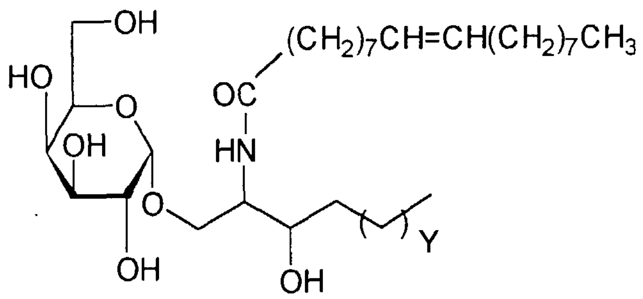

(XIX)

wobei Y eine ganze Zahl von 11 bis 15 bezeichnet.

**41.** Glycosphingolipid gemäss Anspruch 40, wobei Y eine ganze Zahl von 12 bis 14 bezeichnet.

**42.** Glycosphingolipid gemäss Anspruch 40, dargestellt durch die Formel (XX):

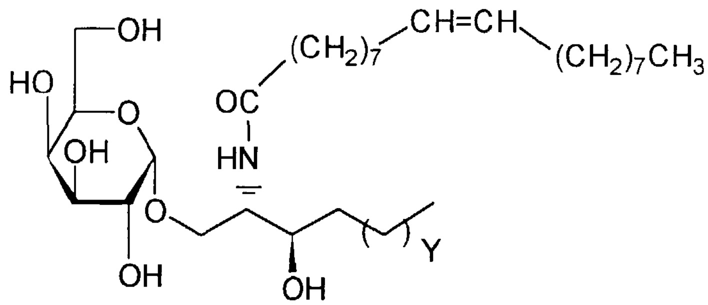

(XX)

wobei Y eine ganze Zahl von 11 bis 15 bezeichnet.

**43.** Glycosphingolipid gemäss Anspruch 42, wobei Y eine ganze Zahl von 12 bis 14 bezeichnet.

**44.** Glycosphingolipid gemäss Anspruch 1, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol,
(10) (2S,3R)-1-(α-D-Galactopyranosyloxy)-2-tetradecanoylamino-3-hexadecanol,
(11) (2R,3S)-1-(α-D-Galactopyranosyloxy)-2-tetradecanoylamino-3-hexadecanol,
(12) (2S,3S)-1-(α-D-Galactopyranosyloxy)-2-tetradecanoylamino-3-hexadecanol,
(13) (2R,3R)-1-(α-D-Galactopyranosyloxy)-2-tetradecanoylamino-3-hexadecanol,
(14) (2S,3R)-1-(α-D-Galactopyranosyloxy)-2-[(R)-2-hydroxytetracosanoylamino]-3-octadecanol,
(15) (2S,3R,4E)-1-(α-D-Galactopyranosyloxy)-2-octadecanoylamino-4-octadecen-3-ol,

(16) (2S,3R,4E)-1-(α-D-Galactopyranosyloxy)-2-tetradecanoylamino-4-octadecen-3-ol,
(17) (2S,3S,4R)-1-(α-D-Galactopyranosyloxy)-2-tetracosanoylamino-3,4-octadecandiol,
(18) (2S,3S,4R)-1-(α-D-Galactopyranosyloxy)-2-tetracosanoylamino-3,4-heptadecandiol,
(19) (2S,3S,4R)-1-(α-D-Galactopyranosyloxy)-2-tetracosanoylamino-3,4-pentadecandiol,
(20) (2S,3S,4R)-1-(α-D-Galactopyranosyloxy)-2-tetracosanoylamino-3,4-undecandiol,
(21) (2S,3S,4R)-1-(α-D-Galactopyranosyloxy)-2-hexacosanoylamino-3,4-heptadecandiol,
(22) (2S,3S,4R)-1-(α-D-Galactopyranosyloxy)-2-[(R)-2-hydroxytetracosanoylamino]-3,4-octadecandiol,
(23) (2S,3S,4R)-1-(α-D-Galactopyranosyloxy)-2-[(R)-2-hydroxytetracosanoylamino]-3,4-heptadecandiol,
(24) (2S,3S,4R)-1-(α-D-Galactopyranosyloxy)-2-[(R)-2-hydroxytetracosanoylamino]-3,4-pentadecandiol,
(25) (2S,3S,4R)-1-(α-D-Galactopyranosyloxy)-2-[(R)-2-hydroxytetracosanoylamino]-3,4-undecandiol,
(26) (2S,3S,4R)-1-(α-D-Galactopyranosyloxy)-2-[(R)-2-hydroxyhexacosanoylamino]-3,4-octadecandiol,
(27) (2S,3S,4R)-1-(α-D-Galactopyranosyloxy)-2-[(R)-2-hydroxyhexacosanoylamino]-3,4-nonadecandiol,
(28) (2S,3S,4R)-1-(α-D-Galactopyranosyloxy)-2-[(R)-2-hydroxyhexacosanoylamino]-3,4-icosandiol,
(29) (2S,3S,4R)-1-(α-D-Galactopyranosyloxy)-2-[(S)-2-hydroxytetracosanoylamino]-3,4-heptadecandiol,
(30) (2S,3S,4R)-1-(α-D-Galactopyranosyloxy)-2-[(R)-2-hydroxytetracosanoylamino]-3,4-hexadecandiol,
(31) (2S,3S,4R)-1-(α-D-Galactopyranosyloxy)-2-[(S)-2-hydroxytetracosanoylamino]-16-methyl-3,4-heptadecandiol,
(32) (2S,3S,4R)-1-(α-D-Galactopyranosyloxy)-16-methyl-2-tetracosanoylamino-3,4-heptadecandiol,
(33) (2S,3S,4R)-1-(α-D-Galactopyranosyloxy)-2-[(R)-2-hydroxytricosanoylamino]-16-methyl-3,4-heptadecandiol,
(34) (2S,3S,4R)-1-(α-D-Galactopyranosyloxy)-2-[(R)-2-hydroxypentacosanoylamino]-16-methyl-3,4-octadecandiol und
(35) (2S,3R)-1-(α-D-Galactopyranosyloxy)-2-oleoylamino-3-octadecanol.

**45.** Glycosphingolipid gemäss Anspruch 44, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol und
(10) (2S,3R)-1-(α-D-Galactopyranosyloxy)-2-tetradecanoylamino-3-hexadecanol.

**46.** Glycosphingolipid gemäss Anspruch 45, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-tetracosanoylamino-3-tetradecanol und
(8) (2S,3R)-1-(α-D-Galactopyranosyloxy)-2-tetradecanoylamino-3-hexadecanol.

**47.** Glycosphingolipid gemäss Anspruch 46, ausgewählt aus der Gruppe bestehend aus den folgenden Verbindungen:

(1) (2S,3R)-1-(α-D-Galactopyranosyloxy)-2-octadecanoylamino-3-octadecanol,
(2) (2S,3R)-1-(α-D-Galactopyranosyloxy)-2-tetradecanoylamino-3-octadecanol und
(3) (2S,3R)-1-(α-D-Galactopyranosyloxy)-2-tetradecanoylamino-3-hexadecanol.

**48.** Glycosphingolipid gemäss Anspruch 44, nämlich (2S,3R)-1-(α-D-Galactopyranosyloxy)-2-[(R)-2-hydroxytetracosanoylamino]-3-octadecanol.

**49.** Glycosphingolipid gemäss Anspruch 44, ausgewählt aus der Gruppe bestehend aus den folgenden Verbindungen:

(1) (2S,3R,4E)-1-(α-D-Galactopyranosyloxy)-2-octadecanoylamino-4-octadecen-3-ol und
(2) (2S,3R,4E)-1-(α-D-Galactopyranosyloxy)-2-tetradecanoylamino-4-octadecen-3-ol.

**50.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-tetracosanoylamino-3,4-octadecandiol,
(2) (2S,3S,4R)-1-(α-D-Galactopyranosyloxy)-2-tetracosanoylamino-3,4-heptadecandiol,
(3) (2S,3S,4R)-1-(α-D-Galactopyranosyloxy)-2-tetracosanoylamino-3,4-pentadecandiol,
(4) (2S,3S,4R)-1-(α-D-Galactopyranosyloxy)-2-tetracosanoylamino-3,4-undecandiol und
(5) (2S,3S,4R)-1-(α-D-Galactopyranosyloxy)-2-hexacosanoylamino-3,4-heptadecandiol.

**51.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-[(R)-2-hydroxytetracosanoylamino]-3,4-octadecandiol,
(2) (2S,3S,4R)-1-(α-D-Galactopyranosyloxy)-2-[(R)-2-hydroxytetracosanoylamino]-3,4-heptadecandiol,
(3) (2S,3S,4R)-1-(α-D-Galactopyranosyloxy)-2-[(R)-2-hydroxytetracosanoylamino]-3,4-pentadecandiol,
(4) (2S,3S,4R)-1-(α-D-Galactopyranosyloxy)-2-[(R)-2-hydroxytetracosanoylamino]-3,4-undecandiol,
(5) (2S,3S,4R)-1-(α-D-Galactopyranosyloxy)-2-[(R)-2-hydroxyhexacosanoylamino]-3,4-octadecandiol,
(6) (2S,3S,4R)-1-(α-D-Galactopyranosyloxy)-2-[(R)-2-hydroxyhexacosanoylamino]-3,4-nonadecandiol,
(7) (2S,3S,4R)-1-(α-D-Galactopyranosyloxy)-2-[(R)-2-hydroxyhexacosanoylamino]-3,4-icosandiol,
(8) (2S,3S,4R)-1-(α-D-Galactopyranosyloxy)-2-[(S)-2-hydroxytetracosanoylamino]-3,4-heptadecandiol und
(9) (2S,3S,4R)-1-(α-D-Galactopyranosyloxy)-2-[(R)-2-hydroxytetracosanoylamino]-3,4-hexadecandiol.

**52.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-[(S)-2-hydroxytetracosanoylamino]-16-methyl-3,4-heptadecandiol,
(2) (2S,3S,4R)-1-(α-D-Galactopyranosyloxy)-16-methyl-2-tetracosanoylamino-3,4-heptadecandiol und
(3) (2S,3S,4R)-1-(α-D-Galactopyranosyloxy)-2-[(R)-2-hydroxytricosanoylamino]-16-methyl-3,4-heptadecandiol.

**53.** Glycosphingolipid gemäss Anspruch 44, nämlich (2S,3S,4R)-1-(α-D-Galactopyranosyloxy)-2-[(R)-2-hydroxypentacosanoylamino]-16-methyl-3,4-octadecandiol.

**54.** Glycosphingolipid gemäss Anspruch 44, nämlich (2S,3R)-1-(α-D-Galactopyranosyloxy)-2-oleoylamino-3-octandecanol.

**55.** Verfahren für ein Glycosphingolipid, umfassend das Aufsammeln des Meeresschwammes Agelas mauritianus, Unterwerfen desselben einem Extraktionsvorgang mit einem organischen Lösungsmittel, und Isolieren aus dem Extrakt mindestens eines der folgenden Glycosphingolipide, dargestellt durch die Formel (I) gemäss Anspruch 2:

(1) (2S,3S,4R)-1-(α-D-Galactopyranosyloxy)-2-[(R)-2-hydroxytetracosanoylamino]-3,4-heptadecandiol,
(2) (2S,3S,4R)-1-(α-D-Galactopyranosyloxy)-2-[(R)-2-hydroxytetracosanoylamino]-3,4-hexadecandiol,
(3) (2S,3S,4R)-1-(α-D-Galactopyranosyloxy)-2-[(R)-2-hydroxytricosanoylamino]-16-methyl-3,4-heptadecandiol,
(4) (2S,3S,4R)-1-(α-D-Galactopyranosyloxy)-2-[(R)-2-hydroxypentacosanoylamino]-16-methyl-3,4-octadecandiol.

**56.** Cytostatisches Mittel, umfassend ein oder mehrere der Verbindungen gemäss Anspruch 1 als Wirkstoff.

**57.** Immunstimulator, umfassend ein oder mehrere Verbindungen gemäss Anspruch 1 als Wirkstoff.

**58.** Cytostatisches Mittel, umfassend eine wirksame Menge einer oder mehrerer der Verbindungen gemäss Anspruch 1 und einen Träger oder ein Verdünnungsmittel.

**59.** Immunstimulator, umfassend eine wirksame Menge einer oder mehrerer der Verbindungen gemäss Anspruch 1 und einen Träger oder ein Verdünnungsmittel.

**60.** Verwendung einer oder mehrerer Verbindungen gemäss Anspruch 1 bei der Herstellung eines Cytostatischen Mittels.

**61.** Verwendung einer oder mehrerer Verbindungen gemäss Anspruch 1 bei der Herstellung eines immunstimulierenden Medikaments.

**Patentansprüche für folgenden Vertragsstaat : ES**

**1.** Verfahren zur Herstellung eines Glycosphingolipids mit einem α-Galactoserest, dargestellt durch die Formel (A):

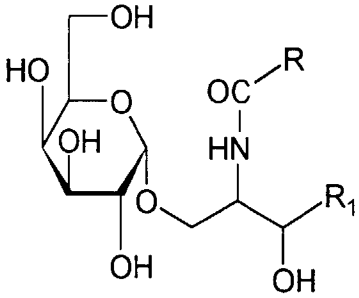

(A)

wobei R

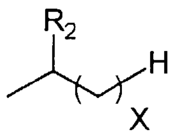

,

wobei $R_2$ H oder OH darstellt und X eine ganze Zahl von 0 bis 26 bezeichnet, oder $-(CH_2)_7CH{=}CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

und

(e) $-CH(OH)(CH_2)_YCH(CH_3)CH_2CH_3$,

wobei Y eine ganze Zahl von 5 bis 17 bezeichnet,

welches eines der folgenden Verfahren umfasst:

(a) Extrahieren von (A) aus einer Spezies des Meeresschwammes Algelas mauretianus mit mindestens einem geeigneten organischen Lösungsmittel und Wasser;

(b) Modifizieren von Sphingosin; und

(c) allgemeine chemische Reaktionen für die Synthese von Glycosphingolipiden.

**2.** Verfahren gemäss Anspruch 1, wobei ein Glycosphingolipid, dargestellt durch die Formel (I), hergestellt wird:

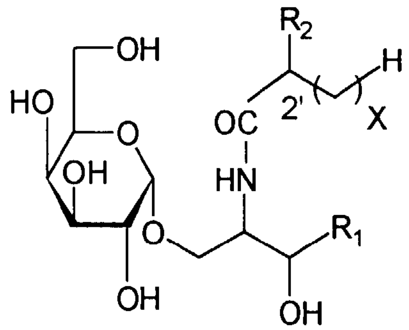

(I)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH{=}CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)\ (CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**3.** Verfahren gemäss Anspruch 2, wobei ein Glycosphingolipid, dargestellt durch die Formel (II), hergestellt wird:

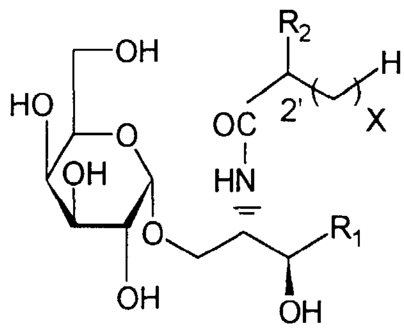

(II)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH=CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**4.** Verfahren gemäss Anspruch 2, wobei ein Glycosphingolipid, dargestellt durch die Formel (III), hergestellt wird:

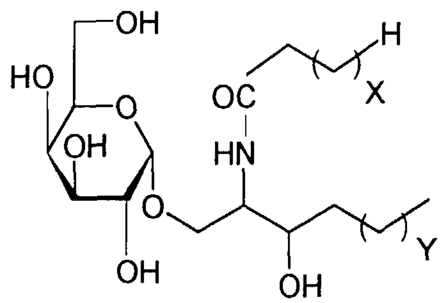

( III )

wobei X eine ganze Zahl von 0 bis 24 bezeichnet und Y eine ganze Zahl von 7 bis 15 bezeichnet.

5. Verfahren gemäss Anspruch 4, wobei X eine ganze Zahl von 8 bis 22 bezeichnet und Y eine ganze Zahl von 9 bis 13 bezeichnet.

6. Verfahren gemäss Anspruch 4, wobei ein Glycosphingolipid, dargestellt durch die Formel (IV), hergestellt wird:

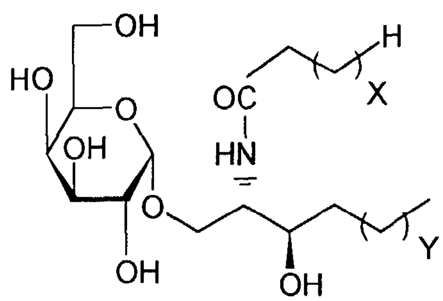

( IV )

wobei X eine ganze Zahl von 0 bis 24 bezeichnet und Y eine ganze Zahl von 7 bis 15 bezeichnet.

7. Verfahren gemäss Anspruch 6, wobei X eine ganze Zahl von 8 bis 22 bezeichnet und Y eine ganze Zahl von 9 bis 13 bezeichnet.

8. Verfahren gemäss Anspruch 2, wobei ein Glycosphingolipid, dargestellt durch die Formel (V), hergestellt wird:

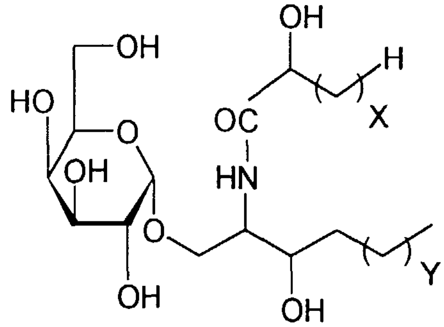

( V )

wobei X eine ganze Zahl von 20 bis 24 bezeichnet und Y eine ganze Zahl von 11 bis 15 bezeichnet.

9. Verfahren gemäss Anspruch 8, wobei X eine ganze Zahl von 21 bis 23 bezeichnet und Y eine ganze Zahl von 12 bis 14 bezeichnet.

10. Verfahren gemäss Anspruch 8, wobei ein Glycosphingolipid, dargestellt durch die Formel (VI), hergestellt wird:

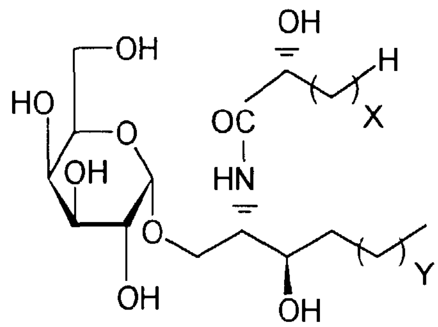

(VI)

wobei X eine ganze Zahl von 20 bis 24 bezeichnet und Y eine ganze Zahl von 11 bis 15 bezeichnet.

**11.** Verfahren gemäss Anspruch 10, wobei X eine ganze Zahl von 21 bis 23 bezeichnet und Y eine ganze Zahl von 12 bis 14 bezeichnet.

**12.** Verfahren gemäss Anspruch 2, wobei ein Glycosphingolipid, dargestellt durch die Formel (VII), hergestellt wird:

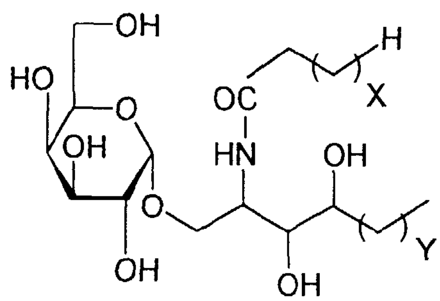

(VII)

wobei X eine ganze Zahl von 18 bis 26 bezeichnet und Y eine ganze Zahl von 5 bis 15 bezeichnet.

**13.** Verfahren gemäss Anspruch 12, wobei X eine ganze Zahl von 21 bis 25 bezeichnet und Y eine ganze Zahl von 6 bis 14 bezeichnet.

**14.** Verfahren gemäss Anspruch 12, wobei ein Glycosphingolipid, dargestellt durch die Formel (VIII), hergestellt wird:

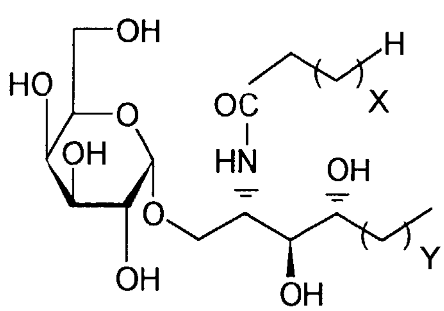

(VIII)

wobei X eine ganze Zahl von 18 bis 26 bezeichnet und Y eine ganze Zahl von 5 bis 15 bezeichnet.

**15.** Verfahren gemäss Anspruch 14, wobei X eine ganze Zahl von 21 bis 25 bezeichnet und Y eine ganze Zahl von 6 bis 14 bezeichnet.

**16.** Verfahren gemäss Anspruch 2, wobei ein Glycosphingolipid, dargestellt durch die Formel (IX), hergestellt wird:

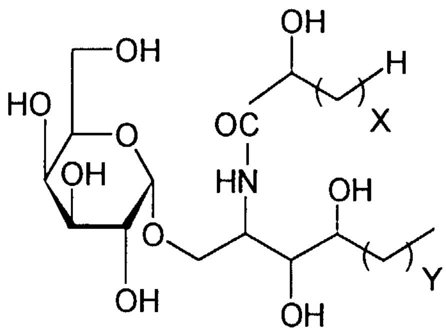

( IX )

wobei X eine ganze Zahl von 18 bis 26 bezeichnet und Y eine ganze Zahl von 5 bis 17 bezeichnet.

**17.** Verfahren gemäss Anspruch 16, wobei X eine ganze Zahl von 21 bis 25 bezeichnet und Y eine ganze Zahl von 6 bis 16 bezeichnet.

**18.** Verfahren gemäss Anspruch 16,, wobei ein Glycosphingolipidm dargestellt durch die Formel (X), hergestellt wird:

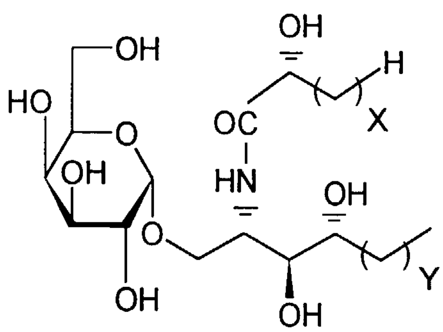

( X )

wobei X eine ganze Zahl von 18 bis 26 bezeichnet und Y eine ganze Zahl von 5 bis 17 bezeichnet.

**19.** Verfahren gemäss Anspruch 16,, wobei ein Glycosphingolipid, dargestellt durch die Formel (X'), hergestellt wird:

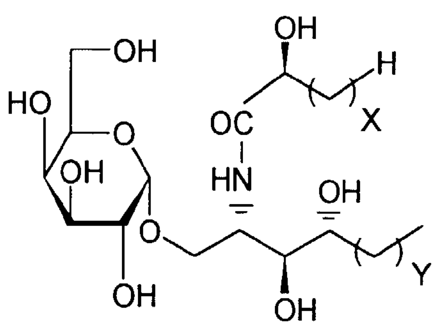

( X ' )

wobei X eine ganze Zahl von 20 bis 24 bezeichnet und Y eine ganze Zahl von 10 bis 14 bezeichnet.

**20.** Verfahren gemäss Anspruch 18, wobei X eine ganze Zahl von 21 bis 25 bezeichnet und Y eine ganze Zahl von 6 bis 16 bezeichnet.

21. Verfahren gemäss Anspruch 19, wobei X eine ganze Zahl von 21 bis 23 bezeichnet und Y eine ganze Zahl von 11 bis 13 bezeichnet.

22. Verfahren gemäss Anspruch 2, wobei ein Glycosphingolipid, dargestellt durch die Formel (XI), hergestellt wird:

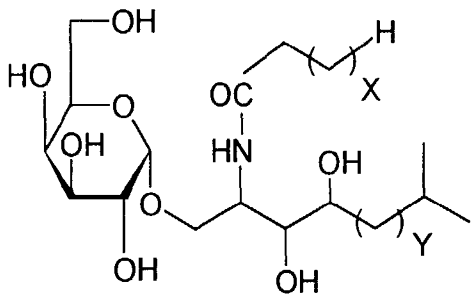

(XI)

wobei X eine ganze Zahl von 20 bis 24 bezeichnet und Y eine ganze Zahl von 9 bis 13 bezeichnet.

23. Verfahren gemäss Anspruch 22, wobei X eine ganze Zahl von 21 bis 23 bezeichnet und Y eine ganze Zahl von 10 bis 12 bezeichnet.

24. Verfahren gemäss Anspruch 22, wobei ein Glycosphingolipid, dargestellt durch die Formel (XII), hergestellt wird:

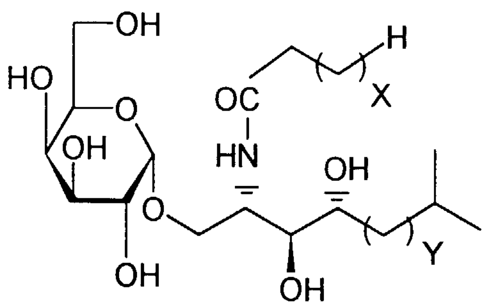

(XII)

wobei X eine ganze Zahl von 20 bis 24 bezeichnet und Y eine ganze Zahl von 9 bis 13 bezeichnet.

25. Verfahren gemäss Anspruch 24, wobei X eine ganze Zahl von 21 bis 23 bezeichnet und Y eine ganze Zahl von 10 bis 12 bezeichnet.

26. Verfahren gemäss Anspruch 2, wobei ein Glycosphingolipid, dargestellt durch die Formel (XIII), hergestellt wird:

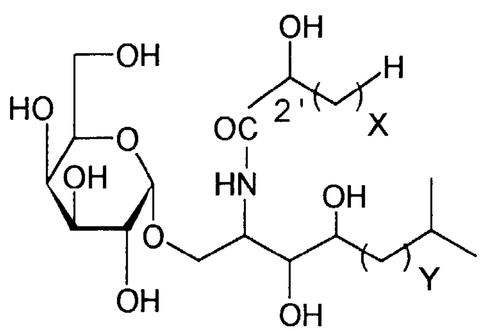

(XIII)

wobei X eine ganze Zahl von 18 bis 24 bezeichnet und Y eine ganze Zahl von 9 bis 13 bezeichnet.

**27.** Verfahren gemäss Anspruch 26, wobei X eine ganze Zahl von 20 bis 23 bezeichnet und Y eine ganze Zahl von 10 bis 12 bezeichnet.

**28.** Verfahren gemäss Anspruch 26, wobei ein Glycosphingolipid, dargestellt durch die Formel (XIV), hergestellt wird:

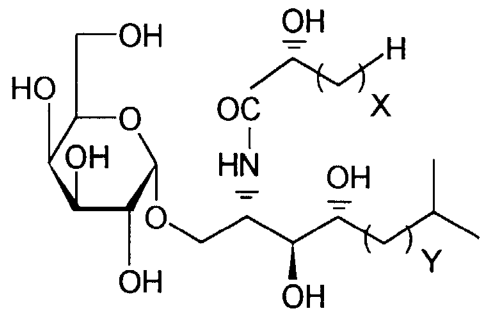

(XIV)

wobei X eine ganze Zahl von 19 bis 23 bezeichnet und Y eine ganze Zahl von 9 bis 13 bezeichnet.

**29.** Verfahren gemäss Anspruch 26, wobei ein Glycosphingolipid, dargestellt durch die Formel (XIV'), hergestellt wird:

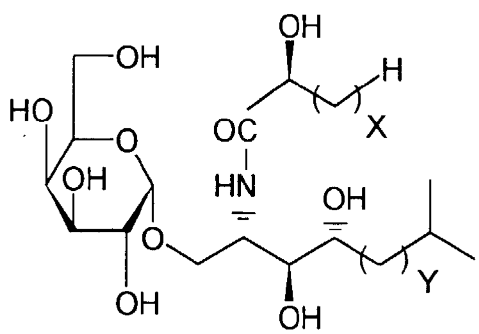

(XIV')

wobei X eine ganze Zahl von 20 bis 24 bezeichnet und Y eine ganze Zahl von 9 bis 13 bezeichnet.

**30.** Verfahren gemäss Anspruch 28, wobei X eine ganze Zahl von 20 bis 22 bezeichnet und Y eine ganze Zahl von 10 bis 12 bezeichnet.

**31.** Verfahren gemäss Anspruch 29, wobei X eine ganze Zahl von 21 bis 23 bezeichnet und Y eine ganze Zahl von 10 bis 12 bezeichnet.

**32.** Verfahren gemäss Anspruch 2, wobei ein Glycosphingolipid, dargestellt durch die Formel (XV), hergestellt wird:

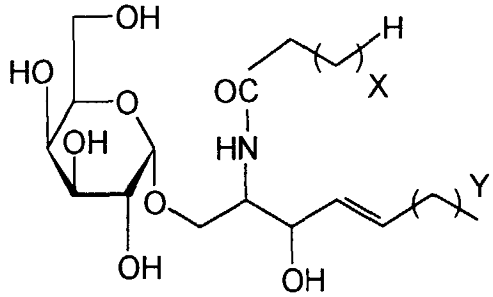

(XV)

wobei X eine ganze Zahl von 10 bis 18 bezeichnet und Y eine ganze Zahl von 10 bis 14 bezeichnet.

**33.** Verfahren gemäss Anspruch 32, wobei X eine ganze Zahl von 11 bis 17 bezeichnet und Y eine ganze Zahl von 11 bis 13 bezeichnet.

**34.** Verfahren gemäss Anspruch 32, wobei ein Glycosphingolipid, dargestellt durch die Formel (XVI), hergestellt wird:

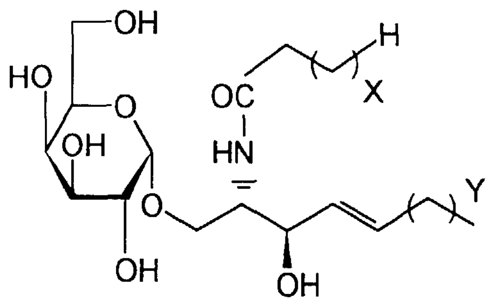

(XVI)

wobei X eine ganze Zahl von 10 bis 18 bezeichnet und Y eine ganze Zahl von 10 bis 14 bezeichnet.

**35.** Verfahren gemäss Anspruch 34, wobei X eine ganze Zahl von 11 bis 17 bezeichnet und Y eine ganze Zahl von 11 bis 13 bezeichnet.

**36.** Verfahren gemäss Anspruch 2, wobei ein Glycosphingolipid, dargestellt durch die Formel (XVII), hergestellt wird:

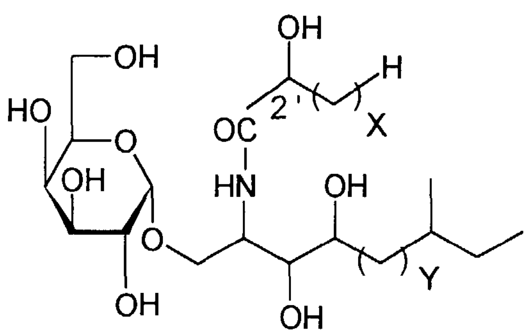

(XVII)

wobei X eine ganze Zahl von 21 bis 25 bezeichnet und Y eine ganze Zahl von 9 bis 13 bezeichnet.

**37.** Verfahren gemäss Anspruch 36, wobei X eine ganze Zahl von 22 bis 24 bezeichnet und Y eine ganze Zahl von 10 bis 12 bezeichnet.

**38.** Verfahren gemäss Anspruch 36, wobei ein Glycosphingolipid, dargestellt durch die Formel (XVIII), hergestellt wird:

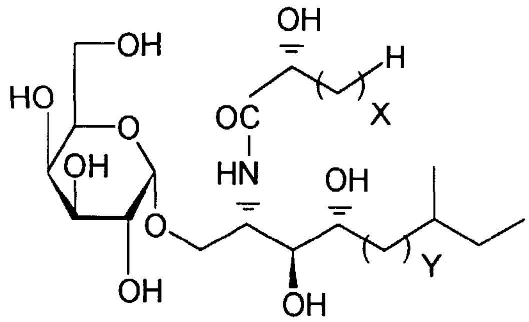

( XVIII )

wobei X eine ganze Zahl von 21 bis 25 bezeichnet und Y eine ganze Zahl von 9 bis 13 bezeichnet.

**39.** Verfahren gemäss Anspruch 38, wobei X eine ganze Zahl von 22 bis 24 bezeichnet und Y eine ganze Zahl von 10 bis 12 bezeichnet.

**40.** Verfahren gemäss Anspruch 1, wobei ein Glycosphingolipid, dargestellt durch die Formel (XIX), hergestellt wird:

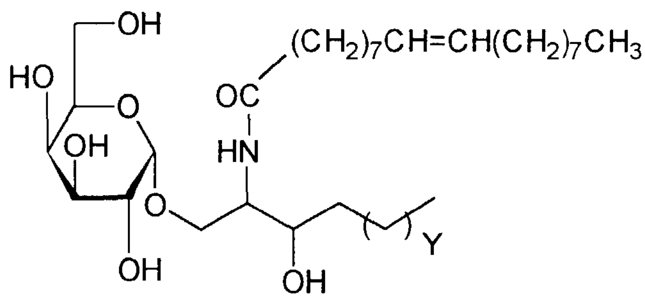

( XIX )

wobei Y eine ganze Zahl von 11 bis 15 bezeichnet.

**41.** Verfahren gemäss Anspruch 40, wobei Y eine ganze Zahl von 12 bis 14 bezeichnet.

**42.** Verfahren gemäss Anspruch 40, wobei ein Glycosphingolipid, dargestellt durch die Formel (XX), hergestellt wird:

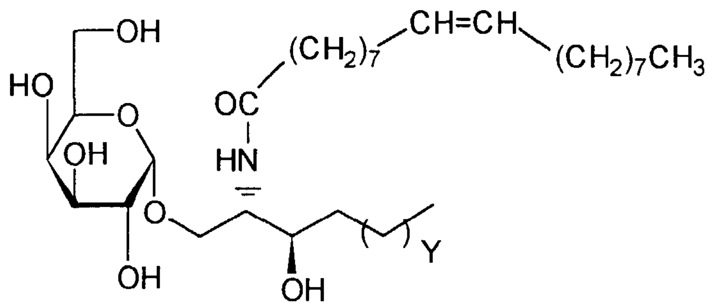

( XX )

wobei Y eine ganze Zahl von 11 bis 15 bezeichnet.

**43.** Verfahren gemäss Anspruch 42, wobei Y eine ganze Zahl von 12 bis 14 bezeichnet.

**44.** Verfahren gemäss Anspruch 1, wobei ein Glycosphingolipid, ausgewählt aus der Gruppe bestehend aus den folgenden Verbindungen, hergestellt wird:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol,
(10) (2S,3R)-1-(α-D-Galactopyranosyloxy)-2-tetradecanoylamino-3-hexadecanol,
(11) (2R,3S)-1-(α-D-Galactopyranosyloxy)-2-tetradecanoylamino-3-hexadecanol,
(12) (2S,3S)-1-(α-D-Galactopyranosyloxy)-2-tetradecanoylamino-3-hexadecanol,
(13) (2R,3R)-1-(α-D-Galactopyranosyloxy)-2-tetradecanoylamino-3-hexadecanol,
(14) (2S,3R)-1-(α-D-Galactopyranosyloxy)-2-[(R)-2-hydroxytetracosanoylamino]-3-octadecanol,
(15) (2S,3R,4E)-1-(α-D-Galactopyranosyloxy)-2-octadecanoylamino-4-octadecen-3-ol,
(16) (2S,3R,4E)-1-(α-D-Galactopyranosyloxy)-2-tetradecanoylamino-4-octadecen-3-ol,
(17) (2S,3S,4R)-1-(α-D-Galactopyranosyloxy)-2-tetracosanoylamino-3,4-octadecandiol,
(18) (2S,3S,4R)-1-(α-D-Galactopyranosyloxy)-2-tetracosanoylamino-3,4-heptadecandiol,
(19) (2S,3S,4R)-1-(α-D-Galactopyranosyloxy)-2-tetracosanoylamino-3,4-pentadecandiol,
(20) (2S,3S,4R)-1-(α-D-Galactopyranosyloxy)-2-tetracosanoylamino-3,4-undecandiol,
(21) (2S,3S,4R)-1-(α-D-Galactopyranosyloxy)-2-hexacosanoylamino-3,4-heptadecandiol,
(22) (2S,3S,4R)-1-(α-D-Galactopyranosyloxy)-2-[(R)-2-hydroxytetracosanoylamino]-3,4-octadecandiol,
(23) (2S,3S,4R)-1-(α-D-Galactopyranosyloxy)-2-[(R)-2-hydroxytetracosanoylamino]-3,4-heptadecandiol,
(24) (2S,3S,4R)-1-(α-D-Galactopyranosyloxy)-2-[(R)-2-hydroxytetracosanoylamino]-3,4-pentadecandiol,
(25) (2S,3S,4R)-1-(α-D-Galactopyranosyloxy)-2-[(R)-2-hydroxytetracosanoylamino]-3,4-undecandiol,
(26) (2S,3S,4R)-1-(α-D-Galactopyranosyloxy)-2-[(R)-2-hydroxyhexacosanoylamino]-3,4-octadecandiol,
(27) (2S,3S,4R)-1-(α-D-Galactopyranosyloxy)-2-[(R)-2-hydroxyhexacosanoylamino]-3,4-nonadecandiol,
(28) (2S,3S,4R)-1-(α-D-Galactopyranosyloxy)-2-[(R)-2-hydroxyhexacosanoylamino]-3,4-icosandiol,
(29) (2S,3S,4R)-1-(α-D-Galactopyranosyloxy)-2-[(S)-2-hydroxytetracosanoylamino]-3,4-heptadecandiol,
(30) (2S,3S,4R)-1-(α-D-Galactopyranosyloxy)-2-[(R)-2-hydroxytetracosanoylamino]-3,4-hexadecandiol,
(31) (2S,3S,4R)-1-(α-D-Galactopyranosyloxy)-2-[(S)-2-hydroxytetracosanoylamino]-16-methyl-3,4-heptadecandiol,
(32) (2S,3S,4R)-1-(α-D-Galactopyranosyloxy)-16-methyl-2-tetracosanoylamino-3,4-heptadecandiol,
(33) (2S,3S,4R)-1-(α-D-Galactopyranosyloxy)-2-[(R)-2-hydroxytricosanoylamino]-16-methyl-3,4-heptadecandiol,
(34) (2S,3S,4R)-1-(α-D-Galactopyranosyloxy)-2-[(R)-2-hydroxypentacosanoylamino]-16-methyl-3,4-octadecandiol und
(35) (2S,3R)-1-(α-D-Galactopyranosyloxy)-2-oleoylamino-3-octadecanol.

**45.** Verfahren für ein Glycosphingolipid, umfassend das Aufsammeln des Meeresschwammes Agelas mauritianus, Unterwerfen desselben einem Extraktionsvorgang mit einem organischen Lösungsmittel, und Isolieren aus dem Extrakt mindestens eines der folgenden Glycosphingolipide, dargestellt durch die Formel (I) gemäss Anspruch 2:

(1) (2S,3S,4R)-1-(α-D-Galactopyranosyloxy)-2-[(R)-2-hydroxytetracosanoylamino]-3,4-heptadecandiol,
(2) (2S,3S,4R)-1-(α-D-Galactopyranosyloxy)-2-[(R)-2-hydroxytetracosanoylamino]-3,4-hexadecandiol,
(3) (2S,3S,4R)-1-(α-D-Galactopyranosyloxy)-2-[(R)-2-hydroxytricosanoylamino]-16-methyl-3,4-heptadecandiol,
(4) (2S,3S,4R)-1-(α-D-Galactopyranosyloxy)-2-[(R)-2-hydroxypentacosanoylamino]-16-methyl-3,4-octadecandiol.

**46.** Verfahren zur Herstellung eines cytostatischen Mittels, umfassend das Mischen einer oder mehrerer Verbindungen, dargestellt durch die Formel (A):

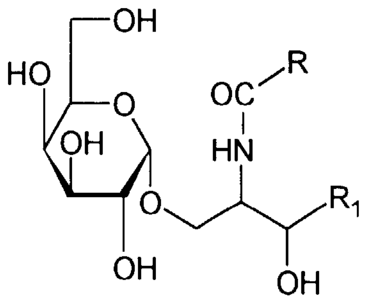

(A)

wobei R

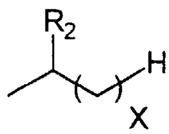

,

wobei $R_2$ H oder OH darstellt und X eine ganze Zahl von 0 bis 26 bezeichnet, oder $-(CH_2)_7CH{=}CH(CH_2)_7CH_3$ darstellt und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

und

(e) $-CH(OH)(CH_2)_YCH(CH_3)CH_2CH_3$,

wobei Y eine ganze Zahl von 5 bis 17 bezeichnet,

als Wirkstoff(e) mit pharmazeutisch annehmbaren Additiven.

**47.** Verfahren zur Herstellung eines Immunstimulators, umfassend das Mischen einer oder mehrerer Verbindungen, dargestellt durch die Formel (A)

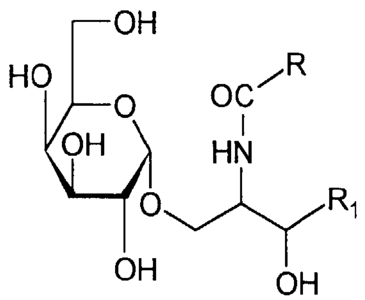 (A)

wobei R

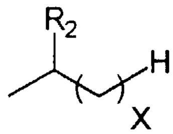 ,

wobei $R_2$ H oder OH darstellt und X eine ganze Zahl von 0 bis 26 bezeichnet, oder $-(CH_2)_7CH{=}CH(CH_2)_7CH_3$ darstellt und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

und

(e) $-CH(OH)(CH_2)_YCH(CH_3)CH_2CH_3$,

wobei Y eine ganze Zahl von 5 bis 17 bezeichnet,

als Wirkstoff(e) mit pharmazeutisch annehmbaren Additiven.

**48.** Verwendung einer oder mehrerer Verbindungen, dargestellt durch die Formel (A)

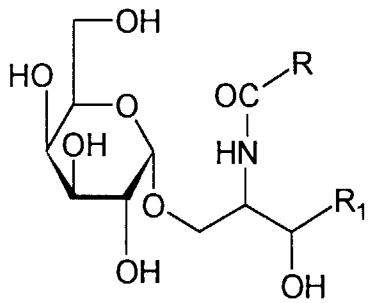

(A)

wobei R

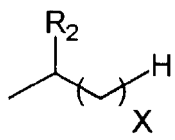

,

wobei $R_2$ H oder OH darstellt und X eine ganze Zahl von 0 bis 26 bezeichnet, oder $-(CH_2)_7CH=CH(CH_2)_7CH_3$ darstellt und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet,

bei der Herstellung eines cytostatischen Mittels.

**49.** Verwendung einer oder mehrerer Verbindungen, dargestellt durch die Formel (A)

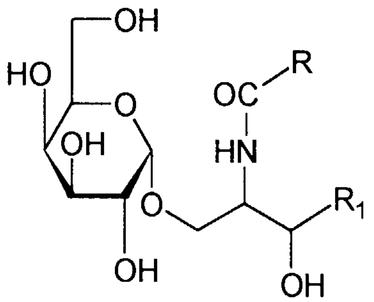

(A)

wobei R

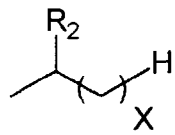

,

wobei $R_2$ H oder OH darstellt und X eine ganze Zahl von 0 bis 26 bezeichnet, oder $-(CH_2)_7CH{=}CH(CH_2)_7CH_3$ darstellt und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)\ (CH_2)_YCH(CH_3)_2$,

(d) $-CH{=}CH(CH_2)_YCH_3$

und

(e) $-CH(OH)(CH_2)_YCH(CH_3)CH_2CH_3$,

wobei Y eine ganze Zahl von 5 bis 17 bezeichnet,

bei der Herstellung eines immunstimulierenden Medikaments.

**Patentansprüche für folgenden Vertragsstaat : GR**

1. Glycosphingolipid mit einem α-Galactoserest, dargestellt durch die Formel (A):

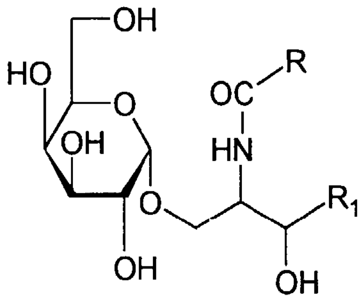

(A)

wobei R

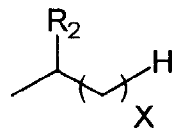

,

wobei $R_2$ H oder OH darstellt und X eine ganze Zahl von 0 bis 26 bezeichnet, oder $-(CH_2)_7CH{=}CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

und

(e) $-CH(OH)(CH_2)_YCH(CH_3)CH_2CH_3$,

wobei Y eine ganze Zahl von 5 bis 17 bezeichnet.

**2.** Glycosphingolipid gemäss Anspruch 1, dargestellt durch die Formel (I):

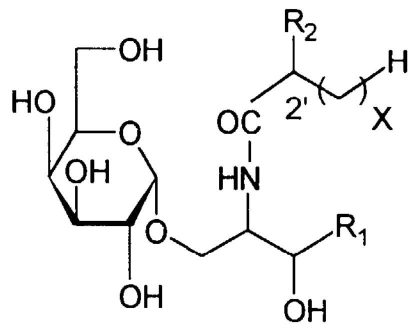

(I)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH=CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**3.** Glycosphingolipid gemäss Anspruch 2, dargestellt durch die Formel (II):

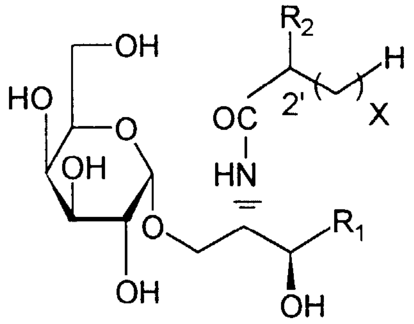

( II )

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH{=}CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**4.** Glycosphingolipid gemäss Anspruch 2, dargestellt durch die Formel (III):

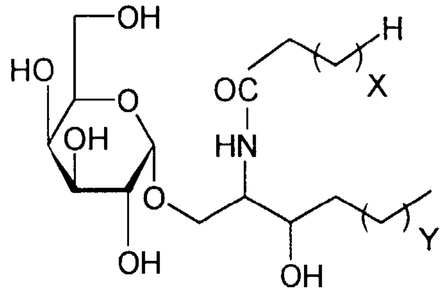

( III )

wobei X eine ganze Zahl von 0 bis 24 bezeichnet und Y eine ganze Zahl von 7 bis 15 bezeichnet.

**5.** Glycosphingolipid gemäss Anspruch 4, wobei X eine ganze Zahl von 8 bis 22 bezeichnet und Y eine ganze Zahl von 9 bis 13 bezeichnet.

**6.** Glycosphingolipid gemäss Anspruch 4, dargestellt durch die Formel (IV):

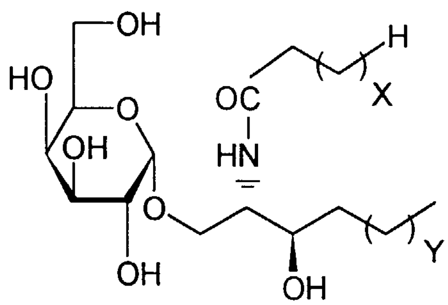

( IV )

wobei X eine ganze Zahl von 0 bis 24 bezeichnet und Y eine ganze Zahl von 7 bis 15 bezeichnet.

**7.** Glycosphingolipid gemäss Anspruch 6, wobei X eine ganze Zahl von 8 bis 22 bezeichnet und Y eine ganze Zahl von 9 bis 13 bezeichnet.

**8.** Glycosphingolipid gemäss Anspruch 2, dargestellt durch die Formel (V):

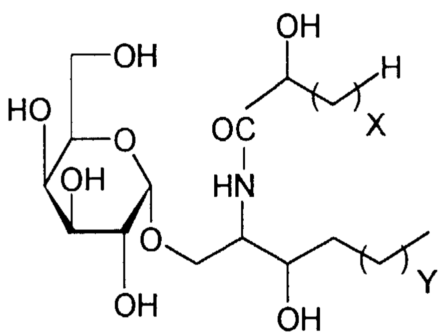

( V )

wobei X eine ganze Zahl von 20 bis 24 bezeichnet und Y eine ganze Zahl von 11 bis 15 bezeichnet.

**9.** Glycosphingolipid gemäss Anspruch 8, wobei X eine ganze Zahl von 21 bis 23 bezeichnet und Y eine ganze Zahl von 12 bis 14 bezeichnet.

**10.** Glycosphingolipid gemäss Anspruch 8, dargestellt durch die Formel (VI):

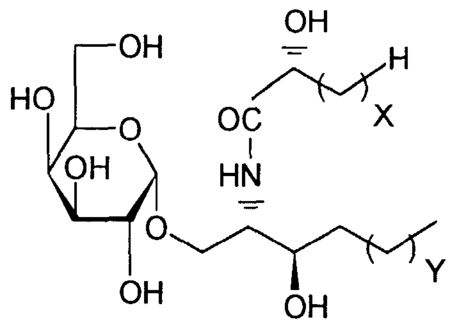

(VI)

wobei X eine ganze Zahl von 20 bis 24 bezeichnet und Y eine ganze Zahl von 11 bis 15 bezeichnet.

**11.** Glycosphingolipid gemäss Anspruch 10, wobei X eine ganze Zahl von 21 bis 23 bezeichnet und Y eine ganze Zahl von 12 bis 14 bezeichnet.

**12.** Glycosphingolipid gemäss Anspruch 2, dargestellt durch die Formel (VII):

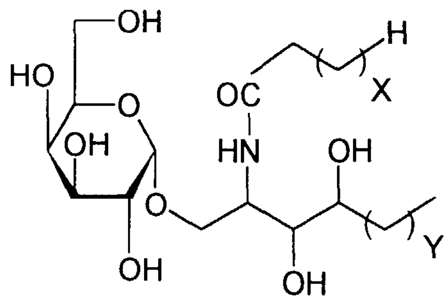

(VII)

wobei X eine ganze Zahl von 18 bis 26 bezeichnet und Y eine ganze Zahl von 5 bis 15 bezeichnet.

**13.** Glycosphingolipid gemäss Anspruch 12, wobei X eine ganze Zahl von 21 bis 25 bezeichnet und Y eine ganze Zahl von 6 bis 14 bezeichnet.

**14.** Glycosphingolipid gemäss Anspruch 12, dargestellt durch die Formel (VIII):

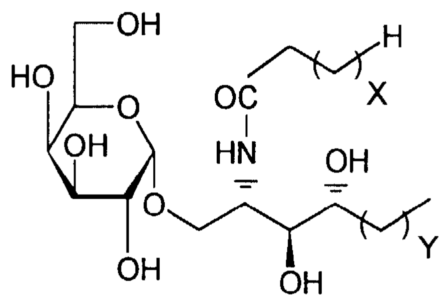

(VIII)

wobei X eine ganze Zahl von 18 bis 26 bezeichnet und Y eine ganze Zahl von 5 bis 15 bezeichnet.

**15.** Glycosphingolipid gemäss Anspruch 14, wobei X eine ganze Zahl von 21 bis 25 bezeichnet und Y eine ganze Zahl von 6 bis 14 bezeichnet.

**16.** Glycosphingolipid gemäss Anspruch 2, dargestellt durch die Formel (IX):

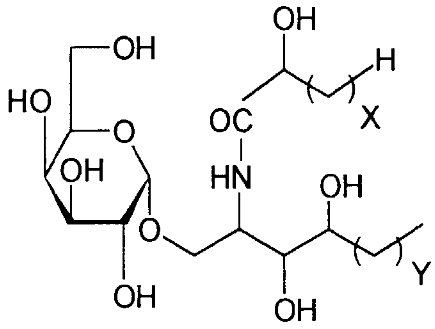

( IX )

wobei X eine ganze Zahl von 18 bis 26 bezeichnet und Y eine ganze Zahl von 5 bis 17 bezeichnet.

**17.** Glycosphingolipid gemäss Anspruch 16, wobei X eine ganze Zahl von 21 bis 25 bezeichnet und Y eine ganze Zahl von 6 bis 16 bezeichnet.

**18.** Glycosphingolipid gemäss Anspruch 16, dargestellt durch die Formel (X):

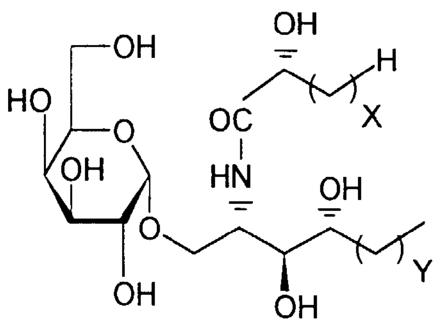

( X )

wobei X eine ganze Zahl von 18 bis 26 bezeichnet und Y eine ganze Zahl von 5 bis 17 bezeichnet.

**19.** Glycosphingolipid gemäss Anspruch 16, dargestellt durch die Formel (X'):

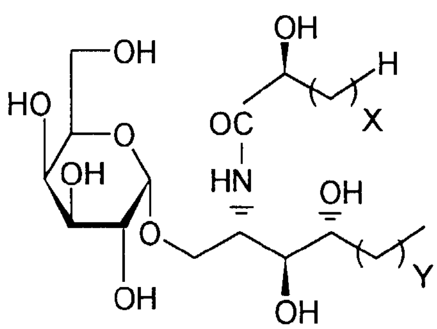

( X ' )

wobei X eine ganze Zahl von 20 bis 24 bezeichnet und Y eine ganze Zahl von 10 bis 14 bezeichnet.

**20.** Glycosphingolipid gemäss Anspruch 18, wobei X eine ganze Zahl von 21 bis 25 bezeichnet und Y eine ganze Zahl von 6 bis 16 bezeichnet.

**21.** Glycosphingolipid gemäss Anspruch 19, wobei X eine ganze Zahl von 21 bis 23 bezeichnet und Y eine ganze Zahl

von 11 bis 13 bezeichnet.

**22.** Glycosphingolipid gemäss Anspruch 2, dargestellt durch die Formel (XI):

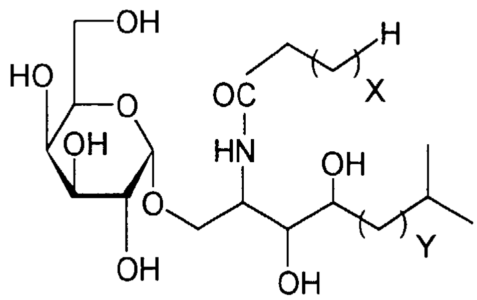

(XI)

wobei X eine ganze Zahl von 20 bis 24 bezeichnet und Y eine ganze Zahl von 9 bis 13 bezeichnet.

**23.** Glycosphingolipid gemäss Anspruch 22, wobei X eine ganze Zahl von 21 bis 23 bezeichnet und Y eine ganze Zahl von 10 bis 12 bezeichnet.

**24.** Glycosphingolipid gemäss Anspruch 22, dargestellt durch die Formel (XII):

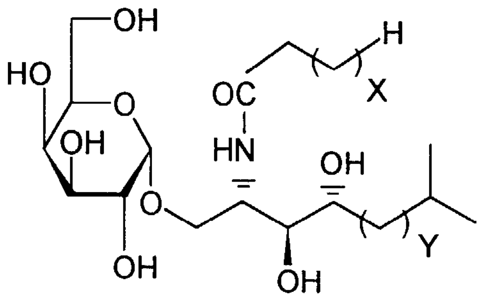

(XII)

wobei X eine ganze Zahl von 20 bis 24 bezeichnet und Y eine ganze Zahl von 9 bis 13 bezeichnet.

**25.** Glycosphingolipid gemäss Anspruch 24, wobei X eine ganze Zahl von 21 bis 23 bezeichnet und Y eine ganze Zahl von 10 bis 12 bezeichnet.

**26.** Glycosphingolipid gemäss Anspruch 2, dargestellt durch die Formel (XIII):

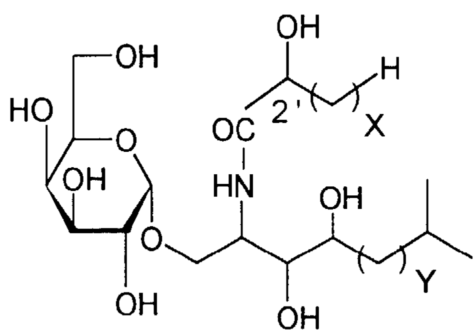

(XIII)

wobei X eine ganze Zahl von 18 bis 24 bezeichnet und Y eine ganze Zahl von 9 bis 13 bezeichnet.

**27.** Glycosphingolipid gemäss Anspruch 26, wobei X eine ganze Zahl von 20 bis 23 bezeichnet und Y eine ganze Zahl von 10 bis 12 bezeichnet.

**28.** Glycosphingolipid gemäss Anspruch 26, dargestellt durch die Formel (XIV):

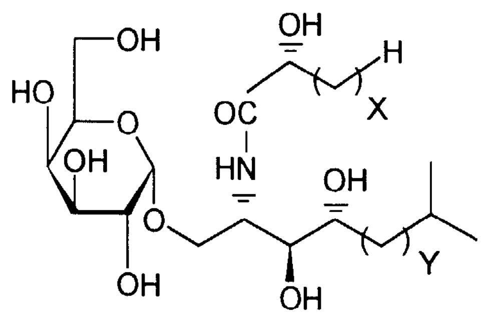

(XIV)

wobei X eine ganze Zahl von 19 bis 23 bezeichnet und Y eine ganze Zahl von 9 bis 13 bezeichnet.

**29.** Glycosphingolipid gemäss Anspruch 26, dargestellt durch die Formel (XIV'):

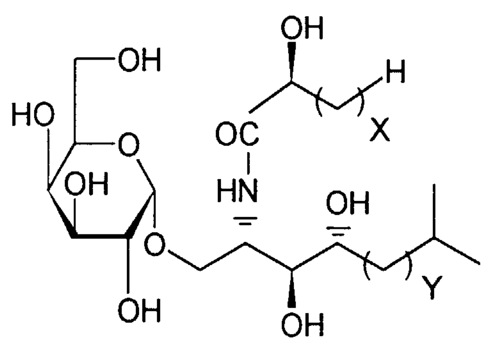

(XIV')

wobei X eine ganze Zahl von 20 bis 24 bezeichnet und Y eine ganze Zahl von 9 bis 13 bezeichnet.

**30.** Glycosphingolipid gemäss Anspruch 28, wobei X eine ganze Zahl von 20 bis 22 bezeichnet und Y eine ganze Zahl von 10 bis 12 bezeichnet.

**31.** Glycosphingolipid gemäss Anspruch 29, wobei X eine ganze Zahl von 21 bis 23 bezeichnet und Y eine ganze Zahl von 10 bis 12 bezeichnet.

**32.** Glycosphingolipid gemäss Anspruch 2, dargestellt durch die Formel (XV):

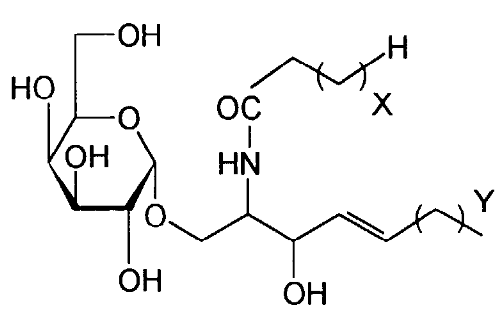

(XV)

wobei X eine ganze Zahl von 10 bis 18 bezeichnet und Y eine ganze Zahl von 10 bis 14 bezeichnet.

**33.** Glycosphingolipid gemäss Anspruch 32, wobei X eine ganze Zahl von 11 bis 17 bezeichnet und Y eine ganze Zahl von 11 bis 13 bezeichnet.

**34.** Glycosphingolipid gemäss Anspruch 32, dargestellt durch die Formel (XVI):

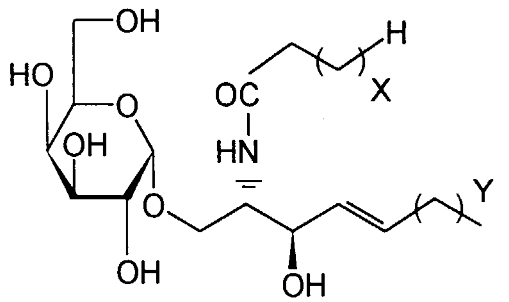

(XVI)

wobei X eine ganze Zahl von 10 bis 18 bezeichnet und Y eine ganze Zahl von 10 bis 14 bezeichnet.

**35.** Glycosphingolipid gemäss Anspruch 34, wobei X eine ganze Zahl von 11 bis 17 bezeichnet und Y eine ganze Zahl von 11 bis 13 bezeichnet.

**36.** Glycosphingolipid gemäss Anspruch 2, dargestellt durch die Formel (XVII):

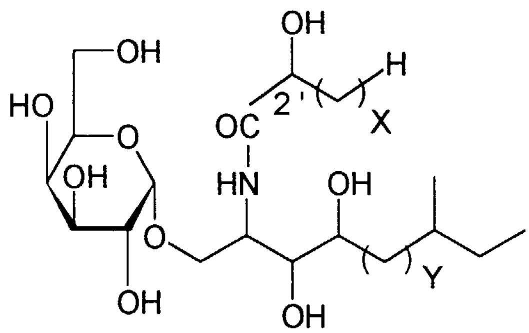

(XVII)

wobei X eine ganze Zahl von 21 bis 25 bezeichnet und Y eine ganze Zahl von 9 bis 13 bezeichnet.

**37.** Glycosphingolipid gemäss Anspruch 36, wobei X eine ganze Zahl von 22 bis 24 bezeichnet und Y eine ganze Zahl von 10 bis 12 bezeichnet.

**38.** Glycosphingolipid gemäss Anspruch 36, dargestellt durch die Formel (XVIII):

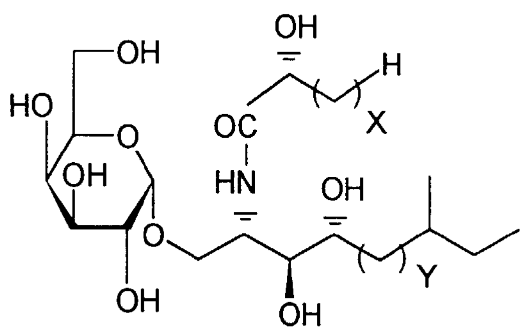

(XVIII)

wobei X eine ganze Zahl von 21 bis 25 bezeichnet und Y eine ganze Zahl von 9 bis 13 bezeichnet.

**39.** Glycosphingolipid gemäss Anspruch 38, wobei X eine ganze Zahl von 22 bis 24 bezeichnet und Y eine ganze Zahl von 10 bis 12 bezeichnet.

**40.** Glycosphingolipid gemäss Anspruch 1, dargestellt durch die Formel (XIX):

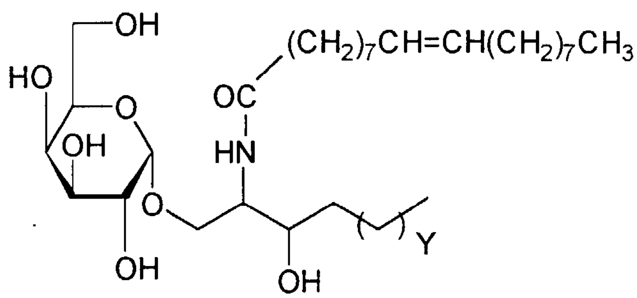

(XIX)

wobei Y eine ganze Zahl von 11 bis 15 bezeichnet.

**41.** Glycosphingolipid gemäss Anspruch 40, wobei Y eine ganze Zahl von 12 bis 14 bezeichnet.

**42.** Glycosphingolipid gemäss Anspruch 40, dargestellt durch die Formel (XX):

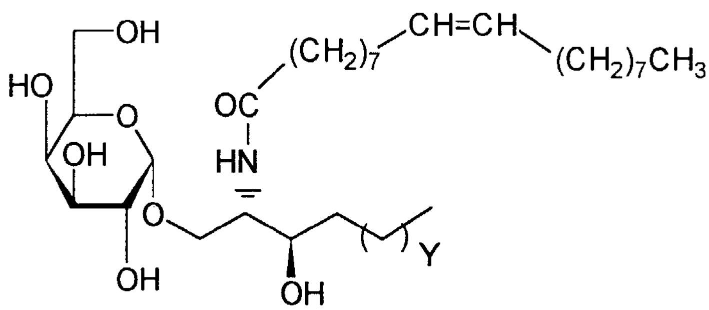

(XX)

wobei Y eine ganze Zahl von 11 bis 15 bezeichnet.

**43.** Glycosphingolipid gemäss Anspruch 42, wobei Y eine ganze Zahl von 12 bis 14 bezeichnet.

**44.** Glycosphingolipid gemäss Anspruch 1, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol,
(10) (2S,3R)-1-(α-D-Galactopyranosyloxy)-2-tetradecanoylamino-3-hexadecanol,
(11) (2R,3S)-1-(α-D-Galactopyranosyloxy)-2-tetradecanoylamino-3-hexadecanol,
(12) (2S,3S)-1-(α-D-Galactopyranosyloxy)-2-tetradecanoylamino-3-hexadecanol,
(13) (2R,3R)-1-(α-D-Galactopyranosyloxy)-2-tetradecanoylamino-3-hexadecanol,
(14) (2S,3R)-1-(α-D-Galactopyranosyloxy)-2-[(R)-2-hydroxytetracosanoylamino]-3-octadecanol,

(15) (2S,3R,4E)-1-(α-D-Galactopyranosyloxy)-2-octadecanoylamino-4-octadecen-3-ol,
(16) (2S,3R,4E)-1-(α-D-Galactopyranosyloxy)-2-tetradecanoylamino-4-octadecen-3-ol,
(17) (2S,3S,4R)-1-(α-D-Galactopyranosyloxy)-2-tetracosanoylamino-3,4-octadecandiol,
(18) (2S,3S,4R)-1-(α-D-Galactopyranosyloxy)-2-tetracosanoylamino-3,4-heptadecandiol,
(19) (2S,3S,4R)-1-(α-D-Galactopyranosyloxy)-2-tetracosanoylamino-3,4-pentadecandiol,
(20) (2S,3S,4R)-1-(α-D-Galactopyranosyloxy)-2-tetracosanoylamino-3,4-undecandiol,
(21) (2S,3S,4R)-1-(α-D-Galactopyranosyloxy)-2-hexacosanoylamino-3,4-heptadecandiol,
(22) (2S,3S,4R)-1-(α-D-Galactopyranosyloxy)-2-[(R)-2-hydroxytetracosanoylamino]-3,4-octadecandiol,
(23) (2S,3S,4R)-1-(α-D-Galactopyranosyloxy)-2-[(R)-2-hydroxytetracosanoylamino]-3,4-heptadecandiol,
(24) (2S,3S,4R)-1-(α-D-Galactopyranosyloxy)-2-[(R)-2-hydroxytetracosanoylamino]-3,4-pentadecandiol,
(25) (2S,3S,4R)-1-(α-D-Galactopyranosyloxy)-2-[(R)-2-hydroxytetracosanoylamino]-3,4-undecandiol,
(26) (2S,3S,4R)-1-(α-D-Galactopyranosyloxy)-2-[(R)-2-hydroxyhexacosanoylamino]-3,4-octadecandiol,
(27) (2S,3S,4R)-1-(α-D -Galactopyranosyloxy)-2-[(R)-2-hydroxyhexacosanoylamino]-3,4-nonadecandiol,
(28) (2S,3S,4R)-1-(α-D-Galactopyranosyloxy)-2-[(R)-2-hydroxyhexacosanoylamino]-3,4-icosandiol,
(29) (2S,3S,4R)-1-(α-D-Galactopyranosyloxy)-2-[(S)-2-hydroxytetracosanoylamino]-3,4-heptadecandiol,
(30) (2S,3S,4R)-1-(α-D-Galactopyranosyloxy)-2-[(R)-2-hydroxytetracosanoylamino]-3,4-hexadecandiol,
(31) (2S,3S,4R)-1-(α-D-Galactopyranosyloxy)-2-[(S)-2-hydroxytetracosanoylamino]-16-methyl-3,4-heptadecandiol,
(32) (2S,3S,4R)-1-(α-D-Galactopyranosyloxy)-16-methyl-2-tetracosanoylamino-3,4-heptadecandiol,
(33) (2S,3S,4R)-1-(α-D-Galactopyranosyloxy)-2-[(R)-2-hydroxytricosanoylamino]-16-methyl-3,4-heptadecandiol,
(34) (2S,3S,4R)-1-(α-D-Galactopyranosyloxy)-2-[(R)-2-hydroxypentacosanoylamino]-16-methyl-3,4-octadecandiol und
(35) (2S,3R)-1-(α-D-Galactopyranosyloxy)-2-oleoylamino-3-octadecanol.

**45.** Glycosphingolipid gemäss Anspruch 44, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol und
(10) (2S,3R)-1-(α-D-Galactopyranosyloxy)-2-tetradecanoylamino-3-hexadecanol.

**46.** Glycosphingolipid gemäss Anspruch 45, ausgewählt aus der Gruppe bestehend aus den folgenden Verbindungen:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-tetracosanoylamino-3-tetradecanol und
(8) (2S,3R)-1-(α-D-Galactopyranosyloxy)-2-tetradecanoylamino-3-hexadecanol.

**47.** Glycosphingolipid gemäss Anspruch 46, ausgewählt aus der Gruppe bestehend aus den folgenden Verbindungen:

(1) (2S,3R)-1-(α-D-Galactopyranosyloxy)-2-octadecanoylamino-3-octadecanol,
(2) (2S,3R)-1-(α-D-Galactopyranosyloxy)-2-tetradecanoylamino-3-octadecanol und
(3) (2S,3R)-1-(α-D-Galactopyranosyloxy)-2-tetradecanoylamino-3-hexadecanol.

**48.** Glycosphingolipid gemäss Anspruch 44, nämlich (2S,3R)-1-(α-D-Galactopyranosyloxy)-2-[(R)-2-hydroxytetracosanoylamino]-3-octadecanol.

**49.** Glycosphingolipid gemäss Anspruch 44, ausgewählt aus der Gruppe bestehend aus den folgenden Verbindungen:

(1) (2S,3R,4E)-1-(α-D-Galactopyranosyloxy)-2-octadecanoylamino-4-octadecen-3-ol und
(2) (2S,3R,4E)-1-(α-D-Galactopyranosyloxy)-2-tetradecanoylamino-4-octadecen-3-ol.

**50.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-tetracosanoylamino-3,4-octadecandiol,
(2) (2S,3S,4R)-1-(α-D-Galactopyranosyloxy)-2-tetracosanoylamino-3,4-heptadecandiol,
(3) (2S,3S,4R)-1-(α-D-Galactopyranosyloxy)-2-tetracosanoylamino-3,4-pentadecandiol,
(4) (2S,3S,4R)-1-(α-D-Galactopyranosyloxy)-2-tetracosanoylamino-3,4-undecandiol und
(5) (2S,3S,4R)-1-(α-D-Galactopyranosyloxy)-2-hexacosanoylamino-3,4-heptadecandiol.

**51.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-[(R)-2-hydroxytetracosanoylamino]-3,4-octadecandiol,
(2) (2S,3S,4R)-1-(α-D-Galactopyranosyloxy)-2-[(R)-2-hydroxytetracosanoylamino]-3,4-heptadecandiol,
(3) (2S,3S,4R)-1-(α-D-Galactopyranosyloxy)-2-[(R)-2-hydroxytetracosanoylamino]-3,4-pentadecandiol,
(4) (2S,3S,4R)-1-(α-D-Galactopyranosyloxy)-2-[(R)-2-hydroxytetracosanoylamino]-3,4-undecandiol,
(5) (2S,3S,4R)-1-(α-D-Galactopyranosyloxy)-2-[(R)-2-hydroxyhexacosanoylamino]-3,4-octadecandiol,
(6) (2S,3S,4R)-1-(α-D-Galactopyranosyloxy)-2-[(R)-2-hydroxyhexacosanoylamino]-3,4-nonadecandiol,
(7) (2S,3S,4R)-1-(α-D-Galactopyranosyloxy)-2-[(R)-2-hydroxyhexacosanoylamino]-3,4-icosandiol,
(8) (2S,3S,4R)-1-(α-D-Galactopyranosyloxy)-2-[(S)-2-hydroxytetracosanoylamino]-3,4-heptadecandiol und
(9) (2S,3S,4R)-1-(α-D-Galactopyranosyloxy)-2-[(R)-2-hydroxytetracosanoylamino]-3,4-hexadecandiol.

**52.** Glycosphingolipid gemäss Anspruch 44, ausgewählt aus der Gruppe bestehend aus den folgenden Verbindungen:

(1) (2S,3S,4R)-1-(α-D-Galactopyranosyloxy)-2-[(S)-2-hydroxytetracosanoylamino]-16-methyl-3,4-heptadecandiol,
(2) (2S,3S,4R)-1-(α-D-Galactopyranosyloxy)-16-methyl-2-tetracosanoylamino-3,4-heptadecandiol und
(3) (2S,3S,4R)-1-(α-D-Galactopyranosyloxy)-2-[(R)-2-hydroxytricosanoylamino]-16-methyl-3,4-heptadecandiol.

**53.** Glycosphingolipid gemäss Anspruch 44, nämlich (2S,3S,4R)-1-(α-D-Galactopyranosyloxy)-2-[(R)-2-hydroxypentacosanoylamino]-16-methyl-3,4-octadecandiol.

**54.** Glycosphingolipid gemäss Anspruch 44, nämlich (2S,3R)-1-(α-D-Galactopyranosyloxy)-2-oleoylamino-3-octandecanol.

**55.** Verfahren zur Herstellung eines Glycosphingolipids mit einem α-Galactoserest, dargestellt durch die Formel (A):

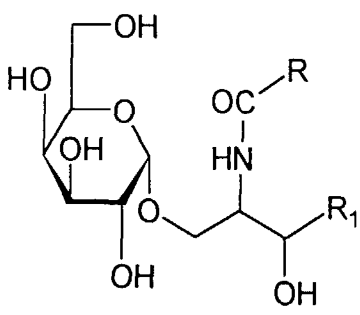

(A)

wobei R

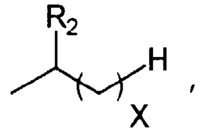

wobei $R_2$ H oder OH darstellt und X eine ganze Zahl von 0 bis 26 bezeichnet, oder $-(CH_2)_7CH=CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet,

welches eines der folgenden Verfahren umfasst:

(a) Extrahieren von (A) aus einer Spezies des Meeresschwammes Algelas mauretianus mit mindestens einem geeigneten organischen Lösungsmittel und Wasser;

(b) Modifizieren von Sphingosin; und

(c) allgemeine chemische Reaktionen für die Synthese von Glycosphingolipiden.

**56.** Verfahren gemäss Anspruch 55, wobei ein Glycosphingolipid, dargestellt durch die Formel (I), hergestellt wird:

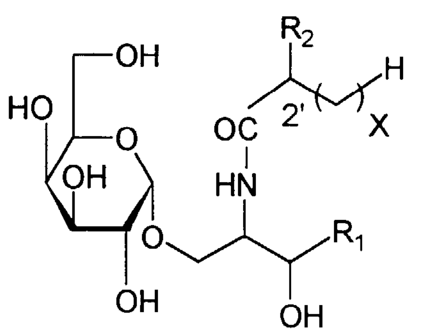

(I)

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH{=}CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**57.** Verfahren gemäss Anspruch 56, wobei ein Glycosphingolipid, dargestellt durch die Formel (II), hergestellt wird:

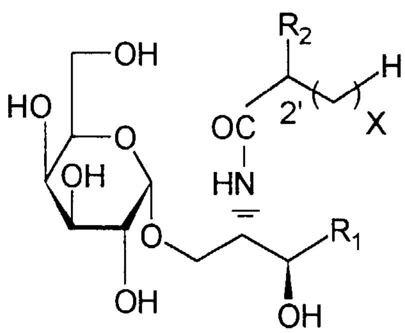

( II )

wobei $R_1$ einen der durch (a) bis (e) nachstehend definierten Substituenten darstellt, und $R_2$ H oder OH darstellt (X wird nachstehend in (a) bis (e) definiert):

(a) $-CH_2(CH_2)_YCH_3$,

wobei, wenn $R_2$ H darstellt, X eine ganze Zahl von 0 bis 24 bezeichnet und Y eine ganze Zahl von 7 bis 15 bezeichnet; wenn $R_2$ OH darstellt, X eine ganze Zahl von 20 bis 24 bezeichnet und Y eine ganze Zahl von 11 bis 15 bezeichnet;

(b) $-CH(OH)(CH_2)_YCH_3$,

wobei, wenn $R_2$ H darstellt, X eine ganze Zahl von 18 bis 26 bezeichnet und Y eine ganze Zahl von 5 bis 15 bezeichnet; wenn $R_2$ OH darstellt, X eine ganze Zahl von 18 bis 26 bezeichnet und Y eine ganze Zahl von 5 bis 17 bezeichnet;

(c) $-CH(OH)(CH_2)_YCH(CH_3)_2$,

wobei, wenn $R_2$ H darstellt, X eine ganze Zahl von 20 bis 24 bezeichnet und Y eine ganze Zahl von 9 bis 13 bezeichnet; wenn $R_2$ OH darstellt, X eine ganze Zahl von 18 bis 24 bezeichnet und Y eine ganze Zahl von 9 bis 13 bezeichnet;

(d) $-CH=CH(CH_2)_YCH_3$

wobei $R_2$ H darstellt, X eine ganze Zahl von 10 bis 18 bezeichnet und Y eine ganze Zahl von 10 bis 14 bezeichnet; und

(e) $-CH(OH)(CH_2)_YCH(CH_3)CH_2CH_3$,

wobei $R_2$ OH darstellt, X eine ganze Zahl von 21 bis 25 bezeichnet und Y eine ganze Zahl von 9 bis 13 bezeichnet.

**58.** Verfahren gemäss Anspruch 56, wobei ein Glycosphingolipid, dargestellt durch die Formel (III), hergestellt wird:

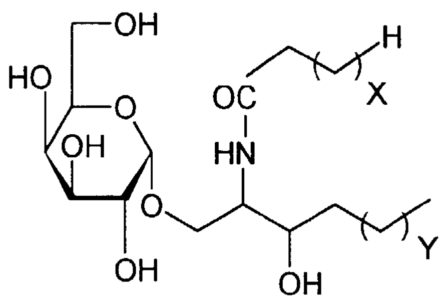

( III )

wobei X eine ganze Zahl von 0 bis 24 bezeichnet und Y eine ganze Zahl von 7 bis 15 bezeichnet.

**59.** Verfahren gemäss Anspruch 58, wobei X eine ganze Zahl von 8 bis 22 bezeichnet und Y eine ganze Zahl von 9 bis 13 bezeichnet.

**60.** Verfahren gemäss Anspruch 58, wobei ein Glycosphingolipid, dargestellt durch die Formel (IV), hergestellt wird:

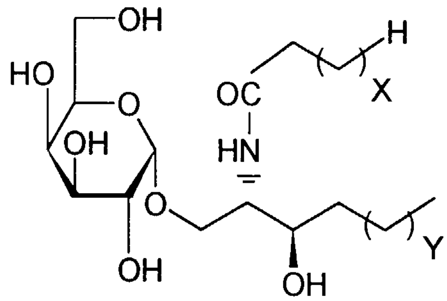

( IV )

wobei X eine ganze Zahl von 0 bis 24 bezeichnet und Y eine ganze Zahl von 7 bis 15 bezeichnet.

**61.** Verfahren gemäss Anspruch 60, wobei X eine ganze Zahl von 8 bis 22 bezeichnet und Y eine ganze Zahl von 9 bis 13 bezeichnet.

**62.** Verfahren gemäss Anspruch 56, wobei ein Glycosphingolipid, dargestellt durch die Formel (V), hergestellt wird:

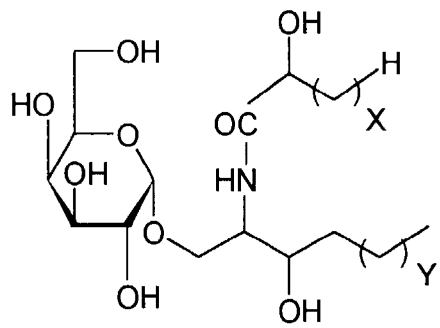

( V )

wobei X eine ganze Zahl von 20 bis 24 bezeichnet und Y eine ganze Zahl von 11 bis 15 bezeichnet.

**63.** Verfahren gemäss Anspruch 62, wobei X eine ganze Zahl von 21 bis 23 bezeichnet und Y eine ganze Zahl von 12 bis 14 bezeichnet.

**64.** Verfahren gemäss Anspruch 62, wobei ein Glycosphingolipid, dargestellt durch die Formel (VI), hergestellt wird:

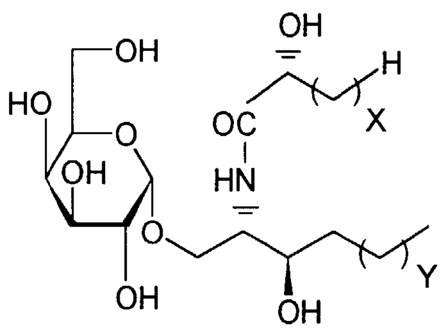

( VI )

wobei X eine ganze Zahl von 20 bis 24 bezeichnet und Y eine ganze Zahl von 11 bis 15 bezeichnet.

**65.** Verfahren gemäss Anspruch 64, wobei X eine ganze Zahl von 21 bis 23 bezeichnet und Y eine ganze Zahl von 12 bis 14 bezeichnet.

**66.** Verfahren gemäss Anspruch 56 wobei ein Glycosphingolipid, dargestellt durch die Formel (VII), hergestellt wird:

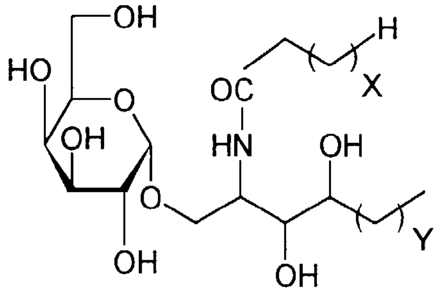

(VII)

wobei X eine ganze Zahl von 18 bis 26 bezeichnet und Y eine ganze Zahl von 5 bis 15 bezeichnet.

**67.** Verfahren gemäss Anspruch 66, wobei X eine ganze Zahl von 21 bis 25 bezeichnet und Y eine ganze Zahl von 6 bis 14 bezeichnet.

**68.** Verfahren gemäss Anspruch 66, wobei ein Glycosphingolipid, dargestellt durch die Formel (VIII), hergestellt wird:

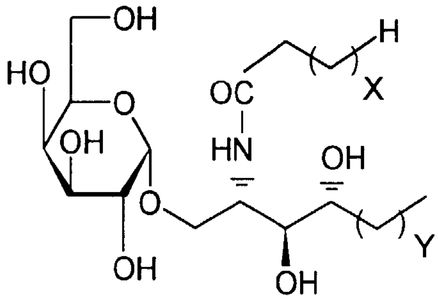

(VIII)

wobei X eine ganze Zahl von 18 bis 26 bezeichnet und Y eine ganze Zahl von 5 bis 15 bezeichnet.

**69.** Verfahren gemäss Anspruch 68, wobei X eine ganze Zahl von 21 bis 25 bezeichnet und Y eine ganze Zahl von 6 bis 14 bezeichnet.

**70.** Verfahren gemäss Anspruch 56, wobei ein Glycosphingolipid, dargestellt durch die Formel (IX), hergestellt wird:

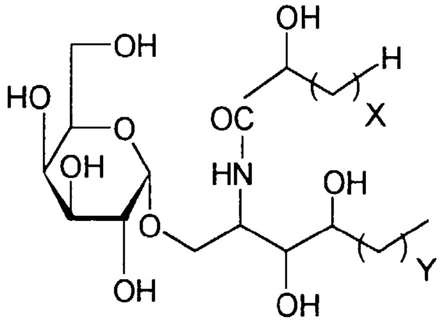

(IX)

wobei X eine ganze Zahl von 18 bis 26 bezeichnet und Y eine ganze Zahl von 5 bis 17 bezeichnet.

**71.** Verfahren gemäss Anspruch 70, wobei X eine ganze Zahl von 21 bis 25 bezeichnet und Y eine ganze Zahl von 6

bis 16 bezeichnet.

**72.** Verfahren gemäss Anspruch 70, wobei ein Glycosphingolipidm dargestellt durch die Formel (X), hergestellt wird:

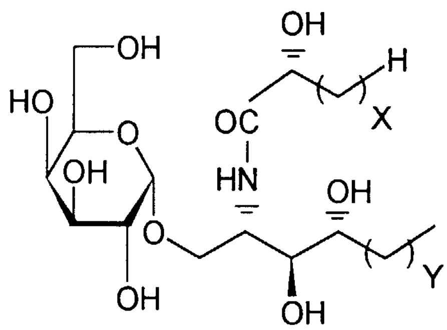

(X)

wobei X eine ganze Zahl von 18 bis 26 bezeichnet und Y eine ganze Zahl von 5 bis 17 bezeichnet.

**73.** Verfahren gemäss Anspruch 70,, wobei ein Glycosphingolipid, dargestellt durch die Formel (X'), hergestellt wird:

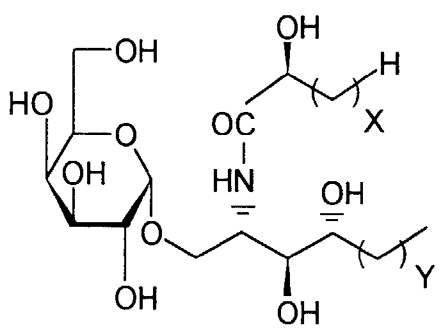

(X')

wobei X eine ganze Zahl von 20 bis 24 bezeichnet und Y eine ganze Zahl von 10 bis 14 bezeichnet.

**74.** Verfahren gemäss Anspruch 72, wobei X eine ganze Zahl von 21 bis 25 bezeichnet und Y eine ganze Zahl von 6 bis 16 bezeichnet.

**75.** Verfahren gemäss Anspruch 74, wobei X eine ganze Zahl von 21 bis 23 bezeichnet und Y eine ganze Zahl von 11 bis 13 bezeichnet.

**76.** Verfahren gemäss Anspruch 56, wobei ein Glycosphingolipid, dargestellt durch die Formel (XI), hergestellt wird:

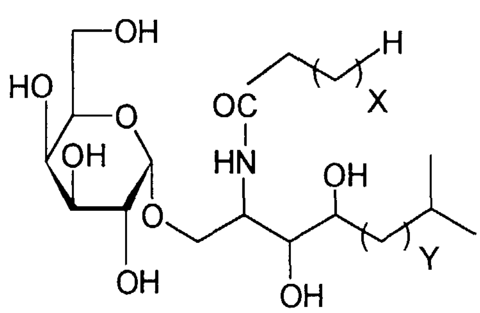

(XI)

wobei X eine ganze Zahl von 20 bis 24 bezeichnet und Y eine ganze Zahl von 9 bis 13 bezeichnet.

**77.** Verfahren gemäss Anspruch 76, wobei X eine ganze Zahl von 21 bis 23 bezeichnet und Y eine ganze Zahl von 10 bis 12 bezeichnet.

**78.** Verfahren gemäss Anspruch 76, wobei ein Glycosphingolipid, dargestellt durch die Formel (XII), hergestellt wird:

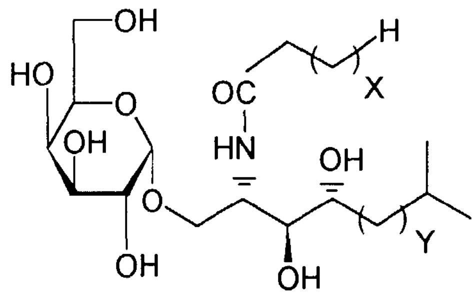

(XII)

wobei X eine ganze Zahl von 20 bis 24 bezeichnet und Y eine ganze Zahl von 9 bis 13 bezeichnet.

**79.** Verfahren gemäss Anspruch 78, wobei X eine ganze Zahl von 21 bis 23 bezeichnet und Y eine ganze Zahl von 10 bis 12 bezeichnet.

**80.** Verfahren gemäss Anspruch 56, wobei ein Glycosphingolipid, dargestellt durch die Formel (XIII), hergestellt wird:

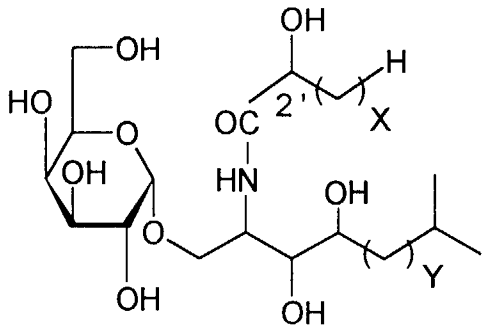

(XIII)

wobei X eine ganze Zahl von 18 bis 24 bezeichnet und Y eine ganze Zahl von 9 bis 13 bezeichnet.

**81.** Verfahren gemäss Anspruch 80, wobei X eine ganze Zahl von 20 bis 23 bezeichnet und Y eine ganze Zahl von 10 bis 12 bezeichnet.

**82.** Verfahren gemäss Anspruch 80, wobei ein Glycosphingolipid, dargestellt durch die Formel (XIV), hergestellt wird:

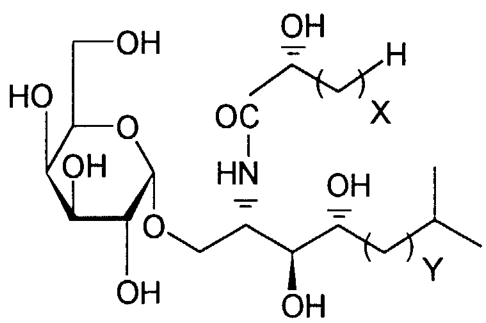

(XIV)

wobei X eine ganze Zahl von 19 bis 23 bezeichnet und Y eine ganze Zahl von 9 bis 13 bezeichnet.

**83.** Verfahren gemäss Anspruch 80, wobei ein Glycosphingolipid, dargestellt durch die Formel (XIV'), hergestellt wird:

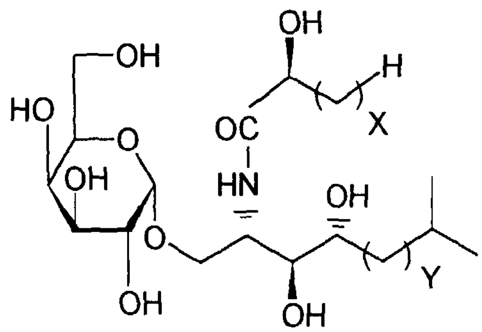

(XIV')

wobei X eine ganze Zahl von 20 bis 24 bezeichnet und Y eine ganze Zahl von 9 bis 13 bezeichnet.

**84.** Verfahren gemäss Anspruch 82, wobei X eine ganze Zahl von 20 bis 24 bezeichnet und Y eine ganze Zahl von 10 bis 12 bezeichnet.

**85.** Verfahren gemäss Anspruch 83, wobei X eine ganze Zahl von 21 bis 23 bezeichnet und Y eine ganze Zahl von 10 bis 12 bezeichnet.

**86.** Verfahren gemäss Anspruch 56, wobei ein Glycosphingolipid, dargestellt durch die Formel (XV), hergestellt wird:

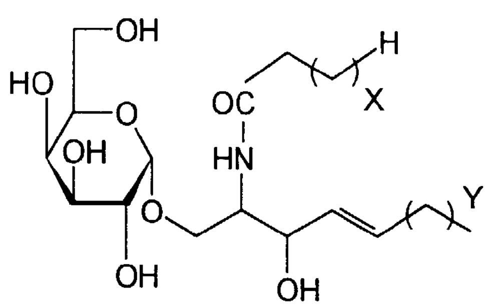

(XV)

wobei X eine ganze Zahl von 10 bis 18 bezeichnet und Y eine ganze Zahl von 10 bis 14 bezeichnet.

**87.** Verfahren gemäss Anspruch 86, wobei X eine ganze Zahl von 11 bis 17 bezeichnet und Y eine ganze Zahl von 11 bis 13 bezeichnet.

**88.** Verfahren gemäss Anspruch 86, wobei ein Glycosphingolipid, dargestellt durch die Formel (XVI), hergestellt wird:

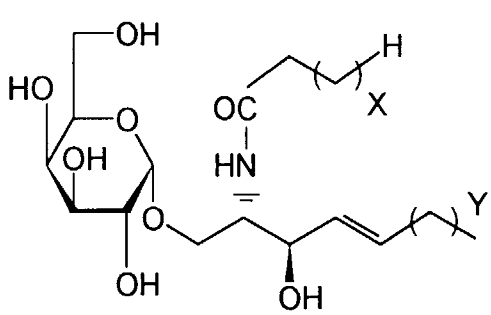

(XVI)

wobei X eine ganze Zahl von 10 bis 18 bezeichnet und Y eine ganze Zahl von 10 bis 14 bezeichnet.

**89.** Verfahren gemäss Anspruch 88, wobei X eine ganze Zahl von 11 bis 17 bezeichnet und Y eine ganze Zahl von 11 bis 13 bezeichnet.

**90.** Verfahren gemäss Anspruch 56, wobei ein Glycosphingolipid, dargestellt durch die Formel (XVII), hergestellt wird:

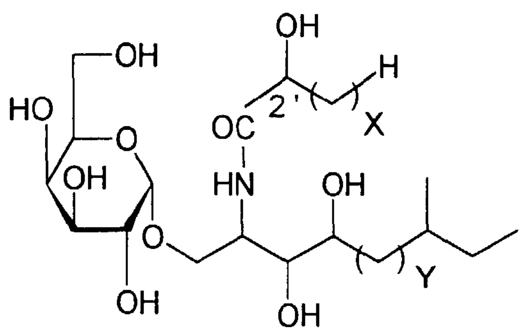

(XVII)

wobei X eine ganze Zahl von 21 bis 25 bezeichnet und Y eine ganze Zahl von 9 bis 13 bezeichnet.

**91.** Verfahren gemäss Anspruch 90, wobei X eine ganze Zahl von 22 bis 24 bezeichnet und Y eine ganze Zahl von 10 bis 12 bezeichnet.

**92.** Verfahren gemäss Anspruch 90, wobei ein Glycosphingolipid, dargestellt durch die Formel (XVIII), hergestellt wird:

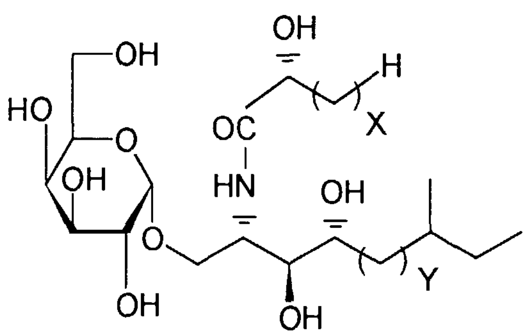

(XVIII)

wobei X eine ganze Zahl von 21 bis 25 bezeichnet und Y eine ganze Zahl von 9 bis 13 bezeichnet.

**93.** Verfahren gemäss Anspruch 92, wobei X eine ganze Zahl von 22 bis 24 bezeichnet und Y eine ganze Zahl von 10 bis 12 bezeichnet.

**94.** Verfahren gemäss Anspruch 55, wobei ein Glycosphingolipid, dargestellt durch die Formel (XIX), hergestellt wird:

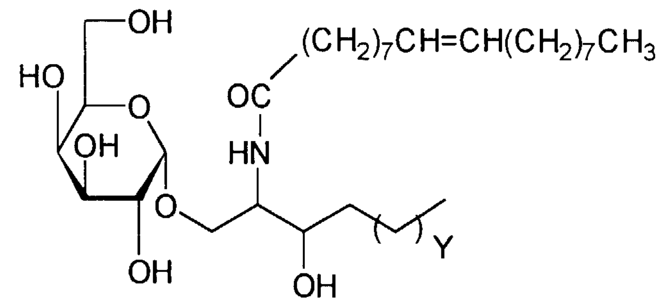

(XIX)

wobei Y eine ganze Zahl von 11 bis 15 bezeichnet.

**95.** Verfahren gemäss Anspruch 94, wobei Y eine ganze Zahl von 12 bis 14 bezeichnet.

**96.** Verfahren gemäss Anspruch 94, wobei ein Glycosphingolipid, dargestellt durch die Formel (XX), hergestellt wird:

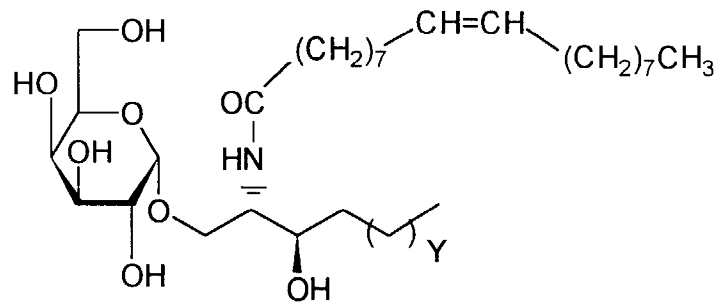

(XX)

wobei Y eine ganze Zahl von 11 bis 15 bezeichnet.

**97.** Verfahren gemäss Anspruch 96, wobei Y eine ganze Zahl von 12 bis 14 bezeichnet.

**98.** Verfahren gemäss Anspruch 55, wobei ein Glycosphingolipid, ausgewählt aus der Gruppe bestehend aus den folgenden Verbindungen, hergestellt wird:

(1) (2S,3R)-1-(α-D-Galactopyranosyloxy)-2-tetracosanoylamino-3-octadecanol,
(2) (2S,3R)-2-Docosanoylamino-1-(α-D-galactopyranosyloxy)-3-octadecanol,
(3) (2S,3R)-1-(α-D-Galactopyranosyloxy)-2-icosanoylamino-3-octadecanol,
(4) (2S,3R)-1-(α-D-Galactopyranosyloxy)-2-octadecanoylamino-3-octadecanol,
(5) (2S,3R)-1-(α-D-Galactopyranosyloxy)-2-tetradecanoylamino-3-octadecanol,
(6) (2S,3R)-2-Decanoylamino-1-(α-D-galactopyranosyloxy)-3-octadecanol,
(7) (2S,3R)-1-(α-D-Galactopyranosyloxy)-2-octanoylamino-3-octadecanol,
(8) (2S,3R)-2-Acetamido-1-(α-D-galactopyranosyloxy)-3-octadecanol,
(9) (2S,3R)-1-(α-D-Galactopyranosyloxy)-2-tetracosanoylamino-3-tetradecanol,
(10) (2S,3R)-1-(α-D-Galactopyranosyloxy)-2-tetradecanoylamino-3-hexadecanol,
(11) (2R,3S)-1-(α-D-Galactopyranosyloxy)-2-tetradecanoylamino-3-hexadecanol,
(12) (2S,3S)-1-(α-D-Galactopyranosyloxy)-2-tetradecanoylamino-3-hexadecanol,
(13) (2R,3R)-1-(α-D-Galactopyranosyloxy)-2-tetradecanoylamino-3-hexadecanol,
(14) (2S,3R)-1-(α-D-Galactopyranosyloxy)-2-[(R)-2-hydroxytetracosanoylamino]-3-octadecanol,
(15) (2S,3R,4E)-1-(α-D-Galactopyranosyloxy)-2-octadecanoylamino-4-octadecen-3-ol,
(16) (2S,3R,4E)-1-(α-D-Galactopyranosyloxy)-2-tetradecanoylamino-4-octadecen-3-ol,
(17) (2S,3S,4R)-1-(α-D-Galactopyranosyloxy)-2-tetracosanoylamino-3,4-octadecandiol,
(18) (2S,3S,4R)-1-(α-D-Galactopyranosyloxy)-2-tetracosanoylamino-3,4-heptadecandiol,
(19) (2S,3S,4R)-1-(α-D-Galactopyranosyloxy)-2-tetracosanoylamino-3,4-pentadecandiol,

(20) (2S,3S,4R)-1-(α-D-Galactopyranosyloxy)-2-tetracosanoylamino-3,4-undecandiol,
(21) (2S,3S,4R)-1-(α-D-Galactopyranosyloxy)-2-hexacosanoylamino-3,4-heptadecandiol,
(22) (2S,3S,4R)-1-(α-D-Galactopyranosyloxy)-2-[(R)-2-hydroxytetracosanoylamino]-3,4-octadecandiol,
(23) (2S,3S,4R)-1-(α-D-Galactopyranosyloxy)-2-[(R)-2-hydroxytetracosanoylamino]-3,4-heptadecandiol,
(24) (2S,3S,4R)-1-(α-D-Galactopyranosyloxy)-2-[(R)-2-hydroxytetracosanoylamino]-3,4-pentadecandiol,
(25) (2S,3S,4R)-1-(α-D-Galactopyranosyloxy)-2-[(R)-2-hydroxytetracosanoylamino]-3,4-undecandiol,
(26) (2S,3S,4R)-1-(α-D-Galactopyranosyloxy)-2-[(R)-2-hydroxyhexacosanoylamino]-3,4-octadecandiol,
(27) (2S,3S,4R)-1-(α-D-Galactopyranosyloxy)-2-[(R)-2-hydroxyhexacosanoylamino]-3,4-nonadecandiol,
(28) (2S,3S,4R)-1-(α-D-Galactopyranosyloxy)-2-[(R)-2-hydroxyhexacosanoylamino]-3,4-icosandiol,
(29) (2S,3S,4R)-1-(α-D-Galactopyranosyloxy)-2-[(S)-2-hydroxytetracosanoylamino]-3,4-heptadecandiol,
(30) (2S,3S,4R)-1-(α-D-Galactopyranosyloxy)-2-[(R)-2-hydroxytetracosanoylamino]-3,4-hexadecandiol,
(31) (2S,3S,4R)-1-(α-D-Galactopyranosyloxy)-2-[(S)-2-hydroxytetracosanoylamino]-16-methyl-3,4-heptadecandiol,
(32) (2S,3S,4R)-1-(α-D-Galactopyranosyloxy)-16-methyl-2-tetracosanoylamino-3,4-heptadecandiol,
(33) (2S,3S,4R)-1-(α-D-Galactopyranosyloxy)-2-[(R)-2-hydroxytricosanoylamino]-16-methyl-3,4-heptadecandiol,
(34) (2S,3S,4R)-1-(α-D-Galactopyranosyloxy)-2-[(R)-2-hydroxypentacosanoylamino]-16-methyl-3,4-octadecandiol und
(35) (2S,3R)-1-(α-D-Galactopyranosyloxy)-2-oleoylamino-3-octadecanol.

**99.** Verfahren für ein Glycosphingolipid, umfassend das Aufsammeln des Meeresschwammes Agelas mauritianus, Unterwerfen desselben einem Extraktionsvorgang mit einem organischen Lösungsmittel, und Isolieren aus dem Extrakt mindestens eines der folgenden Glycosphingolipide, dargestellt durch die Formel (I) gemäss Anspruch 56:

(1) (2S,3S,4R)-1-(α-D-Galactopyranosyloxy)-2-[(R)-2-hydroxytetracosanoylamino]-3,4-heptadecandiol,
(2) (2S,3S,4R)-1-(α-D-Galactopyranosyloxy)-2-[(R)-2-hydroxytetracosanoylamino]-3,4-hexadecandiol,
(3) (2S,3S,4R)-1-(α-D-Galactopyranosyloxy)-2-[(R)-2-hydroxytricosanoylamino]-16-methyl-3,4-heptadecandiol,
(4) (2S,3S,4R)-1-(α-D-Galactopyranosyloxy)-2-[(R)-2-hydroxypentacosanoylamino]-16-methyl-3,4-octadecandiol.

**100.** Verfahren zur Herstellung eines cytostatischen Mittels, umfassend das Mischen einer oder mehrerer Verbindungen, dargestellt durch die Formel (A):

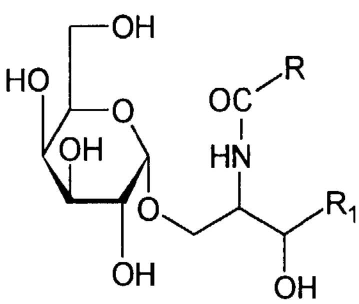

(A)

wobei R

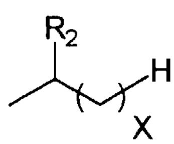

,

wobei $R_2$ H oder OH darstellt und X eine ganze Zahl von 0 bis 26 bezeichnet, oder $-(CH_2)_7CH{=}CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet,

als Wirkstoff(e) mit pharmazeutisch annehmbaren Additiven.

**101.** Verfahren zur Herstellung eines Immunstimulators, umfassend das Mischen einer oder mehrerer Verbindungen, dargestellt durch die Formel (A)

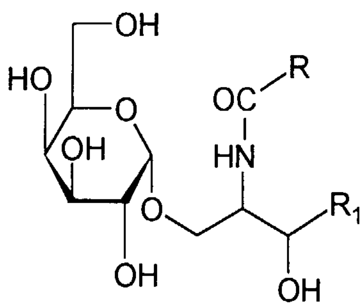

(A)

wobei R

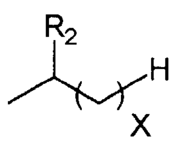

,

wobei $R_2$ H oder OH darstellt und X eine ganze Zahl von 0 bis 26 bezeichnet, oder $-(CH_2)_7CH=CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet,

als Wirkstoff(e) mit pharmazeutisch annehmbaren Additiven.

**102.** Verwendung einer oder mehrerer Verbindungen, dargestellt durch die Formel (A)

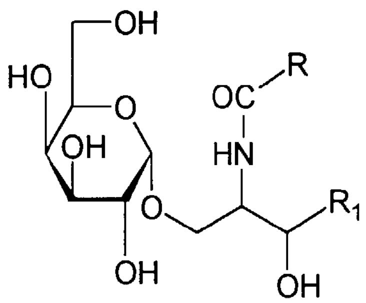

(A)

wobei R

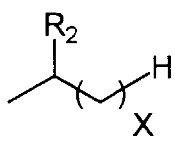

,

wobei $R_2$ H oder OH darstellt und X eine ganze Zahl von 0 bis 26 bezeichnet, oder $-(CH_2)_7CH=CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)\ (CH_2)_YCH(CH_3)CH_2CH_3$,

wobei Y eine ganze Zahl von 5 bis 17 bezeichnet,

bei der Herstellung eines cytostatischen Mittels.

**103.** Verwendung einer oder mehrerer Verbindungen, dargestellt durch die Formel (A)

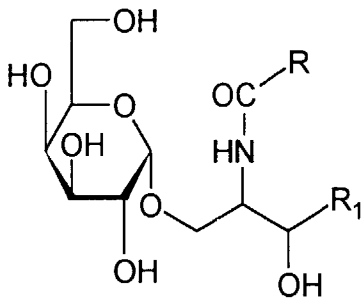

(A)

wobei R

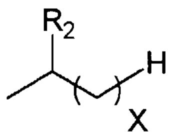

,

wobei $R_2$ H oder OH darstellt und X eine ganze Zahl von 0 bis 26 bezeichnet, oder $-(CH_2)_7CH=CH(CH_2)_7CH_3$ darstellt, und

$R_1$ einen der nachstehend unter (a) bis (e) definierten Substituenten darstellt:

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

und

(e) $-CH(OH)(CH_2)_YCH(CH_3)CH_2CH_3$,

wobei Y eine ganze Zahl von 5 bis 17 bezeichnet,

bei der Herstellung eines immunstimulierenden Medikaments.

## Revendications

**Revendications pour les Etats contractants suivants : AT, BE, CH, LI, DE, DK, FR, GB, IT, LU, MC, NL, SE**

**1.** Glycosphingolipide comprenant un résidu alpha-galactose représenté par la formule (A) :

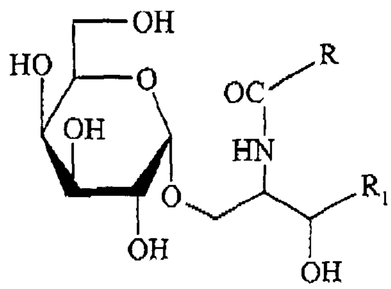

(A)

dans laquelle

R représente

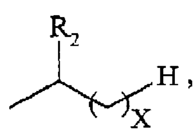

où $R_2$ représente H ou OH et X signifie un entier de 0 à 26, ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17.

**2.** Glycosphingolipide selon la revendication 1, qui est représenté par la formule (I) :

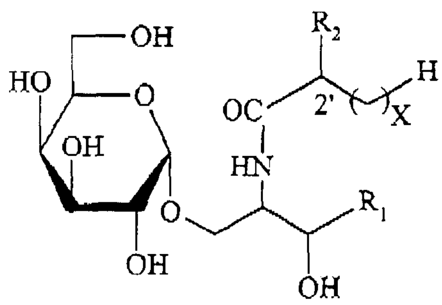

(I)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**3.** Glycosphingolipide selon la revendication 2, qui est représenté par la formule (II) :

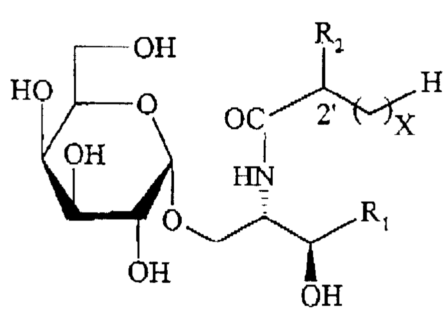

(II)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_1$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH=CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**4.** Glycosphingolipide selon la revendication 2, qui est représenté par la formule (III) :

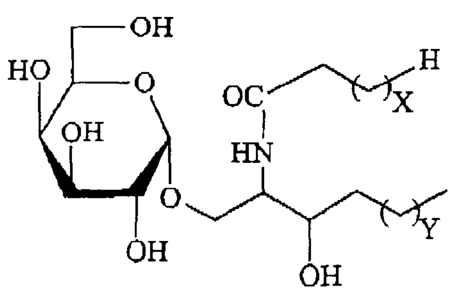

(III)

dans laquelle X signifie un entier de 0 à 24 et Y signifie un entier de 7 à 15.

**5.** Glycosphingolipide selon la revendication 4, dans lequel X signifie un entier de 8 à 22 et Y signifie un entier de 9 à 13.

**6.** Glycosphingolipide selon la revendication 4, qui est représenté par la formule (IV) :

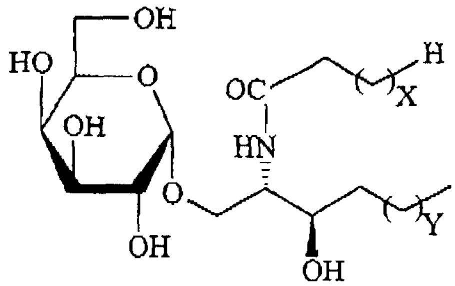

(IV)

dans laquelle X signifie un entier de 0 à 24 et Y signifie un entier de 7 à 15.

7. Glycosphingolipide selon la revendication 6, dans lequel X signifie un entier de 8 à 22 et Y signifie un entier de 9 à 13.

8. Glycosphingolipide selon la revendication 2, qui est représenté par la formule (V) :

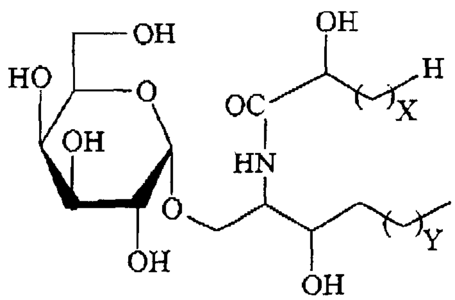

(V)

dans laquelle X signifie un entier de 20 à 24 et Y signifie un entier de 11 à 15.

9. Glycosphingolipide selon la revendication 8, dans lequel X signifie un entier de 21 à 23 et Y signifie un entier de 12 à 14.

10. Glycosphingolipide selon la revendication 8, qui est représenté par la formule (VI) :

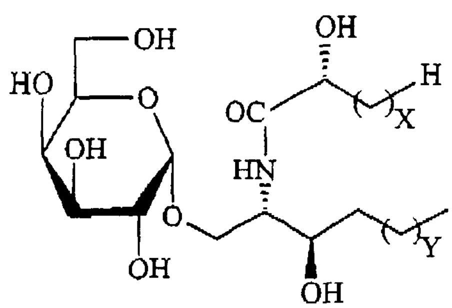

(VI)

dans laquelle X signifie un entier de 20 à 24 et Y signifie un entier de 11 à 15.

11. Glycosphingolipide selon la revendication 10, dans lequel X signifie un entier de 21 à 23 et Y signifie un entier de 12 à 14.

12. Glycosphingolipide selon la revendication 2, qui est représenté par la formule (VII) :

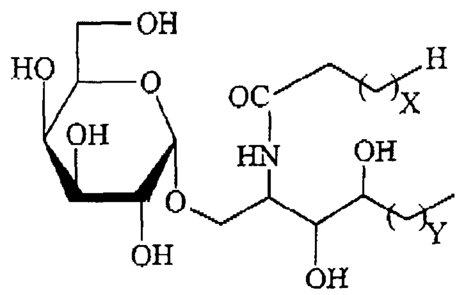

(VII)

dans laquelle X signifie un entier de 18 à 26 et Y signifie un entier de 5 à 15.

**13.** Glycosphingolipide selon la revendication 12, dans lequel X signifie un entier de 21 à 25 et Y signifie un entier de 6 à 14.

**14.** Glycosphingolipide selon la revendication 12, qui est représenté par la formule (VIII) :

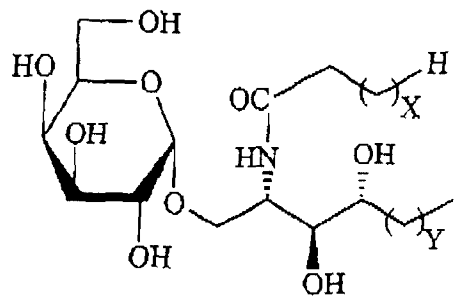

(VIII)

dans laquelle X signifie un entier de 18 à 26 et Y signifie un entier de 5 à 15.

**15.** Glycosphingolipide selon la revendication 14, dans lequel X signifie un entier de 21 à 25 et Y signifie un entier de 6 à 14.

**16.** Glycosphingolipide selon la revendication 2, qui est représenté par la formule (IX) :

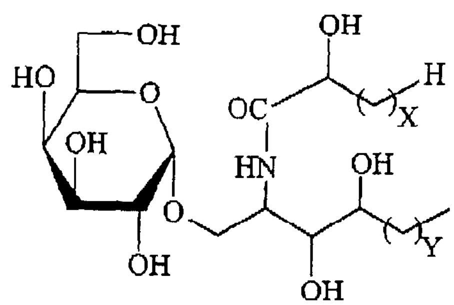

(IX)

dans laquelle X signifie un entier de 18 à 26 et Y signifie un entier de 5 à 17.

**17.** Glycosphingolipide selon la revendication 16, dans lequel X signifie un entier de 21 à 25 et Y signifie un entier de 6 à 16.

**18.** Glycosphingolipide selon la revendication 16, qui est représenté par la formule (X) :

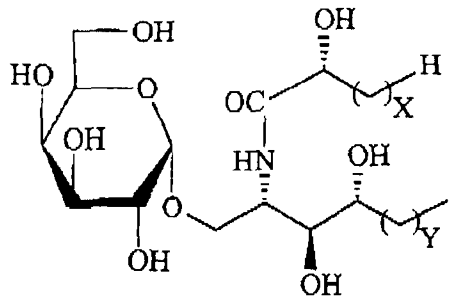

(X)

dans laquelle X signifie un entier de 18 à 26 et Y signifie un entier de 5 à 17.

**19.** Glycosphingolipide selon la revendication 16, qui est représenté par la formule (X'):

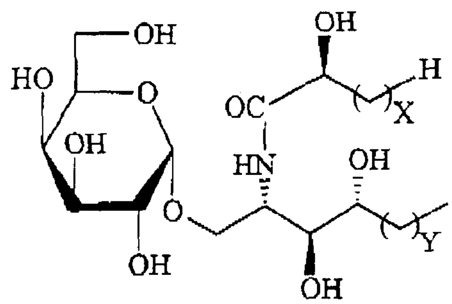

(X')

dans laquelle X signifie un entier de 20 à 24 et Y signifie un entier de 10 à 14.

**20.** Glycosphingolipide selon la revendication 18, dans lequel X signifie un entier de 21 à 25 et Y signifie un entier de 6 à 16.

**21.** Glycosphingolipide selon la revendication 19, dans lequel X signifie un entier de 21 à 23 et Y signifie un entier de 11 à 13.

**22.** Glycosphingolipide selon la revendication 2, qui est représenté par la formule (XI):

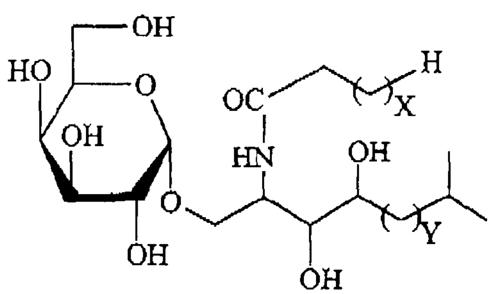

(XI)

dans laquelle X signifie un entier de 20 à 24 et Y signifie un entier de 9 à 13.

**23.** Glycosphingolipide selon la revendication 22, dans lequel X signifie un entier de 21 à 23 et Y signifie un entier de 10 à 12.

**24.** Glycosphingolipide selon la revendication 22, qui est représenté par la formule (XII) :

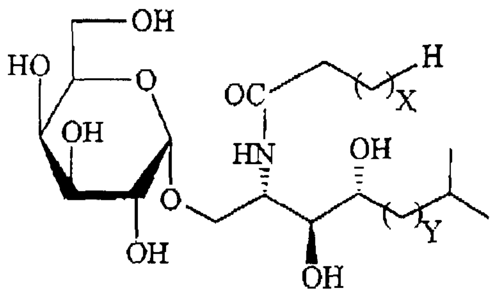

(XII)

dans laquelle X signifie un entier de 20 à 24 et Y signifie un entier de 9 à 13.

**25.** Glycosphingolipide selon la revendication 24, dans lequel X signifie un entier de 21 à 23 et Y signifie un entier de 10 à 12.

**26.** Glycosphingolipide selon la revendication 2, qui est représenté par la formule (XIII):

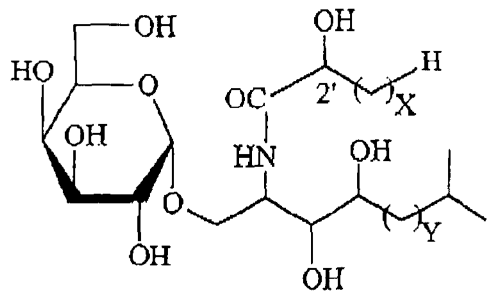

(XIII)

dans laquelle X signifie un entier de 18 à 24 et Y signifie un entier de 9 à 13.

**27.** Glycosphingolipide selon la revendication 26, dans lequel X signifie un entier de 20 à 23 et Y signifie un entier de 10 à 12.

**28.** Glycosphingolipide selon la revendication 26, qui est représenté par la formule (XIV) :

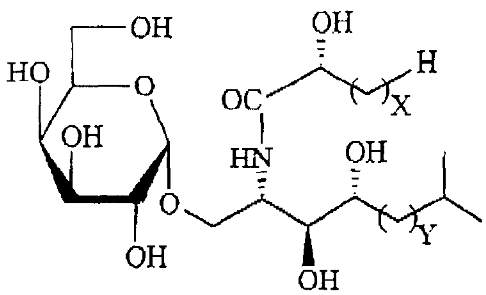

(XIV)

dans laquelle X signifie un entier de 19 à 23 et Y signifie un entier de 9 à 13.

**29.** Glycosphingolipide selon la revendication 26, qui est représenté par la formule (XIV') :

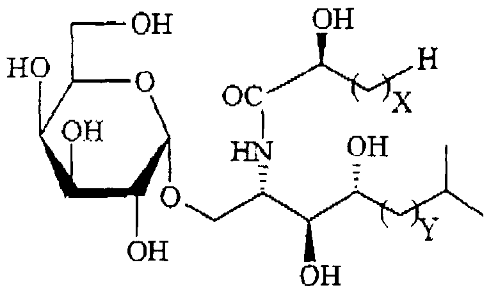

(XIV')

dans laquelle X signifie un entier de 20 à 24 et Y signifie un entier de 9 à 13.

**30.** Glycosphingolipide selon la revendication 28, dans lequel X signifie un entier de 20 à 22 et Y signifie un entier de 10 à 12.

**31.** Glycosphingolipide selon la revendication 29, dans lequel X signifie un entier de 21 à 23 et Y signifie un entier de 10 à 12.

**32.** Glycosphingolipide selon la revendication 2, qui est représenté par la formule (XV) :

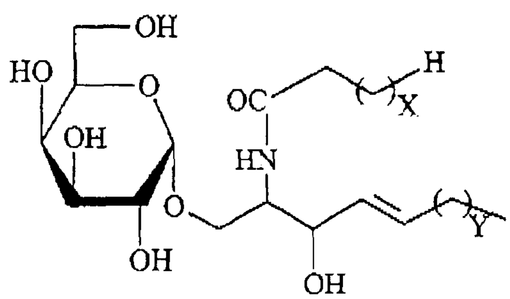

(XV)

dans laquelle X signifie un entier de 10 à 18 et Y signifie un entier de 10 à 14.

**33.** Glycosphingolipide selon la revendication 32, dans lequel X signifie un entier de 11 à 17 et Y signifie un entier de 11 à 13.

**34.** Glycosphingolipide selon la revendication 32, qui est représenté par la formule (XVI) :

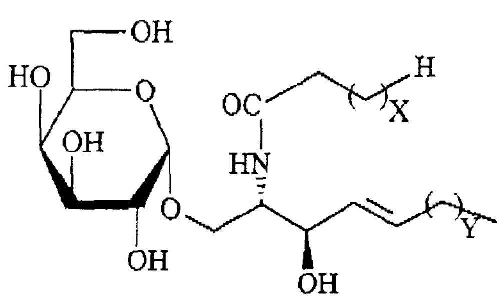

(XVI)

dans laquelle X signifie un entier de 10 à 18 et Y signifie un entier de 10 à 14.

**35.** Glycosphingolipide selon la revendication 34, dans lequel X signifie un entier de 11 à 17 et Y signifie un entier de 11 à 13.

**36.** Glycosphingolipide selon la revendication 2, qui est représenté par la formule (XVII) :

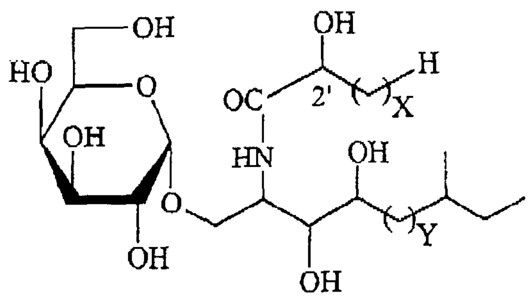

(XVII)

dans laquelle X signifie un entier de 21 à 25 et Y signifie un entier de 9 à 13.

**37.** Glycosphingolipide selon la revendication 36, dans lequel X signifie un entier de 22 à 24 et Y signifie un entier de 10 à 12.

**38.** Glycosphingolipide selon la revendication 36, qui est représenté par la formule (XVIII) :

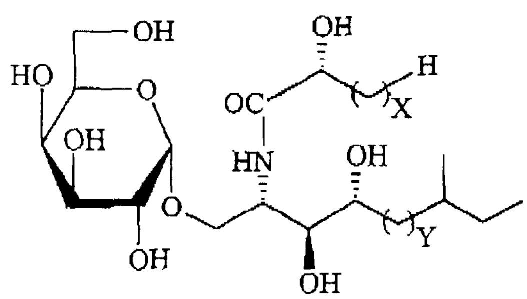

(XVIII)

dans laquelle X signifie un entier de 21 à 25 et Y signifie un entier de 9 à 13.

**39.** Glycosphingolipide selon la revendication 38, dans lequel X signifie un entier de 22 à 24 et Y signifie un entier de 10 à 12.

**40.** Glycosphingolipide selon la revendication 1, qui est représenté par la formule (XIX) :

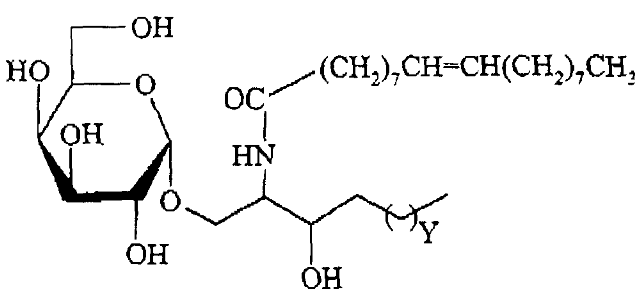

(XIX)

dans laquelle Y signifie un entier de 11 à 15.

**41.** Glycosphingolipide selon la revendication 40, dans lequel Y signifie un entier de 12 à 14.

**42.** Glycosphingolipide selon la revendication 40, qui est représenté par la formule (XX) :

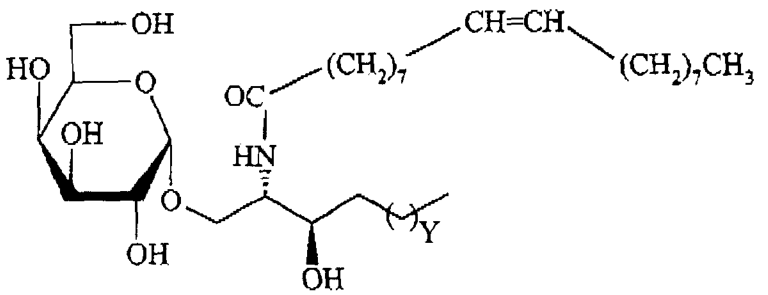

(XX)

dans laquelle Y signifie un entier de 11 à 15.

**43.** Glycosphingolipide selon la revendication 42, dans lequel Y signifie un entier de 12 à 14.

**44.** Glycosphingolipide selon la revendication 1, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol,
(10) (2S,3R)-1-(α-D-galactopyranosyloxy)-2-tétradécanoylamino-3-hexadécanol,
(11) (2R,3S)-1-(α-D-galactopyranosyloxy)-2-tétradécanoylamino-3-hexadécanol,
(12) (2S,3S)-1-(α-D-galactopyranosyloxy)-2-tétradécanoylamino-3-hexadécanol,
(13) (2R,3R)-1-(α-D-galactopyranosyloxy)-2-tétradécanoylamino-3-hexadécanol,
(14) (2S,3R)-1-(α-D-galactopyranosyloxy)-2-[(R)-2-hydroxytétracosanoylamino]-3-octadécanol,
(15) (2S,3R,4E)-1-(α-D-galactopyranosyloxy)-2-octadécanoylamino-4-octadécèn-3-ol,
(16) (2S,3R,4E)-1-(α-D-galactopyranosyloxy)-2-tétradécanoylamino-4-octadécèn-3-ol,
(17) (2S,3S,4R)-1-(α-D-galactopyranosyloxy)-2-tétracosanoylamino-3,4-octadécanediol,
(18) (2S,3S,4R)-1-(α-D-galactopyranosyloxy)-2-tétracosanoylamino-3,4-heptadécanediol,
(19) (2S,3S,4R)-1-(α-D-galactopyranosyloxy)-2-tétracosanoylamino-3,4-pentadécanediol,
(20) (2S,3S,4R)-1-(α-D-galactopyranosyloxy)-2-tétracosanoylamino-3,4-undécanediol,
(21) (2S,3S,4R)-1-(α-D-galactopyranosyloxy)-2-hexacosanoylamino-3,4-heptadécanediol,
(22) (2S,3S,4R)-1-(α-D-galactopyranosyloxy)-2-[(R)-2-hydroxytétracosanoylamino]-3,4-octadécanediol,
(23) (2S,3S,4R)-1-(α-D-galactopyranosyloxy)-2-[(R)-2-hydroxytétracosanoylamino]-3,4-heptadécanediol,
(24) (2S,3S,4R)-1-(α-D-galactopyranosyloxy)-2-[(R)-2-hydroxytétracosanoylamino]-3,4-pentadécanediol,
(25) (2S,3S,4R)-1-(α-D-galactopyranosyloxy)-2-[(R)-2-hydroxytétracosanoylamino]-3,4-undécanediol,
(26) (2S,3S,4R)-1-(α-D-galactopyranosyloxy)-2-[(R)-2-hydroxyhexacosanoylamino]-3,4-octadécanediol,
(27) (2S,3S,4R)-1-(α-D-galactopyranosyloxy)-2-[(R)-2-hydroxyhexacosanoylamino]-3,4-nonadécanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytétracosanoylamino]-3,4-heptadécanediol,
(30) (2S,3S,4R)-1-(α-D-galactopyranosyloxy)-2-[(R)-2-hydroxytétracosanoylamino]-3,4-hexadécanediol,
(31) (2S,3S,4R)-1-(α-D-galactopyranosyloxy)-2-[(S)-2-hydroxytétracosanoylamino]-16-méthyl-3,4-heptadécanediol,
(32) (2S,3S,4R)-1-(α-D-galactopyranosyloxy)-16-méthyl-2-tétracosanoylamino-3,4-heptadécanediol,
(33) (2S,3S,4R)-1-(α-D-galactopyranosyloxy)-2-[(R)-2-hydroxytricosanoylamino]-16-méthyl-3,4-heptadécanediol,
(34) (2S,3S,4R)-1-(α-D-galactopyranosyloxy)-2-[(R)-2-hydroxypentacosanoylamino]-16-méthyl-3,4-octadécanediol
et
(35) (2S,3R)-1-(α-D-galactopyranosyloxy)-2-oléoylamino-3-octadécanol.

**45.** Glycosphingolipide selon la revendication 44, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol et
(10) (2S,3R)-1-(α-D-galactopyranosyloxy)-2-tétradécanoylamino-3-hexadécanol.

**46.** Glycosphingolipide selon la revendication 45, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-tétracosanoylamino-3-tétradécanol et
(8) (2S,3R)-1-(α-D-galactopyranosyloxy)-2-tétradécanoylamino-3-hexadécanol.

**47.** Glycosphingolipide selon la revendication 46, qui est choisi dans le groupe constitué par les composés suivants :

(1) (2S,3R)-1-(α-D-galactopyranosyloxy)-2-octadécanoylamino-3-octadécanol,
(2) (2S,3R)-1-(α-D-galactopyranosyloxy)-2-tétradécanoylamino-3-octadécanol et
(3) (2S,3R)-1-(α-D-galactopyranosyloxy)-2-tétradécanoylamino-3-hexadécanol.

**48.** Glycosphingolipide selon la revendication 44, qui est le (2S,3R)-1-(α-D-galactopyranosyloxy)-2-[(R)-2-hydroxytétracosanoylamino]-3-octadécanol.

**49.** Glycosphingolipide selon la revendication 44, qui est choisi dans le groupe constitué par les composés suivants :

(1) (2S,3R,4E)-1-(α-D-galactopyranosyloxy)-2-octadécanoylamino-4-octadécèn-3-ol et
(2) (2S,3R,4E)-1-(α-D-galactopyranosyloxy)-2-tétradécanoylamino-4-octadécèn-3-ol.

**50.** Glycosphingolipide selon la revendication 44, qui est choisi dans le groupe constitué par les composés suivants :

(1) (2S,3S,4R)-1-(α-D-galactopyranosyloxy)-2-tétracosanoylamino-3,4-octadécanediol,
(2) (2S,3S,4R)-1-(α-D-galactopyranosyloxy)-2-tétracosanoylamino-3,4-heptadécanediol,
(3) (2S,3S,4R)-1-(α-D-galactopyranosyloxy)-2-tétracosanoylamino-3,4-pentadécanediol,
(4) (2S,3S,4R)-1-(α-D-galactopyranosyloxy)-2-tétracosanoylamino-3,4-undécanediol et
(5) (2S,3S,4R)-1-(α-D-galactopyranosyloxy)-2-hexacosanoylamino-3,4-heptadécanediol.

**51.** Glycosphingolipide selon la revendication 44, qui est choisi dans le groupe constitué par les composés suivants :

(1) (2S,3S,4R)-1-(α-D-galactopyranosyloxy)-2-[(R)-2-hydroxytétracosanoylamino]-3,4-octadécanediol,
(2) (2S,3S,4R)-1-(α-D-galactopyranosyloxy)-2-[(R)-2-hydroxytétracosanoylamino]-3,4-heptadécanediol,
(3) (2S,3S,4R)-1-(α-D-galactopyranosyloxy)-2-[(R)-2-hydroxytétracosanoylamino]-3,4-pentadécanediol,
(4) (2S,3S,4R)-1-(α-D-galactopyranosyloxy)-2-[(R)-2-hydroxytétracosanoylamino]-3,4-undécanediol,
(5) (2S,3S,4R)-1-(α-D-galactopyranosyloxy)-2-[(R)-2-hydroxyhexacosanoylamino]-3,4-octadécanediol,
(6) (2S,3S,4R)-1-(α-D-galactopyranosyloxy)-2-[(R)-2-hydroxyhexacosanoylamino]-3,4-nonadécanediol,
(7) (2S,3S,4R)-1-(α-D-galactopyranosyloxy)-2-[(R)-2-hydroxyhexacosanoylamino]-3,4-icosanediol,
(8) (2S,3S,4R)-1-(α-D-galactopyranosyloxy)-2-[(S)-2-hydroxytétracosanoylamino]-3,4-heptadécanediol et
(9) (2S,3S,4R)-1-(α-D-galactopyranosyloxy)-2-[(R)-2-hydroxytétracosanoylamino]-3,4-hexadécanediol.

**52.** Glycosphingolipide selon la revendication 44, qui est choisi dans le groupe constitué par les composés suivants :

(1) (2S,3S,4R)-1-(α-D-galactopyranosyloxy)-2-[(S)-2-hydroxytétracosanoylamino]-16-méthyl-3,4-heptadécanediol,
(2) (2S,3S,4R)-1-(α-D-galactopyranosyloxy)-16-méthyl-2-tétracosanoylamino-3,4-heptadécanediol et
(3) (2S,3S,4R)-1-(α-D-galactopyranosyloxy)-2-[(R)-2-hydroxytricosanoylamino]-16-méthyl-3,4-heptadécanediol,

**53.** Glycosphingolipide selon la revendication 44, qui est le (2S,3S,4R)-1-(α-D-galactopyranosyloxy)-2-[(R)-2-hydroxypentacosanoylamino]-16-méthyl-3,4-octadécanediol.

**54.** Glycosphingolipide selon la revendication 44, qui est le (2S,3R)-1-(α-D-galactopyranosyloxy)-2-oléoylamino-3-octadécanol.

**55.** Procédé pour la préparation d'un glycosphingolipide comprenant la récolte de l'éponge marine Agelas mauritianus, sa soumission à une opération d'extraction avec un solvant organique et l'isolement à partir de l'extrait d'au moins un des glycosphingolipides suivants représentés par la formule (I) selon la revendication 2 :

(1) (2S,3S,4R)-1-(α-D-galactopyranosyloxy)-2-[(R)-2-hydroxytétracosanoylamino]-3,4-heptadécanediol,
(2) (2S,3S,4R)-1-(α-D-galactopyranosyloxy)-2-[(R)-2-hydroxytétracosanoylamino]-3,4-hexadécanediol.
(3) (2S,3S,4R)-1-(α-D-galactopyranosyloxy)-2-[(R)-2-hydroxytricosanoylamino]-16-méthyl-3,4-heptadécanediol et
(4) (2S,3S,4R)-1-(α-D-galactopyranosyloxy)-2-[(R)-2-hydroxypentacosanoylamino]-16-méthyl-3,4-octadécanediol.

**56.** Agent antitumoral comprenant un ou plusieurs des composés selon la revendication 1 en tant qu'ingrédient actif.

**57.** Agent immunostimulant comprenant un ou plusieurs des composés selon la revendication 1 en tant qu'ingrédient actif.

**58.** Agent antitumoral comprenant une quantité efficace d'un ou plusieurs des composés selon la revendication 1 et un excipient ou diluant.

**59.** Agent immunostimulant comprenant une quantité efficace d'un ou plusieurs des composés selon la revendication 1 et un excipient ou diluant.

**60.** Utilisation d'un ou plusieurs des composés selon la revendication 1 dans la préparation d'un agent antitumoral.

**61.** Utilisation d'un ou plusieurs des composés selon la revendication 1 dans la préparation d'un médicament immunostimulant.

**Revendications pour l'Etat contractant suivant : ES**

**1.** Procédé pour préparer un glycosphingolipide comprenant un résidu alpha-galactose représenté par la formule (A) :

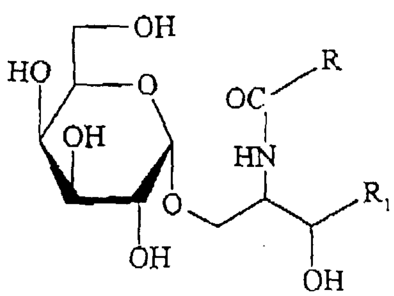

(A)

dans laquelle

R représente

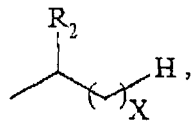

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17,
lequel comprend l'un des procédés suivants :

a) extraction de (A) à partir de l'espèce d'éponge marine Algelas mauretianus à l'aide d'au moins un solvant organique approprié et d'eau;
b) modification de la sphingosine; et
c) réactions chimiques générales pour la synthèse des glycosphingolipides.

**2.** Procédé selon la revendication 1, dans lequel on prépare un glycosphingolipide représenté par la formule (I) :

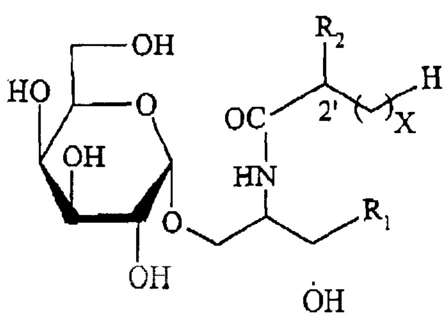

(I)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**3.** Procédé selon la revendication 2, dans lequel on prépare un glycosphingolipide représenté par la formule (II) :

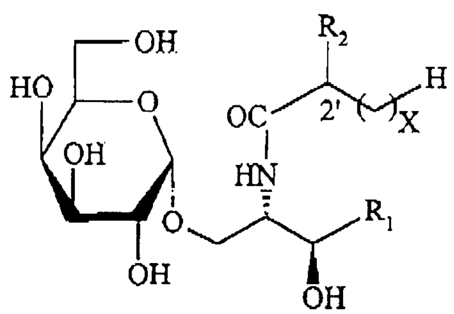

(II)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$

représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**4.** Procédé selon la revendication 2, dans lequel on prépare un glycosphingolipide représenté par la formule (III) :

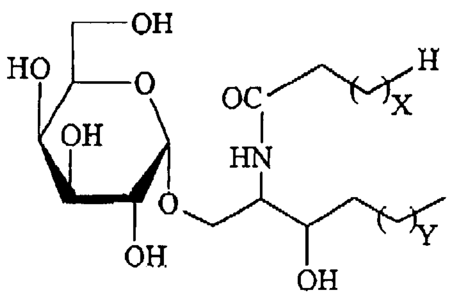

(III)

dans laquelle X signifie un entier de 0 à 24 et Y signifie un entier de 7 à 15.

**5.** Procédé selon la revendication 4, dans lequel X signifie un entier de 8 à 22 et Y signifie un entier de 9 à 13.

**6.** Procédé selon la revendication 4, dans lequel on prépare un glycosphingolipide représenté par la formule (IV) :

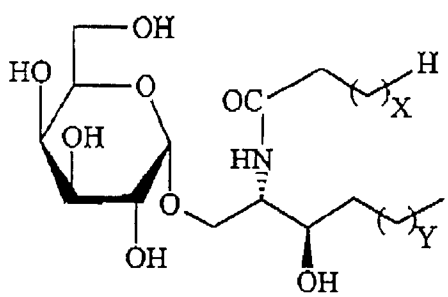

(IV)

dans laquelle X signifie un entier de 0 à 24 et Y signifie un entier de 7 à 15.

**7.** Procédé selon la revendication 6, dans lequel X signifie un entier de 8 à 22 et Y signifie un entier de 9 à 13.

**8.** Procédé selon la revendication 2, dans lequel on prépare un glycosphingolipide représenté par la formule (V) :

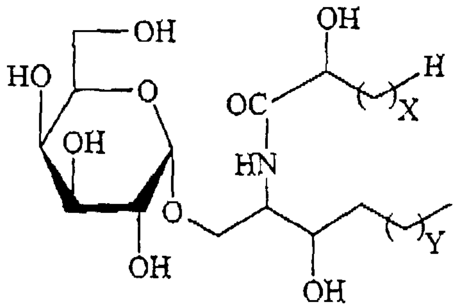

(V)

dans laquelle X signifie un entier de 0 à 24 et Y signifie un entier de 11 à 15.

**9.** Procédé selon la revendication 8, dans lequel X signifie un entier de 21 à 23 et Y signifie un entier de 12 à 14.

**10.** Procédé selon la revendication 8, dans lequel on prépare un glycosphingolipide représenté par la formule (VI) :

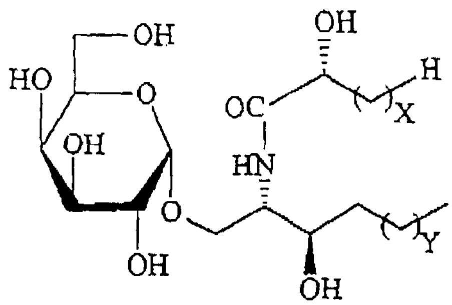

(VI)

dans laquelle X signifie un entier de 20 à 24 et Y signifie un entier de 11 à 15.

**11.** Procédé selon la revendication 10, dans lequel X signifie un entier de 21 à 23 et Y signifie un entier de 12 à 14.

**12.** Procédé selon la revendication 2, dans lequel on prépare un glycosphingolipide représenté par la formule (VII) :

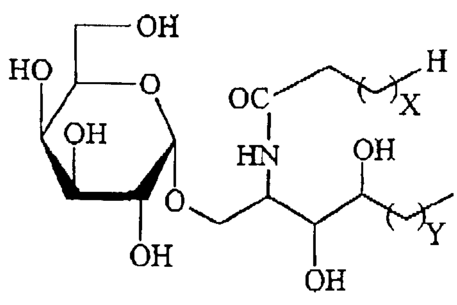

(VII)

dans laquelle X signifie un entier de 18 à 26 et Y signifie un entier de 5 à 15.

**13.** Procédé selon la revendication 12, dans lequel X signifie un entier de 21 à 25 et Y signifie un entier de 6 à 14.

**14.** Procédé selon la revendication 12, dans lequel on prépare un glycosphingolipide représenté par la formule (VIII) :

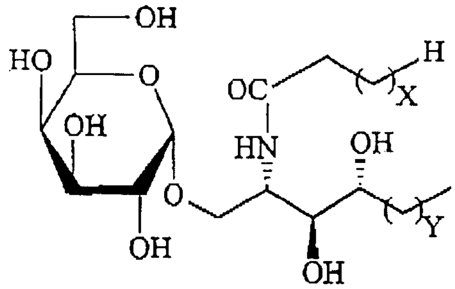

(VIII)

dans laquelle X signifie un entier de 18 à 26 et Y signifie un entier de 5 à 15.

**15.** Procédé selon la revendication 14, dans lequel X signifie un entier de 21 à 25 et Y signifie un entier de 6 à 14.

**16.** Procédé selon la revendication 2, dans lequel on prépare un glycosphingolipide représenté par la formule (IX) :

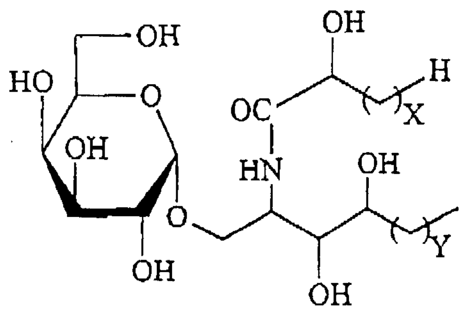

(IX)

dans laquelle X signifie un entier de 18 à 26 et Y signifie un entier de 5 à 17.

**17.** Procédé selon la revendication 16, dans lequel X signifie un entier de 21 à 25 et Y signifie un entier de 6 à 16.

**18.** Procédé selon la revendication 16, dans lequel on prépare un glycosphingolipide représenté par la formule (X) :

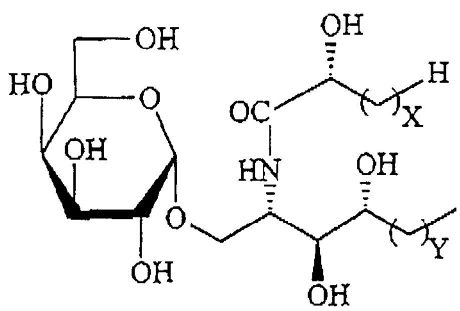

(X)

dans laquelle X signifie un entier de 18 à 26 et Y signifie un entier de 5 à 17.

**19.** Procédé selon la revendication 16, dans lequel on prépare un glycosphingolipide représenté par la formule (X') :

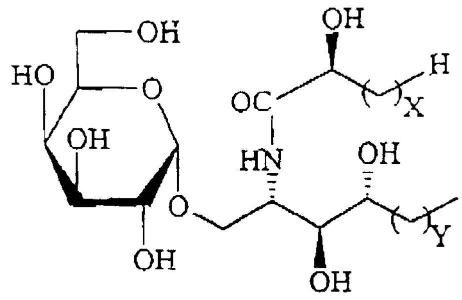

(X')

dans laquelle X signifie un entier de 20 à 24 et Y signifie un entier de 10 à 14.

**20.** Procédé selon la revendication 18, dans lequel X signifie un entier de 21 à 25 et Y signifie un entier de 6 à 16.

**21.** Procédé selon la revendication 19, dans lequel X signifie un entier de 21 à 23 et Y signifie un entier de 11 à 13.

**22.** Procédé selon la revendication 2, dans lequel on prépare un glycosphingolipide représenté par la formule (XI) :

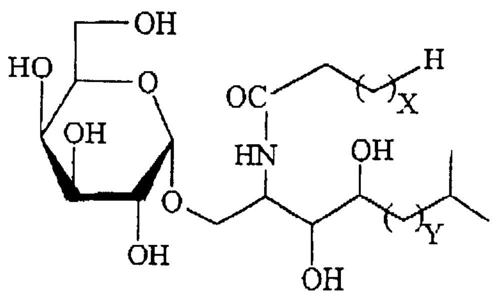

(XI)

dans laquelle X signifie un entier de 20 à 24 et Y signifie un entier de 9 à 13.

**23.** Procédé selon la revendication 22, dans lequel X signifie un entier de 21 à 23 et Y signifie un entier de 10 à 12.

**24.** Procédé selon la revendication 22, dans lequel on prépare un glycosphingolipide représenté par la formule (XII) :

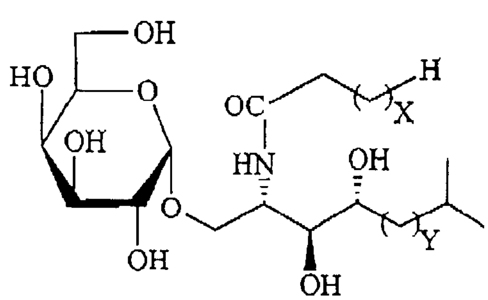

(XII)

dans laquelle X signifie un entier de 20 à 24 et Y signifie un entier de 9 à 13.

**25.** Procédé selon la revendication 24, dans lequel X signifie un entier de 21 à 23 et Y signifie un entier de 10 à 12.

**26.** Procédé selon la revendication 2, dans lequel on prépare un glycosphingolipide représenté par la formule (XIII) :

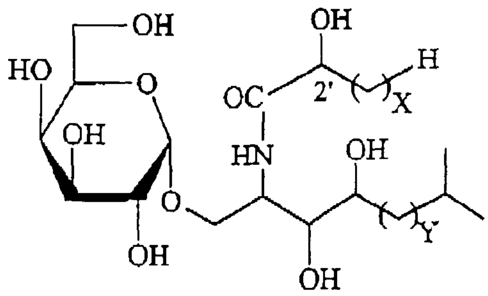

(XIII)

dans laquelle X signifie un entier de 18 à 24 et Y signifie un entier de 9 à 13.

**27.** Procédé selon la revendication 26, dans lequel X signifie un entier de 20 à 23 et Y signifie un entier de 10 à 12.

**28.** Procédé selon la revendication 26, dans lequel on prépare un glycosphingolipide représenté par la formule (XIV) :

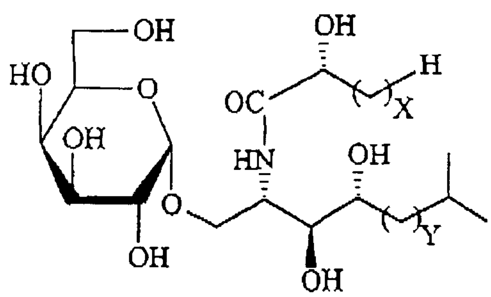

(XIV)

dans laquelle X signifie un entier de 19 à 23 et Y signifie un entier de 9 à 13.

**29.** Procédé selon la revendication 26, dans lequel on prépare un glycosphingolipide représenté par la formule (XIV') :

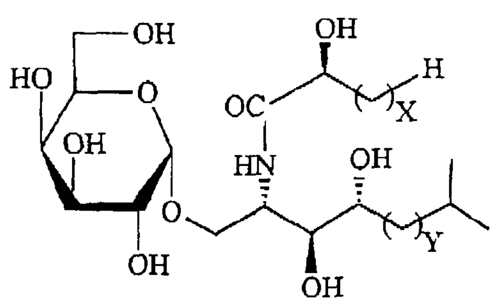

(XIV')

dans laquelle X signifie un entier de 20 à 24 et Y signifie un entier de 9 à 13.

**30.** Procédé selon la revendication 28, dans lequel X signifie un entier de 20 à 22 et Y signifie un entier de 10 à 12.

**31.** Procédé selon la revendication 29, dans lequel X signifie un entier de 21 à 23 et Y signifie un entier de 10 à 12.

**32.** Procédé selon la revendication 2, dans lequel on prépare un glycosphingolipide représenté par la formule (XV) :

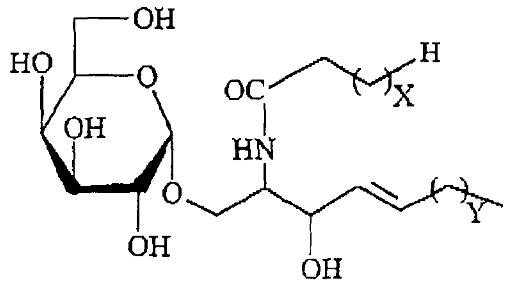

(XV)

dans laquelle X signifie un entier de 10 à 18 et Y signifie un entier de 10 à 14.

**33.** Procédé selon la revendication 32, dans lequel X signifie un entier de 11 à 17 et Y signifie un entier de 11 à 13.

**34.** Procédé selon la revendication 32, dans lequel on prépare un glycosphingolipide représenté par la formule (XVI):

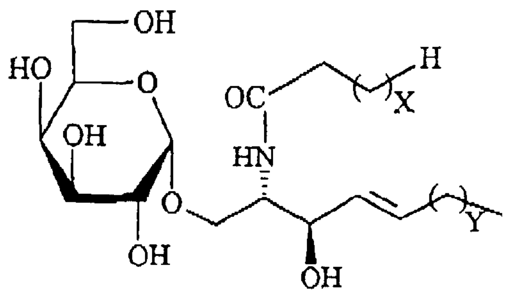

(XVI)

dans laquelle X signifie un entier de 10 à 18 et Y signifie un entier de 10 à 14.

**35.** Procédé selon la revendication 34, dans lequel X signifie un entier de 11 à 17 et Y signifie un entier de 11 à 13.

**36.** Procédé selon la revendication 2, dans lequel on prépare un glycosphingolipide représenté par la formule (XVII) :

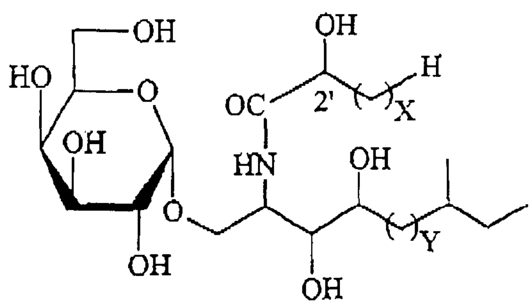

(XVII)

dans laquelle X signifie un entier de 21 à 25 et Y signifie un entier de 9 à 13.

**37.** Procédé selon la revendication 36, dans lequel X signifie un entier de 22 à 24 et Y signifie un entier de 10 à 12.

**38.** Procédé selon la revendication 36, dans lequel on prépare un glycosphingolipide représenté par la formule (XVIII) :

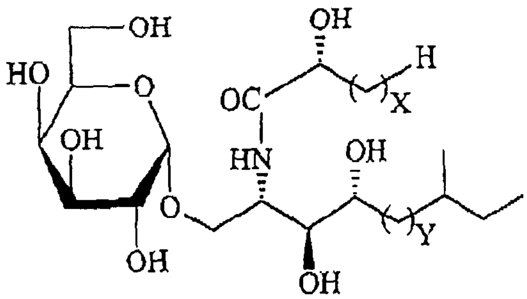

(XVIII)

dans laquelle X signifie un entier de 21 à 25 et Y signifie un entier de 9 à 13.

**39.** Procédé selon la revendication 38, dans lequel X signifie un entier de 22 à 24 et Y signifie un entier de 10 à 12.

**40.** Procédé selon la revendication 1, dans lequel on prépare un glycosphingolipide représenté par la formule (XIX) :

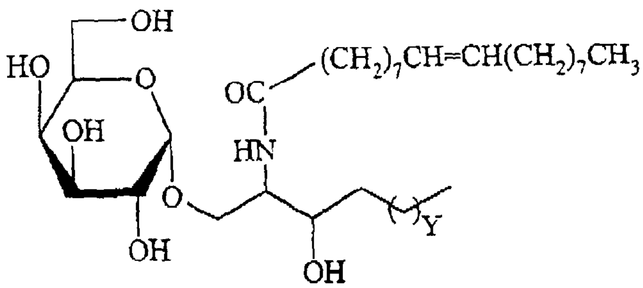

(XIX)

dans laquelle Y signifie un entier de 11 à 15.

**41.** Procédé selon la revendication 40, dans lequel Y signifie un entier de 12 à 14.

**42.** Procédé selon la revendication 40, dans lequel on prépare un glycosphingolipide représenté par la formule (XX) :

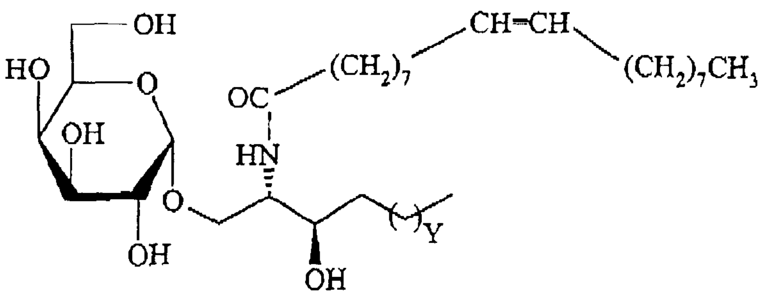

(XX)

dans laquelle Y signifie un entier de 11 à 15.

**43.** Procédé selon la revendication 42, dans lequel Y signifie un entier de 12 à 14.

**44.** Procédé selon la revendication 1, dans lequel on prépare un glycosphingolipide choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,

(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol,
(10) (2S,3R)-1-(α-D-galactopyranosyloxy)-2-tétradécanoylamino-3-hexadécanol,
(11) (2R,3S)-1-(α-D-galactopyranosyloxy)-2-tétradécanoylamino-3-hexadécanol,
(12) (2S,3S)-1-(α-D-galactopyranosyloxy)-2-tétradécanoylamino-3-hexadécanol,
(13) (2R,3R)-1-(α-D-galactopyranosyloxy)-2-tétradécanoylamino-3-hexadécanol,
(14) (2S,3R)-1-(α-D-galactopyranosyloxy)-2-[(R)-2-hydroxytétracosanoylamino]-3-octadécanol,
(15) (2S,3R,4E)-1-(α-D-galactopyranosyloxy)-2-octadécanoylamino-4-octadécèn-3-ol,
(16) (2S,3R,4E)-1-(α-D-galactopyranosyloxy)-2-tétradécanoylamino-4-octadécèn-3-ol,
(17) (2S,3S,4R)-1-(α-D-galactopyranosyloxy)-2-tétracosanoylamino-3,4-octadécanediol,
(18) (2S,3S,4R)-1-(α-D-galactopyranosyloxy)-2-tétracosanoylamino-3,4-heptadécanediol,
(19) (2S,3S,4R)-1-(α-D-galactopyranosyloxy)-2-tétracosanoylamino-3,4-pentadécanediol,
(20) (2S,3S,4R)-1-(α-D-galactopyranosyloxy)-2-tétracosanoylamino-3,4-undécanediol,
(21) (2S,3S,4R)-1-(α-D-galactopyranosyloxy)-2-hexacosanoylamino-3,4-heptadécanediol,
(22) (2S,3S,4R)-1-(α-D-galactopyranosyloxy)-2-[(R)-2-hydroxytétracosanoylamino]-3,4-octadécanediol,
(23) (2S,3S,4R)-1-(α-D-galactopyranosyloxy)-2-[(R)-2-hydroxytétracosanoylamino]-3,4-heptadécanediol,
(24) (2S,3S,4R)-1-(α-D-galactopyranosyloxy)-2-[(R)-2-hydroxytétracosanoylamino]-3,4-pentadécanediol,
(25) (2S,3S,4R)-1-(α-D-galactopyranosyloxy)-2-[(R)-2-hydroxytétracosanoylamino]-3,4-undécanediol,
(26) (2S,3S,4R)-1-(α-D-galactopyranosyloxy)-2-[(R)-2-hydroxyhexacosanoylamino]-3,4-octadécanediol,
(27) (2S,3S,4R)-1-(α-D-galactopyranosyloxy)-2-[(R)-2-hydroxyhexacosanoylamino]-3,4-nonadécanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytétracosanoylamino]-3,4-heptadécanediol,
(30) (2S,3S,4R)-1-(α-D-galactopyranosyloxy)-2-[(R)-2-hydroxytétracosanoylamino]-3,4-hexadécanediol,
(31) (2S,3S,4R)-1-(α-D-galactopyranosyloxy)-2-[(S)-2-hydroxytétracosanoylamino]-16-méthyl-3,4-heptadécanediol,
(32) (2S,3S,4R)-1-(α-D-galactopyranosyloxy)-16-méthyl-2-tétracosanoylamino-3,4-heptadécanediol,
(33) (2S,3S,4R)-1-(α-D-galactopyranosyloxy)-2-[(R)-2-hydroxytricosanoylamino]-16-méthyl-3,4-heptadécanediol,
(34) (2S,3S,4R)-1-(α-D-galactopyranosyloxy)-2-[(R)-2-hydroxypentacosanoylamino]-16-méthyl-3,4-octadécanediol
et
(35) (2S,3R)-1-(α-D-galactopyranosyloxy)-2-oléoylamino-3-octadécanol.

**45.** Procédé pour la préparation d'un glycosphingolipide comprenant la récolte de l'éponge marine <u>Agelas</u> <u>mauritianus</u>, sa soumission à une opération d'extraction avec un solvant organique et l'isolement à partir de l'extrait d'au moins un des glycosphingolipides suivants représentés par la formule (I) selon la revendication 2 :

(1) (2S,3S,4R)-1-(α-D-galactopyranosyloxy)-2-[(R)-2-hydroxytétracosanoylamino]-3,4-heptadécanediol,
(2) (2S,3S,4R)-1-(α-D-galactopyranosyloxy)-2-[(R)-2-hydroxytétracosanoylamino]-3,4-hexadécanediol,
(3) (2S,3S,4R)-1-(α-D-galactopyranosyloxy)-2-[(R)-2-hydroxytricosanoylamino]-16-méthyl-3,4-heptadécanediol et
(4) (2S,3S,4R)-1-(α-D-galactopyranosyloxy)-2-[(R)-2-hydroxypentacosanoylamino]-16-méthyl-3,4-octadécanediol.

**46.** Procédé pour préparer un agent antitumoral comprenant le mélange d'un ou plusieurs composés représentés par la formule (A) :

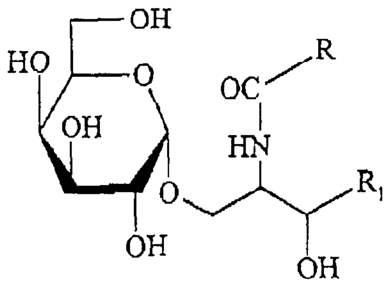

(A)

dans laquelle

R représente

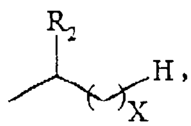

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17

en tant qu'ingrédients actifs avec des additifs pharmaceutiquement acceptables.

**47.** Procédé pour préparer un agent immunostimulant comprenant le mélange d'un ou plusieurs composés représentés par la formule (A) :

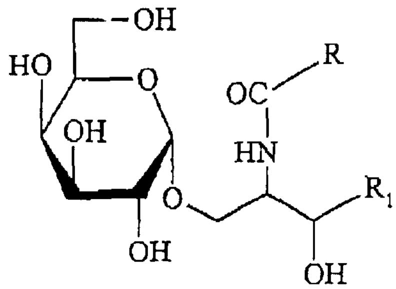 (A)

dans laquelle

R représente

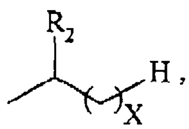

où $R_2$ représente H ou OH et X signifie un entier de 0 à 26 ou $-(CH_2)_7CH{=}CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

et

(e) $-CH(OH)(CH_2)_YCH(CH_3)CH_2CH_3$,

dans lesquels Y signifie un entier de 5 à 17

en tant qu'ingrédients actifs avec des additifs pharmaceutiquement acceptables.

**48.** Utilisation d'un ou plusieurs composés représentés par la formule (A) :

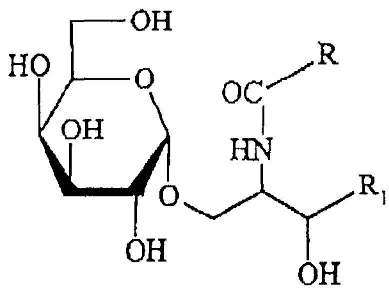

(A)

dans laquelle

R représente

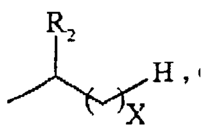

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17

dans la préparation d'un agent antitumoral.

**49.** Utilisation d'un ou plusieurs composés représentés par la formule (A) :

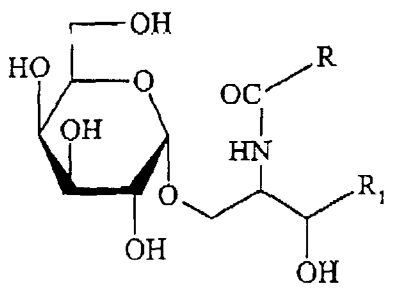

(A)

dans laquelle

R représente

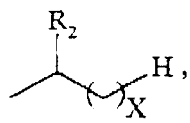

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17

dans la préparation d'un médicament immunostimulant.

**Revendications pour l'Etat contractant suivant : GR**

1. Glycosphingolipide comprenant un résidu alpha-galactose représenté par la formule (A) :

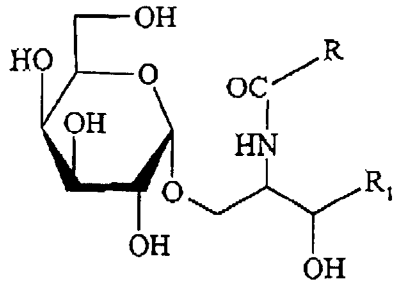

(A)

dans laquelle

R représente

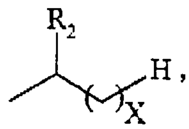

où $R_2$ représente H ou OH et X signifie un entier de 0 à 26 ou $-(CH_2)_7CH{=}CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

et

(e) $-CH(OH)(CH_2)_YCH(CH_3)CH_2CH_3$,

dans lesquels Y signifie un entier de 5 à 17.

**2.** Glycosphingolipide selon la revendication 1, qui est représenté par la formule (I) :

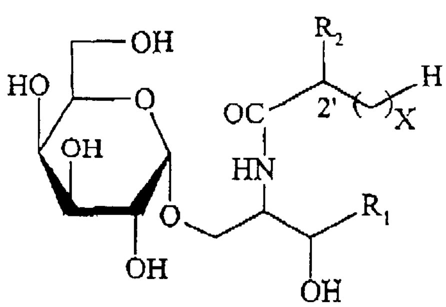

(I)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15 ; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**3.** Glycosphingolipide selon la revendication 2, qui est représenté par la formule (II) :

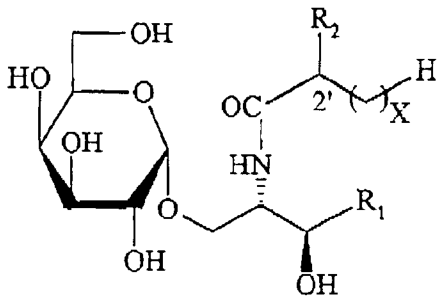

(II)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

4. Glycosphingolipide selon la revendication 2, qui est représenté par la formule (III) :

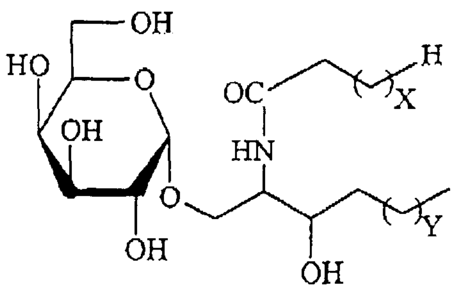

(III)

dans laquelle X signifie un entier de 0 à 24 et Y signifie un entier de 7 à 15.

5. Glycosphingolipide selon la revendication 4, dans lequel X signifie un entier de 8 à 22 et Y signifie un entier de 9 à 13.

6. Glycosphingolipide selon la revendication 4, qui est représenté par la formule (IV) :

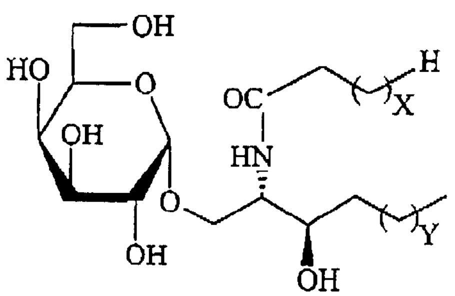

(IV)

dans laquelle X signifie un entier de 0 à 24 et Y signifie un entier de 7 à 15.

7. Glycosphingolipide selon la revendication 6, dans lequel X signifie un entier de 8 à 22 et Y signifie un entier de 9

à 13.

8. Glycosphingolipide selon la revendication 2, qui est représenté par la formule (V) :

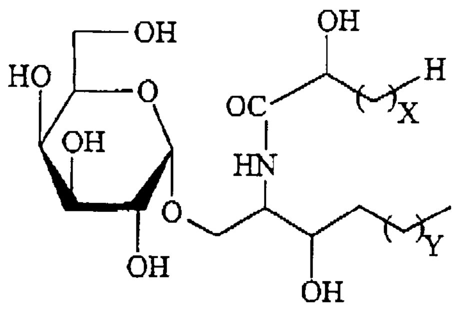

(V)

dans laquelle X signifie un entier de 20 à 24 et Y signifie un entier de 11 à 15.

9. Glycosphingolipide selon la revendication 8, dans lequel X signifie un entier de 21 à 23 et Y signifie un entier de 12 à 14.

10. Glycosphingolipide selon la revendication 8, qui est représenté par la formule (VI) :

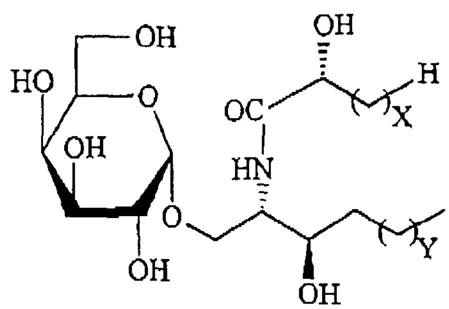

(VI)

dans laquelle X signifie un entier de 20 à 24 et Y signifie un entier de 11 à 15.

11. Glycosphingolipide selon la revendication 10, dans lequel X signifie un entier de 21 à 23 et Y signifie un entier de 12 à 14.

12. Glycosphingolipide selon la revendication 2, qui est représenté par la formule (VII) :

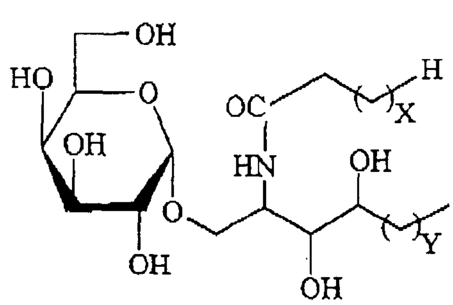

(VII)

dans laquelle X signifie un entier de 18 à 26 et Y signifie un entier de 5 à 15.

**13.** Glycosphingolipide selon la revendication 12, dans lequel X signifie un entier de 21 à 25 et Y signifie un entier de 6 à 14.

**14.** Glycosphingolipide selon la revendication 12, qui est représenté par la formule (VIII) :

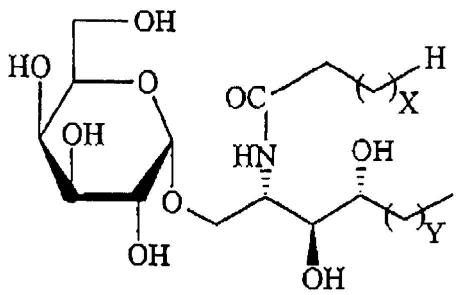

(VIII)

dans laquelle X signifie un entier de 18 à 26 et Y signifie un entier de 5 à 15.

**15.** Glycosphingolipide selon la revendication 14, dans lequel X signifie un entier de 21 à 25 et Y signifie un entier de 6 à 14.

**16.** Glycosphingolipide selon la revendication 2, qui est représenté par la formule (IX) :

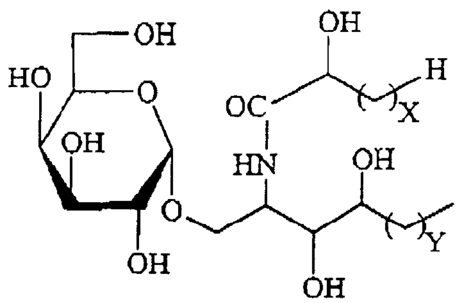

(IX)

dans laquelle X signifie un entier de 18 à 26 et Y signifie un entier de 5 à 17.

**17.** Glycosphingolipide selon la revendication 16, dans lequel X signifie un entier de 21 à 25 et Y signifie un entier de 6 à 16.

**18.** Glycosphingolipide selon la revendication 16, qui est représenté par la formule (X) :

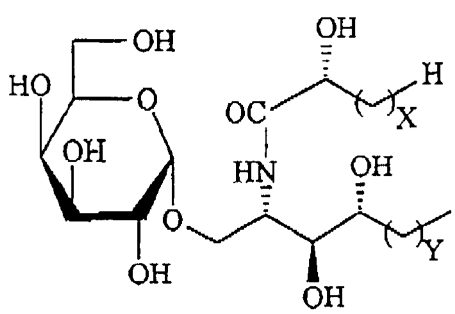

(X)

dans laquelle X signifie un entier de 18 à 26 et Y signifie un entier de 5 à 17.

**19.** Glycosphingolipide selon la revendication 16, qui est représenté par la formule (X'):

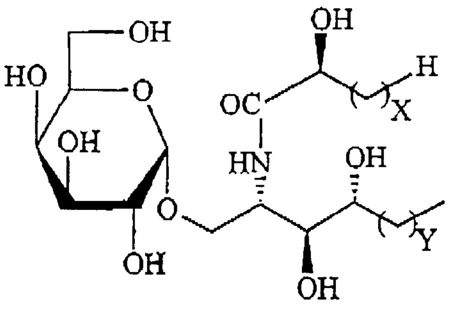

(X')

dans laquelle X signifie un entier de 20 à 24 et Y signifie un entier de 10 à 14.

**20.** Glycosphingolipide selon la revendication 18, dans lequel X signifie un entier de 21 à 25 et Y signifie un entier de 6 à 16.

**21.** Glycosphingolipide selon la revendication 19, dans lequel X signifie un entier de 21 à 23 et Y signifie un entier de 11 à 13.

**22.** Glycosphingolipide selon la revendication 2, qui est représenté par la formule (XI) :

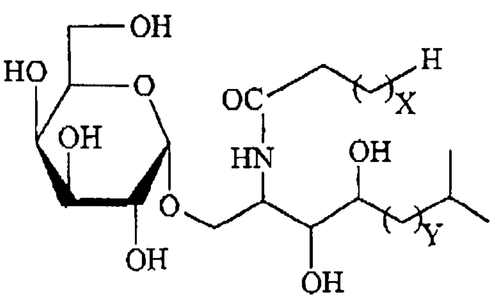

(XI)

dans laquelle X signifie un entier de 20 à 24 et Y signifie un entier de 9 à 13.

**23.** Glycosphingolipide selon la revendication 22, dans lequel X signifie un entier de 21 à 23 et Y signifie un entier de 10 à 12.

**24.** Glycosphingolipide selon la revendication 22, qui est représenté par la formule (XII):

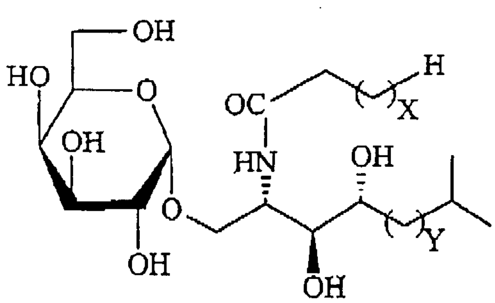

(XII)

dans laquelle X signifie un entier de 20 à 24 et Y signifie un entier de 9 à 13.

**25.** Glycosphingolipide selon la revendication 24, dans lequel X signifie un entier de 21 à 23 et Y signifie un entier de

10 à 12.

**26.** Glycosphingolipide selon la revendication 2, qui est représenté par la formule (XIII) :

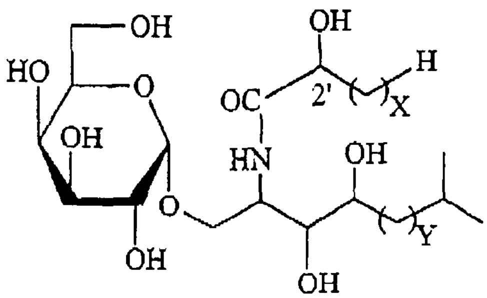

(XIII)

dans laquelle X signifie un entier de 18 à 24 et Y signifie un entier de 9 à 13.

**27.** Glycosphingolipide selon la revendication 26, dans lequel X signifie un entier de 20 à 23 et Y signifie un entier de 10 à 12.

**28.** Glycosphingolipide selon la revendication 26, qui est représenté par la formule (XIV) :

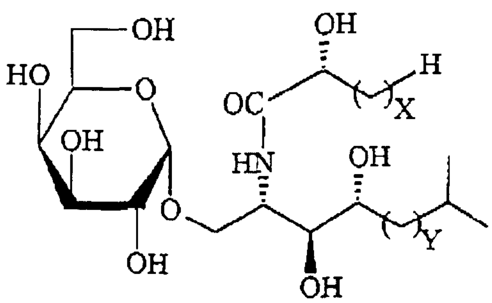

(XIV)

dans laquelle X signifie un entier de 19 à 23 et Y signifie un entier de 9 à 13.

**29.** Glycosphingolipide selon la revendication 26, qui est représenté par la formule (XIV') :

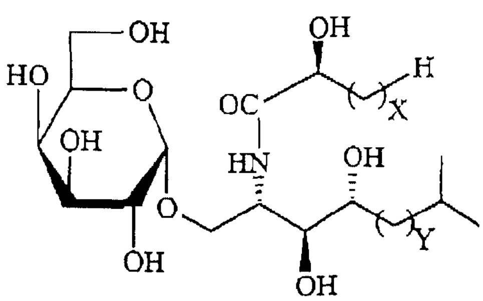

(XIV')

dans laquelle X signifie un entier de 20 à 24 et Y signifie un entier de 9 à 13.

**30.** Glycosphingolipide selon la revendication 28, dans lequel X signifie un entier de 20 à 22 et Y signifie un entier de 10 à 12.

**31.** Glycosphingolipide selon la revendication 29, dans lequel X signifie un entier de 21 à 23 et Y signifie un entier de

10 à 12.

**32.** Glycosphingolipide selon la revendication 2, qui est représenté par la formule (XV) :

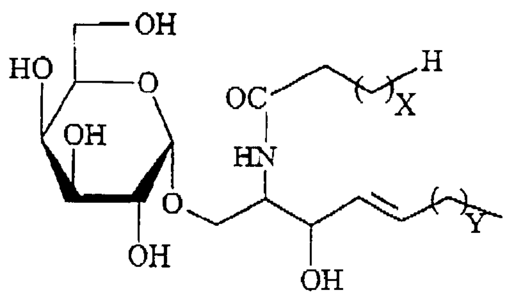

(XV)

dans laquelle X signifie un entier de 10 à 18 et Y signifie un entier de 10 à 14.

**33.** Glycosphingolipide selon la revendication 32, dans lequel X signifie un entier de 11 à 17 et Y signifie un entier de 11 à 13.

**34.** Glycosphingolipide selon la revendication 32, qui est représenté par la formule (XVI):

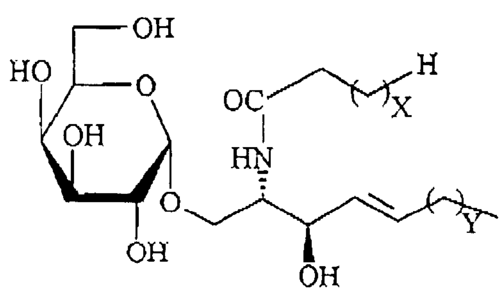

(XVI)

dans laquelle X signifie un entier de 10 à 18 et Y signifie un entier de 10 à 14.

**35.** Glycosphingolipide selon la revendication 34, dans lequel X signifie un entier de 11 à 17 et Y signifie un entier de 11 à 13.

**36.** Glycosphingolipide selon la revendication 2, qui est représenté par la formule (XVII):

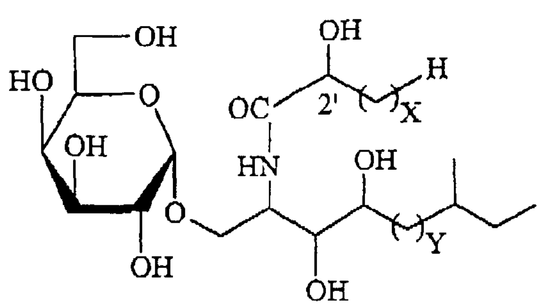

(XVII)

dans laquelle X signifie un entier de 21 à 25 et Y signifie un entier de 9 à 13.

**37.** Glycosphingolipide selon la revendication 36, dans lequel X signifie un entier de 22 à 24 et Y signifie un entier de

10 à 12.

**38.** Glycosphingolipide selon la revendication 36, qui est représenté par la formule (XVIII) :

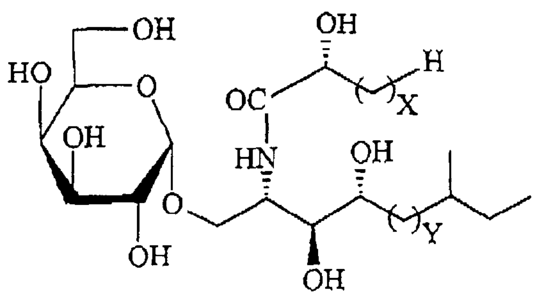

(XVIII)

dans laquelle X signifie un entier de 21 à 25 et Y signifie un entier de 9 à 13.

**39.** Gljcosphingolipide selon la revendication 38, dans lequel X signifie un entier de 22 à 24 et Y signifie un entier de 10 à 12.

**40.** Glycosphingolipide selon la revendication 1, qui est représenté par la formule (XIX) :

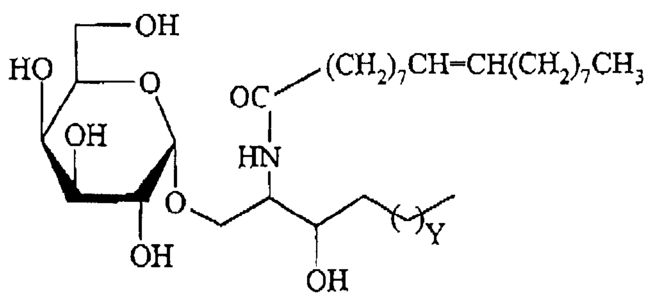

(XIX)

dans laquelle Y signifie un entier de 11 à 15.

**41.** Glycosphingolipide selon la revendication 40, dans lequel Y signifie un entier de 12 à 14.

**42.** Glycosphingolipide selon la revendication 40, qui est représenté par la formule (XX):

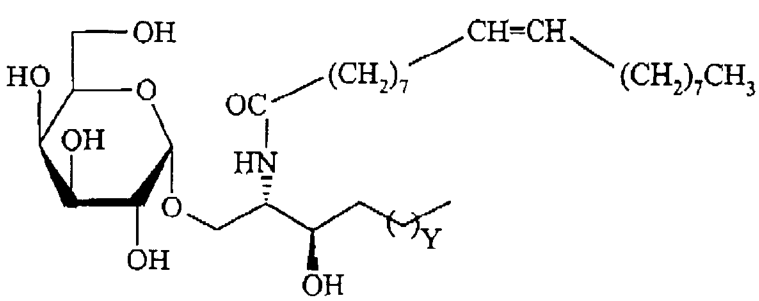

(XX)

dans laquelle Y signifie un entier de 11 à 15.

**43.** Glycosphingolipide selon la revendication 42, dans lequel Y signifie un entier de 12 à 14.

**44.** Glycosphingolipide selon la revendication 1, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol,
(10) (2S,3R)-1-(α-D-galactopyranosyloxy)-2-tétradécanoylamino-3-hexadécanol,
(11) (2R,3S)-1-(α-D-galactopyranosyloxy)-2-tétradécanoylamino-3-hexadécanol,
(12) (2S,3S)-1-(α-D-galactopyranosyloxy)-2-tétradécanoylamino-3-hexadécanol,
(13) (2R,3R)-1-(α-D-galactopyranosyloxy)-2-tétradécanoylamino-3-hexadécanol,
(14) (2S,3R)-1-(α-D-galactopyranosyloxy)-2-[(R)-2-hydroxytétracosanoylamino]-3-octadécanol,
(15) (2S,3R,4E)-1-(α-D-galactopyranosyloxy)-2-octadécanoylamino-4-octadécèn-3-ol,
(16) (2S,3R,4E)-1-(α-D-galactopyranosyloxy)-2-tétradécanoylamino-4-octadécèn-3-ol,
(17) (2S,3S,4R)-1-(α-D-galactopyranosyloxy)-2-tétracosanoylamino-3,4-octadécanediol,
(18) (2S,3S,4R)-1-(α-D-galactopyranosyloxy)-2-tétracosanoylamino-3,4-heptadécanediol,
(19) (2S,3S,4R)-1-(α-D-galactopyranosyloxy)-2-tétracosanoylamino-3,4-pentadécanediol,
(20) (2S,3S,4R)-1-(α-D-galactopyranosyloxy)-2-tétracosanoylamino-3,4-undécanediol,
(21) (2S,3S,4R)-1-(α-D-galactopyranosyloxy)-2-hexacosanoylamino-3,4-heptadécanediol,
(22) (2S,3S,4R)-1-(α-D-galactopyranosyloxy)-2-[(R)-2-hydroxytétracosanoylamino]-3,4-octadécanediol,
(23) (2S,3S,4R)-1-(α-D-galactopyranosyloxy)-2-[(R)-2-hydroxytétracosanoylamino]-3,4-heptadécanediol,
(24) (2S,3S,4R)-1-(α-D-galactopyranosyloxy)-2-[(R)-2-hydroxytétracosanoylamino]-3,4-pentadécanediol,
(25) (2S,3S,4R)-1-(α-D-galactopyranosyloxy)-2- [(R)-2-hydroxytétracosanoylamino)-3,4-undécanediol,
(26) (2S,3S,4R)-1-(α-D-galactopyranosyloxy)-2-[(R)-2-hydroxyhexacosanoylamino]-3,4-octadécanediol,
(27) (2S,3S,4R)-1-(α-D-galactopyranosyloxy)-2-[(R)-2-hydroxyhexacosanoylamino]-3,4-nonadécanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytétracosanoylamino]-3,4-heptadécanediol,
(30) (2S,3S,4R)-1-(α-D-galactopyranosyloxy)-2-[(R)-2-hydroxytétracosanoylamino]-3,4-hexadécanediol,
(31) (2S,3S,4R)-1-(α-D-galactopyranosyloxy)-2-[(S)-2-hydroxytétracosanoylamino]-16-méthyl-3,4-heptadécanediol,
(32) (2S,3S,4R)-1-(α-D-galactopyranosyloxy)-16-méthyl-2-tétracosanoylamino-3,4-heptadécanediol,
(33) (2S,3S,4R)-1-(α-D-galactopyranosyloxy)-2-[(R)-2-hydroxytricosanoylamino]-16-méthyl-3,4-heptadécanediol,
(34) (2S,3S,4R)-1-(α-D-galactopyranosyloxy)-2-[(R)-2-hydroxypentacosanoylamino]-16-méthyl-3,4-octadécanediol
et
(35) (2S,3R)-1-(α-D-galactopyranosyloxy)-2-oléoylamino-3-octadécanol.

**45.** Glycosphingolipide selon la revendication 44, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol et
(10) (2S,3R)-1-(α-D-galactopyranosyloxy)-2-tétradécanoylamino-3-hexadécanol.

**46.** Glycosphingolipide selon la revendication 45, qui est choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,

(4) (2S,3R)-1-(α-D-galactopyranosyloxy)-2-octadécanoylamino-3-octadécanol,
(5) (2S,3R)-1-(α-D-galactopyranosyloxy)-2-tetradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-tétracosanoylamino-3-tétradécanol et
(8) (2S,3R)-1-(α-D-galactopyranosyloxy)-2-tétradécanoylamino-3-hexadécanol.

**47.** Glycosphingolipide selon la revendication 46, qui est choisi dans le groupe constitué par les composés suivants :

(1) (2S,3R)-1-(α-D-galactopyranosyloxy)-2-octadécanoylamino-3-octadécanol,
(2) (2S,3R)-1-(α-D-galactopyranosyloxy)-2-tétradécanoylamio-3-octadécanol et
(3) (2S,3R)-1-(α-D-galactopyranosyloxy)-2-tétradécanoylamino-3-hexadécanol.

**48.** Glycosphingolipide selon la revendication 44, qui est le (2S,3R)-1-(α-D-galactopyranosyloxy)-2-[(R)-2-hydroxytétracosanoylamino]-3-octadécanol,

**49.** Glycosphingolipide selon la revendication 44, qui est choisi dans le groupe constitué par les composés suivants :

(1) (2S,3R,4E)-1-(α-D-galactopyranosyloxy)-2-octadécanoylamino-4-octadécèn-3-ol et
(2) (2S,3R,4E)-1-(α-D-galactopyranosyloxy)-2-tétradécanoylamino-4-octadécèn-3-ol.

**50.** Glycosphingolipide selon la revendication 44, qui est choisi dans le groupe constitué par les composés suivants :

(1) (2S,3S,4R)-1-(α-D-galactopyranosyloxy)-2-tétracosanoylamino-3,4-octadécanediol,
(2) (2S,3S,4R)-1-(α-D-galactopyranosyloxy)-2-tétracosanoylamino-3,4-heptadécanediol,
(3) (2S,3S,4R)-1-(α-D-galactopyranosyloxy)-2-tétracosanoylamino-3,4-pentadécanediol,
(4) (2S,3S,4R)-1-(α-D-galactopyranosyloxy)-2-tétracosanoylamino-3,4-undécanediol et
(5) (2S,3S,4R)-1-(α-D-galactopyranosyloxy)-2-hexacosanoylamino-3,4-heptadécanediol.

**51.** Glycosphingolipide selon la revendication 44, qui est choisi dans le groupe constitué par les composés suivants :

(1) (2S,3S,4R)-1-(α-D-galactopyranosyloxy)-2-[(R)-2-hydroxytétracosanoylamino]-3,4-octadécanediol,
(2) (2S,3S,4R)-1-(α-D-galactopyranosyloxy)-2-[(R)-2-hydroxytétracosanoylamino]-3,4-heptadécanediol,
(3) (2S,3S,4R)-1-(α-D-galactopyranosyloxy)-2-[(R)-2-hydroxytétracosanoylamino]-3,4-pentadécanediol,
(4) (2S,3S,4R)-1-(α-D-galactopyranosyloxy)-2-[(R)-2-hydroxytétracosanoylamino]-3,4-undécanediol,
(5) (2S,3S,4R)-1-(α-D-galactopyranosyloxy)-2-[(R)-2-hydroxyhexacosanoylamino]-3,4-octadécanediol,
(6) (2S,3S,4R)-1-(α-D-galactopyranosyloxy)-2-[(R)-2-hydroxyhexacosanoylamino]-3,4-nonadécanediol,
(7) (2S,3S,4R)-1-(α-D-galactopyranosyloxy)-2-[(R)-2-hydroxyhexacosanoylamino]-3,4-icosanediol,
(8) (2S,3S,4R)-1-(α-D-galactopyranosyloxy)-2-[(S)-2-hydroxytétracosanoylamino]-3,4-heptadécanediol et
(9) (2S,3S,4R)-1-(α-D-galactopyranosyloxy)-2-[(R)-2-hydroxytétracosanoylamino]-3,4-hexadécanediol,

**52.** Glycosphingolipide selon la revendication 44, qui est choisi dans le groupe constitué par les composés suivants :

(1) (2S,3S,4R)-1-(α-D-galactopyranosyloxy)-2-[(S)-2-hydroxytétracosanoylamino]-16-méthyl-3,4-heptadécanediol,
(2) (2S,3S,4R)-1-(α-D-galactopyranosyloxy)-16-méthyl-2-tétracosanoylamino-3,4-heptadécanediol et
(3) (2S,3S,4R)-1-(α-D-galactopyranosyloxy)-2-[(R)-2-hydroxytricosanoylamino]-16-méthyl-3,4-heptadécanediol.

**53.** Glycosphingolipide selon la revendication 44, qui est le (2S,3S,4R)-1-(α-D-galactopyranosyloxy)-2-[(R)-2-hydroxypentacosanoylamino]-16-méthyl-3,4-octadécanediol.

**54.** Glycosphingolipide selon la revendication 44, qui est le (2S,3R)-1-(α-D-galactopyranosyloxy)-2-oléoylamino-3-octadécanol.

**55.** Procédé pour préparer un glycosphingolipide comprenant un résidu alpha-galactose représenté par la formule (A) :

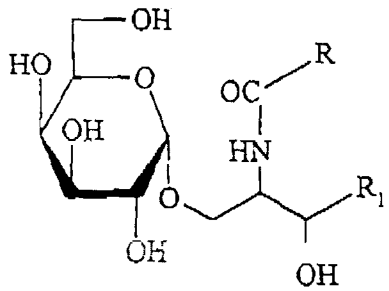

(A)

dans laquelle

R représente

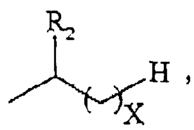

où $R_2$ représente H ou OH et X signifie un entier de 0 à 26 ou $-(CH_2)_7CH{=}CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

et

(e) $-CH(OH)(CH_2)_YCH(CH_3)CH_2CH_3$,

dans lesquels Y signifie un entier de 5 à 17, lequel comprend l'un des procédés suivants :

a) extraction de (A) à partir de l'espèce d'éponge marine Algelas mauretianus à l'aide d'au moins un solvant organique approprié et d'eau;
b) modification de la sphingosine; et
c) réactions chimiques générales pour la synthèse des glycosphingolipides.

**56.** Procédé selon la revendication 55, dans lequel on prépare un glycosphingolipide représenté par la formule (I) :

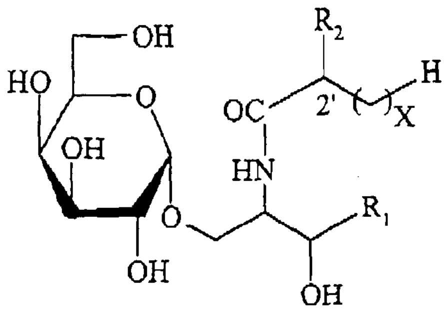

(I)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH=CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**57.** Procédé selon la revendication 56, dans lequel on prépare un glycosphingolipide représenté par la formule (II) :

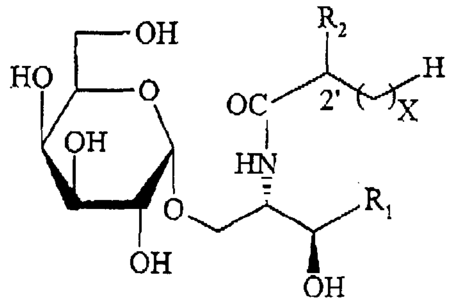

(II)

dans laquelle

$R_1$ représente l'un des substituants définis par (a) à (e) ci-dessous et $R_2$ représente H ou OH (X est défini dans les paragraphes (a) à (e) suivants) :

(a) $-CH_2(CH_2)_YCH_3$,

où lorsque $R_2$ représente H, X signifie un entier de 0 à 24 et Y signifie un entier de 7 à 15; lorsque $R_2$ représente OH, X signifie un entier de 20 à 24 et Y signifie un entier de 11 à 15;

(b) $-CH(OH)(CH_2)_YCH_3$,

où lorsque $R_2$ représente H, X signifie un entier de 18 à 26 et Y signifie un entier de 5 à 15; lorsque $R_2$ représente OH, X signifie un entier de 18 à 26 et Y signifie un entier de 5 à 17;

(c) $-CH(OH)(CH_2)_YCH(CH_3)_2$,

où lorsque $R_2$ représente H, X signifie un entier de 20 à 24 et Y signifie un entier de 9 à 13; lorsque $R_2$ représente OH, X signifie un entier de 18 à 24 et Y signifie un entier de 9 à 13;

(d) $-CH{=}CH(CH_2)_YCH_3$,

où lorsque $R_2$ représente H, X signifie un entier de 10 à 18 et Y signifie un entier de 10 à 14; et

(e) $-CH(OH)(CH_2)_YCH(CH_3)CH_2CH_3$,

où lorsque $R_2$ représente OH, X signifie un entier de 21 à 25 et Y signifie un entier de 9 à 13.

**58.** Procédé selon la revendication 56, dans lequel on prépare un glycosphingolipide représenté par la formule (III) :

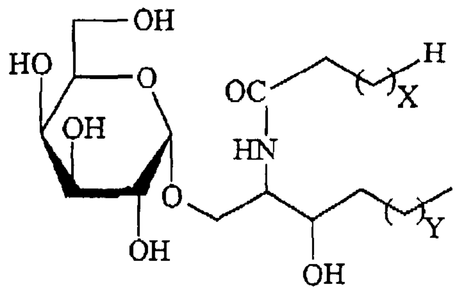

(III)

dans laquelle X signifie un entier de 0 à 24 et Y signifie un entier de 7 à 15.

**59.** Procédé selon la revendication 58, dans lequel X signifie un entier de 8 à 22 et Y signifie un entier de 9 à 13.

**60.** Procédé selon la revendication 58, dans lequel on prépare un glycosphingolipide représenté par la formule (IV) :

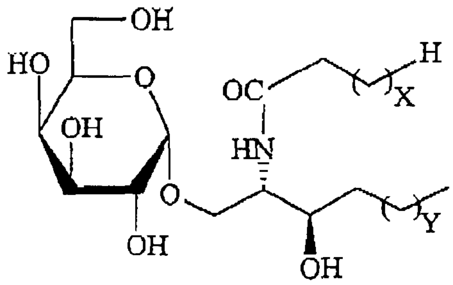

(IV)

dans laquelle X signifie un entier de 0 à 24 et Y signifie un entier de 7 à 15.

**61.** Procédé selon la revendication 60, dans lequel X signifie un entier de 8 à 22 et Y signifie un entier de 9 à 13.

**62.** Procédé selon la revendication 56, dans lequel on prépare un glycosphingolipide représenté par la formule (V) :

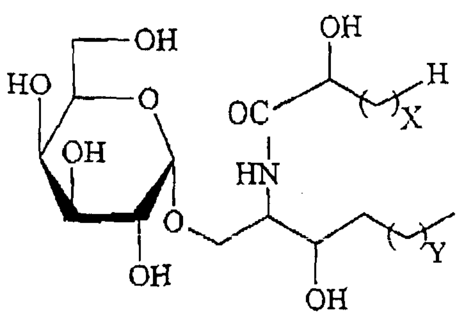

(V)

dans laquelle X signifie un entier de 20 à 24 et Y signifie un entier de 11 à 15.

**63.** Procédé selon la revendication 62, dans lequel X signifie un entier de 21 à 23 et Y signifie un entier de 12 à 14.

**64.** Procédé selon la revendication 62, dans lequel on prépare un glycosphingolipide représenté par la formule (VI) :

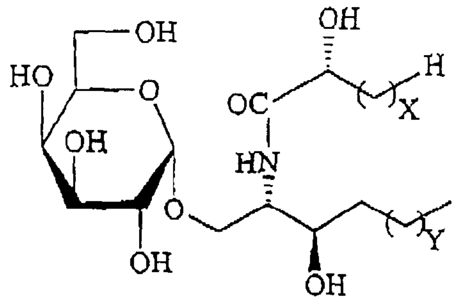

(VI)

dans laquelle X signifie un entier de 20 à 24 et Y signifie un entier de 11 à 15.

**65.** Procédé selon la revendication 64, dans lequel X signifie un entier de 21 à 23 et Y signifie un entier de 12 à 14.

**66.** Procédé selon la revendication 56, dans lequel on prépare un glycosphingolipide représenté par la formule (VII) :

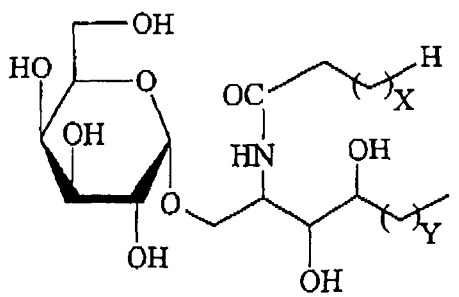

(VII)

dans laquelle X signifie un entier de 18 à 26 et Y signifie un entier de 5 à 15.

**67.** Procédé selon la revendication 66, dans lequel X signifie un entier de 21 à 25 et Y signifie un entier de 6 à 14.

**68.** Procédé selon la revendication 66, dans lequel on prépare un glycosphingolipide représenté par la formule (VIII) :

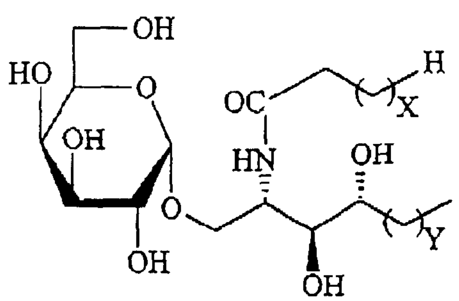

(VIII)

dans laquelle X signifie un entier de 18 à 26 et Y signifie un entier de 5 à 15.

**69.** Procédé selon la revendication 68, dans lequel X signifie un entier de 21 à 25 et Y signifie un entier de 6 à 14.

**70.** Procédé selon la revendication 56, dans lequel on prépare un glycosphingolipide représenté par la formule (IX) :

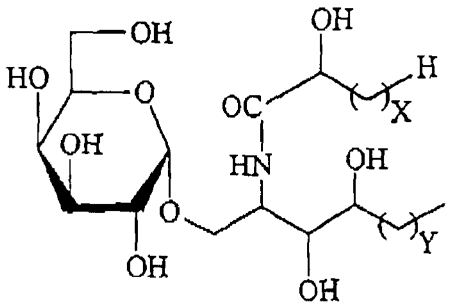

(IX)

dans laquelle X signifie un entier de 18 à 26 et Y signifie un entier de 5 à 17.

**71.** Procédé selon la revendication 70, dans lequel X signifie un entier de 21 à 25 et Y signifie un entier de 6 à 16.

**72.** Procédé selon la revendication 70, dans lequel on prépare un glycosphingolipide représenté par la formule (X) :

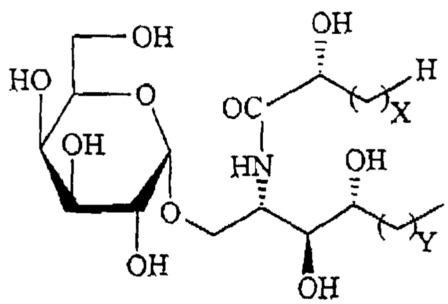

(X)

dans laquelle X signifie un entier de 18 à 26 et Y signifie un entier de 5 à 17.

**73.** Procédé selon la revendication 70, dans lequel on prépare un glycosphingolipide représenté par la formule (X') :

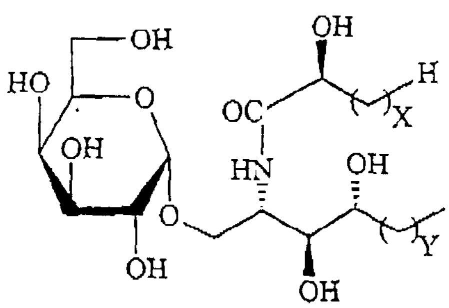

(X')

dans laquelle X signifie un entier de 20 à 24 et Y signifie un entier de 10 à 14.

**74.** Procédé selon la revendication 72, dans lequel X signifie un entier de 21 à 25 et Y signifie un entier de 6 à 16.

**75.** Procédé selon la revendication 74, dans lequel X signifie un entier de 21 à 23 et Y signifie un entier de 11 à 13.

**76.** Procédé selon la revendication 56, dans lequel on prépare un glycosphingolipide représenté par la formule (XI):

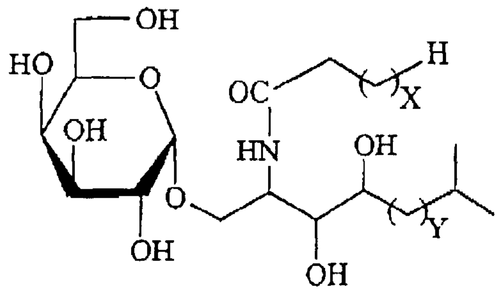

(XI)

dans laquelle X signifie un entier de 20 à 24 et Y signifie un entier de 9 à 13.

**77.** Procédé selon la revendication 76, dans lequel X signifie un entier de 21 à 23 et Y signifie un entier de 10 à 12.

**78.** Procédé selon la revendication 76, dans lequel on prépare un glycosphingolipide représenté par la formule (XII) :

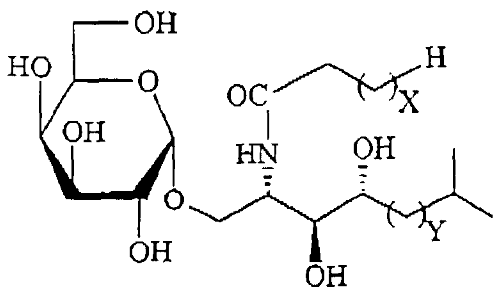

(XII)

dans laquelle X signifie un entier de 20 à 24 et Y signifie un entier de 9 à 13.

**79.** Procédé selon la revendication 78, dans lequel X signifie un entier de 21 à 23 et Y signifie un entier de 10 à 12.

**80.** Procédé selon la revendication 56, dans lequel on prépare un glycosphingolipide représenté par la formule (XIII) :

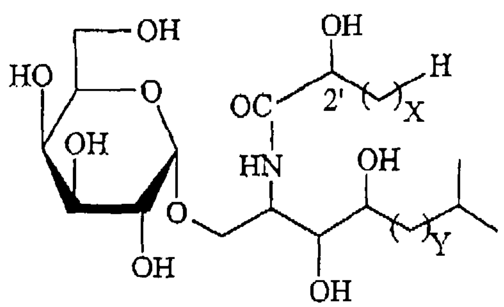

(XIII)

dans laquelle X signifie un entier de 18 à 24 et Y signifie un entier de 9 à 13.

**81.** Procédé selon la revendication 80, dans lequel X signifie un entier de 20 à 23 et Y signifie un entier de 10 à 12.

**82.** Procédé selon la revendication 80, dans lequel on prépare un glycosphingolipide représenté par la formule (XIV) :

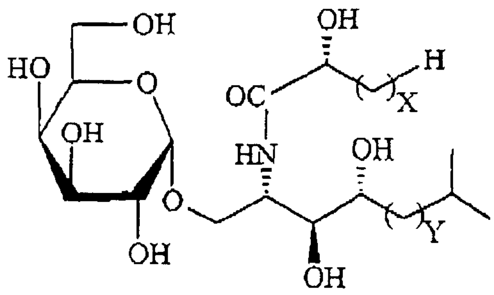

(XIV)

dans laquelle X signifie un entier de 19 à 23 et Y signifie un entier de 9 à 13.

**83.** Procédé selon la revendication 80, dans lequel on prépare un glycosphingolipide représenté par la formule (XIV') :

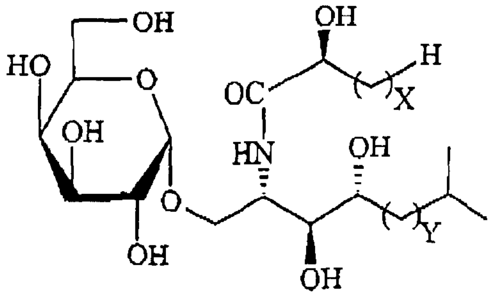

(XIV')

dans laquelle X signifie un entier de 20 à 24 et Y signifie un entier de 9 à 13.

**84.** Procédé selon la revendication 82, dans lequel X signifie un entier de 20 à 22 et Y signifie un entier de 10 à 12.

**85.** Procédé selon la revendication 83, dans lequel X signifie un entier de 21 à 23 et Y signifie un entier de 10 à 12.

**86.** Procédé selon la revendication 56, dans lequel on prépare un glycosphingolipide représenté par la formule (XV) :

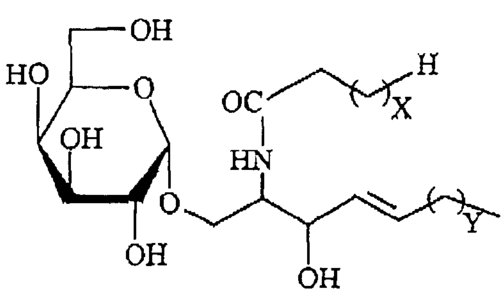

(XV)

dans laquelle X signifie un entier de 10 à 18 et Y signifie un entier de 10 à 14.

**87.** Procédé selon la revendication 86, dans lequel X signifie un entier de 11 à 17 et Y signifie un entier de 11 à 13.

**88.** Procédé selon la revendication 86, dans lequel on prépare un glycosphingolipide représenté par la formule (XVI):

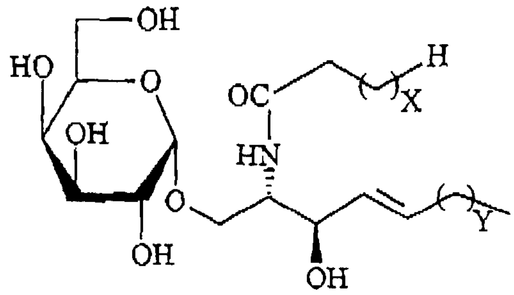

(XVI)

dans laquelle X signifie un entier de 10 à 18 et Y signifie un entier de 10 à 14.

**89.** Procédé selon la revendication 88, dans lequel X signifie un entier de 11 à 17 et Y signifie un entier de 11 à 13.

**90.** Procédé selon la revendication 56, dans lequel on prépare un glycosphingolipide représenté par la formule (XVII) :

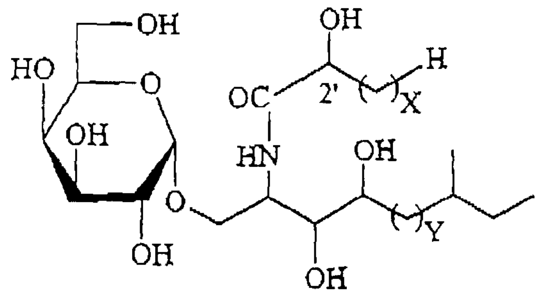

(XVII)

dans laquelle X signifie un entier de 21 à 25 et Y signifie un entier de 9 à 13.

**91.** Procédé selon la revendication 90, dans lequel X signifie un entier de 22 à 24 et Y signifie un entier de 10 à 12.

**92.** Procédé selon la revendication 90, dans lequel on prépare un glycosphingolipide représenté par la formule (XVIII) :

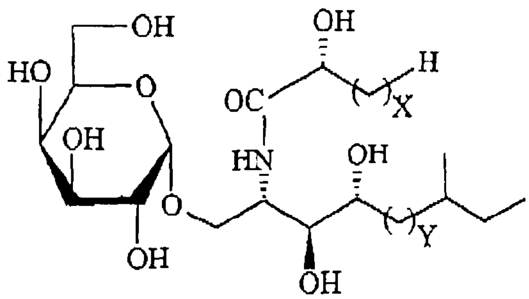

(XVIII)

dans laquelle X signifie un entier de 21 à 25 et Y signifie un entier de 9 à 13.

**93.** Procédé selon la revendication 92, dans lequel X signifie un entier de 22 à 24 et Y signifie un entier de 10 à 12.

**94.** Procédé selon la revendication 55, dans lequel on prépare un glycosphingolipide représenté par la formule (XIX) :

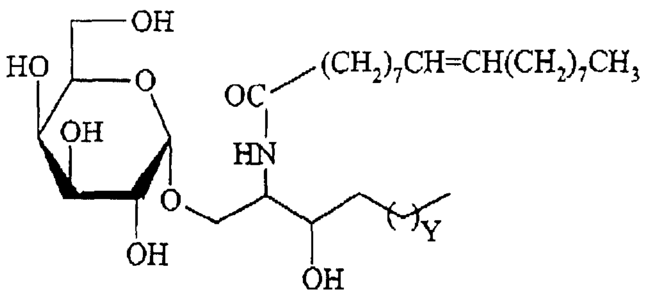

(XIX)

dans laquelle Y signifie un entier de 11 à 15.

**95.** Procédé selon la revendication 94, dans lequel Y signifie un entier de 12 à 14.

**96.** Procédé selon la revendication 94, dans lequel on prépare un glycosphingolipide représenté par la formule (XX) :

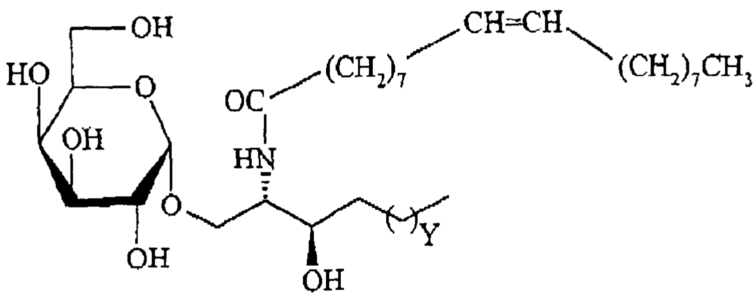

(XX)

dans laquelle Y signifie un entier de 11 à 15.

**97.** Procédé selon la revendication 96, dans lequel Y signifie un entier de 12 à 14.

**98.** Procédé selon la revendication 55, dans lequel on prépare un glycosphingolipide choisi dans le groupe constitué par les composés suivants :

(1) (2S,3R)-1-(α-D-galactopyranosyloxy)-2-tétracosanoylamino-3-octadécanol,
(2) (2S,3R)-2-docosanoylamino-1-(α-D-galactopyranosyloxy)-3-octadécanol,
(3) (2S,3R)-1-(α-D-galactopyranosyloxy)-2-icosanoylamino-3-octadécanol,
(4) (2S,3R)-1-(α-D-galactopyranosyloxy)-2-octadécanoylamino-3-octadécanol,
(5) (2S,3R)-1-(α-D-galactopyranosyloxy)-2-tétradécanoylamino-3-octadécanol,
(6) (2S,3R)-2-décanoylamino-1-(α-D-galactopyranosyloxy)-3-octadécanol,
(7) (2S,3R)-1-(α-D-galactopyranosyloxy)-2-octanoylamino-3-octadécanol,
(8) (2S,3R)-2-acétamino-1-(α-D-galactopyranosyloxy)-3-octadécanol,
(9) (2S,3R)-1-(α-D-galactopyranosyloxy)-2-tétracosanoylamino-3-tétradécanol,
(10) (2S,3R)-1-(α-D-galactopyranosyloxy)-2-tétradécanoylamino-3-hexadécanol,
(11) (2R,3S)-1-(α-D-galactopyranosyloxy)-2-tétradécanoylamino-3-hexadécanol,
(12) (2S,3S)-1-(α-D-galactopyranosyloxy)-2-tétradécanoylamino-3-hexadécanol,
(13) (2R,3R)-1-(α-D-galactopyranosyloxy)-2-tétradécanoylamino-3-hexadécanol,
(14) (2S,3R)-1-(α-D-galactopyranosyloxy)-2-[(R)-2-hydroxytétracosanoylamino]-3-octadécanol,
(15) (2S,3R,4E)-1-(α-D-galactopyranosyloxy)-2-octadécanoylamino-4-octadécèn-3-ol,
(16) (2S,3R,4E)-1-(α-D-galactopyranosyloxy)-2-tétradécanoylamino-4-octadécèn-3-ol,
(17) (2S,3S,4R)-1-(α-D-galactopyranosyloxy)-2-tétracosanoylamino-3,4-octadécanediol,
(18) (2S,3S,4R)-1-(α-D-galactopyranosyloxy)-2-tétracosanoylamino-3,4-heptadécanediol,
(19) (2S,3S,4R)-1-(α-D-galactopyranosyloxy)-2-tétracosanoylamino-3,4-pentadécanediol,
(20) (2S,3S,4R)-1-(α-D-galactopyranosyloxy)-2-tétracosanoylamino-3,4-undécanediol,

(21) (2S,3S,4R)-1-(α-D-galactopyranosyloxy)-2-hexacosanoylamino-3,4-heptadécanediol,
(22) (2S,3S,4R)-1-(α-D-galactopyranosyloxy)-2-[(R)-2-hydroxytétracosanoylamino]-3,4-octadécanediol,
(23) (2S,3S,4R)-1-(α-D-galactopyranosyloxy)-2-[(R)-2-hydroxytétracosanoylamino]-3,4-heptadécanediol,
(24) (2S,3S,4R)-1-(α-D-galactopyranosyloxy)-2-[(R)-2-hydroxytétracosanoylamino]-3,4-pentadécanediol,
(25) (2S,3S,4R)-1-(α-D-galactopyranosyloxy)-2-[(R)-2-hydroxytétracosanoylamino]-3,4-undécanediol,
(26) (2S,3S,4R)-1-(α-D-galactopyranosyloxy)-2-[(R)-2-hydroxyhexacosanoylamino]-3,4-octadécanediol,
(27) (2S,3S,4R)-1-(α-D-galactopyranosyloxy)-2-[(R)-2-hydroxyhexacosanoylamino]-34-nonadécanediol,
(28) (2S,3S,4R)-1-(α-D-galactopyranosyloxy)-2-[(R)-2-hydroxyhexacosanoylamino]-3,4-icosanediol,
(29) (2S,3S,4R)-1-(α-D-galactopyranosyloxy)-2-[(S)-2-hydroxytétracosanoylamino]-3,4-heptadécanediol,
(30) (2S,3S,4R)-1-(α-D-galactopyranosyloxy)-2-[(R)-2-hydroxytétracosanoylamino]-3,4-hexadécanediol,
(31) (2S,3S,4R)-1-(α-D-galactopyranosyloxy)-2-[(S)-2-hydroxytétracosanoylamino]-16-méthyl-3,4-heptadécanediol,
(32) (2S,3S,4R)-1-(α-D-galactopyranosyloxy)-16-méthyl-2-tétracosanoylamino-3,4-heptadécanediol,
(33) (2S,3S,4R)-1-(α-D-galactopyranosyloxy)-2-[(R)-2-hydroxytricosanoylamino]-16-méthyl-3,4-heptadécanediol,
(34) (2S,3S,4R)-1-(α-D-galactopyranosyloxy)-2-[(R)-2-hydroxypentacosanoylamino]-16-méthyl-3,4-octadécanediol
et
(35) (2S,3R)-1-(α-D-galactopyranosyloxy)-2-oléoylamino-3-octadécanol.

**99.** Procédé pour la préparation d'un glycosphingolipide comprenant la récolte de l'éponge marine Agelas mauritianus, sa soumission à une opération d'extraction avec un solvant organique et l'isolement à partir de l'extrait d'au moins un des glycosphingolipides suivants représentés par la formule (I) selon la revendication 56 :

(1) (2S,3S,4R)-1-(α-D-galactopyranosyloxy)-2-[(R)-2-hydroxytétracosanoylamino]-3,4-heptadécanediol,
(2) (2S,3S,4R)-1-(α-D-galactopyranosyloxy)-2-[(R)-2-hydroxytétracosanoylamino]-3,4-hexadécanediol,
(3) (2S,3S,4R)-1-(α-D-galactopyranosyloxy)-2-[(R)-2-hydroxytricosanoylamino]-16-méthyl-3,4-heptadécanediol et
(4) (2S,3S,4R)-1-(α-D-galactopyranosyloxy)-2-[(R)-2-hydroxypentacosanoylamino]-16-méthyl-3,4-octadécanediol.

**100.** Procédé pour préparer un agent antitumoral comprenant le mélange d'un ou plusieurs composés représentés par la formule (A) :

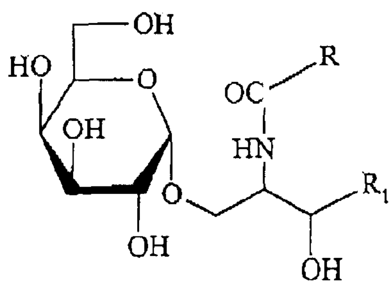

(A)

dans laquelle

R représente

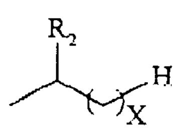

,

où $R_2$ représente H ou OH et X signifie un entier de 0 à 26 ou $-(CH_2)_7CH=CH(CH_2)_7CH_3$ et

$R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17

en tant qu'ingrédients actifs avec des additifs pharmaceutiquement acceptables.

**101.** Procédé pour préparer un agent immunostimulant comprenant le mélange d'un ou plusieurs composés représentés par la formule (A) :

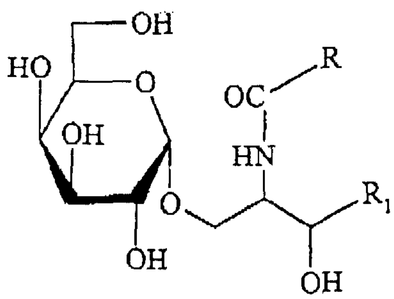

(A)

dans laquelle

R représente

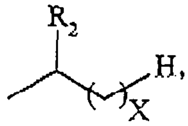

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

et

(e) $-CH(OH)(CH_2)_YCH(CH_3)CH_2CH_3$,

dans lesquels Y signifie un entier de 5 à 17

en tant qu'ingrédients actifs avec des additifs pharmaceutiquement acceptables.

**102.** Utilisation d'un ou plusieurs composés représentés par la formule (A) :

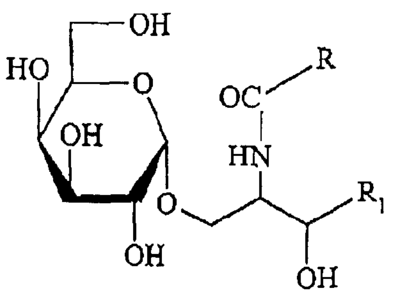

(A)

dans laquelle

R représente

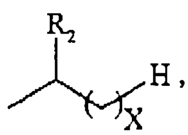

où $R_2$ représente H ou OH et X signifie un entier de 0 à 26 ou $-(CH_2)_7CH{=}CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH{=}CH(CH_2)_YCH_3$

et

(e) $-CH(OH)(CH_2)_YCH(CH_3)CH_2CH_3$,

dans lesquels Y signifie un entier de 5 à 17

dans la préparation d'un agent antitumoral.

**103.** Utilisation d'un ou plusieurs composés représentés par la formule (A) :

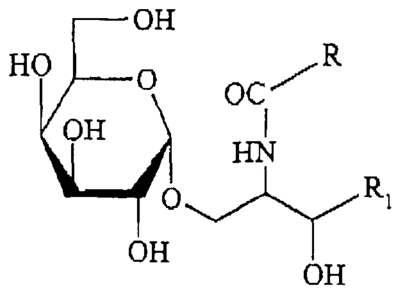

(A)

dans laquelle

R représente

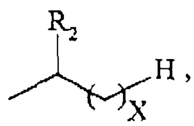

où $R_2$ représente H ou OH et X signifie un entier de 0 à 26 ou $-(CH_2)_7CH=CH(CH_2)_7CH_3$ et $R_1$ représente l'un des substituants définis par les paragraphes (a) à (e) suivants :

(a) $-CH_2(CH_2)_YCH_3$,

(b) $-CH(OH)(CH_2)_YCH_3$,

(c) $-CH(OH)(CH_2)_YCH(CH_3)_2$,

(d) $-CH=CH(CH_2)_YCH_3$

et

(e) $-CH(OH)(CH_2)_YCH(CH_3)CH_2CH_3$,

dans lesquels Y signifie un entier de 5 à 17

dans la préparation d'un médicament immunostimulant.

SYNTHETIC ROUTE A

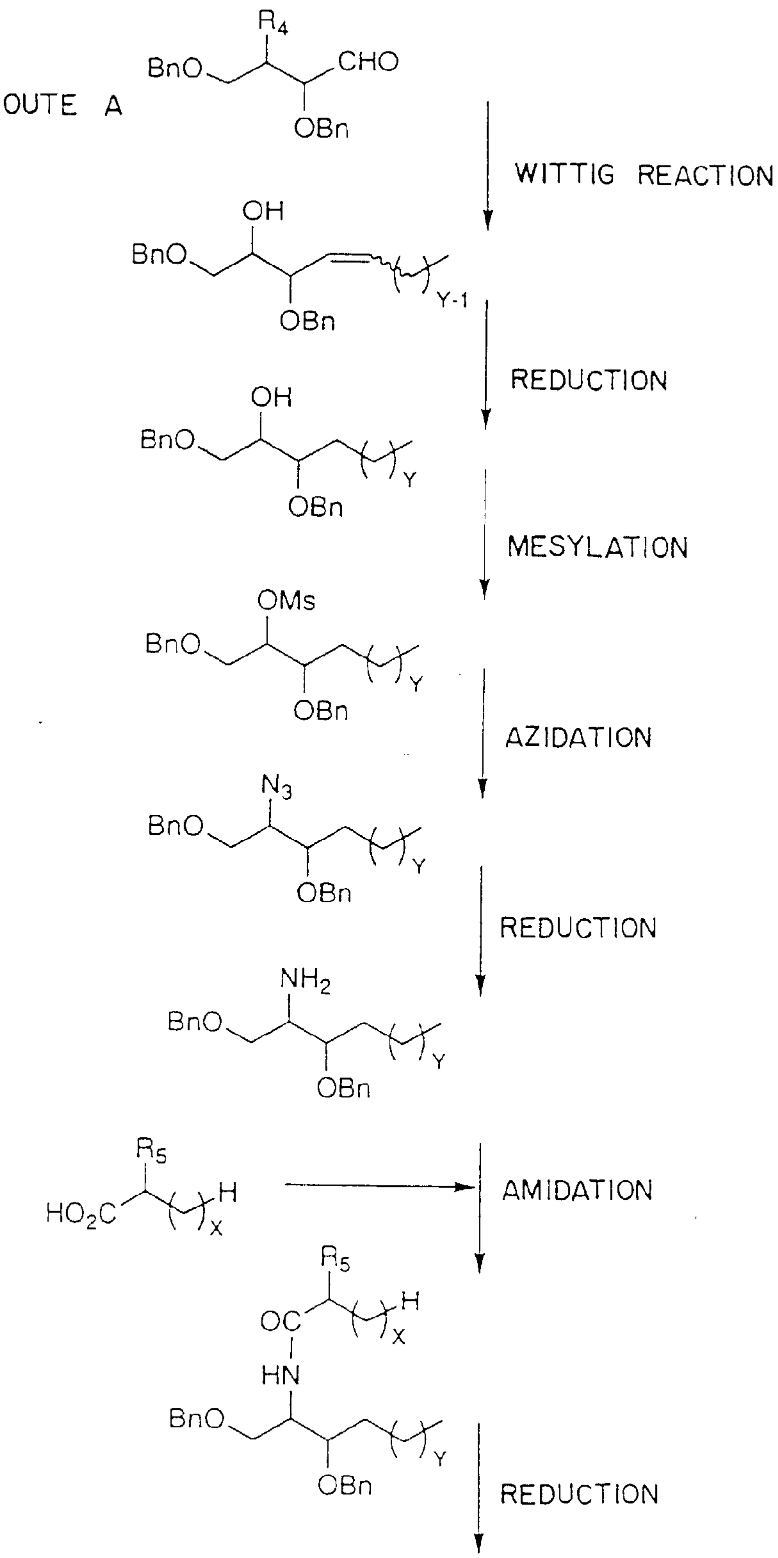


FIG. 1a

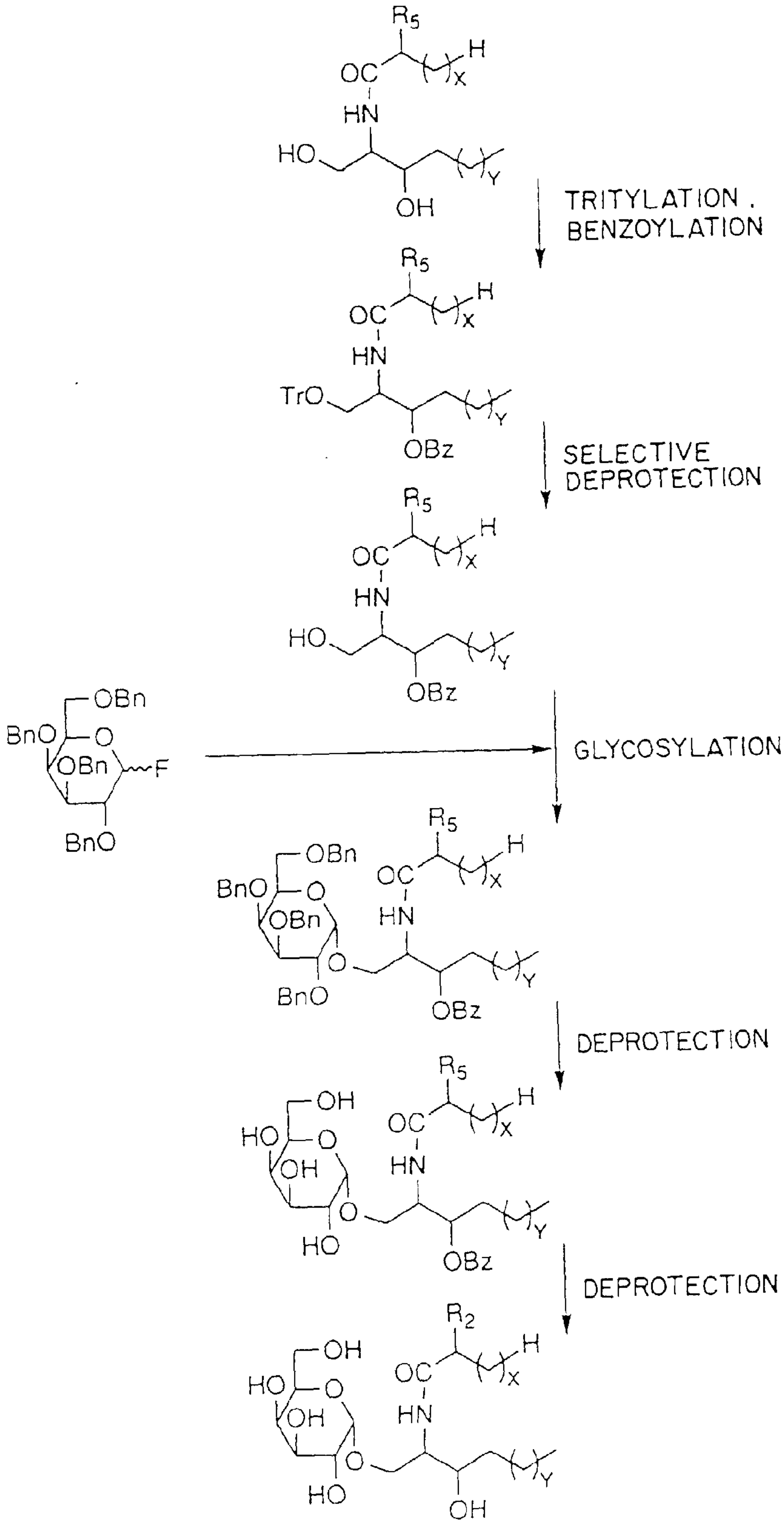
TRITYLATION .
BENZOYLATION
SELECTIVE
DEPROTECTION
GLYCOSYLATION
DEPROTECTION
DEPROTECTION

FIG. 1 b

SYNTHETIC ROUTE B

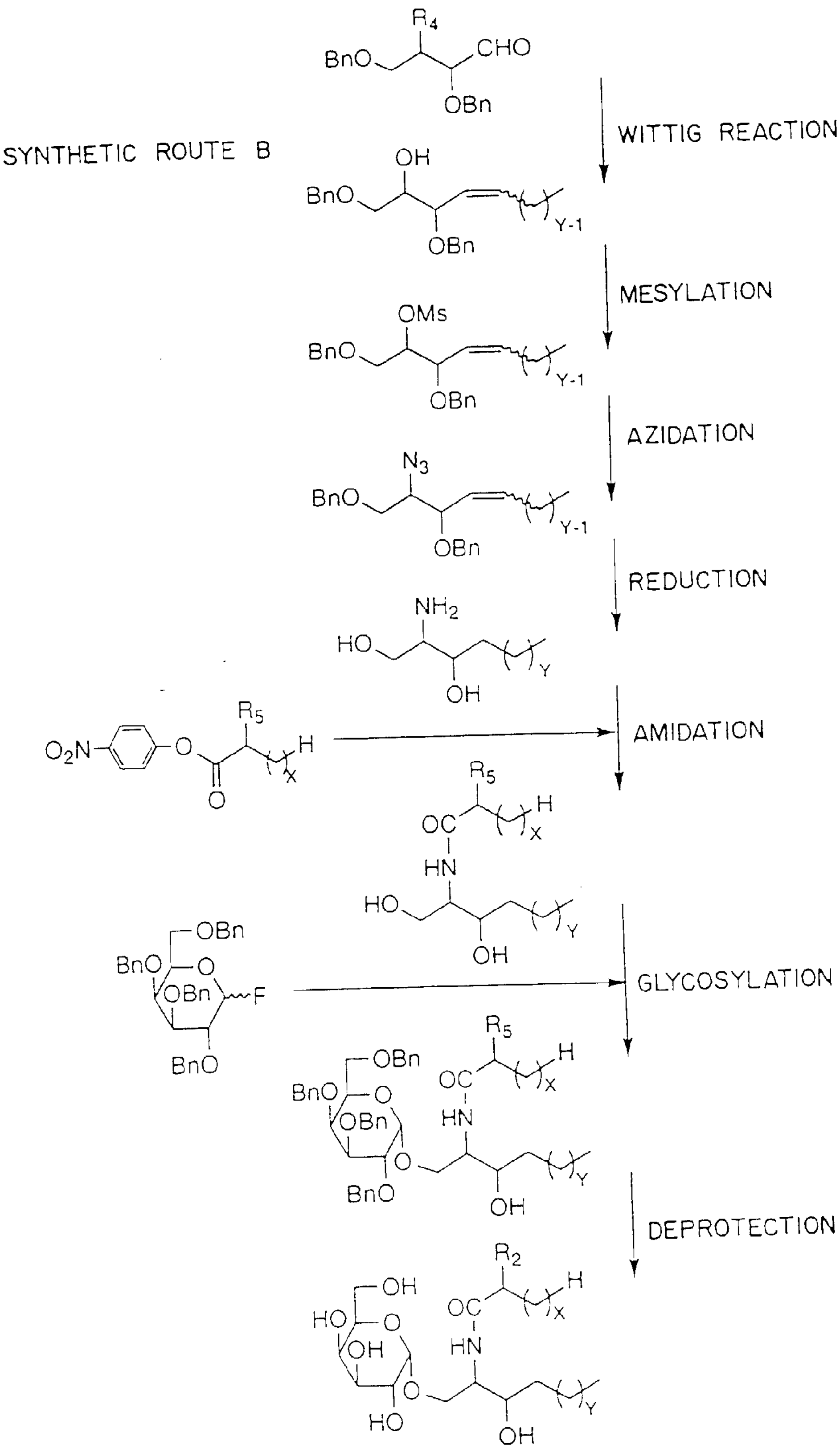


FIG. 2

SYNTHETIC ROUTE C

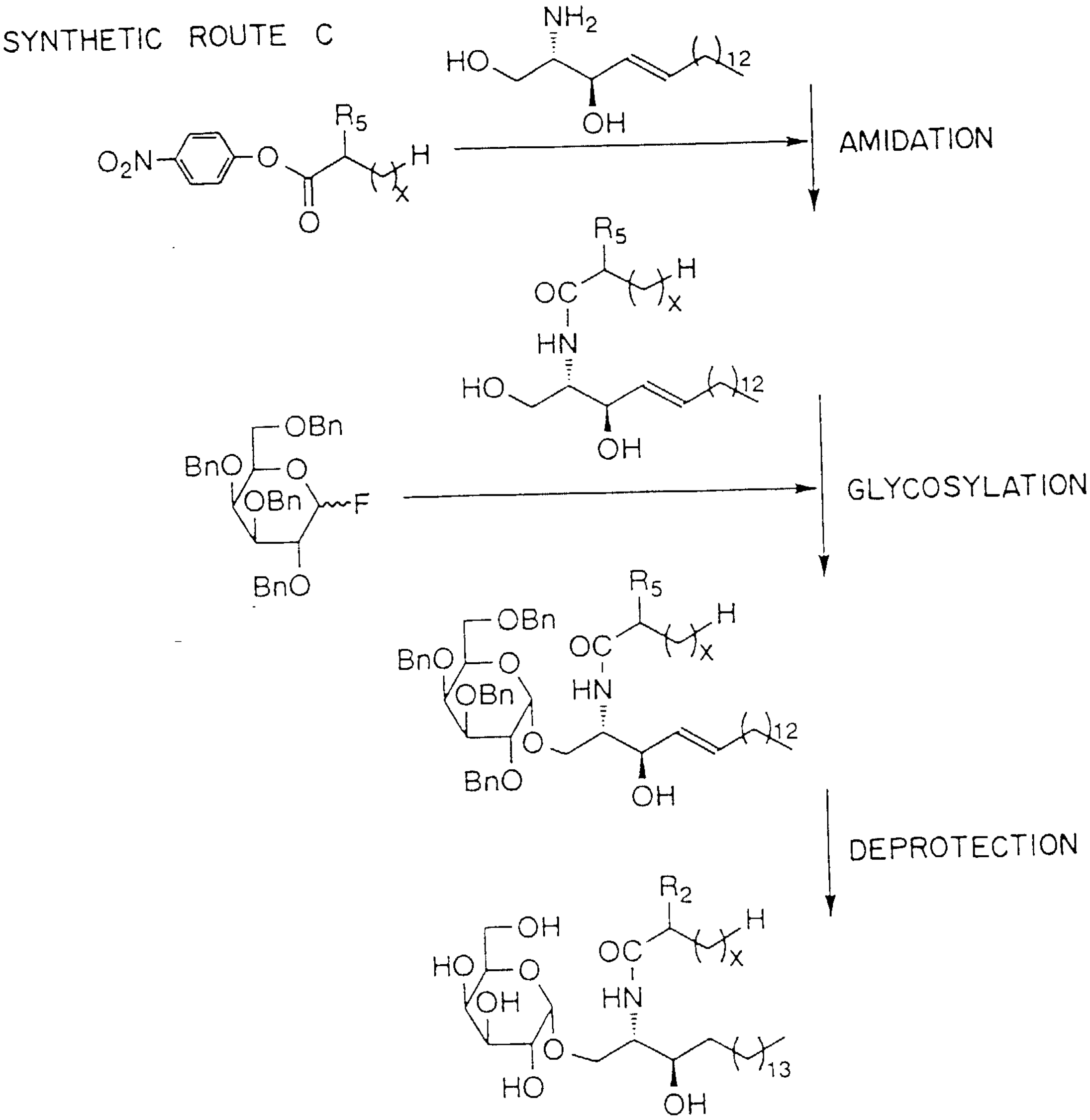


FIG. 3

SYNTHETIC ROUTE D

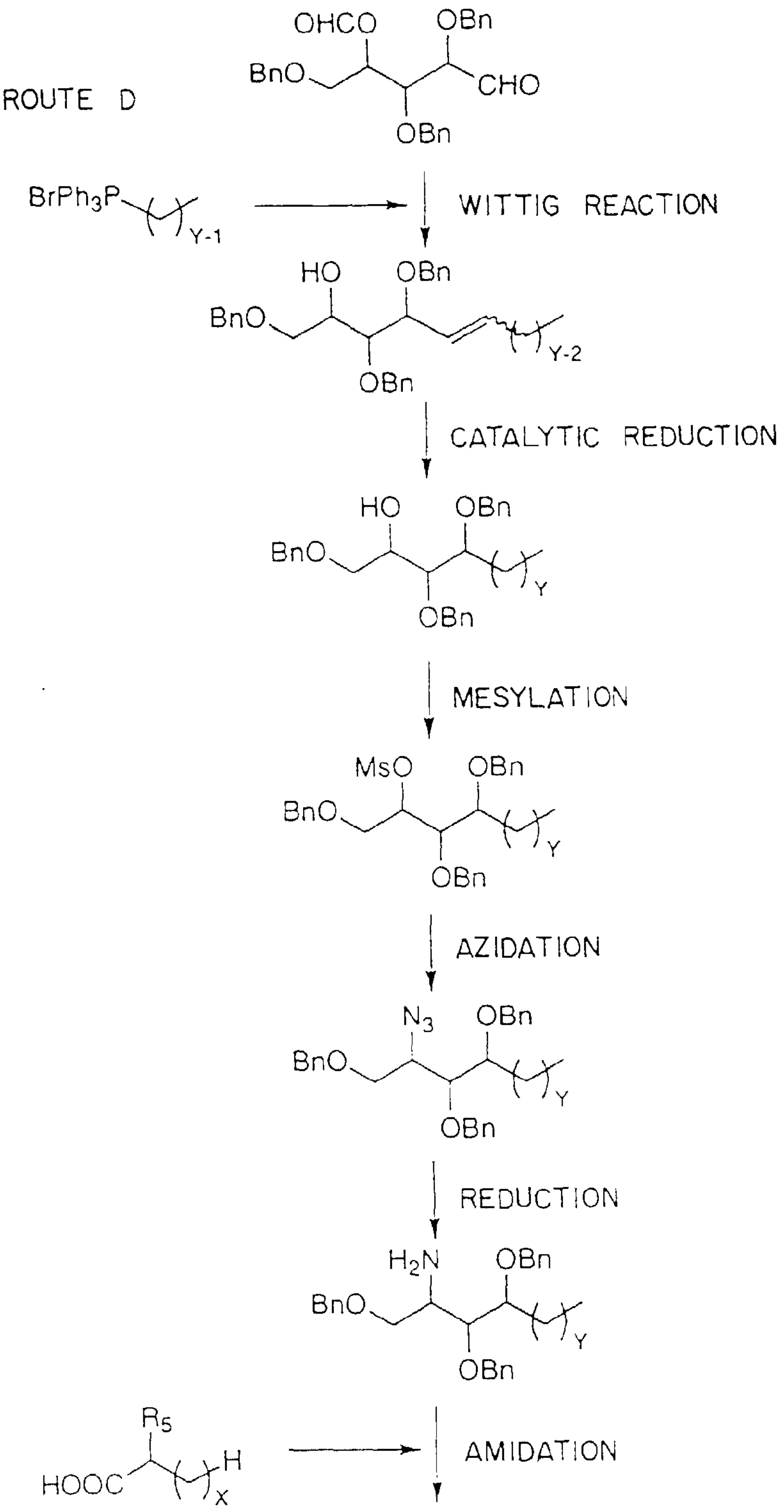


FIG. 4 a

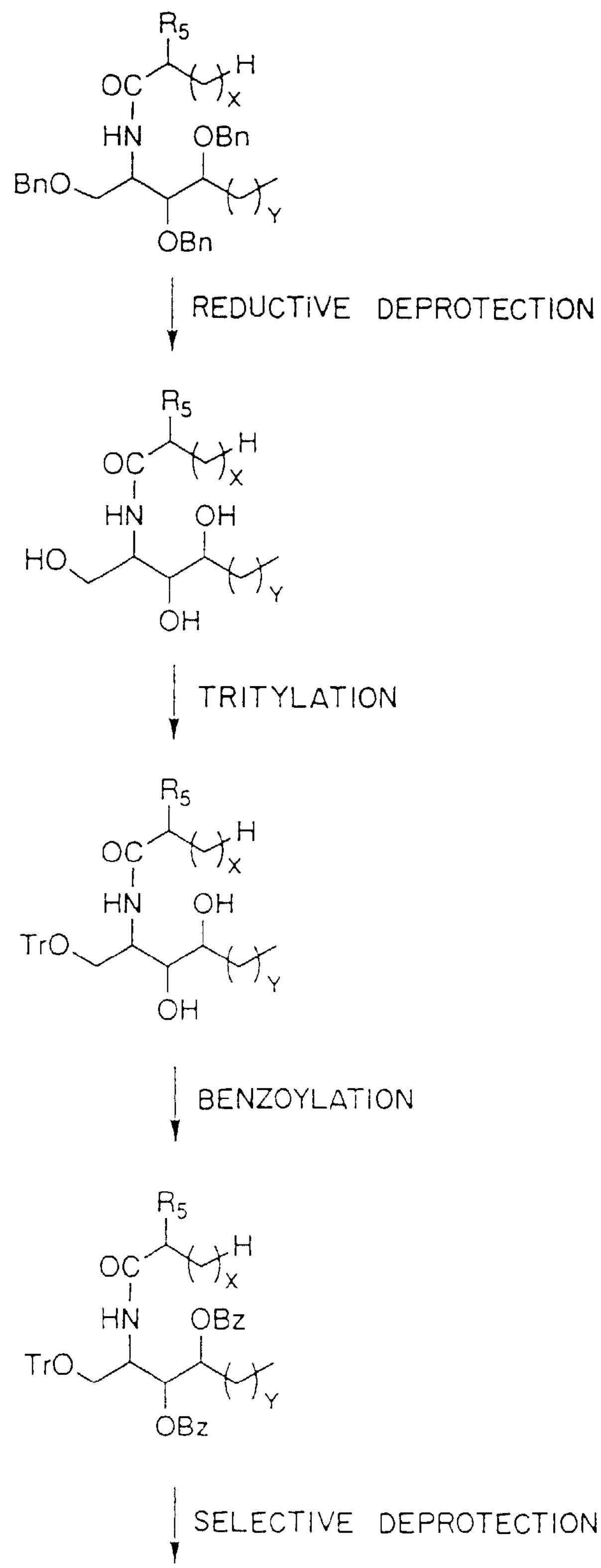
REDUCTIVE DEPROTECTION
TRITYLATION
BENZOYLATION
SELECTIVE DEPROTECTION

FIG. 4b

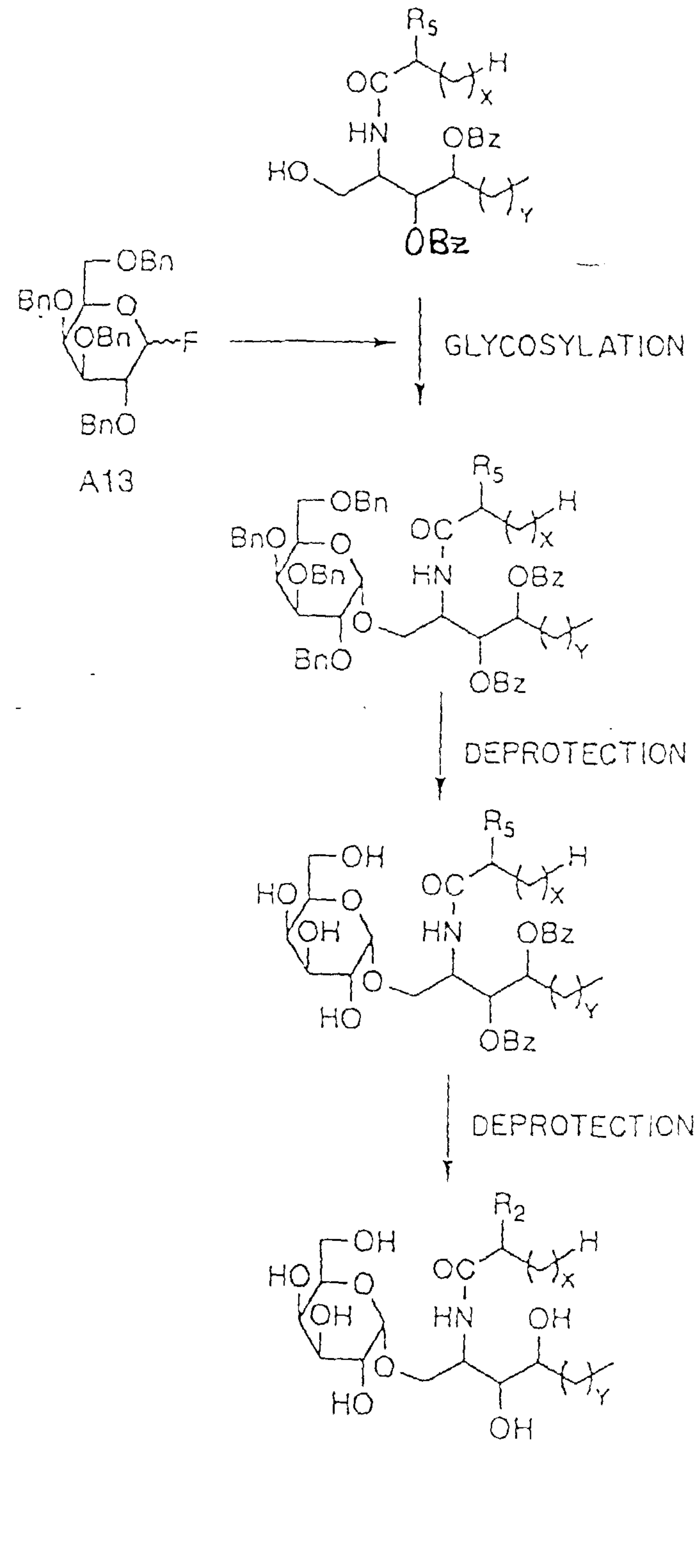


FIG. 4 c

SYNTHESIS OF COMPOUND 9

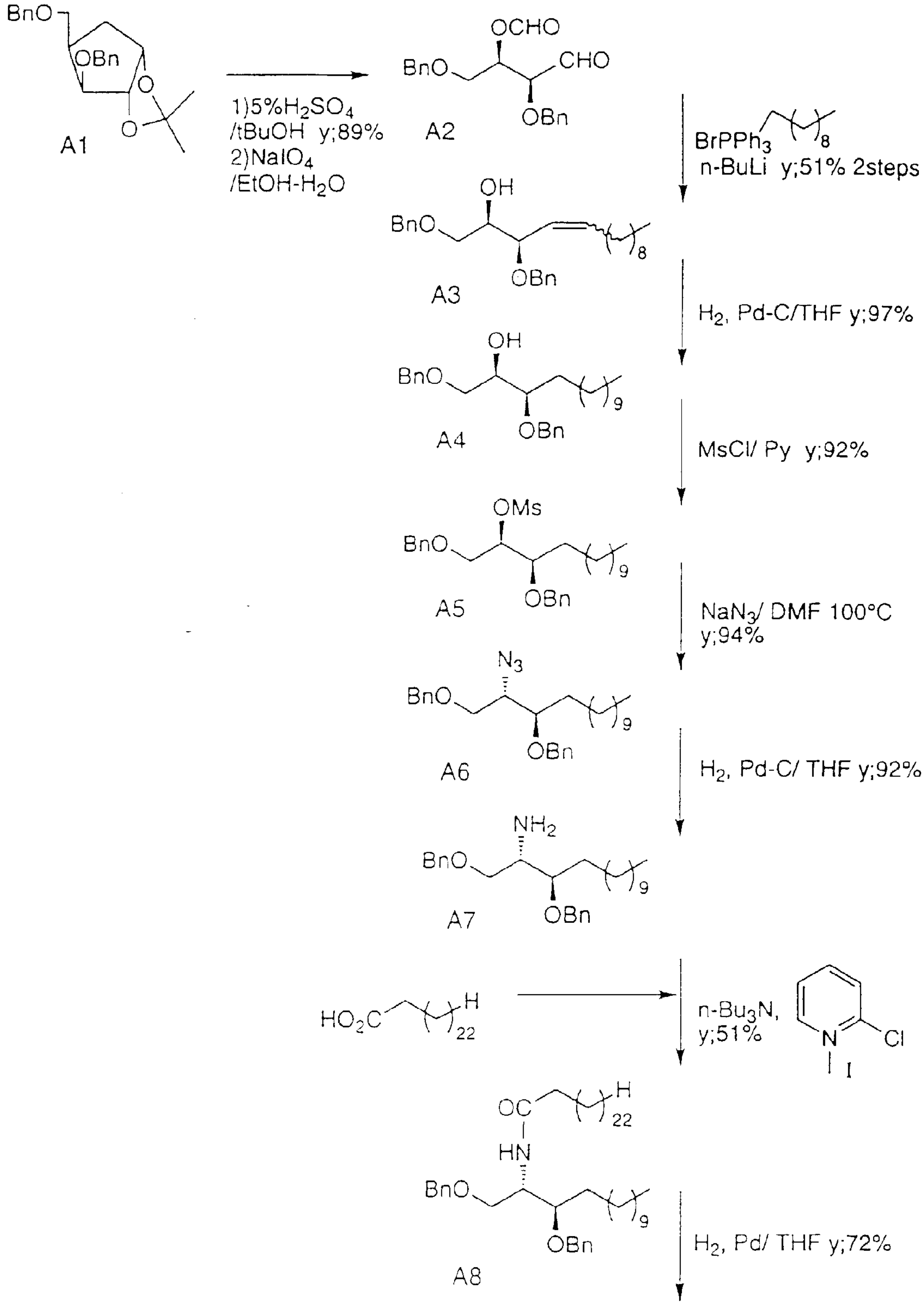


FIG. 5a

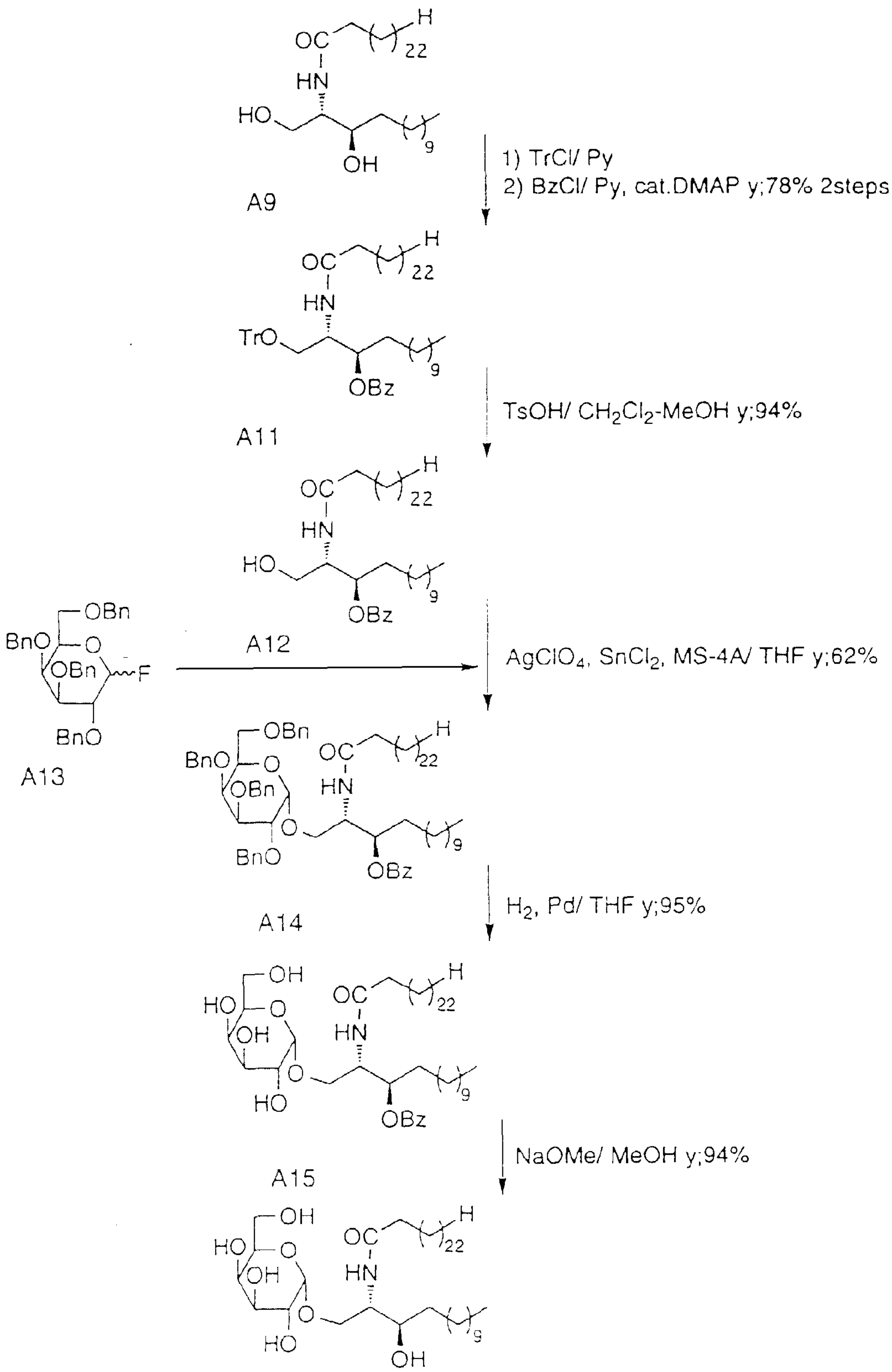


A9

A11

A12

A13

A14

A15

COMPOUND 9

FIG. 5 b

SYNTHESIS OF COMPOUND 7

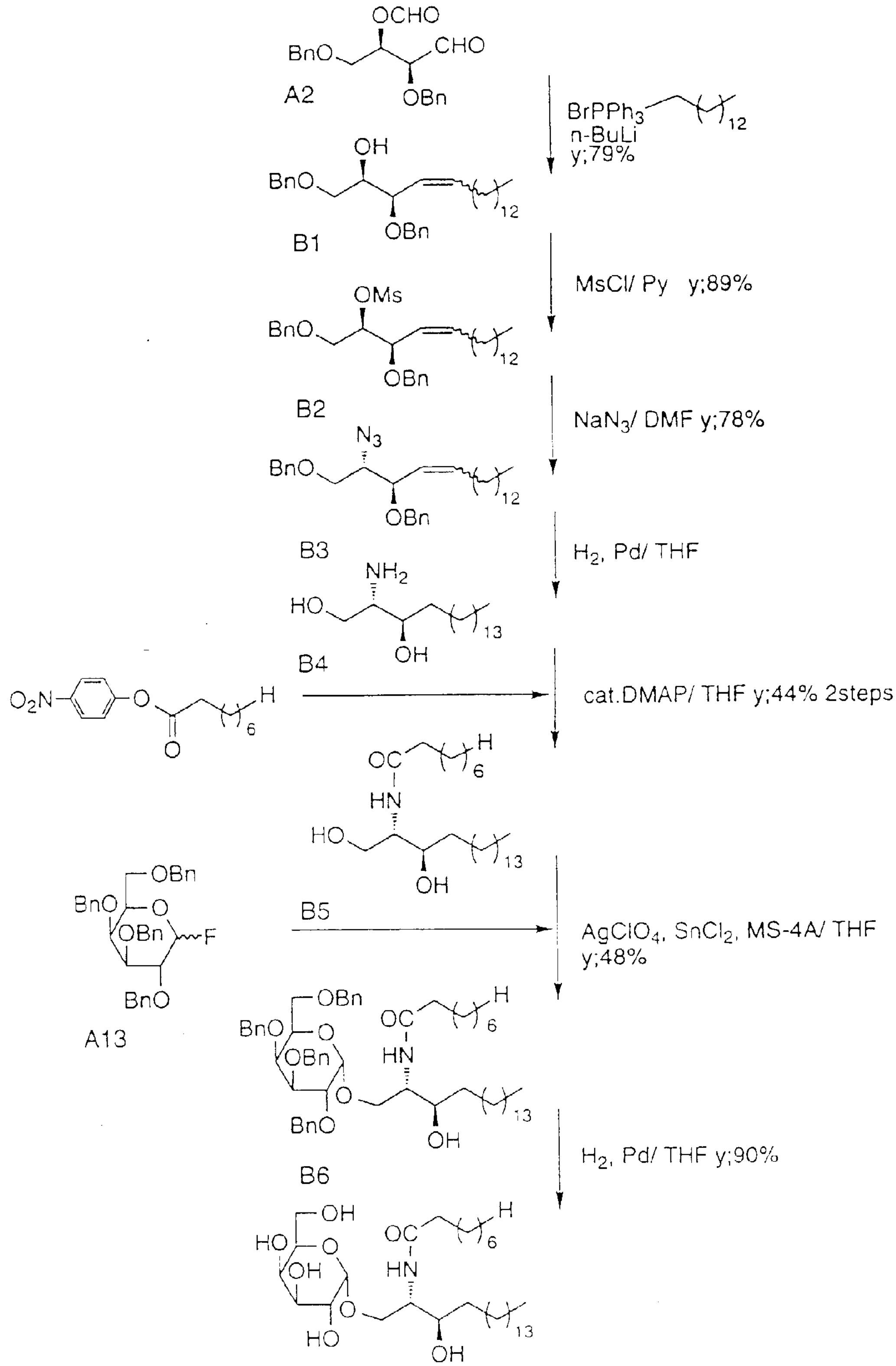


COMPOUND 7

FIG. 6

SYNTHESIS OF COMPOUND 5

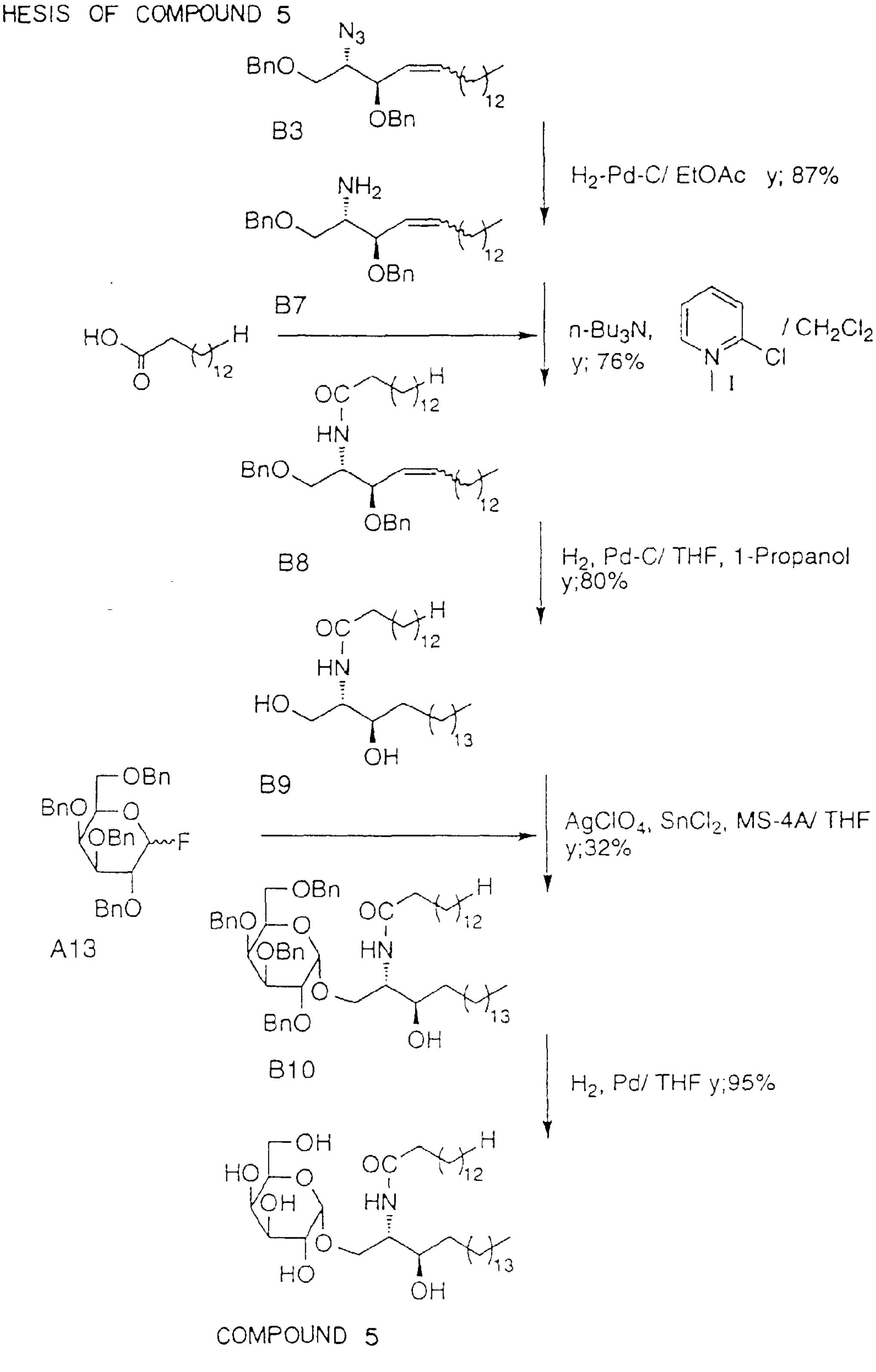


COMPOUND 5

FIG. 7

SYNTHESIS OF COMPOUND 1

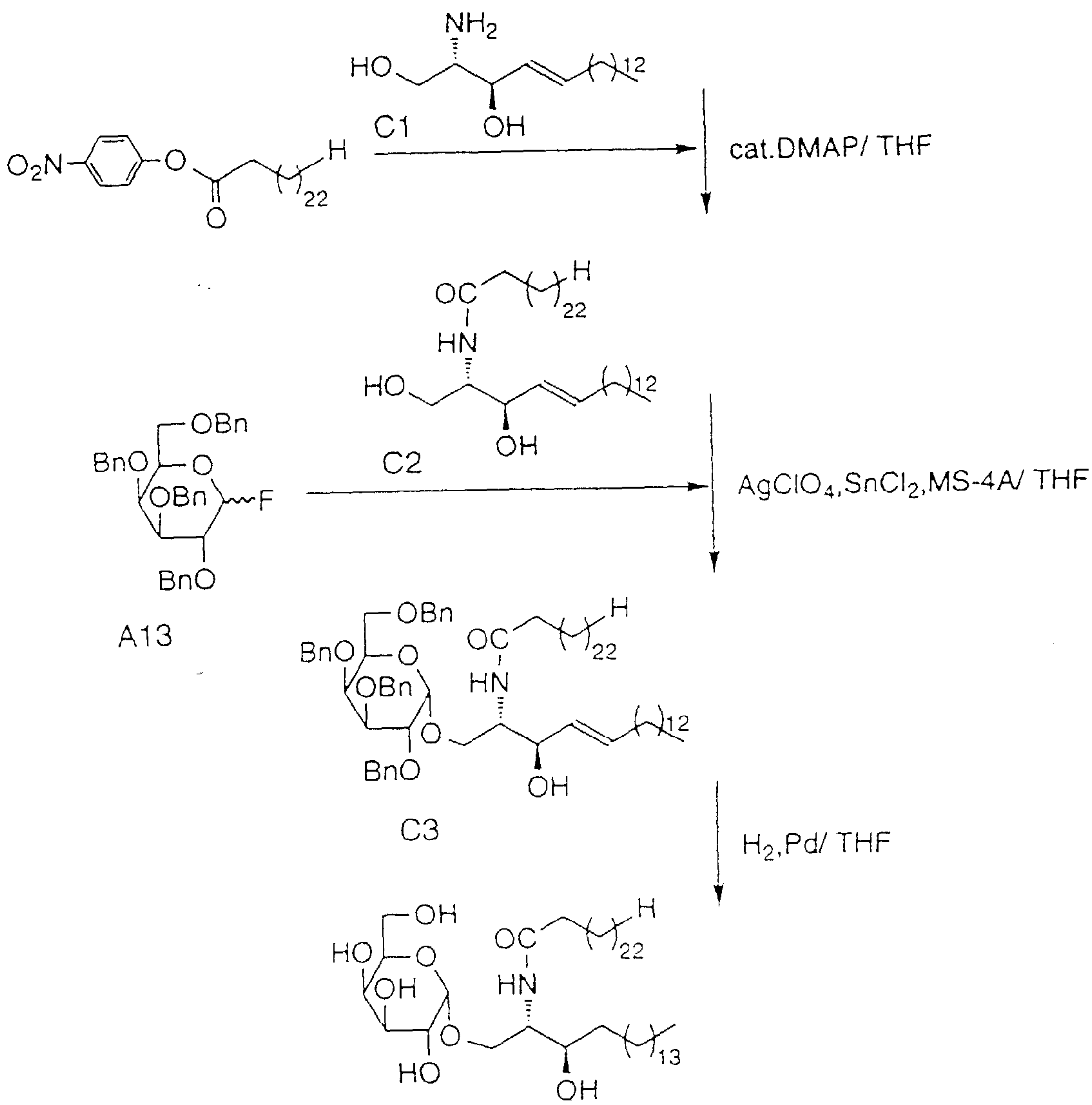


COMPOUND 1

FIG. 8

SYNTHESIS OF COMPOUND 5

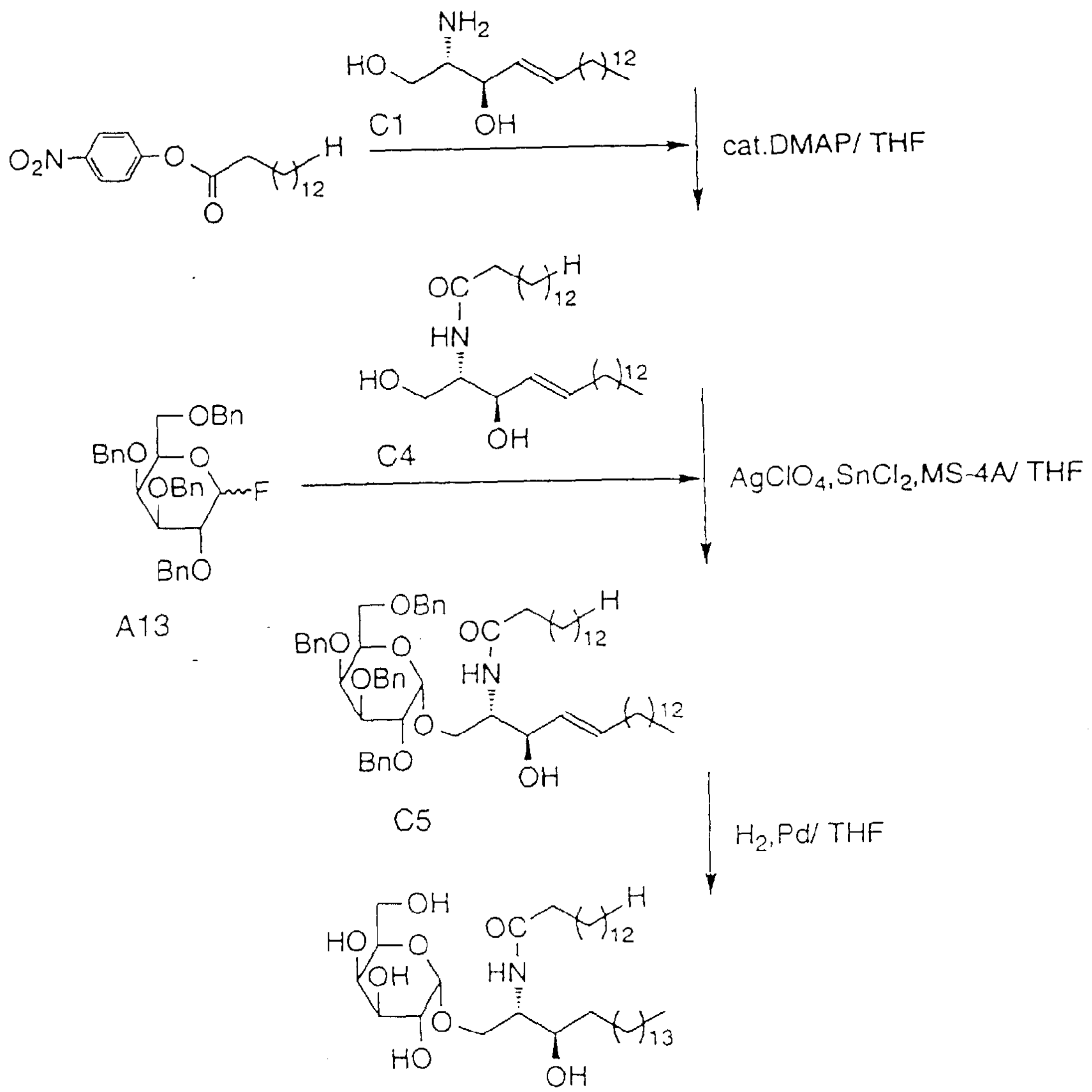


C1

C4

A13

C5

COMPOUND 5

FIG. 9

SYNTHESIS OF COMPOUND 22

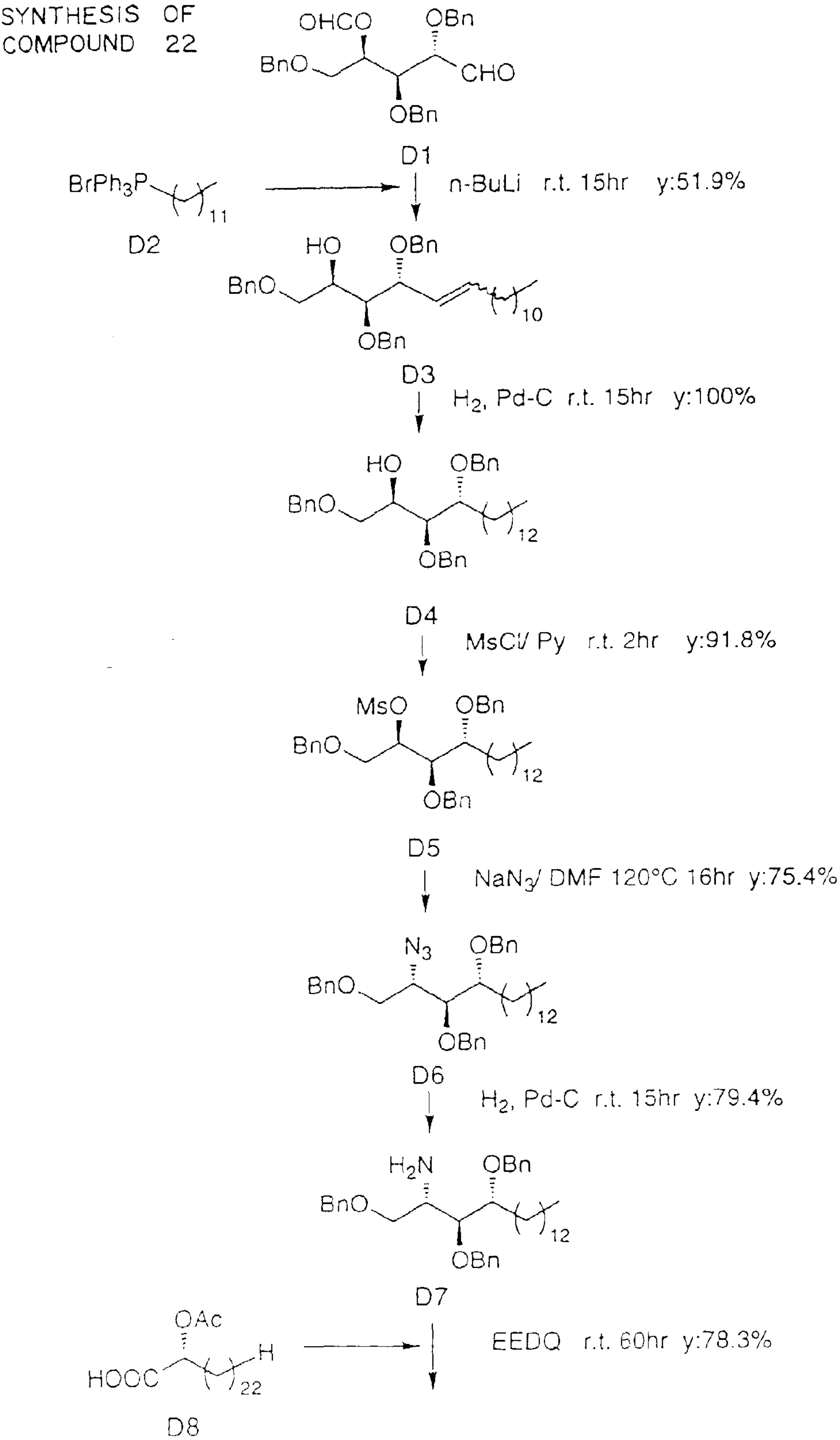


FIG.10 a

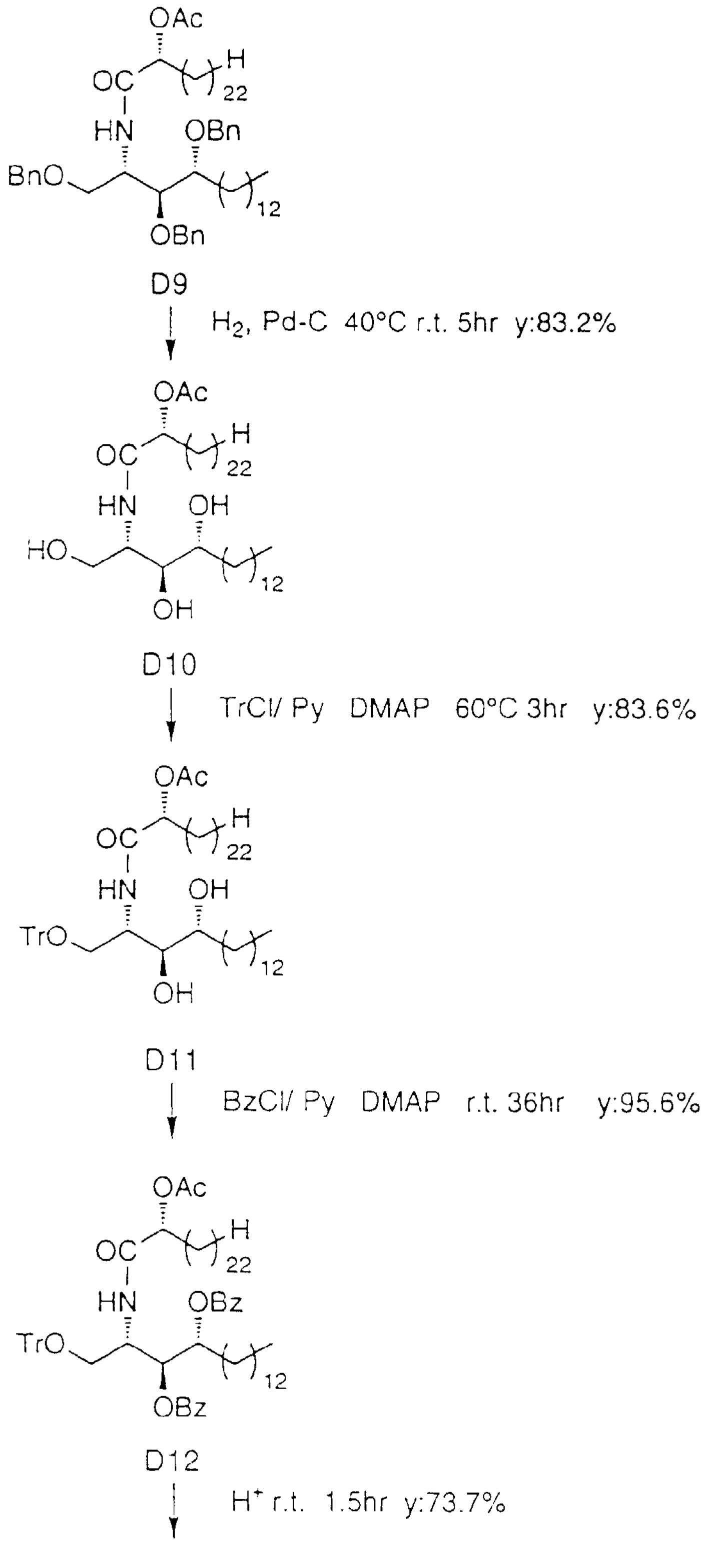
D9
H2, Pd-C 40°C r.t. 5hr y:83.2%
D10
TrCl/ Py DMAP 60°C 3hr y:83.6%
D11
BzCl/ Py DMAP r.t. 36hr y:95.6%
D12
H+ r.t. 1.5hr y:73.7%

FIG.10 b

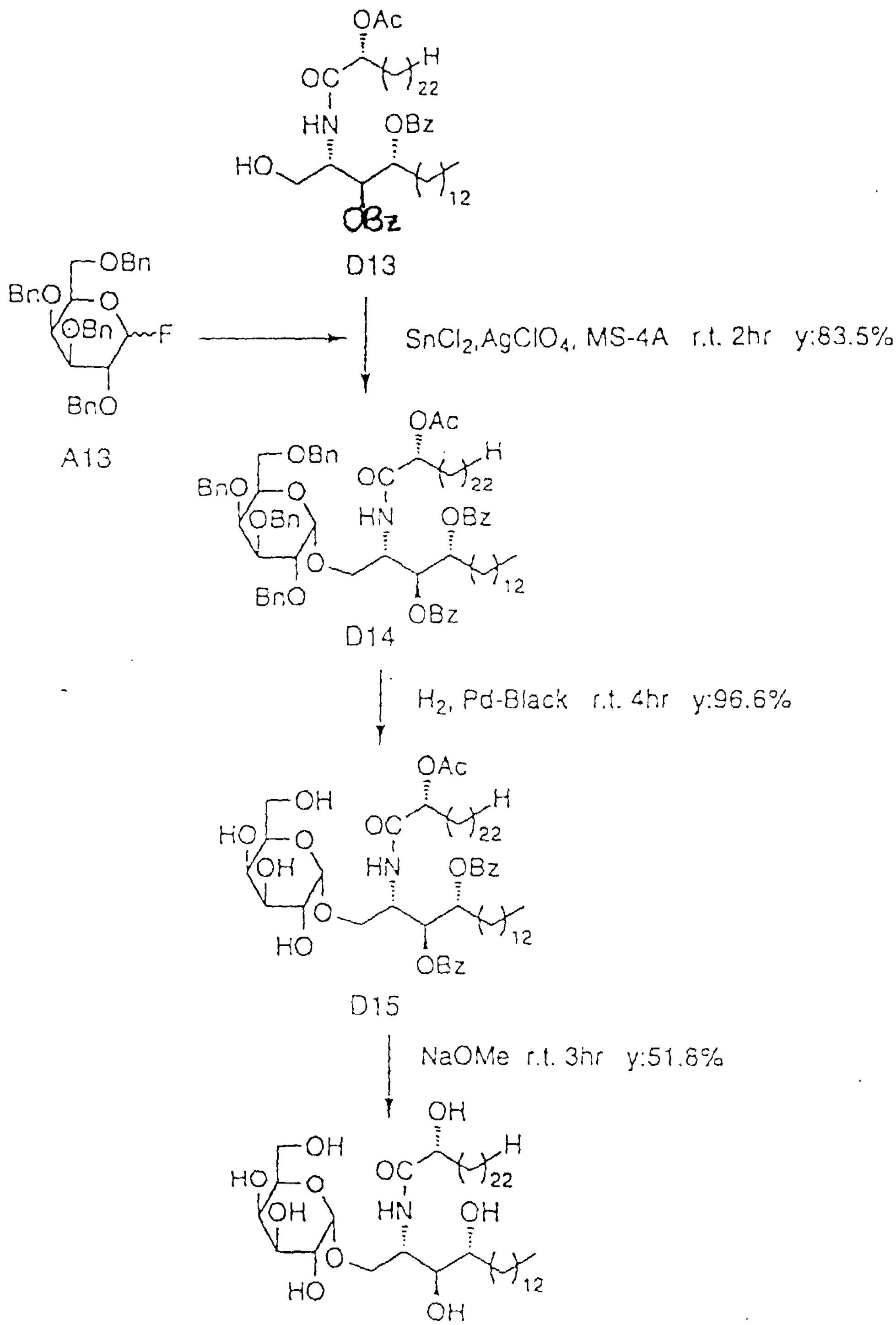


COMPOUND 22

FIG.10c